(19) Europäisches Patentamt
European Patent Office
Office européen des brevets

(11) Publication number : 0 618 221 A2

# (12) EUROPEAN PATENT APPLICATION

(21) Application number : 94302255.8

(22) Date of filing : 29.03.94

(51) Int. Cl.[5] : C07K 5/00, A61K 37/02

(30) Priority : 02.04.93 US 42377
29.06.93 US 85338

(43) Date of publication of application :
05.10.94 Bulletin 94/40

(84) Designated Contracting States :
AT BE CH DE DK ES FR GB GR IE IT LI LU MC NL PT SE

(71) Applicant : BRISTOL-MYERS SQUIBB COMPANY
P.O. Box 4000
Princeton, NJ 08543-4000 (US)

(72) Inventor : Patel, Dinesh V.
10253 Parkwood Drive, Apt. No. 7
Cupertino, CA (US)
Inventor : Kline, Toni B.
79th Street Boat Basin
New York, NY (US)
Inventor : Meyers, Chester A.
5 Fox Trail
Medford, NJ (US)
Inventor : Leftheris, Katerina
92 Richmond Drive
Skillman, NJ (US)
Inventor : Bhide, Rajeev S.
156 Barnsbury Road
Langhorne, PA (US)

(74) Representative : Thomas, Roger Tamlyn et al
D. Young & Co.
21 New Fetter Lane
London EC4A 1DA (GB)

## (54) Heterocyclic inhibitors of farnesyl protein transferase.

(57) Inhibition of farnesyl protein transferase is effected by compounds of the formula

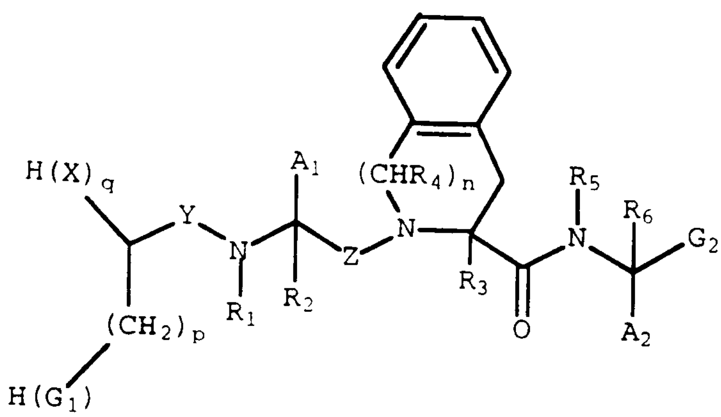

its enantiomers, diastereomers, pharmaceutically acceptable salts, prodrugs or solvates thereof, wherein :

$A_1$ and $A_2$ are each independently H, alkyl, substituted alkyl, cycloalkyl, substituted cycloalkyl, phenyl or substituted phenyl ;

$G_1$ is S or O ;

$G_2$ is H, -C(O)OH, $-C(O)NH_2$, 5-tetrazolyl, $-C(O)N(R_7)OH$ or $-CH_2OH$ ;

X is O or $R_8N$ ;

Y and Z are each independently $-CH_2-$ or -C(O)- ;

$R_1$, $R_2$, $R_3$, $R_4$, $R_5$, $R_6$ and $R_7$ are each independently H or alkyl ;

$R_1$ may also be alkanoyl,

$R_1$ and $A_1$ taken together may be $-(CH_2)_m$ ;

$R_6$ is H, alkyl, phenyl, phenylalkyl, substituted phenyl, (substituted phenyl)alkyl or $-C(O)R_9$ ;

$R_9$ is H, alkyl, phenyl, phenylalkyl, substituted phenyl or (substituted phenyl)alkyl ;

m is 3 or 4 ;

EP 0 618 221 A2

n is 0, 1 or 2 ;
p is 0, 1 or 2 ; and
q is 0 or 1, with the proviso that when p is 0, then q is also 0.

## Field of the Invention

This invention relates to compounds that inhibit farnesyl-protein transferase and Ras protein farnesylation, thereby making them useful as anti-cancer agents. The compounds are also useful in the treatment of diseases, other than cancer, associated with signal transduction pathways operating through Ras and those associated with CAAX-containing proteins other than Ras that are also post-translationally modified by the enzyme farnesyl protein transferase. The compounds may also act as inhibitors of other prenyl transferases, and thus be effective in the treatment of diseases associated with other prenyl modifications of proteins.

## Background of the Invention

The mammalian ras gene family comprises three genes, H-ras, K-ras and N-ras. The Ras proteins are a family of GTP-binding and hydrolyzing proteins that regulate cell growth and differentiation. Overproduction of normal Ras proteins or mutations that inhibit their GTPase activity can lead to uncontrolled cell division.

The transforming activity of ras is dependent on localization of the protein to plasma membranes. This membrane binding occurs via a series of post-translational modifications of the cytosolic Ras proteins. The first and mandatory step in this sequence of events is the farnesylation of these proteins. The reaction is catalyzed by the enzyme farnesyl protein transferase (FPT), and farnesyl pyrophosphate (FPP) serves as the farnesyl group donor in this reaction. The Ras C-terminus contains a sequence motif termed a "Cys-$Aaa_1$-$Aaa_2$-Xaa" box (CAAX box), wherein Cys is cysteine, Aaa is an aliphatic amino acid, and Xaa is a serine or methionine. Farnesylation occurs on the cysteinyl residue of the CAAX box (Cys-186), thereby attaching the prenyl group on the protein via a thio-ether linkage.

In accordance with the present invention, a compound of the formula

I

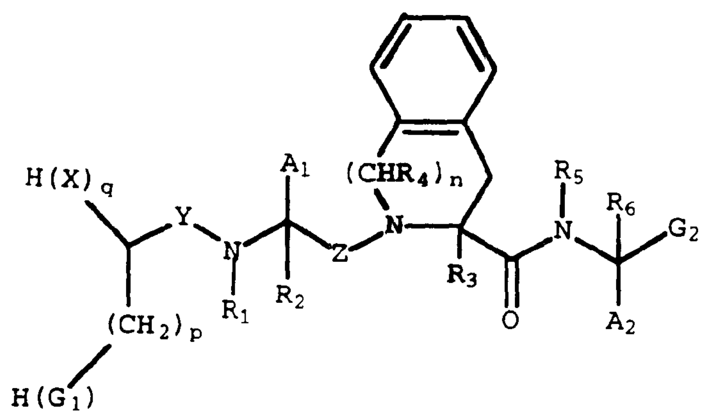

its enantiomers and diastereomers, and pharmaceutically acceptable salts, prodrugs and solvates thereof inhibit S-farnesyl protein transferase, which is an enzyme involved in ras oncogene function. In formula I and throughout this specification, unless otherwise specified, the above symbols are defined as follows:

$A_1$ and $A_2$ are each independently H, alkyl, substituted alkyl, cycloalkyl, substituted cycloalkyl, phenyl or substituted phenyl;
$G_1$ is S or O;
$G_2$ is H, -C(O)OH, -C(O)$NH_2$, 5-tetrazolyl, -C(O)N($R_7$)OH or -$CH_2$OH;
X is O or $R_8$N;
Y and Z are each independently -$CH_2$- or -C(O)-;
$R_1$, $R_2$, $R_3$, $R_4$, $R_5$, $R_6$ and $R_7$ are each independently H or alkyl;
$R_1$ may also be alkanoyl;
$R_1$ and $A_1$ taken together may be $-(CH_2)_m-$;
$R_8$ is H, alkyl, phenyl, phenylalkyl, substituted phenyl, (substituted phenyl)alkyl or -C(O)$R_9$;
$R_9$ is H, alkyl, phenyl, phenylalkyl, substituted phenyl or (substituted phenyl)alkyl;
m is 3 or 4;
n is 0, 1 or 2;
p is 0, 1 or 2; and
q is 0 or 1, with the proviso that when p is 0, then q is also 0.

Listed below are definitions of various terms used to describe this invention. These definitions apply to the terms as they are used throughout this specification, unless otherwise limited in specific instances, either individually or as part of a larger group.

The term "alkyl" or "alk-" refers to straight or branched chain unsubstituted hydrocarbon groups of 1 to 7 carbon atoms. The expression "lower alkyl" refers to unsubstituted alkyl groups of 1 to 4 carbon atoms.

The term "cycloalkyl" refers to cyclic hydrocarbon groups of 3 to 8 carbon atoms.

The term "halogen" or "halo" refers to fluorine, chlorine, bromine and iodine.

The term "alkoxy" refers to alkyl-O-.

The term "alkanoyl" refers to alkyl-C(O)-.

The term "alkanoyloxy" refers to alkyl-C(O)-O-.

The terms "alkylamino" and "dialkylamino" refer to (alkyl)NH- and $(alkyl)_2N$-, respectively.

The term "alkanoylamino" refers to alkyl-C(O)-NH-.

The term "alkylthio" refers to alkyl-S-.

The term "alkylthiono" refers to alkyl-S(O)-.

The term "alkylsulfonyl" refers to alkyl-$S(O)_2$-.

The term "carbamyl" refers to -$C(O)NH_2$.

The term "alkoxycarbonyl" refers to alkyl-O-C(O)-.

The expressions "substituted alkyl" and "substituted cycloalkyl" refer to an alkyl or cycloalkyl, group, respectfully, substituted by, for example, one to four substituents such as halo, hydroxy, alkoxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, N-hydroxycarbamyl, alkoxycarbonyl, phenyl, substituted phenyl, guanidino, indolyl, imidazolyl, furyl, thienyl, thiazolyl, pyrrolidyl, pyridyl, pyrimidyl and the like.

The term "substituted phenyl" refers to a phenyl group substituted by, for example, one to four substituents such as alkyl, halo, hydroxy, alkoxy, alkanoyl, alkanoyloxy, amino, alkylamino, dialkylamino, alkanoylamino, thiol, alkylthio, nitro, cyano, carboxy, carbamyl, alkoxycarbonyl, alkylthiono, alkylsulfonyl, sulfonamido and the like.

The compounds of formula I form salts which are also within the scope of this invention. Pharmaceutically acceptable (i.e., non-toxic, physiologically acceptable) salts are preferred, although other salts are also useful, e.g, in isolating or purifying the compounds of this invention.

The compounds of formula I may form salts with alkali metals such as sodium, potassium and lithium, with alkaline earth metals such as calcium and magnesium, with organic bases such as dicyclohexylamine, tributylamine, pyridine and amino acids such as arginine, lysine and the like. Such salts may be obtained by exchanging the carboxylic acid protons in compound I with the desired ion in a medium in which the salt precipitates or in an aqueous medium followed by evaporation.

When compound I comprises a basic moiety, such as amino or substituted amino, it may form salts with a variety of organic and inorganic acids. Such salts include those formed with hydrogen chloride, hydrogen bromide, methanesulfonic acid, sulfuric acid, acetic acid, trifluoroacetic acid, maleic acid, benzenesulfonic acid, toluenesulfonic acid and various others (e.g., nitrates, phosphates, borates, tartrates, citrates, succinates, benzoates, ascorbates, salicylates and the like). Such salts may be formed by reacting compound I in an equivalent amount of the acid in a medium in which the salt precipitates or in an aqueous medium followed by evaporation.

In addition, zwitterions ("inner salts") may be formed.

A compound of the formula I may also have prodrug forms. Any compound that will be converted in vivo to provide the bioactive agent (i.e., the compound of formula I) is a prodrug within the scope and spirit of the invention. For example, compound I may be in the form of a prodrug having the formula

II

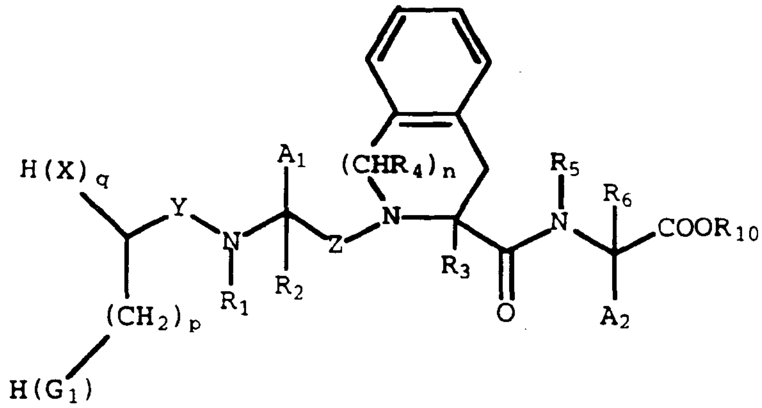

wherein $R_{10}$ is:

lower alkyl, such as methyl, ethyl and the like;

substituted lower alkyl, such as 2-(N-morpholine)ethyl and the like;

lower aralkyl, such as benzyl, biphenylmethyl and the like;

(acyloxy)alkyl, such as (pivalyloxy)methyl, 1-(propanoyloxy)-2-methyl-1-propyl and the like;

(aminoacyloxy)aroyloxyalkyl, such as para-glycyloxybenzoyloxymethyl and the like;

(aminoalkoxy)aroyloxyalkyl, such as para-2-[(N-morpholine)ethoxy]benzoyloxymethyl and the like;

substituted amides, such as N,N-di(2-hydroxyethyl)acetamido, 4-methylpiperazine-1-acetyl, 4-(2-hydroxyethyl)piperazine-1-acetyl and the like; or

a dioxolanemethyl, such as (5-methyl-2-oxo-1,3-dioxolan-4-yl)methyl and the like.

Additionally, for a compound of formula I where $A_2$ is $-(CH_2)_wOH$ (where w is 2 or 3) and $G_2$ is -C(O)OH, $A_2$ and $G_2$ may be joined to form a lactone ring which can be opened in vivo to give a compound of formula I where $A_2$ is $-(CH_2)_wOH$ (where w is 2 or 3) and $G_2$ is -C(O)OH.

Various forms of prodrugs are well known in the art. For examples of such prodrug derivatives, see:

a) Design of Prodrugs, edited by H. Bundgaard, (Elsevier, 1985) and Methods in Enzymology, Vol. 42, p. 309-396, edited by K. Widder, *et al.* (Academic Press, 1985);

b) A Textbook of Drug Design and Development, edited by Krogsgaard-Larsen and H. Bundgaard, Chapter 5, "Design and Application of Prodrugs," by H. Bundgaard, p. 113-191 (1991);

c) H. Bundgaard, Advanced Drug Delivery Reviews, 8, 1-38 (1992);

d) H. Bundgaard, *et al.*, Journal of Pharmaceutical Sciences, 77, 285 (1988); and

e) N. Kakeya, *et al.*, Chem Pharm Bull, 32, 692 (1984).

It should further be understood that solvates (e.g., hydrates) of compound I are also within the scope of the present invention. Methods of solvation are generally known in the art.

**Preferred Moieties**

For compound I, the following moieties are preferred:

$A_1$ and $A_2$ are each independently H or D-, L- or DL- $-CH_3$, $-CH(CH_3)_2$, $-CH_2CH(CH_3)_2$, $-CH(CH_3)CH_2CH_3$, $-C(CH_3)_3$, $-CH_2OH$, $-CH_2CH_2OH$, $-CH_2CH_2CH_2OH$, $-CH(OH)CH_3$,

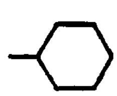 , 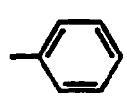 ,

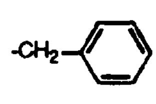 , 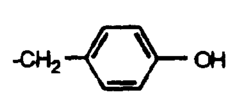 ,

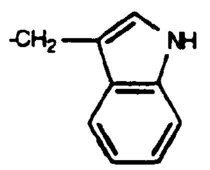

,

$-CH_2\,CH_2\,CH_2\,CH_2\,NH_2$,

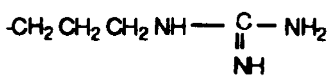

,

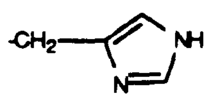

, 

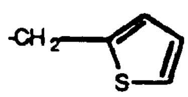

,

$-CH_2C(O)OH$, $-CH_2CH_2C(O)OH$, $-CH_2C(O)NH_2$, $-CH_2CH_2C(O)NR_{11}R_{12}$ where $R_{11}$ and $R_{12}$ are each independently H, alkyl or phenyl, and $R_{11}$ and $R_{12}$ taken together may be $-(CH_2)_o-$ where o is 2 to 6, $-CH_2CH_2C(O)NHOH$, $-CH_2SH$, $-CH_2CH_2SH$, $-CH_2CH_2S(O)_2NH_2$, $-CH_2CH_2OCH_3$ or $-CH_2CH_2SCH_3$.

The following moieties are particularly preferred:

$A_1$ is $-CH_3$, $-CH(CH_3)_2$, $-CH_2CH(CH_3)_2$, $-C(CH_3)_3$, $-CH(CH_3)CH_2CH_3$, $-CH_2OH$ or $-CH(OH)CH_3$;

$A_2$ is $-CH_2CH_2SCH_3$, $-CH_2CH_2OH$, $-CH_2CH_2CH_2OH$, $-CH_2CH_2C(O)NR_{11}R_{12}$ where $R_{11}$ and $R_{12}$ are each independently H, alkyl or phenyl, and $R_{11}$ and $R_{12}$ taken together may be $-(CH_2)_o-$ where o is 2 to 6, $-CH_2CH_2OCH_3$ or $-CH_2CH_2SH$;

$G_1$ is S;

$G_2$ is $-C(O)OH$;

X is $R_8N$;

Y is $-CH_2-$;

$R_1$, $R_2$, $R_3$, $R_4$, $R_5$, $R_6$, $R_7$ and $R_6$ are H;

n is 1; and

p is 1.

## Use and Utility

The compounds of formula I are inhibitors of S-farnesyl protein transferase. They are thus useful in the treatment of a variety of cancers, including (but not limited to) the following:

- carcinoma, including that of the bladder, breast, colon, kidney, liver, lung, ovary, pancreas, stomach, cervix, thyroid and skin;
- hematopoietic tumors of lymphoid lineage, including acute lymphocytic leukemia, B-cell lymphoma and Burketts lymphoma;
- hematopoietic tumors of myeloid lineage, including acute and chronic myelogenous leukemias and promyelocytic leukemia;
- tumors of mesenchymal origin, including fibrosarcoma and rhabdomyosarcoma; and
- other tumors, including melanoma, seminoma, tetratocarcinoma, neuroblastoma and glioma.

The compounds of formula I are especially useful in treatment of tumors having a high incidence of ras involvement, such as colon, lung, and pancreatic tumors. By the administration of a composition having one (or a combination) of the compounds of this invention, development of tumors in a mammalian host is reduced.

Compounds of formula I may also be useful in the treatment of diseases other than cancer that may be associated with signal transduction pathways operating through Ras, e.g., neuro-fibromatosis.

Compounds of formula I may also be useful in the treatment of diseases associated with CAAX-containing proteins other than Ras (e.g., nuclear lamins and transducin) that are also post-translationally modified by the enzyme farnesyl protein transferase.

Compounds of formula I may also act as inhibitors of other prenyl transferases (e.g., geranylgeranyl transferase), and thus be effective in the treatment of diseases associated with other prenyl modifications (e.g., geranylgeranylation) of proteins (e.g., the rap, rab, rac, and rho gene products and the like). For example, they

may find use as drugs against Hepatitis delta virus (HDV) infections, as suggested by the recent finding that geranylgeranylation of the large isoform of the delta antigen of HDV is a requirement for productive viral infection [J. S. Glenn, *et al.*, Science, 256, 1331 (1992)].

The compounds of this invention may also be useful in combination with known anti-cancer and cytotoxic agents. If formulated as a fixed dose, such combination products employ the compounds of this invention within the dosage range described below and the other pharmaceutically active agent within its approved dosage range. Compounds of formula I may be used sequentially with known anticancer or cytotoxic agents when a combination formulation is inappropriate.

The compounds of this invention may be formulated with a pharmaceutical vehicle or diluent for oral, intravenous or subcutaneous administration. The pharmaceutical composition can be formulated in a classical manner using solid or liquid vehicles, diluents and additives appropriate to the desired mode of administration. Orally, the compounds can be administered in the form of tablets, capsules, granules, powders and the like. These compounds may be administered in a dosage range of about 0.05 to 50 mg/kg/day, preferably less than 50 mg/kg/day, in a single dose or in 2 to 4 divided doses.

## Process of Preparation

A compound of formula III below, wherein $Pro_1$ is an amine protecting group (e.g., *t*-butyloxycarbonyl (Boc), benzyloxycarbonyl (Cbz) and the like):

III

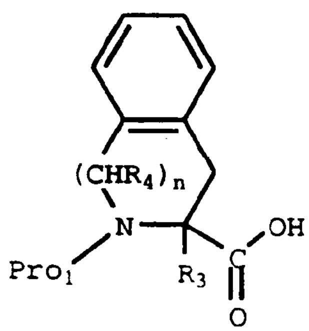

can be coupled with a carboxylic acid protected derivative of an amino acid of formula IV:

IV

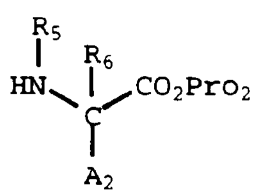

to form a compound of formula V below, wherein $Pro_2$ is a carboxylic acid protecting group (e.g., alkyl, benzyl, p-methoxybenzyl and the like):

V

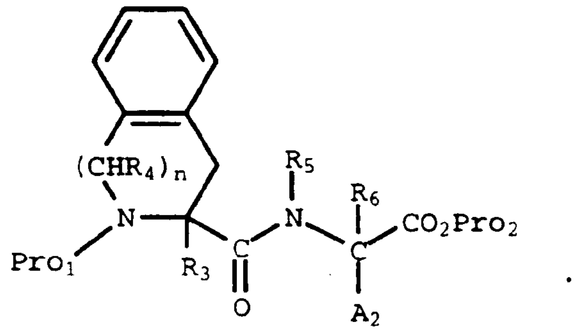

For additional examples of amino and carboxylic acid protecting groups (as well as means of formation and eventual deprotection), see T.W. Greene and P.G.M. Wuts, "Protective Groups in Organic Synthesis," Second Edition, John Wiley & Sons, New York, 1991.

A variety of coupling agents may be used for this coupling, including 1-(3-dimethylaminopropyl)-3-ethylcarbodiimide hydrochloride (EDCI) with 1-hydroxybenzotriazole (HOBT), dicyclohexylcarbodiimide (DCC) with HOBT, benzotriazol-1-yloxytris(dimethylamino)phosphonium hexafluorophosphate (BOP) with or without HOBT, carbonyldiimidazole (CDI), bis(2-oxo-3-oxazolidinyl)phosphinic chloride (BOP chloride), isopropylchloroformate (IPCF) and the like.

Compounds of the formula IV are known in the art. See, for example, R. M. Williams, "Synthesis of Optically Active a α-Amino Acids," Pergamon Press, Oxford, 1989.

Compounds of formula III can be prepared by methods known in the art. For example, for compounds of formula III wherein n is 1 and $R_3$ is alkyl, see, for instance, J.W. Skiles, *et al.*, J. Med. Chem., 29, 784 (1986) and U. Schollkopf, *et al.*, Angew. Chem. Int. Ed. Engl., 26, 143 (1987). For compounds of formula III wherein $R_4$ is alkyl and n is 1, see, for example, I. Huber and D. Seebach, Helvitica Chim Acta, 70, 1944 (1987). For compounds of formula III wherein n is 0 and $R_3$ is H, see, for example, J. L. Stanton, *et al.*, J. Med. Chem., 26 1267 (1983), and for n is 2 and $R_3$ is H, see, for example, M. Okada, *et al.*, JP 02,193,971, 31 July 1990.

A compound of formula V can be treated with a suitable N-deprotecting agent to provide the corresponding free amine of formula VI:

VI

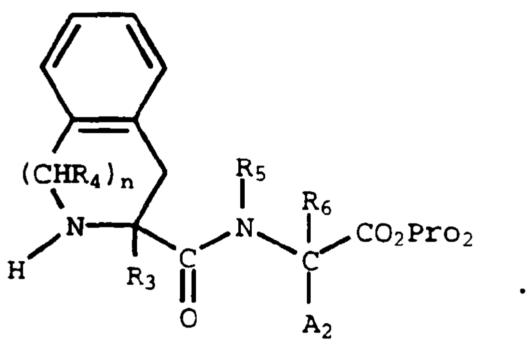

(The $Pro_1$ and $Pro_2$ protecting groups are chosen so that $Pro_1$ can be selectively removed in the presence of $Pro_2$).

An amine of formula VI can be coupled with a suitable amine-protected amino acid of formula VII (wherein $Pro_3$ is an amine protecting group):

VII

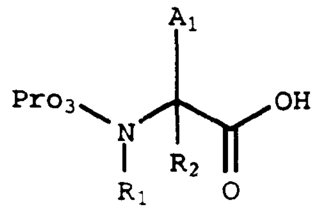

using an appropriate coupling reagent (e.g., BOP-Cl) to form a compound of formula VIII:

VIII

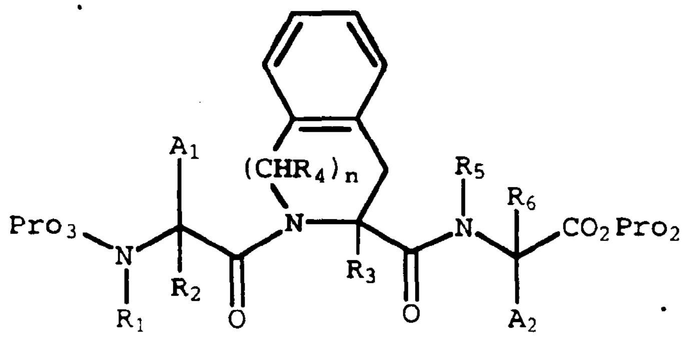

(Compounds of formula VII are known in the art. See, for example, R.M. Williams, "Synthesis of Optically Active α-Amino Acids," Pergamon Press, Oxford, 1989).

Compounds of formula VIII can also be prepared by coupling a C-terminal protected amino acid of the formula IX (wherein $Pro_4$ is a carboxylic acid protecting group):

IX

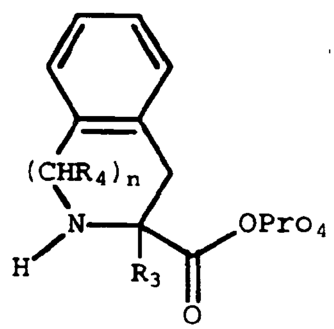

with an amine-protected amino acid of formula VII above to provide a compound of formula X:

X

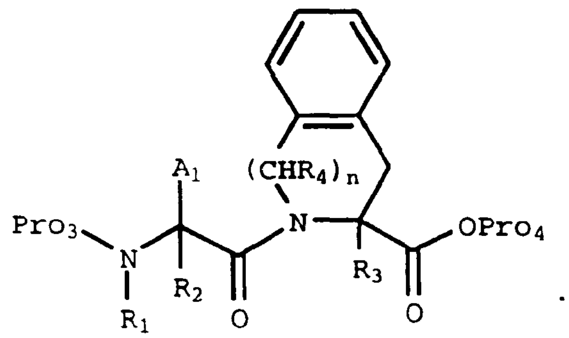

The $Pro_4$ protecting group of compound X can be selectively removed by methods known in the art to provide a compound of formula XI below:

XI

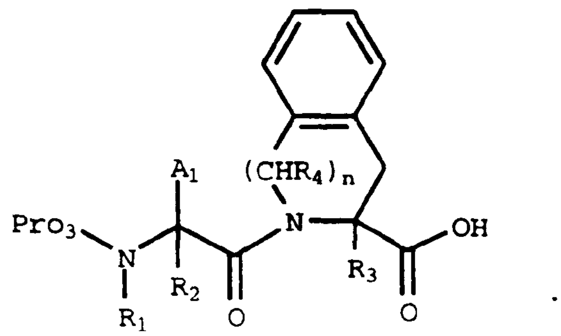

(The $Pro_3$ and $Pro_4$ protecting groups are chosen so that $Pro_4$ can be selectively removed in the presence of $Pro_3$).

Coupling of a compound of formula XI with an amino ester of formula IV would then provide compound VIII.

A compound of formula VIII can be selectively N-deprotected by methods known in the art to provide an amine of formula XII:

XII

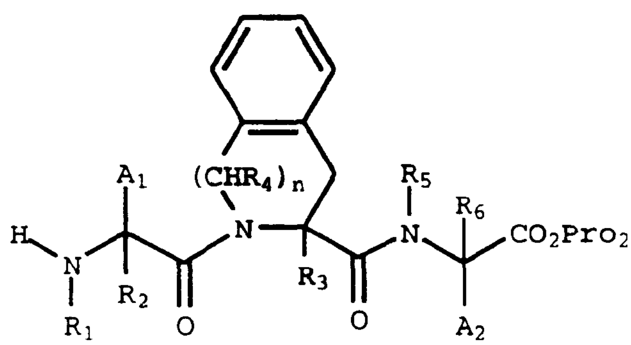

(The $Pro_2$ and $Pro_3$ protecting groups are chosen so that $Pro_3$ can be selectively removed in the presence of $Pro_2$).

Coupling of an amine of formula XII with an acid of formula XIII (wherein X and $G_1$ are optionally protected):

XIII

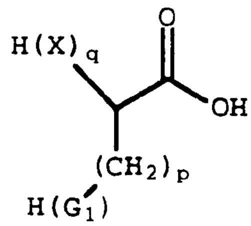

with a suitable coupling agent provides a compound of formula XIV:

XIV

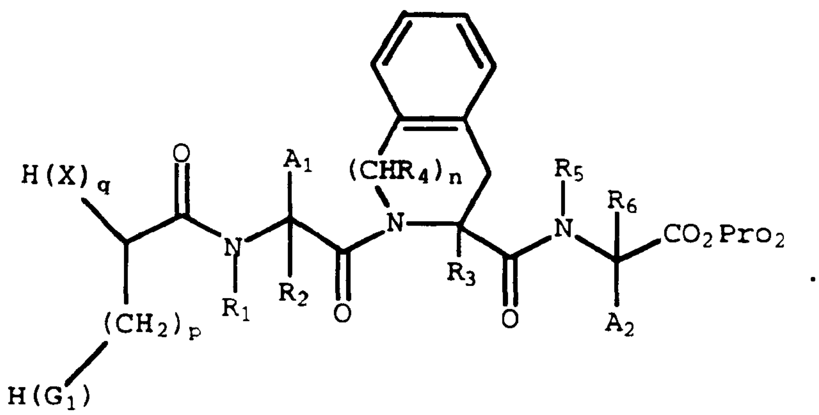

Deprotection of the $Pro_2$ group of XIV, and removal of the optional protecting groups protecting X and $G_1$, then provides a compound of formula I where Y and Z are -C(O)- and $G_2$ is -C(O)OH. (See T.W. Greene and P.G.M. Wuts, "Protective Groups in Organic Synthesis", John Wiley & Sons, New York, 1991, for exemplary protecting groups for X and $G_1$, their formation and removal).

Alternatively, removal of the nitrogen protecting group ($Pro_3$) from a compound of formula X gives an amine of formula XV:

XV

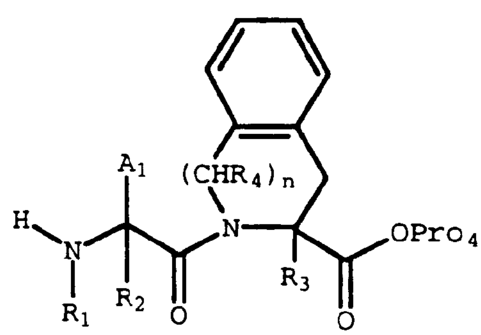

which can then be coupled with an acid of formula XIII to provide a compound of formula XVI wherein X and $G_1$ are optionally protected:

XVI

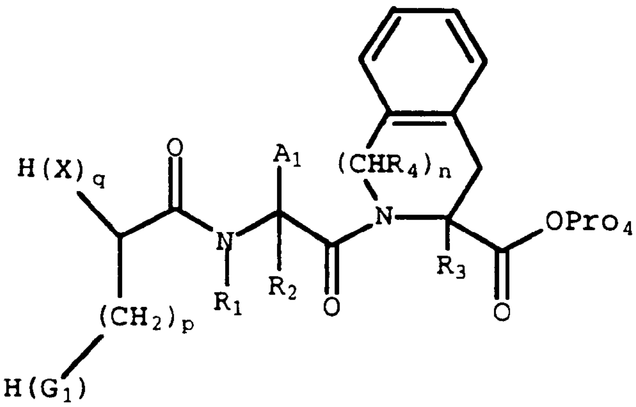

.

Selective deprotection of the $Pro_4$ protecting group of XVI provides an acid of formula XVII:

XVII

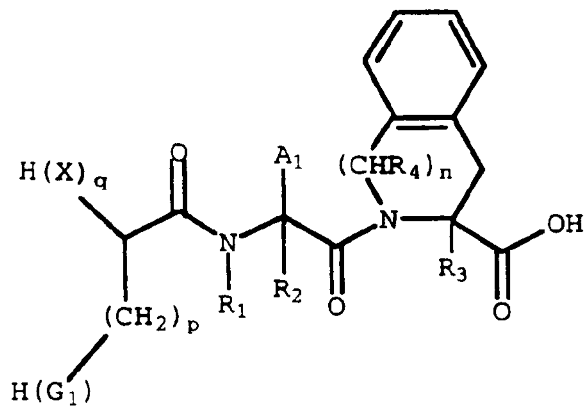

which can be coupled with an amine of formula IV to provide a compound of formula XIV. Deprotection of the $Pro_2$ protecting group of XIV, and removal of the optional protecting groups on X and $G_1$, then also provides a compound of formula I where Y and Z are -C(O)- and $G_2$ is -C(O)OH.

A compound of formula I where Y and Z are -C(O)- and $G_2$ is -C(O)OH or -C(O)$NH_2$, can be prepared by automated solid phase peptide synthesis using methods that are well known in the art. See for example:

a) M. Bodanszky and A. Bodanszky, "The Practice of Peptide Synthesis", Springer-Verlag, Berlin/Heidelberg/New York/ Tokyo, 1984.

b) J. M. Stewart and J. D. Young, "Solid Phase Peptide Synthesis", Pierce Chemical Co., Rockport, Illinois, 1984.

Reduction of a compound of formula XVIII below, wherein $Pro_5$ is an amine protecting group (e.g., *t*-butyloxycarbonyl (Boc), benzyloxycarbonyl (Cbz) and the like):

XVIII

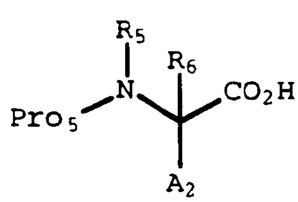

with a reducing agent such as borane provides an alcohol of formula XIX:

XIX

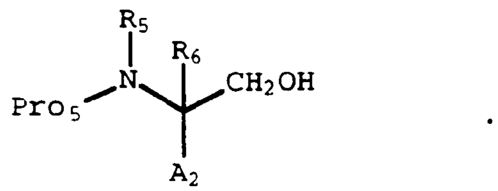

A compound of formula XIX can be N-deprotected by methods known in the art to provide an amine of formula XIXa:

XIXa

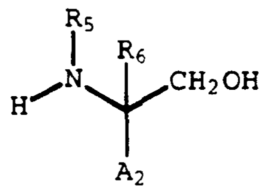

which can be coupled with a compound of formula XVII to provide a compound of formula XX:

XX

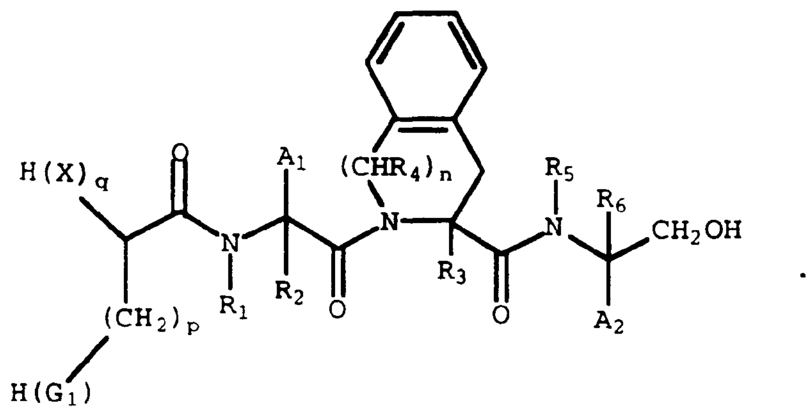

Removal of the optional protecting groups protecting X and $G_1$ then provides a compound of formula I where Y and Z are -C(O)- and $G_2$ is $-CH_2OH$.

Compounds of the formula XVIII are known in the art. See, for example, R. M. Williams, "Synthesis of Optically Active α-Amino Acids", Pergamon Press, Oxford, 1989.

A compound of formula I, where Y and Z are -C(O)- and $G_2$ is $-CH_2OH$, can also be prepared using automated solid phase peptide synthesis by performing the routine final step of releasing the product from its solid support according to the procedure described in J.M. Stewart *et al.*, U.S. Patent No. 4,254,023 (1981). For example, a C-terminal amino acid residue conventionally attached to a phenylacetamidomethyl (PAM) resin would be released as a primary alcohol upon treatment with lithium borohydride in a suitable solvent such as tetrahydrofuran.

Coupling of a compound of formula XVII with a compound of formula XXI:

XXI

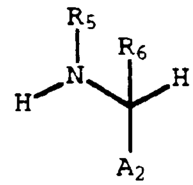

and removal of the optional protecting groups on X and $G_1$ provides a compound of formula I where Y and Z are -C(O)- and $G_2$ is H. Compounds of the formula XXI can be prepared by methods known in the art.

Selective removal of the $Pro_2$ group in a compound of formula XIV by methods known in the art provides a compound of formula XXII wherein X and $G_1$ are optionally protected:

XXII

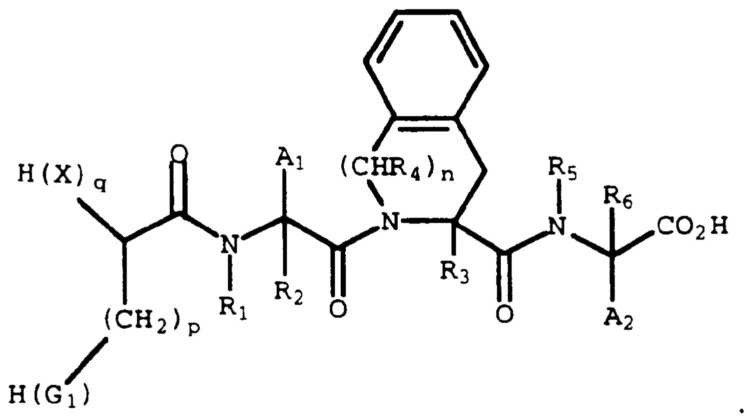

A compound of formula XXII can be coupled with an alkoxyamine of formula XXIII below (wherein $Pro_6$ is a suitable protecting group known in the art, e.g., benzyl, 2-tetrahydropyranyl (THP), and the like):

XXIII

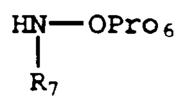

by methods known in the art to provide a hydroxamic ether of formula XXIV wherein X and $G_1$ are optionally protected:

XXIV

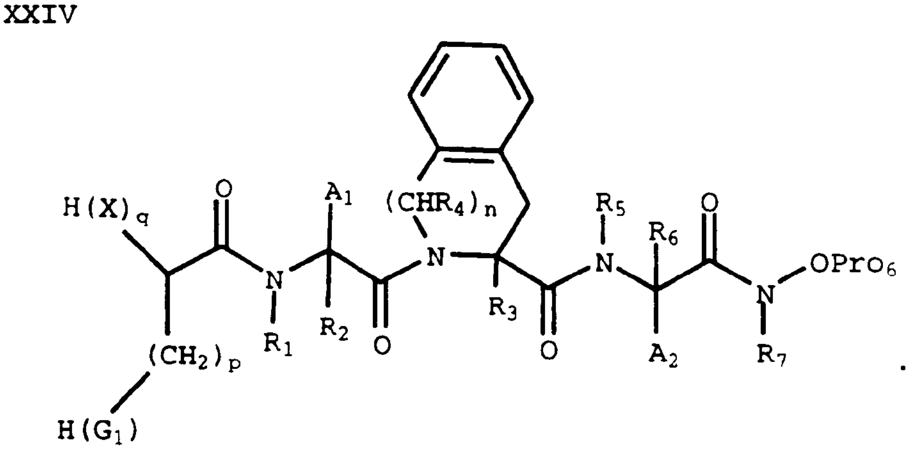

Appropriate deprotection(s) of a compound of formula XXIV as required, depending on the definitions of $Pro_6$, X and $G_1$ provides a compound of formula I where Y and Z are -C(O)- and $G_2$ is $C(O)N(R_7)OH$.

An acid of the formula XVIII can be coupled with ammonia by methods known in the art to form an amide of formula XXV:

XXV

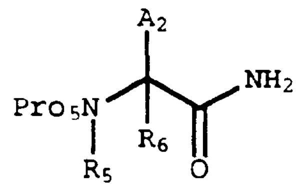

An amide of formula XXV can be dehydrated to form a nitrile of formula XXVI by methods known in the art (e.g., $POCl_3$ in pyridine or p-toluenesulfonyl chloride in pyridine):

XXVI

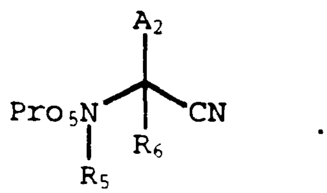

A compound of formula XXVI can be converted to a tetrazole of formula XXVII, wherein $Pro_7$ is a suitable tetrazole protecting group, by methods known in the art:

XXVII

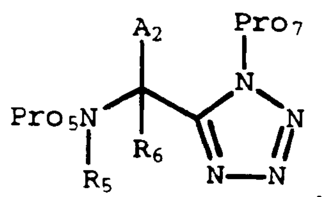

For example, heating a compound of formula XXVI with tributyltin azide in an organic solvent (e.g., toluene), followed by treatment with hydroxide and trityl chloride gives a compound of formula XXVII wherein $Pro_7$ is a trityl group. See, for example, P. Brooks, *et al.*, J. Chem. Soc., 925 (1960); Y. Hirotsu, *et al.*, Bull. Chem. Soc. Japan, 40, 2945 (1967); Z. Grzonka and B. Liberek, Roczniki Chemii. Ann. Soc. Chim. Polonorum, 45, 967 (1971).

Alternatively, a compound of formula XVIII can coupled with an amine of formula XXVIII below by methods known in the art:

XXVIII $NH_2$-$Pro_7$

to give an amide of formula XXIX:

XXIX

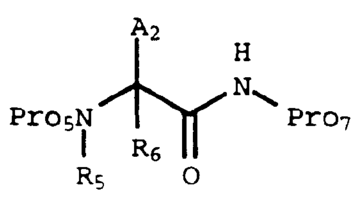

which can be treated under conditions known in the art (e.g., azidotrimethylsilane, triphenylphosphine, diethyl

azodicarboxylate in tetrahydrofuran, as described by J. V. Duncia, *et al.*, J. Org. Chem., 56 2395 (1991)) to provide a compound of formula XXVII. For instance, a compound of formula XXVII where $Pro_7$ is a cyanoethyl group can be prepared using this alternative procedure.

Removal of the $Pro_5$ protecting group from a compound of formula XXVII provides an amine of formula XXX:

XXX

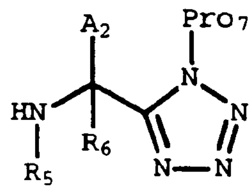

which can be coupled with an acid of formula XVII to provide a compound of formula XXXI wherein X and $G_1$ are optionally protected:

XXXI

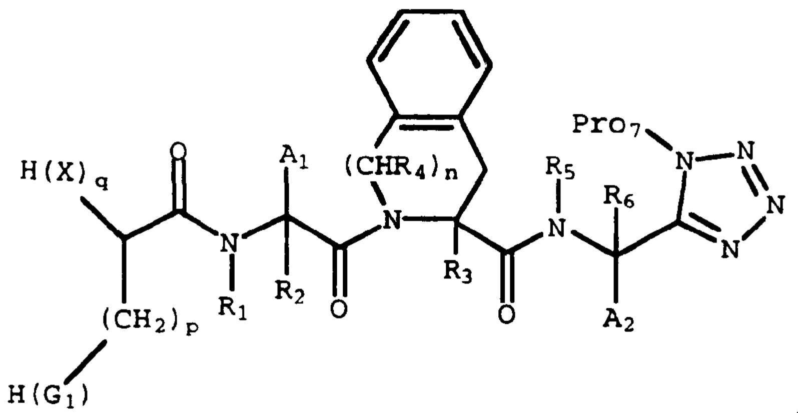

Appropriate deprotection(s) of a compound of formula XXXI as required, depending on the definitions of $Pro_7$, X and $G_1$ by methods known in the art provides a compound of formula I wherein Y and Z are -C(O)- and $G_2$ is 5-tetrazolyl.

An amino acid of formula VII (wherein $Pro_3$ is a suitable amino protecting group) can be converted to the N-methoxy-N-methylamide of formula XXXII by methods known in the art:

XXXII

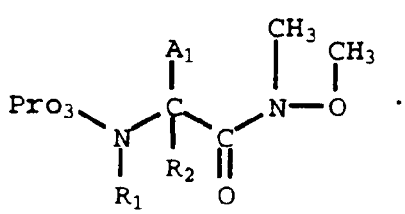

A compound of formula XXXII can be reduced to an aldehyde of formula XXXIII by methods known in the art (e.g., Fehrentz, *et al.*, Synthesis, 676 (1983)):

XXXIII

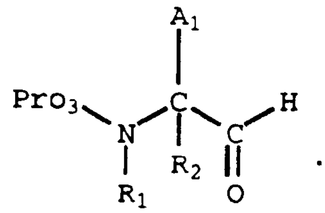

Alternatively, a compound of formula XXXIII can be prepared by reduction of a compound of formula VII with a reducing agent such as borane, followed by oxidation of the resulting alcohol using, for example, the Swern method of oxidation (e.g., Luly, *et al.*, J. Org Chem., 52, 1487 (1987); Stanfield, *et al.*, J. Org. Chem., 46, 4797 (1981)). In yet another alternative, a compound of formula XXXIII can be prepared by reduction of an ester of a compound of formula VII with a reducing agent such as diisobutylaluminum hydride. See, e.g., Rich, *et al.*, J. Org. Chem., 43, 3624 (1978).

An N-protected aldehyde of formula XXXIII can be reductively aminated with an amine of formula VI to form a compound of formula XXXIV using methods known in the art (e.g., Borch, *et al.*, J. Am. Chem. Soc., 93, 2897 (1971)):

XXXIV

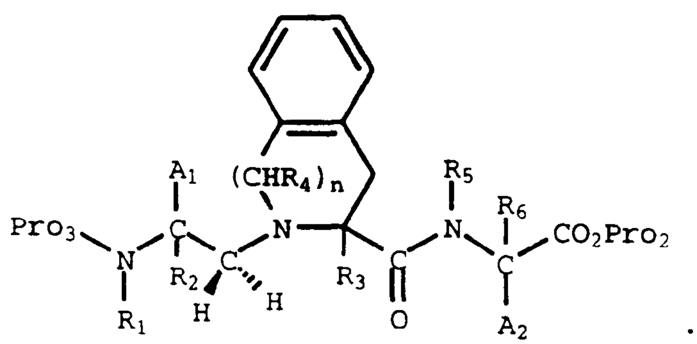

A compound of formula XXXIV can be treated with a suitable N-deprotecting agent to provide the corresponding free amine of formula XXXV:

XXXV

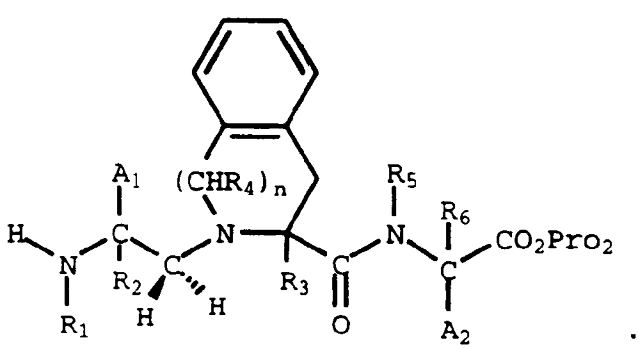

(The $Pro_2$ and $Pro_3$ protecting groups are chosen so that $Pro_3$ can be selectively removed in the presence of $Pro_2$).

A compound of formula XXXV can be coupled with an acid of formula XIII (wherein X and $G_1$ are optionally protected) using an appropriate coupling reagent to form a compound of formula XXXVI:

XXXVI

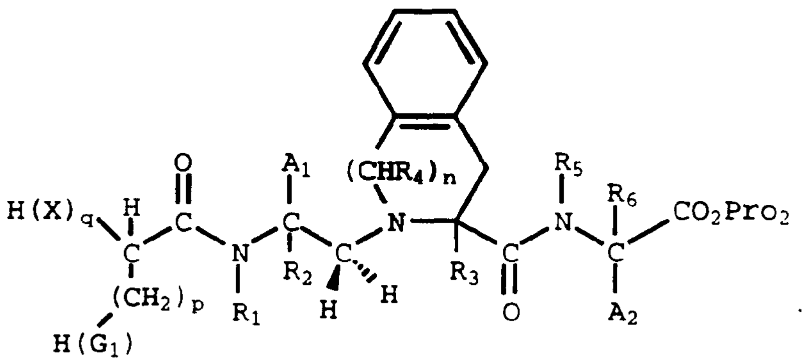

Deprotection of the $Pro_2$ group of XXXVI, and removal of the optional protecting groups on X and $G_1$, provides a compound of formula I where Y is -C(O)-, Z is $-CH_2-$, and $G_2$ is C(O)OH. (See T.W. Greene and P.G.M. Wuts, "Protective Groups in Organic Synthesis", Second Edition, John Wiley & Sons, New York, 1991, for exemplary protecting groups for X and $G_1$, their formation and removal).

An acid of formula XIII (wherein X and $G_1$ are optionally protected) can also be converted to the N-methoxy-N-methylamide of formula XXXVII by methods known in the art (e.g., Fehrentz, *et al.,* Synthesis, 676 (1983)):

XXXVII

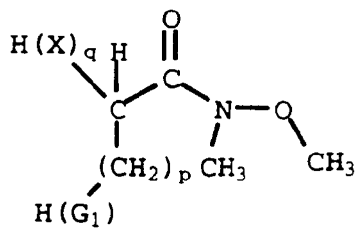

A compound of formula XXXVII can be selectively reduced to an aldehyde of formula XXXVIII by methods known in the art (e.g., Fehrentz, *et al.*, Synthesis, 676 (1983)):

XXXVIII

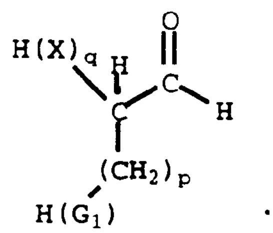

An aldehyde of formula XXXVIII can be reductively aminated with an amine of formula XII to form a compound of formula XXXIX using methods known in the art (Borch, *et al.*, J. Am. Chem. Soc., 93, 2897 (1971)):

XXXIX

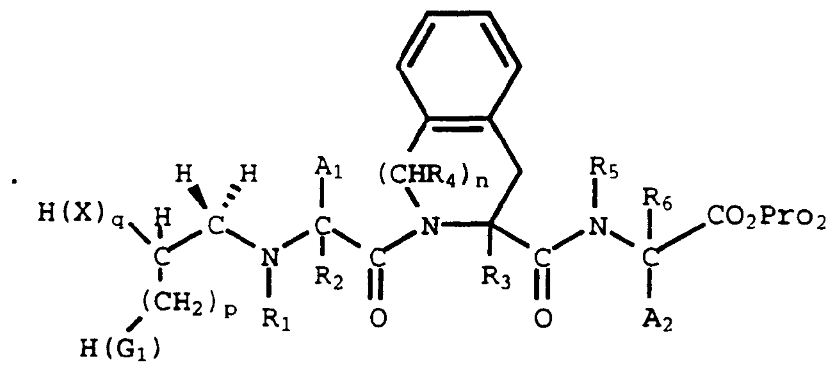

Deprotection of the $Pro_2$ group of XXXIX, and removal of the optional protecting groups on X and $G_1$, provides a compound of formula I where Y is $-CH_2-$, Z is -C(O)-, and $G_2$ is C(O)OH.

By combining the appropriate steps above, one skilled in the art can prepare a compound of formula I where both Y and Z are $-CH_2-$, and $G_2$ is C(O)OH. In addition, by combining the appropriate steps above, one skilled in the art can prepare compounds of formula I where one or both Y and Z are $-CH_2-$, and $G_2$ is H, $-C(O)NH_2$, 5-tetrazolyl, $-C(O)N(R_7)OH$ or $-CH_2OH$.

In yet another process, an aldehyde of formula XXXVIII can be reductively aminated with an amine of formula XXXX, wherein $Pro_8$ is a carboxylic acid protecting group (e.g., alkyl, benzyl, p-methoxybenzyl and the like), to form a compound of formula XXXXI using methods known in the art (Borch, *et al.,* J. Am. Chem. Soc., 93, 2897 (1971)):

XXXX

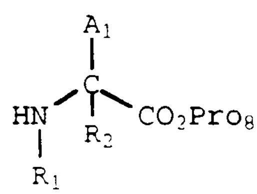

XXXXI

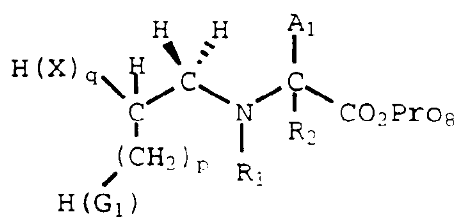

The $Pro_8$ protecting group can be selectively removed by methods known in the art to provide the corresponding carboxylic acid which can be coupled with an amine of formula VI to provide a compound of formula XXXIX. Removal of the protecting groups as described above provides a compound of formula I where Y is $-CH_2-$, Z is -C(O)-, and $G_2$ is C(O)OH.

In yet another process, a compound of formula XXXXI where $R_1$ is H can be protected on the secondary amine with a suitable N-protecting group $Pro_9$ (such as a Cbz group) to provide a compound of formula XXXXII:

XXXXII

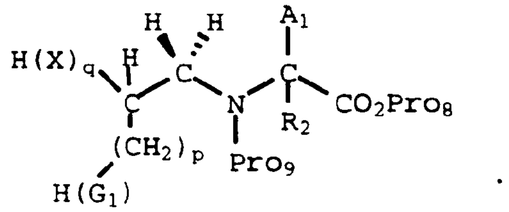

The $Pro_8$ protecting group of a compound of formula XXXXII can be selectively removed by methods known in the art to provide the corresponding carboxylic acid which can be coupled with an amine of formula IX to provide a compound of formula XXXXIII:

XXXXIII

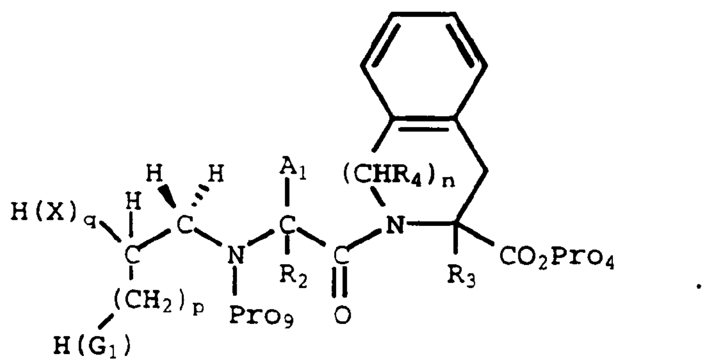

The $Pro_4$ protecting group of a compound of formula XXXXIII can be selectively removed by methods known in the art to provide the corresponding carboxylic acid which can be coupled with an amine of formula IV to provide a compound of formula XXXXIV:

XXXXIV

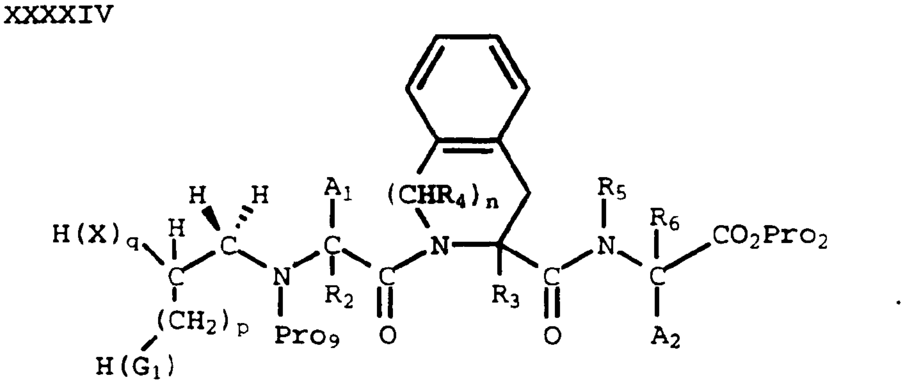

Deprotection of the $Pro_2$ group of XXXIV, removal of the N-protecting group $Pro_9$ (for example, by using a mixture of trifluoroacetic acid, thioanisole and bromotrimethylsilane), and removal of optional protecting groups on X and $G_1$, provide a compound of formula I where Y is $-CH_2-$, Z is -C(O)-, $R_1$ is H, and $G_2$ is C(O)OH.

Side-chain protecting groups may be used in these processes for amino acids having reactive functionalities, such as hydroxyl, carboxyl, amino, mercapto, guanidino, imidazolyl, indolyl and the like. The particular protecting groups used for any amino acid residues depend upon the sidechains to be protected and are generally known in the art. Exemplary sidechain protecting groups include acetyl, benzoyl, benzyl, t-butyl and the like for hydroxyl; cyclohexyl, benzyl, methyl, ethyl, t-butyl and the like for carboxyl; benzyl, 4-methylbenzyl, 4-methoxybenzyl, acetyl, acetamidomethyl, triphenylmethyl (trityl) and the like for mercapto; t-butoxycarbonyl (Boc), benzyloxylcarbonyl (Cbz), N-[(9H-Fluoren-9-ylmethoxy)carbonyl] (Fmoc), phthaloyl (Pht), p-toluenesulfonyl (Tos), trifluoroacetyl, 2-(trimethylsilyl)ethoxycarbonyl (Teoc) and the like for amino; 2,4-dinitrophenyl, benzyloxymethyl, Tos, Boc, trityl and the like for imidazolyl; formyl, Cbz, Teoc, 2,2,2-trichloroethyl carbamate

(TROC) and the like for indolyl; and tosyl, nitro, bis(1-adamantyloxycarbonyl) and the like for guanidino.

Side-chain protecting groups may be removed, if desired, by, for example, treatment with one or more deprotecting agents in an inert solvent or solvent mixture. For examples of protecting groups and suitable deprotecting agents, see M. Bodansky and A. Bodansky, "The Practice of Peptide Synthesis", Springer-Verlag, Inc. (1984); and T. W. Greene and P.G.M. Wuts, "Protective Groups in Organic Synthesis", Second Edition, John Wiley & Sons, New York, 1991.

The invention will now be further described by the following working examples, which are preferred embodiments of the invention. All temperatures are in degrees Celsius (°C) unless otherwise indicated. Compounds exemplified herein, which comprise a basic moiety such as an amine or substituted amine, may exist as a salt of an organic or inorganic acid. Such information is not explicitly described in all the examples, but would be understood by those skilled in the art. These examples are illustrative rather than limiting.

## Example 1

**(R*,S*)-N-[[2-[N-(2-Amino-3-mercaptopropyl)-L-valyl]-1,2,3,4-tetrahydro-3-isoquinolinyl]carbonyl]-L-methionine.**

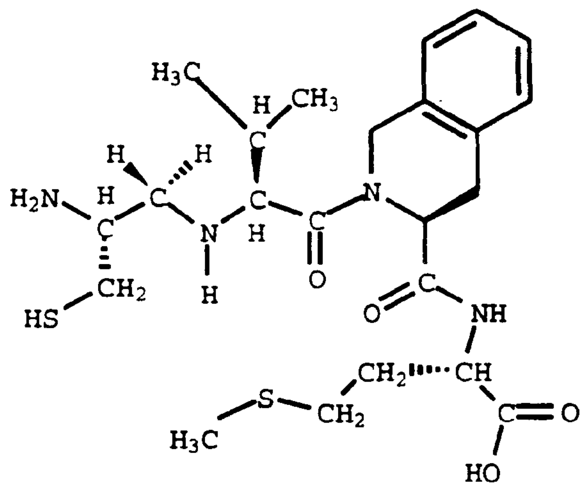

**A. (R*)-N-[[2-[(1,1-Dimethylethoxy)-carbonyl]-1,2,3,4-tetrahydro-3-isoquinolinyl]carbonyl]-L-methionine, methyl ester.**

A solution of (S)-3,4-dihydro-2,3(1H)-isoquinolinedicarboxylic acid, 2-(1,1-dimethylethyl) ester (2.0 g, 7.21 mmol) and L-methionine methyl ester HCl (1.44 g, 7.21 mmol) in 5:15 N-methylpyrolidinone-$CH_2Cl_2$ was stirred at 4°C. N,N-Diisopropylethylamine (1.23 mL, 7.21 mmol) was added, followed by N-hydroxybenzotriazole (974 mg, 7.21 mmol). The reaction mixture was stirred for 5 minutes, then 1-ethyl-3-(3-dimethylaminopropyl)carbodiimide·HCl (1.38 g, 7.21 mmol) was added. The reaction mixture was allowed to come to room temperature, stirred overnight and partitioned between $CH_2Cl_2$ and saturated NaCl. The organic phase was washed successively with citric acid, $NaHCO_3$ and NaCl, dried over $MgSO_4$, and concentrated *in vacuo* to give 2.64 g (86%) of Compound A. This was used in the next step without further purification.

**B. (R*)-N-[(1,2,3,4-Tetrahydro-3-isoquinolinyl)carbonyl]-L-methionine, methyl ester.**

A solution of Compound A (2 g, 6.22 mmol) in 10 mL $CH_2Cl_2$ was treated at room temperature with 10 mL trifluoroacetic acid and 0.5 mL dimethyl sulfide, and stirred for 0.5 hour. The reaction mixture was concentrated *in vacuo*, dissolved in $CH_2Cl_2$ and concentrated. This procedure was repeated five times to yield Compound B (99%) as a clear glass. This was used in the next step without further purification.

**C. (R*)-N-[[2-[N-[(1,1-Dimethylethoxy)carbonyl]-L-valyl]-1,2,3,4-tetrahydro-3-isoquinolinyl]carbonyl]-L-methionine, methyl ester.**

Compound B (4.73 mmol) was dissolved in $CH_2Cl_2$ (20 mL) and cooled to 0°C. N-t-Butyloxycarbonyl-L-valine (2.06 g, 9.466 mmol), bis(2-oxo-3-oxazolidinyl)phosphinic chloride (1.2 g, 4.733 mmol) and N,N-diisopropylethylamine (1.2 g, 1.6 mL, 9.466 mmol) were added. The reaction mixture was stirred for 24 hours at 0°C. Additional bis(2-oxo-3-oxazolidinyl)phosphinic chloride (1.2 g, 4.733 mmol) and N,N-diisopropylethylamine (590 mg, 0.8 mL, 4.733 mmol) were added and the reaction mixture was stirred for an additional 12 hours at 0°C. The reaction mixture was concentrated and chromatographed (silica gel, eluting with 50% ethyl acetate, 50% hexane). The active fractions were collected and concentrated to yield Compound C as a clear oil (2.4 g, 97%).

**D. (R*)-N-[[2-(L-Valyl)-1,2,3,4-tetrahydro-3-isoquinolinyl]carbonyl]-L-methionine, methyl ester.**

Compound C (190 mg, 0.45 mmol) was stirred in methyl sulfide (0.3 mL) and 4N HCl in dioxane (10mL) for 30 minutes at room temperature. The reaction mixture was concentrated *in vacuo*, dissolved in $CH_2Cl_2$ (30 mL) and concentrated. This latter procedure was repeated five times to yield Compound D (99%) as a clear glass. This was used in the next coupling step without further purification.

**E. $N^2$-[(1,1-Dimethylethoxy)carbonyl]-N-methoxy-N-methyl-S-(triphenylmethyl)-L-cysteinamide.**

N-Butyloxycarbonyl-S-trityl-L-cysteine (20.0 g, 43.14 mmol), 1-ethyl-3-(3-dimethylaminopropyl)carbodiimide·HCl (8.3 g, 43.1 mmol), and 1-hydroxybenzotriazole hydrate (5.8 g, 43.1 mmol) were dissolved in dimethylformamide (100 mL). N,O-Dimethylhydroxylamine hydrochloride (4.91 g, 50.6 mmol) and N,N-diisopropylethylamine (6.13 g, 8.3 mL, 47.5 mmol) were then added. The reaction mixture was stirred for 5 hours at room temperature, poured into water (50 mL) and extracted with ethyl acetate (3 X 200 mL). The combined organic layers were washed with water (3 X 500 mL) and brine (200 mL), dried ($MgSO_4$), concentrated and chromatographed (silica gel, eluting with 40% ethyl acetate, 60% hexane). Fractions containing the product were collected and concentrated to yield Compound E as a white solid (19.95 g, 92%).

**F. N-[(1,1-Dimethylethoxy)carbonyl]-S-(triphenylmethyl)-L-cysteinal.**

Compound E (1.00 g, 1.89 mmol) was dissolved in tetrahydrofuran (4 mL) and the solution was cooled to 0°C. 1M $LiAlH_4$ in tetrahydrofuran (4.9 mL, 4.9 mmol) was added dropwise over 20 minutes. The reaction mixture was stirred for 30 minutes at 0°C under nitrogen. Diethyl ether (200 mL) was added and the reaction mixture was quenched with dropwise addition of 1M $KHSO_4$ (40 mL) at 0°C. The mixture was stirred for an additional 30 minutes at 0°C and the layers were separated. The organic layer was washed with saturated $NaHCO_3$ (50 $m_L$), 1M $KHSO_4$ (50 mL) and brine (50 mL), dried ($MgSO_4$), and concentrated to yield Compound F as a white glass (840mg, 99%) which was used immediately in the next step without further purification.

**G. (R*,S*)-N-[[2-[N-[2-[[(1,1-Dimethylethoxy)carbonyl]amino]-3-[(triphenylmethyl)thio]propyl]-L-valyl]-1,2,3,4-tetrahydro-3-isoquinolinyl]carbonyl]-L-methionine, methyl ester.**

Compound D (0.45 mmol) and Compound F (250 mg, 0.54 mmol) were dissolved in methanol (10 mL) and glacial acetic acid (0.2 mL) at room temperature. Sodium cyanoborohydride (28 mg, 0.45 mmol) was dissolved in methanol (2mL) and added dropwise over 30 minutes. The reaction mixture was stirred for 3 hours. More Compound F (125 mg, 0.27 mmol) was then added, and the reaction mixture stirred an additional 3 hours. The mixture was cooled to 0°C, and saturated $NaHCO_3$ (50 mL) was slowly added. The product was then extracted into ethyl acetate (60 mL). The ethyl acetate layer was washed with water (100 mL) and brine (100 mL), dried ($MgSO_4$), concentrated and chromatographed (silica gel, eluting with 40% ethyl acetate, 60% hexane). Fractions containing the product were collected and concentrated to yield Compound G as a clear oil (260 mg, 67%).

**H. (R*,S*)-N-[[2-[N-(2-Amino-3-mercaptopropyl)-L-valyl] -1,2,3,4-tetrahydro-3-isoquinolinyl]carbonyl]-L-methionine.**

Compound G (1.8 g, 2.11 mmol) was dissolved in methanol (30 mL) and 5N NaOH (10 mL) at room temperature. The reaction mixture was stirred for 3 hours at room temperature. The mixture was neutralized to pH 6 using 5N HCl, concentrated and dissolved in ethyl acetate (100 mL) and water (100 mL). The aqueous layer was separated, acidified to pH 2 using 5N HCl, and extracted with ethyl acetate (100 mL). The combined organic layers were washed with brine (50 mL), dried ($MgSO_4$) and concentrated to yield a white solid. This material was dissolved in $CH_2Cl_2$ (10 mL), trifluoroacetic acid (10 mL) and triethylsilane (1.0 mL) and was stirred for 40 minutes at room temperature. The mixture was concentrated, dissolved in $CH_2Cl_2$ (50 mL), and then

concentrated again. This latter procedure was repeated five times to yield the crude product as a white sticky solid (quantitative crude recovery). The crude solid was purified by preparative HPLC (YMC-C18 column, 2.2 X 25 cm, 5 micron, 120 angstrom; solvent A, 0.1% trifluoracetic acid in water; solvent B, 0.1% trifluoracetic acid in acetonitrile: 25-35% B in A over 40 minutes, flow rate 9 mL/min; UV monitored at 220 nm). Fractions containing Compound H were combined and lyophilized to provide a white solid (790 mg, 75% yield). mp 103-104 °C

Optical rotation: - 44.2° (c = 0.5, methanol)

MS: $(M+H)^+$ $497^+$.

Elemental Analysis for 2.20 $C_2HF_3O_2$, 0.80 $H_2O$:

Calc'd: C, 45.03; H, 5.68; N, 7.99; F, 13.40;

Found: C, 45.03; H, 5.44; N, 7.95; F, 12.99.

$^1$H-NMR ($CD_3OD$, 270 MHz) δ(ppm) 7.17 (4H, m), 4.72 (4H, m), 4.48 (1H, m), 4.29 (1H, m), 3.92 (1H, m), 3.25 (2H, m), 2.69 (2H, m), 2.43 (1H, m), 2.12 (3H, m), 1.95 (3H, d), 1.83 (2H, m), 0.98 (6H, m).

## Example 2

**(R*)-N-[[2-[N-(L-Cysteinyl)-L-valyl]-1,2,3,4-tetrahydro-3-isoquinolinyl]carbonyl]-L-methionine.**

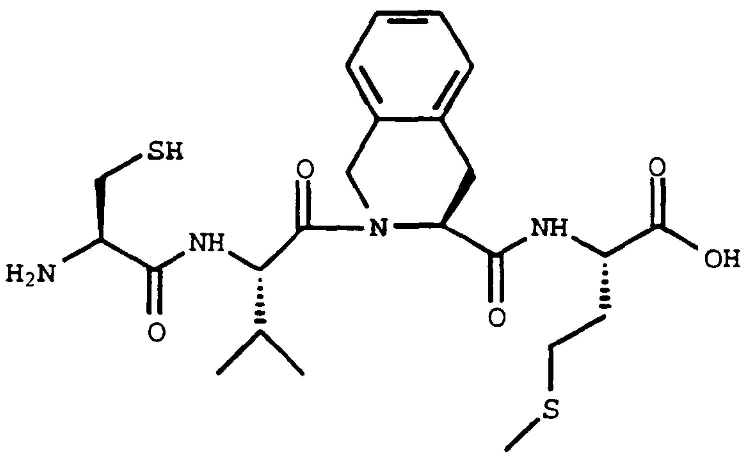

**A. (R*)-N-[[2-[N-[N-[(1,1-Dimethylethoxy)carbonyl]-S-(triphenylmethyl)-L-cysteinyl]-L-valyl]-1,2,3,4-tetrahydro-3-isoquinolinyl]carbonyl]-L-methionine, methyl ester.**

A solution of Compound 1C (1.2 g, 2.3 mmol) in 7.5mL of $CH_2Cl_2$ was treated at 4°C with 7.5 mL of trifluoroacetic acid and stirred for one hour. The reaction mixture was concentrated *in vacuo*, dissolved in $CH_2Cl_2$ and neutralized with N,N-diisopropylethylamine. The resulting solution was added to an ice-bath chilled solution of N-t-butyloxycarbonyl-S-trityl-L-cysteine (3.21 g, 6.9 mmol), benzotriazol-1-yloxy-tris(dimethylamino)phosphonium hexafluorophosphate (3.05 g, 6.9 mmol) N-hydroxybenzotriazole (936 mg, 6.9 mmol), and N,N-diisopropylethylamine (1.2 mL, 9 mmol) in tetrahydrofuran. The reaction mixture was allowed to come to room temperature and stirred overnight. The solvent was removed *in vacuo* and the residue dissolved in ethyl acetate. The organic solution was washed successively with saturated citric acid, $NaHCO_3$ and NaCl, then dried over $MgSO_4$ and concentrated *in vacuo* to a white foamy residue. Purification on a 5 x 16 column of silica, eluting with 25% to 40% ethyl acetate/hexane, gave 1.34 g (1.55 mmol, 67%) of Compound A.

Alternatively, Compound 1C (3 g, 5.75 mmol) was added to a solution of 2N HCl in acetic acid (10 mL) and dimethyl sulfide (1 mL). After stirring for 3 hours, the solvents were removed *in vacuo* and the residue was triturated with ether (3 x 50 mL). A solution of the resulting material (2.4 g) in ethyl acetate (100 mL) was washed with sodium bicarbonate (20 mL), dried over sodium sulfate, filtered and concentrated *in vacuo.* The residue (2.19 g) was dissolved in dimethylformamide (30 mL) and cooled to 0°C under $N_2$. To this was added N-t-butyloxycarbonyl-S-trityl-L-cysteine (2.42 g, 5.21 mmol), N,N-diisopropylethylamine (905 mL, 5.21 mmol), and benzotriazol-1-yloxy-tris(dimethylamino)phosphonium hexafluorophosphate (2.30 g, 5.21 mmol). After 30 minutes, the reaction was allowed to warm to room temperature. After stirring for 6 hours, the reaction was diluted with ethyl acetate (300 mL) and washed sequentially with saturated sodium bicarbonate (100 mL), phosphate buffer (pH=4, 100 mL) and 10% lithium chloride (3 x 100 mL). The organic layer was dried over sodium sulfate, filtered and concentrated *in vacuo*. The residue was purified on silica gel (400 mL eluting with 3:1 to

1:1 hexane/ethyl acetate) to give Compound A (4.23 g, 86%).
TLC: $R_f$ = 0.49 (1:1 hexane/ethyl acetate, visualized by ceric ammonium sulfate); mp: 82-84°C.

**B. (R*)-N-[[2-[N-[N-[(1,1-Dimethylethoxy)carbonyl]-S-(triphenylmethyl)-L-cysteinyl)-L-valyl]-1,2,3,4-tetrahydro-3-isoquinolinyl]carbonyl]-L-methionine.**

Compound A (1.34 g, 1.55 mmol) in 90 mL methanol was treated at room temperature with LiOH (162 mg, 3.88 mmol) in 30 mL $H_2O$ for 3 hours. The methanol was removed *in vacuo*, and the aqueous solution diluted with water, washed with ether and acidified with saturated citric acid. The product was extracted from the aqueous phase with ethyl acetate. The organic solution was washed with saturated NaCl and dried over $MgSO_4$, and the solvent was removed *in vacuo* to give 650 mg (0.76 mmol, 50%) of Compound B which was used in the subsequent step without further purification.

Alternatively, to a solution of Compound A (1.0 g, 1.15 mmol) in tetrahydrofuran (4 mL) at 0°C was added 1N sodium hydroxide (2.30 mL, 2.30 mmol). After stirring for 2 hours, the reaction mixture was poured into 1N hydrochloric acid (10 mL) and extracted with ethyl acetate (3 x 50 mL). The organic layer was dried over sodium sulfate, filtered and concentrated *in vacuo* to give Compound B (970 mg, 99%).
TLC: $R_f$ = 0.75 (9:1:0.05 chloroform/methanol/acetic acid, visualized by ceric ammonium sulfate); mp: 112 -115°C.

**C. (R*)-N-[[2-[N-(L-Cysteinyl)-L-valyl]-1,2,3,4-tetrahydro-3-isoquinolinyl]carbonyl]-L-methionine.**

Compound B was treated at room temperature with a mixture of 95 mL trifluoroacetic acid, 3 mL thioanisole, 1 mL methyl sulfide and 1 mL ethanedithiol, for 3.5 hours. The volatiles were removed *in vacuo* and the residue triturated with cold ether. The crude peptide precipitated, and 286 mg (0.56 mmol, 74%) was collected, washed with cold ether and finally extracted into glacial acetic acid. The acetic acid solution was lyophilized, and the lyophilate was purified by reverse phase HPLC using a gradient of 25-60% solvent B in solvent A over 60 minutes (solvent A, 0.05% trifluoroacetic acid in $H_2O$; solvent B, 0.05% trifluoroacetic acid in $CH_3CN$) to give, after lyophilization, 120 mg (42%) of pure Compound C. FAB-MS: 533 (M $Na^+$), 511($MH^+$),362 (M-Met $H^+$), 309 (M-Tic Met $H^+$).

| Calculated for $C_{28}H_{34}N_4O_5S_2 \cdot C_2HF_3O_2 \cdot H_2O$: | | | | | |
|---|---|---|---|---|---|
| Theory: | C, 47.22; | H, 5.82; | N, 8.85; | S, 10.12; | F, 8.55. |
| Found: | C, 47.21; | H, 5.67; | N, 8.60; | S, 10.15; | F, 8.41. |

**Example 3**

**(R*)-N-[[2-[N-(L-Cysteinyl)-L-valyl]-1,2,3,4-tetrahydro-3-isoquinolinyl]carbonyl]-L-methionine, methyl ester.**

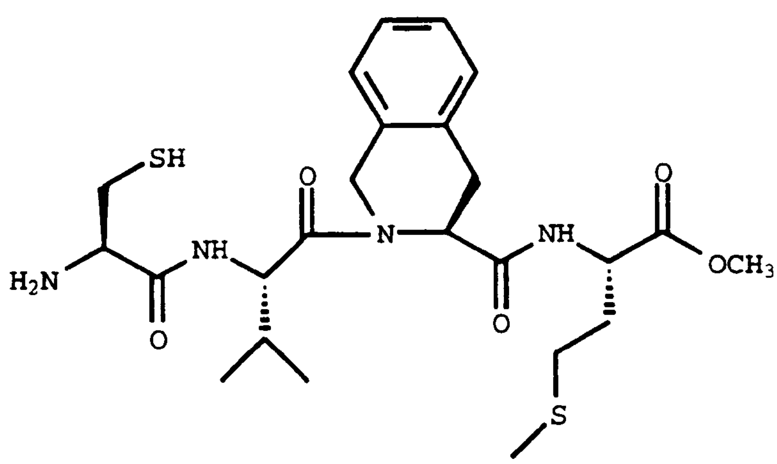

The title Compound (100 mg, 27%) was prepared from Compound 2A (672 mg, 0.72 mmol) using the procedure described for the preparation of Compound 2C from Compound 2B, except that the reaction was carried out at 0°C.
FAB-MS: 525($MH^+$) 547($MNa^+$).

| Calculated for $C_{24}H_{36}N_4O_5S_2 \cdot 1.22C_2HF_3O_2 \cdot 0.14\ H_2O$: | | | | | |
|---|---|---|---|---|---|
| Theory: | C, 47.66; | H, 5.67; | N, 8.41; | S, 9.62; | F, 10.44. |
| Found: | C, 47.66; | H, 5.41; | N, 8.17; | S, 9.85; | F, 10.42. |

## Example 4

**(R*)-N-[[2-[N-(3-Mercapto-1-oxopropyl)-L-valyl]-1,2,3,4-tetrahydro-3-isoquinolinyl]-carbonyl]-L-methionine.**

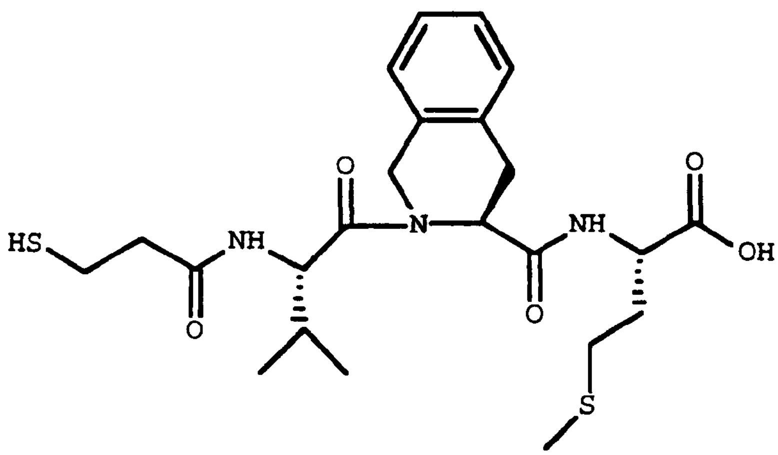

**A. (R*)-N-[[2-[N-[3-[(Triphenylmethyl)thio]-1-oxopropyl]-L-valyl]-1,2,3,4-tetrahydro-3-isoquinolinyl]carbonyl]-L-methionine, methyl ester.**

Compound 1C (2.61 g, 5 mmol) was treated at 0°C with 50 mL 1:1 trifluoroacetic acid/$CH_2Cl_2$ for one hour. The volatiles were removed *in vacuo*, and the residue dissolved in 25 mL tetrahydrofuran, neutralized with N,N-diisopropylethylamine, and added to an ice-cold solution of S-trityl-3-mercaptopropionic acid (5.22 g, 15 mmol, prepared according to Kwang et al., J. Med. Chem., 13, (1970) 414), benzotriazol-1-yl,oxy-tris(dimethylamino)phosphonium hexafluorophosphate (6.65 g, 15 mmol), N-hydroxybenzotriazole (2.03 g, 15 mmol) and N,N-diisopropylethylamine (2.7 mL) in 75 mL tetrahydrofuran. The reaction mixture was allowed to come to room temperature and stirred overnight. The reaction mixture was concentrated *in vacuo*, and the residue partitioned between ethyl acetate and NaCl. The organic phase was washed with citric acid, $NaHCO_3$ and NaCl, dried over $MgSO_4$, and concentrated to a white foamy residue. This was purified on a 5 x 16 column of silica gel, using 20-60% ethyl acetate/hexane as the eluant, to give 2.07 g (55%) of Compound A.

**B. (R*)-N-[[2-[N-[3-[(Triphenylmethyl)thio]-1-oxopropyl]-L-valyl]-1,2,3,4-tetrahydro-3-isoquinolinyl]carbonyl]-L-methionine.**

Compound A (1.59 g, 2.1 mmol) was treated with LiOH as described for preparation of Compound 2B to give 1.25 g (88%) of Compound B.

**C. (R*)-N-[[2-[N-(3-Mercapto-1-oxopropyl)-L-valyl]-1,2,3,4-tetrahydro-3-isoquinolinyl]carbonyl]-L-methionine.**

Compound B (740 mg, 1 mmol) was deprotected in trifluoroacetic acid/thioanisole/methyl sulfide/ethanethiol as described for the preparation of Compound 3A. After removal of the volatiles, the residue was triturated

with ether/pentane, and the precipitated crude product was washed with cold pentane, then extracted into 0.5% trifluoroacetic acid in acetonitrile. The acetonitrile solution was diluted with an equal volume of 0.5% trifluoroacetic acid in water, lyophilized, and the residue purified on reverse phase HPLC, to give Compound C. FAB-MS: 496($MH^+$), 518($MNa^+$).

| Calculated for $C_{23}H_{33}N_3O_5S_2 \cdot 0.46C_2HF_3O_2 \cdot 0.03\ H_2O$: | | | | | |
|---|---|---|---|---|---|
| Theory: | C, 52.40; | H, 6.16; | N, 7.79; | S, 11.91; | F, 4.73. |
| Found: | C, 52.40; | H, 6.24; | N, 7.79; | S, 11.91; | F, 4.72. |

**Example 5**

**(R*,R*)-N-[[2-[2-(L-Cysteinylamino)-3-methylbutyl]-1,2,3,4-tetrahydro-3-isoquinolinyl]carbonyl]-L-methionine.**

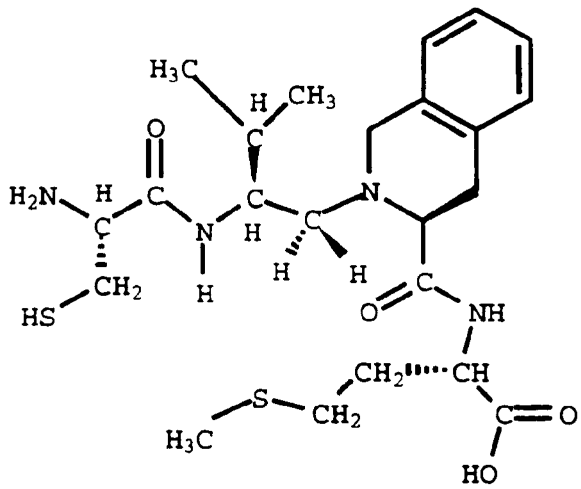

**A. $N^2$-[(1,1-Dimethylethoxy)carbonyl]-N-methoxy-N-methyl-L-valinamide.**

N,O-Dimethylhydroxylamine hydrochloride (4.91 g, 50.6 mmol) was dissolved in $CH_2Cl_2$ (100 mL). N-t-Butyloxycarbonyl-L-valine (10.0 g, 46 mmol), 1-ethyl-3-(3-dimethylaminopropyl)carbodiimide·HCl (9.7 g, 50.6 mmol), and N,N-diisopropylethylamine (6.13 g, 8.3 mL, 47.5 mmol) were added. The reaction mixture was stirred for 16 hours at room temperature, concentrated and chromatographed (silica gel, eluting with 40% ethyl acetate, 60% hexane). The active fractions were collected and concentrated to yield Compound A as a clear oil (3.5 g, 42%).

**B. N-[(1,1-Dimethylethoxy)carbonyl]-L-valinal.**

Compound B (2.6 g, 99%; colorless oil) was prepared from Compound A (3.5 g, 11.8 mmol) using the procedure described for the preparation of Compound 1F from Compound 1E.

**C. (R*)-N-[[2-[2-[[(1,1-Dimethyl ethoxy)carbonyl]amino]-3-methylbutyl]-1,2,3,4-tetrahydro-3-isoquinolinyl]carbonyl]-L-methionine, methyl ester.**

To a solution of Compound B (2.6 g, 11.82 mmol) and Compound 1B (4.66 g, 13.0 mmol) in methanol (100 mL) at room temperature was added glacial acetic acid (2.0 mL). Sodium cyanoborohydride (743 mg, 11.82 mmol) was added portionwise over 30 minutes. The reaction mixture was stirred at room temperature for 4 hours. The mixture was cooled to 0°C. Saturated $NaHCO_3$ (100 mL) was slowly added and the product was extracted with ethyl acetate (200 mL). The ethyl acetate layer was washed with water (200 mL) and brine (100 mL), dried ($MgSO_4$), concentrated and chromatographed (silica gel, eluting with 50% diethyl ether, 50% hexane). Fractions containing the product were collected and concentrated to yield Compound C as a clear oil

(1.5 g, 30%).

**D. (R*,R*)-N-[[2-[2-[[N-[(1,1-Dimethylethoxy)carbonyl]-S-(triphenylmethyl)-L-cysteinyl]amino]-3-methylbutyl]-1,2,3,4-tetrahydro-3-isoquinolinyl] carbonyl]-L-methionine, methyl ester.**

Compound C (1.5 g, 2.96 mmol) was stirred in methyl sulfide (0.4 mL) and 4N HCl in dioxane (10 mL) for 30 minutes at room temperature. The mixture was concentrated, dissolved in $CH_2Cl_2$ (30 mL), and then concentrated again. This latter procedure was repeated five times to yield the amine hydrochloride as a clear glass. This material was dissolved in dimethylformamide (20 mL) and N,N-diisopropylethylamine (383 mg, 0.52 mL, 2.96 mmol). This solution was added dropwise to a stirred solution of N-t-butyloxycarbonyl-S-trityl-L-cysteine (1.65 g, 3.55 mmol), 1-ethyl-3-(3-dimethylaminopropyl)-carbodiimide·HCl (737 mg, 3.85 mmol), and 1-hydroxybenzotriazole hydrate (519 mg, 3.85 mmol) in dimethylformamide (30 mL). The reaction mixture was stirred for 16 hours. The mixture was poured into water (100 mL) and the product was extracted with ethyl acetate (2 X 100 mL). The combined ethyl acetate layer was washed with water (3 X 200 mL) and brine (100 mL), dried ($MgSO_4$), concentrated and chromatographed (silica gel, eluting with 50% ethyl acetate, 50% hexane). Fractions containing the product were collected and concentrated to yield Compound D as a clear oil (1.9 g, 75%).

**E. (R*,R*)-N-[[2-[2-(L-Cysteinylamino)-3-methylbutyl]-1,2,3,4-tetrahydro-3-isoquinolinyl]carbonyl]-L-methionine.**

Compound E (50%) was prepared from Compound D (1.4 g, 1.64 mmol) using the procedure described for the preparation of Compound 1H from Compound 1G.
mp 112-113 °C
Optical rotation: - 55.7° (c = 0.5, methanol)
MS: $(M+H)^+$ $497^+$.
Elemental Analysis for 2.20 $C_2HF_3O_2$, 0.80 $H_2O$:
Calc'd: C, 43.123; H, 5.02; N, 6.84;
Found: C, 42.83; H, 5.12; N, 7.13.
$^1$H-NMR ($CD_3OD$, 270 MHz) δ (ppm) 7.28 (4H, m), 4.57 (2H, m), 4.39 (1H, m), 4.22 (2H, m), 4.11 (1H, m), 3.14 (4H, br m), 2.55 (2H, m), 2.21 (2H, m), 2.10 (3H, s), 1.97 (3H, m), 0.98 (6H, m).

## Example 6

**(R*)-N-[[2-[N-(3-Mercaptopropyl)-L-valyl]-1,2,3,4-tetrahydro-3 -isoquinolinyl] carbonyl]-L-methionine.**

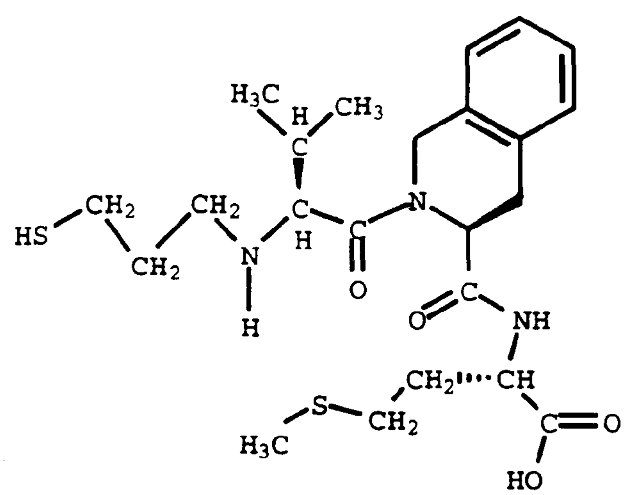

**A. 3-[(Triphenylmethyl)thio]-N-methoxy-N-methylpropanamide.**

To a stirred solution of S-trityl-3-mercaptopropionic acid (4.5 g, 12.9 mmol, prepared according to Kwang et al., J. Med. Chem., 13, (1970) 414) in $CH_2Cl_2$ (40 mL) was added N,N-diisopropylethylamine (2.2 mL, 12.9 mmol) followed by benzotriazol-1-yloxy-tris(dimethylamino)phosphonium hexafluorophosphate (5.7 g, 12.9

mmol). After several minutes, N,O-dimethylhydroxylamine hydrochloride (1.31 g, 13.5 mmol) was added along with additional diisopropylethyl amine (2.2 mL, 12.9 mmol). The solution was stirred for 2 hours at room temperature. Additional $CH_2Cl_2$ was added (80 mL) and the solution was washed with 1N $KHSO_4$ (3x), saturated $NaHCO_3$ (3x) and brine (2x). The organic layer was dried ($MgSO_4$) and concentrated. The residue was recrystallized twice using ethyl acetate/hexanes to yield Compound A (4.12 g, 82%).

**B. [(Triphenylmethyl)thio]propanal.**

Compound B (1.5 g, 88%) was prepared from Compound A (1.8 g, 5.2 mmol) using the procedure described for the preparation of Compound 1F from Compound 1E.

**C. (R*)-N-[[2-[N-[3-[(Triphenylmethyl)-thio]propyl]-L-valyl]-1,2,3,4-tetrahydro-3-isoquinolinyl]carbonyl]-L-methionine, methyl ester.**

Compound C (1.8 g, 53%) was prepared from Compound B (1.5 g, 4.6 mmol) and Compound 1D (2.2 g, 5.2 mmol) by a procedure analogous to that described for the preparation of Compound 5C from Compound 5B and Compound 1B.

**D. (R*)-N-[[2-[N-(3-Mercaptopropyl)-L-valyl]-1,2,3,4-tetrahydro-3-isoquinolinyl]carbonyl]-L-methionine.**

Compound D (white powder, 49%) was prepared from Compound C (700 mg, 0.94 mmol) using a procedure analogous to that described for the preparation of Compound 1H from Compound 1G.
MS $(M+H)^+$ $482^+$
$^1$H-NMR($CD_3OD$, 270 MHz) δ (ppm) 7.23-7.13 (4H, m), 4.65-4.49 (4H, m), 4.26 (1H, m), 3.22 (1H, m), 3.06-2.88 (3H, m), 2.51-2.35 (4H, m), 1.97 (3H, s), 2.33-1.77 (4H, m), 1.10-1.07 (6H, m).
Elemental Analysis for $C_{23}H_{34}N_3O_4S_2$.1.2$C_2HF_3O_2$, 1.35 $H_2O$:
Calc'd: C, 47.53; H, 5.95; N, 6.55; S, 9.99; F, 10.68;
Found: C, 47.54; H, 5.85; N, 6.69; S, 9.76; F, 10.66.
Optical rotation: -33.8° (c = 0.7, methanol).

<u>**Example 7**</u>

<u>**(R*)-N-[[2-[N-(3-Mercaptopropyl)-L-alanyl]-1,2,3,4-tetrahydro-3-isoquinolinyl]carbonyl]-L-methionine.**</u>

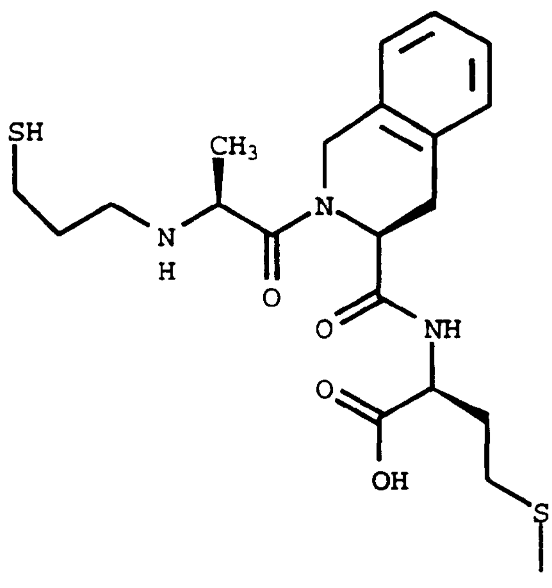

**A. (R*)-N-[[2-[N-[(1,1-Dimethylethoxy)carbonyl]-L-alanyl]-1,2,3,4-tetrahydro-3-isoquinolinyl]carbonyl]-L-methionine, methyl ester.**

Compound 1A (4.0 g, 9.47.mmol) was stirred in dimethyl sulfide (0.5 mL) and 4N HCl in dioxane (10 mL)

for 30 minutes. The mixture was concentrated, dissolved in methylene chloride (50 mL) and concentrated. This latter procedure was repeated five times to yield the amine hydrochloride as a clear glass. This material was dissolved in methylene chloride (40 mL) and cooled to 0°C. t-Butyloxycarbonyl-L-alanine (3.58 g, 18.93 mmol), bis(2-oxo-3-oxazolidinyl)phosphinic chloride (4.82 g, 19.93 mmol) and N,N-diisopropylethylamine (3.68 g, 5.0 mL, 28.40 mmol) were added. The reaction mixture was stirred for 24 hours at 0°C. The reaction mixture was concentrated and chromatographed (silica gel, eluting with 40% ethyl acetate, 60% hexane). Fractions containing the product were collected and concentrated to yield Compound A as a white glass (4.6 g, 99%).

**B. (R*)-N-[[2-[N-[3-[(Triphenylmethyl)thio]propyl]-L-alanyl]-1,2,3,4-tetrahydro-3-isoquinolinyl]carbonyl]-L-methionine, methyl ester.**

Compound A (2.5 g, 5.07 mmol) was stirred in methyl sulfide (0.3 mL) and 4N HCl in dioxane (10 mL) for 30 minutes at room temperature. The mixture was concentrated, dissolved in methylene chloride (30 mL) and concentrated. This latter procedure was repeated five times to yield the amine hydrochloride as a clear glass. This material and Compound 6B (4.4 g, 13.25 mmol) were dissolved in methanol (100 mL) and glacial acetic acid (2.0 mL) at room temperature. Sodium cyanoborohydride (833 mg, 13.25 mmol) was added portionwise over 30 minutes. The reaction mixture was stirred for 4 hours at room temperature. The mixture was cooled to 0°C, and saturated $NaHCO_3$ (100 mL) was slowly added. The product was then extracted into ethyl acetate (200 mL). The ethyl acetate layer was washed with water (200 mL) and brine (100 mL), dried ($MgSO_4$), concentrated and chromatographed (silica gel, eluting with 50% ethyl acetate, 50% hexane). Fractions containing the product were collected and concentrated to yield Compound B as a clear oil (2.0 g, 56%).

**C. (R*)-N-[[2-[N-(3-Mercaptopropyl)-L-alanyl]-1,2,3,4-tetrahydro-3-isoquinolinyl]carbonyl]-L-methionine.**

Compound C was prepared from Compound B using the procedure described for the preparation of Compound 1H from Compound 1G.
mp 81-82 °C
$[a]D^{25}$ = - 43.3° (c=1.0, methanol),
MS: $(M+H)^+$ $454^+$.
Elemental Analysis :
Calc'd: C, 46.82; H, 5.89; N, 7.12.
Found: C, 46.83; H, 5.52; N, 7.05.
$^1$H-NMR (CD3OD, 270 MHz) δ (ppm) 7.16 (4H, m), 4.67 (2H, m), 4.56 (2H, m), 4.51 (1H, m), 4.39 (1H, m), 4.03 (1H, m), 3.19 (2H, m), 3.01 (2H, m), 2.48 (2H, m), 2.35 (1H, m), 1.99 (1H, m), 1.96 (3H, s), 1.86 (2H,m), 1.51 (3H, m).

**Example 8**

**(R*)-N-[[2-[N-(3-Mercaptopropyl)-N-methyl-L-valyl]-1,2,3,4-tetrahydro-3-isoquinolinyl]carbonyl]-L-methionine.**

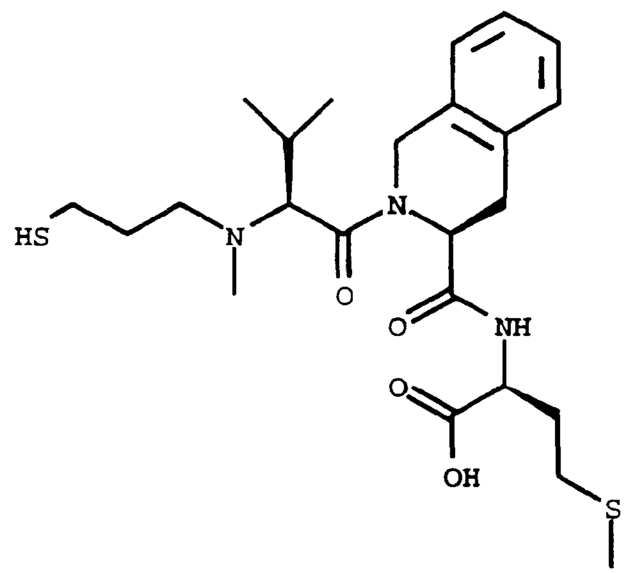

**A. (R*)-N-[[2-[N-[(9H-Fluoren-9-ylmethoxy)carbonyl]-N-methyl-L-valyl]-1,2,3,4-tetrahydro-3-isoquinolinyl]carbonyl]-L-methionine, methyl ester.**

Compound 1A (2.0 g, 4.733 mmol), was N-deprotected using the procedure described in the preparation of Compound 7A. The resulting amine hydrochloride was dissolved in $CH_2Cl_2$ (20 mL) and cooled to 0°C. Fmoc-N-methyl-L-valine (1.67 g, 4.733 mmol), bis-(2-oxo-3-oxazolidinyl)phosphinic chloride (2.41 g, 9.467 mmol) and N,N-diisopropylethylamine (2.45 g, 3.3 mL, 18.9 mmol) were added. The reaction mixture was stirred for 4 hours at 0°C. The reaction mixture was concentrated and chromatographed (silica gel, eluting with 40% ethyl acetate, 60% hexane) to yield Compound A as a white glass (2.4g, 78%).

**B. (R*)-N-[[2-[N-[3-[(Triphenylmethyl)thio]propyl]-N-methyl-L-valyl]-1,2,3,4-tetrahydro-3-isoquinolinyl]carbonyl]-L-methionine, methyl ester.**

Compound A (1.20 g, 2.309 mmol) was stirred in acetonitrile (30 mL) and diethylamine (2.4 mL) for 1 hour at room temperature. The mixture was concentrated, dissolved in methylene chloride (50 mL), and then concentrated again. This latter procedure was repeated seven times to yield the amine as a clear glass. This amine was converted to the hydrochloride salt with 4N HCl in dioxane (10 mL). The resulting solution was concentrated, dissolved in methylene chloride (50 mL), and then concentrated again. This latter procedure was repeated three times to yield the amine hydrochloride as a clear glass/oil mixture. This product and Compound 6B (1.33 g, 4.018 mmol), were dissolved in methanol (35 mL) and glacial acetic acid (0.6 mL) at room temperature. Sodium cyanoborohydride (253 mg, 4.018 mmol) was added portionwise over 1 hour. The reaction mixture was stirred for 16 hours. The mixture was cooled to 0°C and saturated $NaHCO_3$ (50 mL) was slowly added. The product was then extracted into ethyl acetate (100 mL). The ethyl acetate layer was washed with water (100 mL) and brine (50 mL), dried ($MgSO_4$), concentrated and chromatographed (silica gel, eluting with 95% methylene chloride, 5% methanol). Fractions containing the desired compound were collected and concentrated to yield Compound B as a clear oil (1.4 g, 52%).

**C. (R*)-N-[[2-[N-(3-Mercaptopropyl)-N-methyl-L-valyl]-1,2,3,4-tetrahydro-3-isoquinolinyl]carbonyl]-L-methionine.**

Compound C (60% yield) was prepared from Compound B (1.3 g, 1.73 mmol) using the procedure described for the preparation of Compound 1H from Compound 1G.
mp 85-86 °C

$[a]D^{25}$ = -31.9 ° (c=0.7, methanol)
MS: $(M+H)^+$ $496^+$
Elemental Analysis:
Calc'd: C, 49.38; H, 6.17; N, 6.59; F, 9.84;
Found: C, 49.50; H, 6.39; N, 6.41; F, 9.70.
$^1$H-NMR (CD3OD, 270 MHz) δ (ppm) 7.21 (4H, m), 4.87 (2H, m), 4.55 (2H, m), 3.24 (2H, m), 3.06 (2H, m), 2.48 (2H, m), 2.32 (1H, m), 2.07 (1H, m), 1.98 (3H, s), 1.88 (3H, m), 1.22 (3H, d, J=7.03Hz), 0.95 (3H, d, J=6.45Hz).

**Example 9**

**(R*,R*)-N-[[2-[2-[(3-Mercaptopropyl)amino]-3-methylbutyl]-1,2,3,4-tetrahydro-3-isoquinolinyl]carbonyl]-L-methionine.**

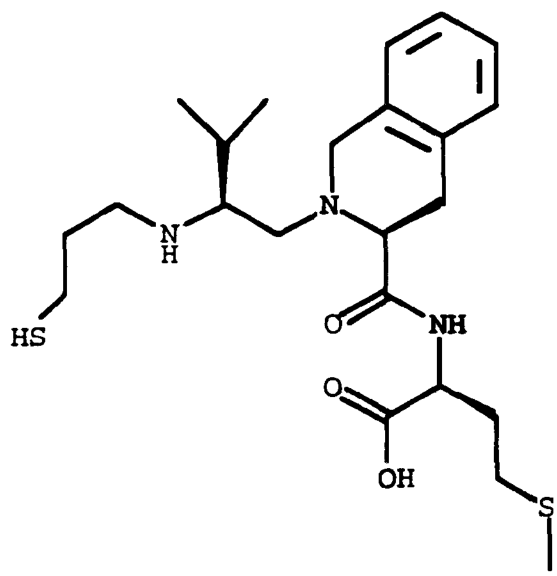

**A. (R*)-N-[[2-[2-[[3-[(Triphenylmethyl)thio]propyl]amino]-3-methylbutyl]-1,2,3,4-tetrahydro-3-isoquinolinyl]carbonyl]-L-methionine, methyl ester.**

Compound A (1.7 g, 54%) was prepared from Compound 5C (2.00 g, 3.95 mmol) using the two step procedure described for the preparation of compound 7B from Compound 7A, with the exception that in the second step only 1.1 equivalents (4.34 mmol) of Compound 6B and 1.1 equivalents (4.34 mmol) of sodium cyanoborohydride were used.

**B. (R*,R*)-N-[[2-[2-[(3-Mercaptopropyl)amino]-3-methylbutyl]-1,2,3,4-tetrahydro-3-isoquinolinyl]carbonyl]-L-methionine.**

Compound B (70% yield) was prepared from Compound A (1.6 g, 2.21 mmol) using the procedure described for the preparation of Compound 1H from Compound 1G.
mp 68-69°C
$[a]_D^{25}$ = -19.2° (c=0.6, methanol)
MS: $(M+H)^+$ $468^+$

| Elemental Analysis: | | | | |
|---|---|---|---|---|
| Calcd: | C, 49.40; | H, 6.25; | N, 6.78; | F, 11.49; |
| Found: | C, 49.40; | H, 6.50; | N, 6.60; | F, 11.33. |

$^1$H-NMR ($CD_3OD$, 270 MHz) δ (ppm) 7.18 (4H, m), 4.64 (1H, m), 4.04 (2H, m), 3.84 (1H, m), 3.25 (2H, m), 3.12 (2H, m), 2.77 (2H, m), 2.67 (2H, m), 2.54 (2H, m), 2.18 (1H, m), 2.08 (3H, s), 2.02 (4H, m), 1.03 (3H,d, J=6.45Hz),

0.95 (3H, d, J=7.03Hz).

**Example 10**

**(R*)-N-[[2-[N-(3-Mercaptopropyl)-L-prolyl]-1,2,3,4-tetrahydro-3-isoquinolinyl]carbonyl]-L-methionine.**

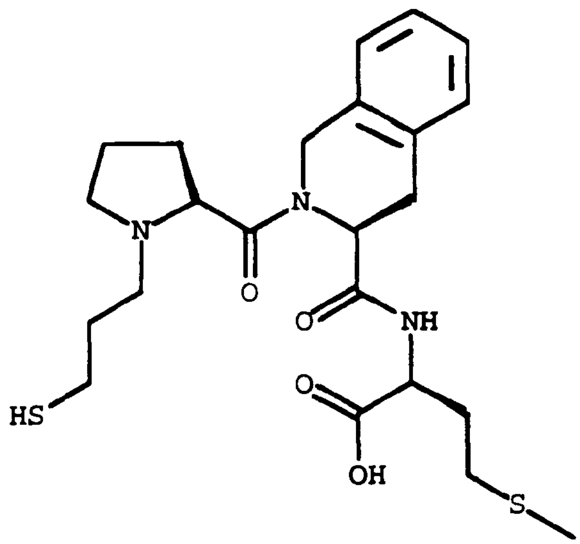

**A. (R*)-N-[[2-[N-[(1,1-Dimethylethoxy)carbonyl]-L-prolyl]-1,2,3,4-tetrahydro-3-isoquinolinyl]carbonyl]-L-methionine, methyl ester.**

Compound A (1.2 g, 49%) was prepared from Compound 1A (2.0 g, 4.73 mmol) using the two step procedure described for the preparation of Compound 8A from Compound 1A, except that in the second step t-butyloxycarbonyl-L-proline (2.04 g, 9.46 mmol) was used instead of Fmoc-N-methyl-L-valine.

**B. (R*)-N-[[2-[N-[3-[(Triphenylmethyl)thio]propyl]-L-prolyl]-1,2,3,4-tetrahydro-3-isoquinolinyl]carbonyl]-L-methionine, methyl ester.**

Compound B (clear oil, 1.5 g, 89%) was prepared from Compound A (1.20 g, 2.31 mmol) using the procedure described for the preparation of Compound 7B from Compound 7A.

**C. (R*)-N-[[2-[N-(3-Mercaptopropyl)-L-prolyl]-1,2,3,4-tetrahydro-3-isoquinolinyl]carbonyl]-L-methionine.**

Compound C (70% yield) was prepared from Compound B (1.5 g, 2.04 mmol) using the procedure described for the preparation of Compound 1H from Compound 1G.
mp 54-55 °C
$[a]_D^{25}$ = -50.0 °(c=0.7, methanol)
MS: $(M+H)^+$ $480^+$
Elemental Analysis:
Calc'd: C, 48.75; H, 5.75; N, 6.74; F, 10.51;
Found: C, 48.63; H, 5.92; N, 6.65; F, 10.66.
$^1$H-NMR ($CD_3OD$, 270 MHz) δ (ppm) 7.18 (4H, m), 4.67 (2H, m), 4.53 (2H, m), 4.41 (1H, m), 4.30 (1H, m), 3.72 (1H, m), 3.24 (4H, m), 2.70 (1H, m), 2.46 (2H, m), 2.34 (1H, m), 2.11 (2H, m), 1.98 (2H, m), 1.96 (3H,s), 1.87 (4H, m).

**Example 11**

**(R*)-N-[[2-[N-(2-Mercaptoethyl)-L-valyl]-1,2,3,4-tetrahydro-3-isoquinolinyl]-carbonyl]-L-methionine.**

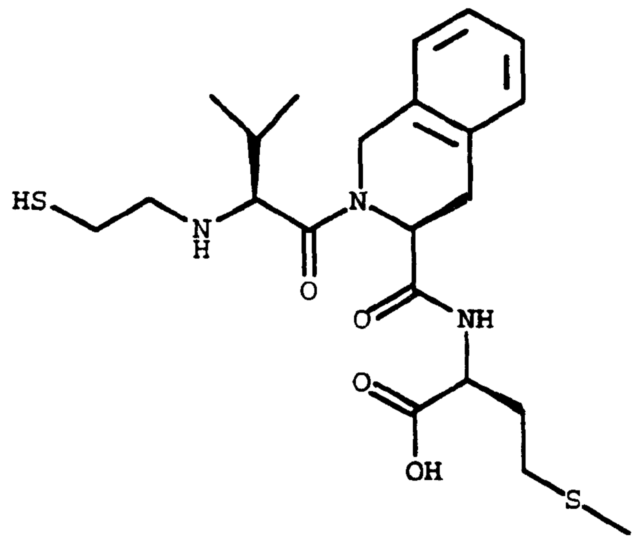

**A. 2-[(Triphenylmethyl)thio]-N-methoxy-N-methylacetamide.**

Compound A (6.0 g, 76%) was prepared using the procedure described for the preparation of Compound 6A, except that triphenylmethylthioacetic acid (7.0 g, 20.96 mmol) was used in place of S-(triphenylmethyl)-3-mercaptopropionic acid.

**B. [(Triphenylmethyl)thio]acetaldehyde.**

Compound B was prepared from Compound A (2.0 g, 5.3 mmol) in quantitative yield using the procedure described for the preparation of Compound 6B from Compound 6A.

**C. (R*)-N-[[2-[N-[2-[(Triphenylmethyl)thio]ethyl]-L-valyl]-1,2,3,4-tetrahydro-3-isoquinolinyl]carbonyl]-L-methionine, methyl ester.**

Compound C (490 mg, 12.8%) was prepared from Compound B (5.3 mmol) and Compound 1C (2.8 g, 5.3 mmol) using the procedure described for the preparation of Compound 6C from Compound 6B.

**D. (R*)-N-[[2-[N-(2-Mercaptoethyl)-L-valyl]-1,2,3,4-tetrahydro-3-isoquinolinyl]carbonyl]-L-methionine.**

Compound D (white powder, 50% yield) was prepared from Compound C (490 mg, 0.68 mmol) using the procedure described for the preparation of Compound 6D from Compound 6C.
mp 94-96°C
$[a]_D^{25}$ = - 36.7° (c=0.81, methanol)
MS: $(M+H)^+$ $468^+$.
Elemental Analysis:
Calc'd: C, 48.38; H, 5.87; N, 7.05; F, 9.57;
Found: C, 48.39; H, 6.09; N, 7.14; F, 9.60.
$^1$H-NMR ($CD_3OD$, 270 MHz) δ (ppm) 7.19 (4H, m), 4.58 (4H, m), 3.07 (2H, m), 2.65 (2H, m), 2.47 (1H, m), 2.29 (1H, m), 2.08 (1H, m), 1.97 (3H, s), 1.90 (2H, m), 1.07 (6H, m).

**Example 12**

**(R*)-N-[[2-[N-(3-Mercapto-1-oxopropyl)-L-valyl]-1,2,3,4-tetrahydro-3-isoquinolinyl]carbonyl]-L-methionine, methyl ester.**

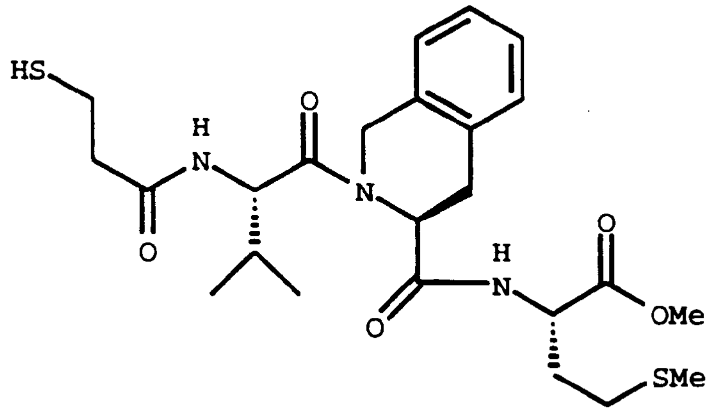

Triethylsilane (223 mg, 306 mL, 1.9 mmol) in 10 mL $CH_2Cl_2$ and trifluoroacetic acid (10 mL) were added dropwise, alternatingly, to a solution of Compound 4A (480 mg, 0.64 mmol) in 5 mL $CH_2Cl_2$. The reaction was stirred under nitrogen at room temperature for 40 minutes, then concentrated *in vacuo.* The residue was triturated with ether/pentane, and the precipitate was collected, washed with more ether and pentane, and then extracted into $CH_3CN$/0.5% trifluoroacetic acid. This solution was diluted with $H_2O$/0.5% trifluoroacetic acid, and lyophilized to give 250 mg (0.5 mmol, 77%) crude ester as a white solid. Purification by reverse phase HPLC (35 to 90% solvent B in solvent A over 100 minutes: solvent A, 0.05% trifluoroacetic acid in $H_2O$; solvent B, 0.05% trifluoroacetic acid in $CH_3CN$) gave 105 mg (0.21 mmol, 32%) pure title Compound as an amorphous white powder.
FAB-MS: 510 $MH^+$, 508 $MH^-$.
$^1H$ NMR: $\delta$ 7.3-7.4 (m), 4.8-5.2 (m), 3.83 (s), 3.77(s),3.2-3.7 (m), 2.5-2.9 (m), 2.2 (s), 2.1 (s), 1.2 (m).
Calculated for $C_{24}H_{35}N_3O_5S_2 \cdot H_2O$:
Theory: C 54.66; H 7.06; N 7.97; S 12.16;
Found: C 54.79; H 6.64; N 7.84; S 11.91.

**Example 13**

**(R*,S*)-2-[N-(2-Amino-3-mercaptopropyl)-L-valyl]-1,2,3,4-tetrahydro-N-[3-(methylthio)propyl]-3-isoquinolinecarboxamide.**

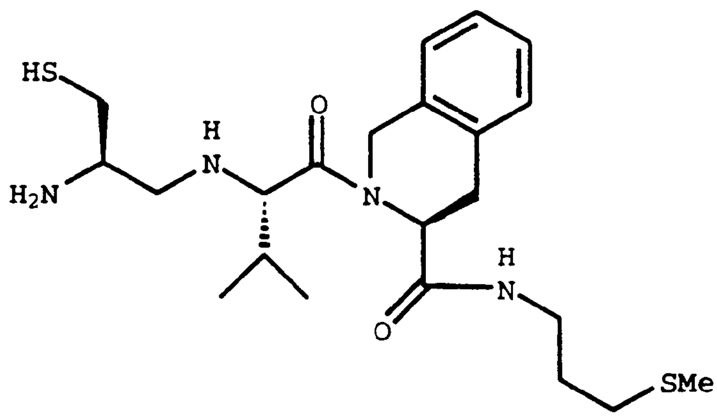

**A. (S)-2-[(1,1-Dimethylethoxy)carbonyl]-1,2,3,4-tetrahydro-N-[3-(methylthio)propyl]-3-isoquinolinecarboxamide.**

To a nitrogen-flushed, ice-cooled suspension of N-t-butyloxycarbonyl-L-(1,2,3,4-tetrahydro)isoquinoline-3-carboxylate (3.0 g, 10.8 mmol) and (3-methylthio)propylamine (1.13 g, 10.8 mmole) in 40:13 $CH_2Cl_2$/N-methylpyrolidinone, was added N,N-diisopropylethylamine (1.85 mL, 10.8 mmol) and N-hydroxybenzotriazole (1.46 g, 10.8 mmol). After five minutes of stirring at 0°C, 1-(3-dimethylaminopropyl)-3-ethylcarbodiimide hydrochloride (2.07 g, 10.8 mmol) was added. Within one hour, a clear solution was obtained. The reaction mixture was allowed to come slowly to room temperature and stirred overnight. The solution was washed successively with saturated NaCl, citric acid, saturated $NaHCO_3$, and saturated NaCl, dried over $MgSO_4$, and concentrated *in vacuo*. The resulting residue was dissolved in $CHCl_3$ and washed several times with saturated NaCl. The organic layer was dried over $MgSO_4$, and concentrated *in vacuo* to give Compound A (3.91 g, 10.2 mmol, 94%) as a colorless oil.

**B. (R*)-2-[N-[(1,1-Dimethylethoxy)carbonyl]-L-valyl]-1,2,3,4-tetrahydro-N-[3-(methylthio)propyl]-3-isoquinolinecarboxamide.**

Compound A (10.2 mmol) was stirred under nitrogen in 40 mL 1:1 trifluoroacetic acid $CH_2Cl_2$ for one hour. The solution was concentrated *in vacuo.* $CH_2Cl_2$ was added and the solution was concentrated again; this latter procedure was repeated twice more. The residue was dissolved in $CH_2Cl_2$, treated with N,N-diisopropylethylamine (1.77 mL, 10.2 mmol), and added to an ice-cooled, nitrogen-flushed *(ca.* 20 minutes) reaction mixture of N-t-butyloxycarbonyl-L-valine (4.42 g, 20.4 mmol), bis(2-oxo-3-oxazolidinyl)phosphinic chloride (5.19 g, 20.4 mol), and N,N-diisopropylethylamine (3.55 mL, 20.4 mmol) in $CH_2Cl_2$. The pH was adjusted to 6 by addition of another 0.9 mL N,N-diisopropylethylamine, and the reaction placed under nitrogen in a 4°C cold room overnight. The solution was washed successively with saturated NaCl, 50 mM $KHSO_4$, saturated $NaHCO_3$ and saturated NaCl. The organic layer was dried over $MgS0_4$, and concentrated *in vacuo* to an oily residue. Flash chromatogrpahy on a 5 x 12 silica column, eluting with 12.5% to 50% ethyl acetate in hexane, gave Compound B (1.9 g, 4.1 mmol).

**C. (R*,S*)-2-[N-[2-[[(1,1-Dimethylethoxy)carbonyl]amino]-3-[(triphenylmethyl)thio]propyl]-L-valyl]-1,2,3,4-tetrahydro-N-[3-(methylthio)propyl]-3-isoquinolinecarboxamide.**

A nitrogen-flushed solution of Compound B (0.93 g, 2.0 mmol) and methylsulfide (1.5 mL, 20 mmol) in 20 mL 1:1 trifluoroacetic acid-$CH_2Cl_2$ was stirred at 0°C for one hour. The mixture was concentrated *in vacuo.* dissolved in $CH_2Cl_2$, and concentrated *in vacuo* again. This latter procedure was repeated several times until the $CH_2Cl_2$ solution was pH 4. This was concentrated a final time, combined with Compound 1F in anhydrous methanol under nitrogen, and brought to pH 7 by dropwise addition of N,N-diisopropylethylamine. Solid $NaBH_3CN$ (160 mg, 2.8 mmol) was added in portions, and the reaction was allowed to proceed at room temperature overnight. The pH was readjusted from 9 to 6 with 4 drops of glacial acetic acid, and the reaction mixture stirred an additonal 1.5 hours. The mixture was concentrated *in vacuo.* The residue was cooled on ice, and partitioned between cold ethyl acetate and saturated $NaHCO_3$. The organic phase was washed with saturated NaCl, dried over $MgSO_4$, and conentrated *in vacuo.* The residue was purified on a flash column of 2 x 18 cm silica, eluting with 25%, then 40%, ethyl acetate in hexane, to provide 625 mg (0.79 mmol, 40%) Compound C.

**D. (R*,S*)-2-[N-(2-Amino-3-mercaptopropyl)-L-valyl]-1,2,3,4-tetrahydro-N-[3-(methylthio)propyl]-3-isoquinolinecarboxamide.**

To a nitrogen-flushed solution of Compound C (0.79 mmol) in 5 mL $CH_2Cl_2$ were added, dropwise and alternatingly, trifluoroacetic acid (15 mL) and triethylsilane (10mL). The reaction mixture was stirred at room temperature for 3 hours, concentrated *in vacuo*, and triturated with ether and pentane to give a solid residue. This solid was collected, washed several times with ether and pentane, and then extracted into glacial acetic acid. The acid solution was lyophilized to give a dark oily residue. This residue was dissolved in 50:50 $CH_3CN$/0.5% trifluoroacetic acid-$H_2O$/0.5% trifluoroacetic acid, filtered through Norit to remove the dark color, and then purified on preparative HPLC, eluting with a gradient of 20% to 50% B in A (A is 0.05% trifluoroacetic acid in $H_2O$; B is 0.05% trifluoroacetic acid in $CH_3CN$). Lyophilization of the product-containing fractions gave 27 mg (0.06 mmol, 8%) of title Compound as a white amorphous powder.

FAB MS: $MH^+$ 453.

$^1H$ NMR ($CD_3OD$): δ 7.0-7.2 (m), 4.5-4.9 (m), 3.9 (d), 3.7 (d), 2.9-3.5 (m), 2.6-2.9 (m), 2.3 (dt), 1.9-2.2 (m), 1.6 (m), 1.4-1.5 (m), 0.9-1.1 (dd).

Calculated for $C_{22}H_{35}N_4O_2S_2 \cdot$ 1.95 trifluoroacetic acid·2.10 $H_2O$:

Theory: C, 43.70; H, 5.83; N, 7.87; S, 9.01; F, 15.61.

Found: C, 43.67; H, 5.52; N, 7.55; S, 8.67; F, 15.83.

## Example 14

**(R*)-2-[N-(3-Mercaptopropyl)-L-valyl]-1,2,3,4-tetrahydro-N-[3-(methylthio)propyl]-3-isoquinolinecarboxamide.**

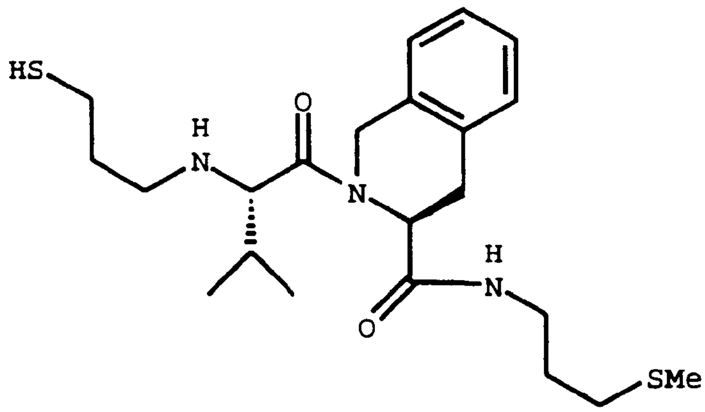

**A. (R*)-2-[N-[3-[(Triphenylmethyl)thio]propyl]-L-valyl]-1,2,3,4-tetrahydro-N-[3-(methylthio)propyl]-3-isoquinolinecarboxamide.**

A nitrogen-flushed solution of Compound 13B (0.95 g, 1 mmol), trifluoroacetic acid (10 mL), and methyl sulfide (1.5 mL, 20 mmol) in 10 mL $CH_2Cl_2$ was stirred for 1 hour at 0°C, and then concentrated *in vacuo.* The residue was dissolved in $CH_2Cl_2$ and concentrated (this process was repeated four times). The amine salt was then dissolved along with Compound 6B in a 15:1 tetrahydrofuran-methanol mixture. The pH was adjusted to 7 with N,N-diisopropylethylamine, and solid $NaBH_3CN$ (160 mg, 2.8 mmole) was added in portions over several minutes. The reaction was stirred at room temperature overnight, at which time the pH was adjusted down to 6 with a few drops of acetic acid. Additional $NaBH_3CN$ (80 mg, 1.4 mmol) was added, and the reaction stirred for an addional 24 hours. The mixture was cooled to 0°C and quenched by addition of ethyl acetate and $NaHCO_3$. The phases were separated, and the organic phase washed with saturated NaCl, dried over $MgSO_4$, and concentrated *in vacuo.* The residue was applied purified on a 2 x 20 cm flash column of silica. Elution with 25% then 40% ethyl acetate in hexane gave 385 mg (0.55 mmol, 28%) Compound A.

**B. (R*)-2-[N-(3-Mercaptopropyl)-L-valyl]-1,2,3,4-tetrahydro-N-[3-(methylthio)propyl]-3-isoquinolinecarboxamide.**

Compound A was stirred at 0°C under nitrogen in a solution of 95:3:1:1 trifluoroacetic acid-thioanisole-methyl sulfide-ethanedithiol (50 mL) for three hours. The volatiles were removed *in vacuo,* and the residue was analyzed by HPLC; significant amounts of starting material remained. The treatment with 95:3:1:1 trifluoroacetic acid-thioanisole-methyl sulfide-ethanedithiol (50 mL) was repeated at room temperature for two hours. The mixture was concentrated *in vacuo*, and the residue was purified by preparative HPLC(C-18). Elution with a gradient of 20 to 50% solvent B in solvent A, and lyophilization of the product-containing fractions (solvent A is 0.05% trifluoroacetic acid in $H_2O$; solvent B is 0.05% trifluoroacetic acid in $CH_3CN$) gave title Compound (63.6mg, 0.15 mmol, 27%) as a fluffy white amorphous powder.

FAB MS: $MH^+$ 438.

$^1H$ NMR ($CD_3OD$): δ 7.0-7.3 (m), 4.5-4.9 (m), 4.15 (d), 2.8-3.4 (m), 2.1-2.5 (m), 1.7-2.1 (m), 1.601.7 (m) 1.4-1.5 (m), 0.9-1.1 (dd).

$[a]^{20}_D$ (methanol) -27.8°.

| Calculated for $C_{22}H_{34}N_3O_2S_2$·trifluoroacetic acid ·$H_2O$: | | | | | |
|---|---|---|---|---|---|
| Theory: | C, 50.69; | H, 6.56; | N, 7.39; | S, 11.28; | F, 10.02; |
| Found: | C, 50.67; | H, 6.60; | N, 6.96; | S, 11.61; | F, 10.03. |

## Example 15

**(R*)-N-[[2-[N-(L-Cysteinyl)-L-valyl]-1,2,3,4-tetrahydro-3-isoquinolinyl]carbonyl]-L-alanine.**

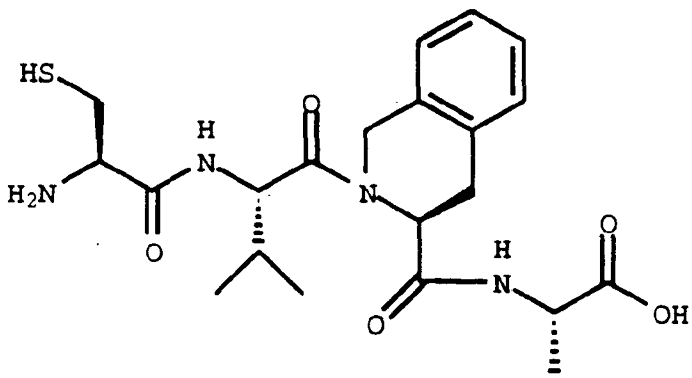

The protected peptide intermediate was assembled stepwise by the solid phase method in the Applied Biosystems Model 430A Peptide Synthesizer using the N-methylpyrrolidone(NMP)HOBt *t*-Boc chemistry program supplied with the instrument. Butyloxycarbonyl-L-Ala-PAM resin (0.5 g, 0.23 mmol) was obtained commercially. Amino acids were coupled as their N-t-butyloxycarbonyl derivatives, and the reactive side chain of cysteine was protected with *p*-methoxybenzyl. N-t-Butyloxycarbonyl groups were removed at each cycle with successive 10 minute treatments of 33% and 50% trifluoroacetic acid in $CH_2Cl_2$. Neutralization of the $\alpha$-amino groups was accomplished with 10% N,N-diisopropylethylamine in $CH_2Cl_2$. Each amino acid was coupled with DDC/HOBT in NMP/$CH_2Cl_2$ mixtures at room temperature. Any remaining free amino groups were acetylated with 10% acetic anhydride/5% N,N-diisopropylethylamine in $CH_2Cl_2$ for 10 minutes. After the final coupling, the N-t-butyloxycarbonyl group was removed as described above. The dried, protected peptidyl-resin weighed 584 mg (93%).

The entire product from above was simultaneously deprotected and cleaved from the resin by treatment with anhydrous liquid HF as follows: the peptidyl-resin (584mg) was placed into a Kel-F HF reaction vessel with a stir bar, and treated with 0.75 mL thioanisole, 0.25 mL ethanedithiol, and 0.25 mL methyl sulfide. The vessel was evacuated in the HF apparatus and cooled in a dry ice/ethanol bath. Approximately 10 mL HF was distilled from a cylinder into the reaction vessel, and the dry ice bath was replaced with an ice water bath. The reaction was stirred at 4°C for 1.5 hours, and then HF was removed under vacuum for 30 minutes. The product was precipitated and washed with ether, and then dissolved in 15% aqueous acetic acid and separated from the resin by filtration. The filtrate was frozen on dry ice and lyophilized to yield 103 mg crude peptide product. Sample aliquots (10 mg) were applied to a YMC ODS-AQ $C_{18}$ column (10 x 250 mm, 5 m), and reverse phase HPLC was performed under the following conditions: Solvent A, 0.05% trifluoroacetic acid in water; Solvent B, 0.05% trifluoroacetic acid in $CH_3CN$: linear gradient from 10 to 50% solvent B in solvent A over 40 min; flow rate: 8 mL/min. Fractions containing the major peak (220 nm) eluting at 30.2 min were combined and lyophilized to yield 7 mg of title Compound as a white, fluffy powder.

Analytical HPLC: YMC ODS-AQ $C_{18}$ (4.6 x 250 mm); 220 nm, 1.0 mL/min: linear gradient from 5% to 60% solvent B in solvent A over 40min (solvent A, 0.05% trifluoroacetic acid in water; solvent B, 0.05% trifluoroacetic acid in $CH_3CN$). RT, 21.7 min.

H.I.; >98%.

MS: $451^+$ $(M+H)^+$.

$^1H$ NMR($CD_3OD$, 270 MHz) $\delta$ (ppm) 7.12 (m, Ar-H), 5.60-5.90 (m), 4.22 (m), 3.95 (m), 3.08 (J= 5.9 Hz), 2.07 (J= 11.1 Hz), 1.86 (J= 16.4 Hz), 1.27 (J= 7.0 Hz), 1.17 ( J= 7.0 Hz), 1.06 (J= 7.6 Hz), 1.00 (J= 6.0 Hz), 0.94 (J= 6.5 Hz).

**Example 16**

**(R*,R*)-N-[[2-[N-(2-Amino-4-hydroxy-1-oxobutyl)-L-valyl]-1,2,3,4-tetrahydro-3-isoquinolinyl]carbonyl]-L-methionine.**

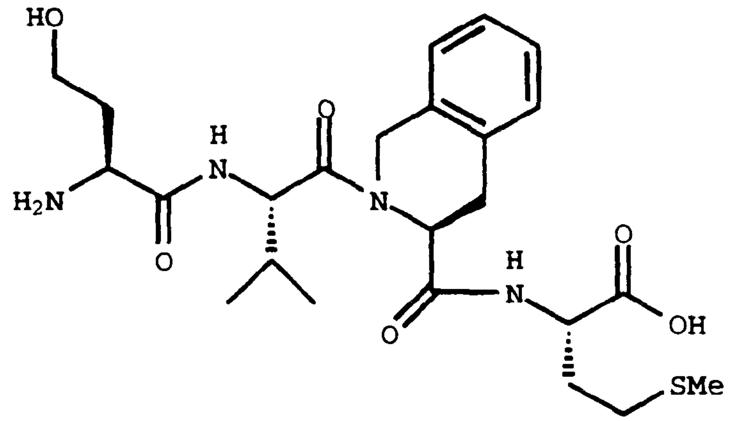

The title Compound (6 mg; white, fluffy powder) was prepared using the solid phase method described in Example 15, with the following modifications:

N-t-Butyloxycarbonyl-L-Met-PAM resin (0.59 g, 0.25 mmol) was obtained commercially. The reactive side chain of homoserine was protected as a benzyl ether.

MS: $(M+H)^+$ $509^{+\cdot}$

$^1$H NMR ($CD_3OD$, 270 MHz): δ (ppm) 7.52 (m), 7.24 (m), 5.14 (m), 5.02 (m), 5.06 (m), 5.09 (m), 4.7 (m), 4.5 (m), 3.78 (m), 3.64 (m), 3.19 (m), 2.72 (m), 2.39 (m), 1.92 (m), 1.12 (m), 1.03 (m).

Rotamers observed.

**Example 17**

**(R*,R*)-2-[N-(L-Cysteinyl)-L-valyl]-1,2,3,4-tetrahydro-N-[1-(hydroxymethyl)-3-(methylthio)propyl]-3-isoquinolinecarboxamide.**

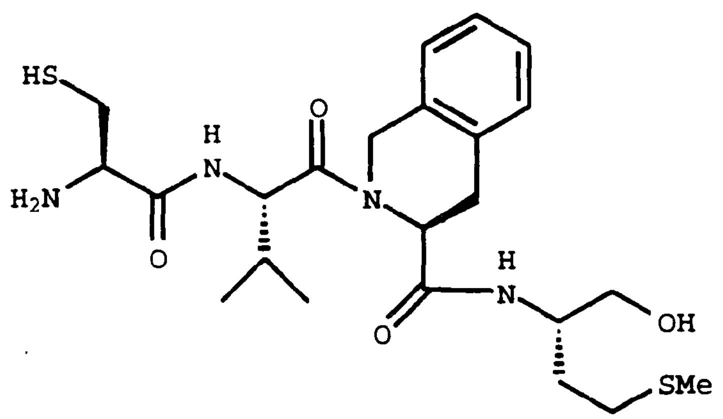

The title Compound (5.5 mg; glassy solid) was prepared using the solid phase method described in Example 15, with the following modifications:

N-t-Butyloxycarbonyl-L-Met-PAM resin (1.0 g, 0.42 mmol) was obtained commercially.

The tetrapeptide product was cleaved from the resin by treatment with $LiBH_4$. A solution of $LiBH_4$ (28 mg, 1.30 mmol) in 5 mL of anhydrous tetrahydrofuran was added dropwise in 4 portions over 1 hour to 0.788 g (0.26 mmol) of tetrapeptide-resin suspended in 30 mL anhydrous tetrahydrofuran at 0°C. The mixture was warmed to room temperature and stirred for 1 hour. The reaction was quenched by the addition of glacial acetic acid (4 x 1 mL). Additional glacial acetic acid (5 mL) was added, followed by 15% aqueous acetic acid (10 mL). The product was separated from the resin by filtration and successively washed with glacial acetic acid, 15%

acetic acid, and $H_2O$. The filtrate was lyophilized to yield the crude protected peptide alcohol as a glassy solid.

The protected peptide alcohol was suspended in 15 mL of $CH_2Cl_2$ and treated with 1.5 mL thioanisole, 0.5 mL ethanedithiol, 0.5 mL methyl sulfide and 19 mL trifluoroacetic acid at room temperature. The mixture was stirred at room temperature for 2 hours, and then concentrated in *vacuo.* The residue was washed with diethyl ether and decanted (4x15 mL). This residue was dissolved in glacial acetic acid (2 mL) and 15% acetic acid (45 mL), and then lyophilized. The lyophilate (20 mg) was purified by reverse phase HPLC as described in Example 15 to yield 5.5 mg of the title compound.

MS: $(M+H)^+$ $497^+$.

$^1H$ NMR (400MHz, d6 DMSO, 30°C): δ (ppm) 8.70 (m, exchangeable), 8.13 ( br s, exch), 7.50 (m, exch.), 7.17 ( m, 5H, Ar-H), 6.49 (br s, exch, CONH), 4.50-5.15 (m, a-H's), 4.15 (br, exch), 3.69 (br, exch), 3.01 (d, J=6.4 Hz), 2.75-2.95 (m), 2.32 (m), 2.16 (m), 1.99 (s), 1.77 (m), 1.50 (m), 1.24 (s), 1.02 d, J=6.84 Hz), 0.96 ( d, J=6.4 Hz).

## Example 18

**(R*)-N-[[2-[N-(L-Cysteinyl)-L-valyl]-1,2,3,4-tetrahydro-3-isoquinolinyl]carbonyl]-L-phenylalanine.**

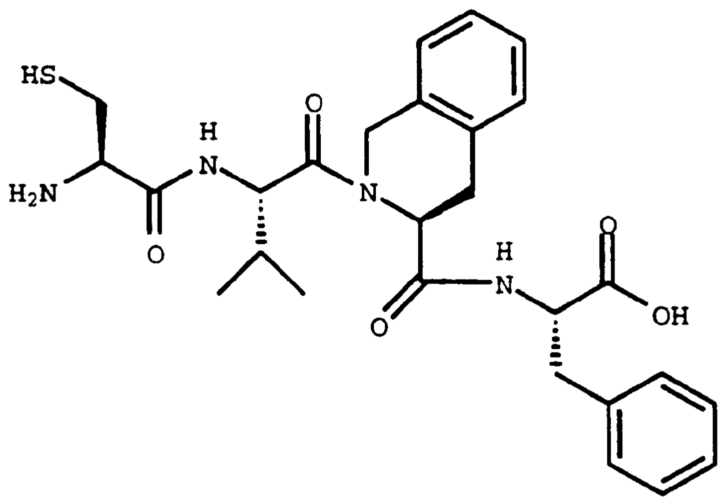

**A. (R*)-2-[N-[(1,1-Dimethylethoxy)carbonyl]-L-valyl]-1,2,3,4-tetrahydro-3-isoquinolinecarboxylic acid, phenylmethyl ester.**

Benzyl (S)-1,2,3,4-tetrahydroisoquinoline-3-carboxylate (S)-mandelic acid salt [as prepared in B.O.T. Kammermeier, *et al.,* Synthesis, 1157 (1992)] (80g, 0.19 mole) was partitioned between 2L of ethyl acetate and 1 L of 0.9 M sodium bicarbonate. The organic layer was dried (magnesium sulfate) and evaporated to an oil. The oil was dissolved in 600 mL of dichloromethane, and the solution was chilled to 5°C under nitrogen. The cold solution was treated sequentially with N-t-butyloxycarbonyl-L-valine (46 g, 0.21 mole), N,N-diisopropylethylamine (60 mL, 44.5 g, 0.35 mole), and bis(2-oxo-3-oxazolidinyl)phosphinic chloride (58.3 g, 0.23 mole), while chilling back to 5°C after each addition. The mixture was stirred overnight at 5°C to give a thin slurry. The mixture was poured into 2 L of ethyl acetate and washed sequentially with 1.5 L of hydrochloric acid, 1 L of water, 1.5 L of 1N sodium bicarbonate and 1 L of brine. A second 1 L ethyl acetate extraction of the aqueous solutions was combined with the first. A third extract contained predominantly a more polar material and was not combined. The product-containing extracts were combined and evaporated to an oil. Pumping *in vacuo* to constant weight gave 82.3 g (93% yield) of the product as a thick colorless oil which partially solidified on standing.

**B. (R*)-2-L-Valyl-1,2,3,4-tetrahydro-3-isoquinolinecarboxylic acid, phenylmethyl ester.**

A solution of Compound A (7.94 g, 0.017 mol) in 1M HCl in acetic acid (34 mL) was stirred for 2 hours at room temperature. Additional 1M HCl in acetic acid (23.5 mL) was added. The reaction was stirred for another 1.5 hours and the solvents were removed in *vacuo.* Ethyl acetate was added and the reaction was concentrated *in vacuo.* The residue was triturated with hexane and dried *in vacuo* to give Compound B (6.26 g, 99%).

mp: 80-83°C
TLC: $R_f$ = 0.65 (9:1:0.05 chloroform/methanol/acetic acid, visualized by ceric ammonium sulfate)
MS: $(M+H)^+$ 367.

**C. (R*)-2-[N-[N-[(1,1-Dimethylethoxy)carbonyl]-S-(triphenylmethyl)-L-cysteinyl]-L-valyl]-1,2,3,4-tetrahydro-3-isoquinolinecarboxylic acid, phenylmethyl ester.**

To a solution of N-t-butyloxycarbonyl-S-trityl-L-cysteine (7.78 g, 16.8 mmol) in dimethylformamide (80 mL) at 0°C under $N_2$ was added benzotriazol-1-yloxy-tris(dimethylamino)phosphonium hexafluorophosphate (7.42 g, 16.8 mmol) and N,N-diisopropylethylamine (2.92 mL, 16.8 mmol). After stirring for 30 minutes, Compound B (6.96 g, 16.8 mmol, 1 eq) and N,N-diisopropylethylamine (2.92 mL, 16.9 mmol) were added. After stirring for 16 hours at 0°, the reaction was diluted with ethyl acetate (500 mL) and washed sequentially with saturated sodium bicarbonate (100 mL) and 10% lithium chloride (4 x 100 mL). The organic layer was dried over sodium sulfate, filtered and concentrated *in vacuo.* The residue was purified on silica gel (400 mL, eluting with 1:4 to 3:7 ethyl actetate/hexane) to give Compound C (10.46 g, 77%). mp: 85-87°C TLC: $R_f$ = 0.40 (2:1 hexane/ethyl acetate, visualized by ceric ammonium sulfate).

**D. (R*)-2-[N-[N-[(1,1-Dimethylethoxy)carbonyl]-S-(triphenylmethyl)-L-cysteinyl]-L-valyl]-1,2,3,4-tetrahydro-3-isoquinolinecarboxylic acid.**

To a solution of Compound C (8.61 g, 10.6 mmol) in tetrahydrofuran/methanol (40 mL 3:1) at 0°C was added 1N lithium hydroxide (15.9 mL, 15.9 mmol). After stirring 1 hour at 0°C and 1 hour at room temperature, the reaction was diluted with water (20 mL) and 1N hydrochloric acid was added (pH=2). The reaction was extracted with ethyl acetate (2 x 100 mL). The organic layers were combined, dried over sodium sulfate, filtered and concentrated *in vacuo* to give Compound D (8.54 g, 73% by HPLC).
MS: $(M-H)^-$ 720.
$^1H$ NMR 400 MHz ($CD_3OD$) : 0.89 (d, 3H, J=5.86), 1.00 (d, 3H, J=5.86), 1.45 (s, 9H), 2.03 (m, 1H), 2.22 (m, 1H), 2.61 (m, 1H), 2.92 (m 1H), 3.14 (m, 1H), 3.93 (m, 1H), 4.62-5.41 (m, 4H), 7.27 (m, 19H). $^{13}C$ NMR 100 MHz ($CD_3OD$): 17.4, 19.0, 28.0, 30.2, 31.0, 33.6, 45.5, 51.5, 54.1, 64.6, 66.5, 79.8, 126.0, 126.6, 126.8, 126.9, 127.2, 127.8, 128.2, 129.3, 131.4, 131.8, 140.6, 144.1, 154.9, 170.5, 171.7, 173.6.

**E. (R*)-N-[[2-[N-[N-[(1,1-Dimethylethoxy)carbonyl]-S-(triphenylmethyl)-L-cysteinyl]-L-valyl]-1,2,3,4-tetrahydro-3-isoquinolinyl]carbonyl]-L-phenylalanine, methyl ester.**

To a solution of Compound D (573 mg, 0.70 mmol, 88% by weight) in dimethylformamide (3 mL) at 0°C under $N_2$ was added L-phenylalanine methyl ester (181 mg, 0.84 mmol), N-methylmorpholine (184 mL, 1.68 mmol) and benzotriazol-1-yloxy-tris(dimethylamino)phosphonium hexafluorophosphate (371 mg, 0.84 mmol). After stirring at 0°C for 16 hours, the reaction was diluted with ethyl acetate (100 mL) and washed sequentially with saturated sodium bicarbonate (30 mL), 1% potassium hydrogen sulfate (30 mL) and 10% lithium chloride (3 x 30 mL). The organic layer was dried over sodium sulfate, filtered and concentrated *in vacuo*. The residue was purified on Merck silica gel (100 mL, eluting with 3:1 to 2:1 hexane/ethyl acetate) to give Compound E (458 mg, 76%). mp: 88-92°C TLC: $R_f$ = 0.15 (2:1 hexane/ethyl acetate, visualized by ceric ammonium sulfate).

**F. (R*)-N-[[2-[N-[N-[(1,1-Dimethylethoxy)carbonyl]-S-(triphenylmethyl)-L-cysteinyl]-L-valyl]-1,2,3,4-tetrahydro-3-isoquinolinyl]carbonyl]-L-phenylalanine.**

To a solution of Compound E (376 mg, 0.426 mmol) in tetrahydrofuran (5 mL) at 0°C was added 1N sodium hydroxide (0.853 mL, 0.853 mmol). After stirring 3 hours, the reaction was diluted with ethyl acetate (100 mL) and water (50 mL) and the pH was adjusted with 1N hydrochloric acid (pH is 2). The layers were separated and the aqueous layer was extracted with ethyl acetate (2 x 100 mL). The organic layers were combined, dried over sodium sulfate, filtered and concentrated *in vacuo* to give Compound F (0.354 g, 96%).
mp: 125-129°C
TLC: $R_f$ = 0.65 (9:1:0.05 chloroform/methanol/acetic acid, visualized by ceric ammonium sulfate).

**G. (R*)-N-[[2-[N-(L-Cysteinyl)-L-valyl]-1,2,3,4-tetrahydro-3-isoquinolinyl]carbonyl]-L-phenylalanine.**

(All solvents used in Example 18G are degassed). To a solution of Compound F (377 mg, 0.434 mmol) in dichloromethane (2 mL) at 0°C under $N_2$ was added triethylsilane (0.69 mL, 4.34 mmol) and trifluoroacetic

acid (2 mL). After stirring for 2 hours at 0° and 1 hour at room temperature, the solvents were removed *in vacuo*. The residue was dissolved in methanol (5 mL), and water (3 mL) was added. The precipitate was filtered and half of the filtrate was purified by preparative HPLC (YMC ODS S-10, 30 x 500 mm, 21 mL/min, gradient of 36-90% aqueous $CH_3OH$, 0.1% trifluoroacetic acid) to give the title Compound (54 mg, 40%).
mp: 137-142°
TLC: $R_f$ = 0.32 (4:1:1 butanol/acetic acid/water, visualized by ceric ammonium sulfate)
MS: $(M+H)^+$ 527.
$[a]_D$ = -9.9° [c=0.102, $CH_3OH$]
$^1H$ NMR 400 MHz ($CD_3OD$): 0.096-1.74 (m, 6H), 2.06-2.20 (m, 2H), 2.88-3.42 (m, 6H), 4.07, 4.23 (dd, 1H, J=5.13, 5.55), 4.44-5.00 (m, 5H, also water peak), 7.06-7.26 (m, 9H).
$^{13}C$ NMR 100 MHz ($CD_3OD$): 18.3, 18.6, 19.7, 19.9, 26.5, 26.9, 31.4, 31.6, 31.7, 32.3, 37.6, 38.4, 44.0, 47.3, 47.5, 54.9, 55.3, 55.5, 55.8, 56.0, 56.9, 57.0, 57.6, 127.1, 127.2, 127.6, 127.8, 127.9, 128.0, 128.8, 129.4, 130.4, 130.5, 131.0, 132.9, 133.3, 134.8, 134.9, 138.0, 138.6, 168.4, 169.2, 171.5, 172.8, 173.1, 173.3, 174.2, 175.1.

## Example 19

**(R*)-N-[[2-(N-(L-Cysteinyl)-L-valyl]-1,2,3,4-tetrahydro-3-isoquinolinyl]carbonyl]-L-serine.**

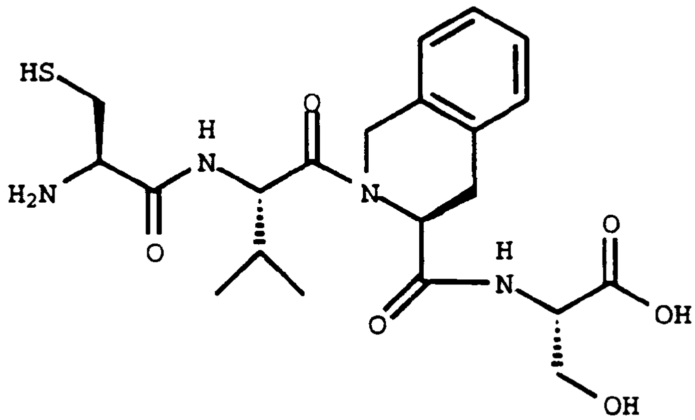

The title compound (137 mg, 37%; colorless solid) was prepared from L-serine methyl ester and Compound 18D (574 mg) by a three step procedure analogous to that described for the preparation of Compound 18G from Compound 18D.
MS: 467 $(M+H)^+$.

## Example 20

**(R*)-N-[[2-[N-(L-Cysteinyl)-L-valyl]-1,2,3,4-tetrahydro-3-isoquinolinyl]carbonyl]-N-hydroxy-L-methioninamide.**

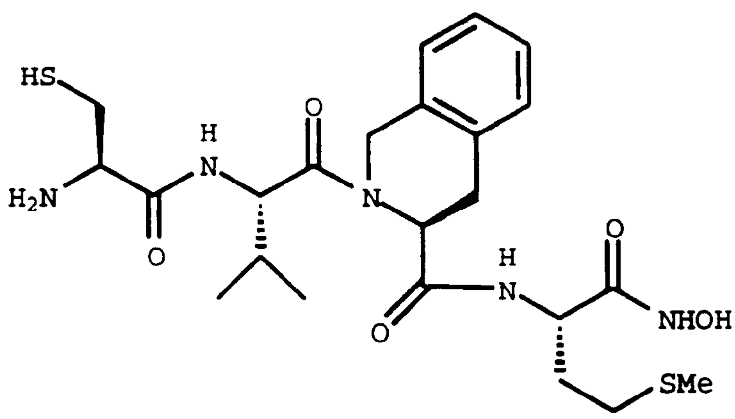

**A. O-Tetrahydropyran-2-yl-hydroxylamine**

To a solution of N-hydroxyphthalimide (20 g, 0.2123 mol) in tetrahydrofuran (350 mL) was added dihyropyran (17.0 mL, 0.187 mol) and p-toluene sulfonic acid monohydrate (400 mg) under $N_2$ at room temperature. Additional dihydropyran (5.6 mL, 0.062 mol) was added after 8 hours and 24 hours. The reaction mixture was filtered and concentrated to 150 mL. Then dihydropyran (11.2 mL, 0.125 mol) and p-toluene sulfonic acid monohydrate (800 mg) was added. After stirring for 16 hours, the reaction was concentrated *in vacuo.* The residue was dissolved in ethyl acetate (400 mL) and washed with saturated sodium bicarbonate (3 x 150 mL). The organic layer was dried over sodium sulfate, filtered and concentrated *in vacuo*. The residue was triturated with hexane (3 x 200 mL) to give the tetrahydropyranyl ether of N-hydroxyphthalimide (23.9 g, 78%).
mp: >210°
TLC: $R_f$ = 0.81 (3:7 hexane/ethyl acetate, visualized by UV 254 mm)
MS: $(M+H)^+$ 248.

To a solution of the intermediate prepared above (23.64 g, 0.095 mol) in toluene (60 mL) was added methyl hydrazine (5.59 mL, 0.105 mol). After heating at 80° for 1 hour, the solvent was removed *in vacuo.* The residue was triturated with ether (20 mL) and filtered. The solid was dissolved in hexane, treated with charcoal, filtered through celite and concentrated to 50 mL. The cyrstals were filtered and dried.to give Compound A (5.542 g, 50%).
mp: 33-38°
TLC: $R_f$ = 0.67 (1:4 hexane/ethyl acetate, visualized by ceric ammonium sulfate).
MS: $(M+H)^+$ 118.
$^1H$ 400 MHz ($CDCl_3$) : 1.53-1.78 (m, 6H), 3.57 (m, 1H), 3.91 (m, 1H), 4.71 (m, 1H), 5.49 (br s, 2H). $^{13}C$ 100 MHz ($CDCl_3$) : 19.6, 25.3, 28.8, 62.5, 102.5

**B. (R*)-N-[[2-[N-[N-[(1,1-Dimethylethoxy)carbonyl]-S-(triphenylmethyl)-L-cysteinyl]-L-valyl]-1,2,3,4-tetrahydro-3-isoquinolinyl]carbonyl]-N-tetrahydropyran-2-yloxy-L-methioninamide.**

To a solution of Compound 2B (400 mg, 0.469 mmol) in dimethylformamide (3 mL) at 0°C under $N_2$, was added sequentially Compound A (55 mg, 0.469 mmol), N,N-diisopropylethylamine (81.5 mL, 0.469 mmol) and benzotriazol-1-yloxy-tris(dimethylamino)phosphonium hexafluorophosphate (207 mg, 0.469 mmol). After stirring at 0°C for 18 hours, the reaction was diluted with ethyl acetate (70 mL) and washed sequentially with saturated sodium bicarbonate (30 mL), 1% $KHSO_4$ (30 mL) and 10% lithium chloride (3 x 30 mL). The organic layer was dried over sodium sulfate, filtered and concentrated *in vacuo* to give Compound B (427 mg, 96%).
mp: 113-115°C
TLC: $R_f$ = 0.85 (9:1:0.05 chloroform/methanol/acetic acid, visualized by ceric ammonium sulfate).
MS: $(M+H)^+$ 952.

**C. (R*)-N-[[2-[N-(L-Cysteinyl)-L-valyl]-1,2,3,4-tetrahydro-3-isoquinolinyl]carbonyl]-N-hydroxy-L-methioninamide.**

Compound C (72 mg, 33%) was prepared from Compound B (323 mg, 0.340 mmol) using a procedure analogous to that described for the preparation of Compound 18G from Compound 18F.
mp: 135-139°C
TLC: $R_f$ = 0.17 (9:1:0.05 chloroform/methanol/acetic acid, visualized by ceric ammonium sulfate)
MS: $(M+H)^+$ 526.
$^1H$ NMR 400 MHz ($CD_3OD$): 1.06 (m, 6H), 1.99, 2.05 (s, 3H), 1.89-2.45 (m 5H), 2.90-3.18 (m, 4H), 4.00-5.20 (m, 6H), 7.22 (b m, 4H).

**Example 21**

**(R*)-N-[[2-[N-(L-Cysteinyl)-L-valyl]-1,2,3,4-tetrahydro-3-isoquinolinyl]carbonyl]-3-(2-thienyl)-DL-alanine.**

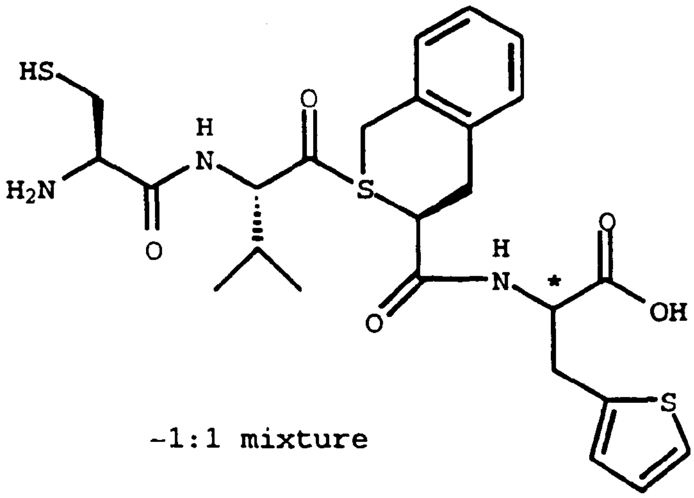

~1:1 mixture

**A. 3-(2-Thienyl)-DL-alanine, methyl ester.**

Anhydrous HCl was bubbled in a slurry of R,S-α-amino-2-thiophenepropionic acid (2 g, 11.68 mmol) and methanol (75 mL) at 0°C until the solution became homogeneous. The mixture was stirred for 16 hours at room temperature, 3 hours at reflux and then concentrated under vacuum. The solid was dissolved in water (50 mL), extracted with $CH_2Cl_2$ (3x50 mL). The aqueous layer was basified with 10% $NaHCO_3$ and extracted with $CH_2Cl_2$ (4x50 mL). The combined organic extracts from the basic portion were dried ($MgSO_4$), filtered and concentrated under vacuum to afford Compound A (2.0 g, 93%).
TLC: $R_f$ = 0.51 (9/1/0.05 $CHCl_3$/methanol/acetic acid, visualization by ceric ammonium sulfate/ammonium molybdate/sulfuric acid/water).
MS: $(M+H)^+$ 186.

**B. (R*)-N-[[2-[N-[N-[(1,1-Dimethylethoxy)carbonyl]-S-(triphenylmethyl)-L-cysteinyl]-L-valyl]-1,2,3,4-tetrahydro-3-isoquinolinyl]carbonyl]-3-(2-thienyl)-DL-alanine, methyl ester.**

N,N-Diisopropylethylamine (0.130 mL, 0.748 mmol) was added to a solution Compound 18D (0.539 g, 0.748 mmol), Compound A (0.138 g, 0.748 mmol), benzotriazol-1-yloxy-tris(dimethylamino)phosphonium hexafluorophosphate (0.331 g, 0.748 mmol) in $CH_3CN$ (15 mL) and dimethylformamide (3 mL), and was stirred at room temperature for 16 hours. The reaction was quenched with 1N HCl (100 mL) and extracted with ethyl acetate (3x100 mL). The combined organic layers were sequentially washed with 10% $NaHCO_3$ (2x100 mL), and 10% LiCl (2x100 mL), dried ($MgSO_4$), filtered and concentrated under vacuum. The residue was purified by flash chromatography (eluting with 4/1 hexane/acetone) to afford Compound B (0.616 g, 93%).
mp: 51-56°C
TLC: $R_f$ = 0.56 (2/1 hexane/acetone, visualization by ceric ammonium sulfate/ammonium molybdate/$H_2SO_4$/water).
MS: $(M+H)^+$ 889.

**C. (R*)-N-[[2-[N-(L-Cysteinyl)-L-valyl]-1,2,3,4-tetrahydro-3-isoquinolinyl]carbonyl]-3-(2-thienyl)-DL-alanine.**

Compound C (57%) was prepared from Compound B (0.61 g, 0.687 mmol) using a two step procedure analogous to that described for the preparation of Compound 18G from Compound 18E.
mp: 140-147°C
TLC: $R_f$ = 0.28 (9/1/0.05 $CHCl_3$/methanol/acetic acid, visualization by ceric ammonium sulfate/ammonium molybdate/ sulfuric acid/ water).

MS: $(M+H)^+$ 533.
$[a]_D^{22}$ = - 20.0° (c 0.05, $CH_3OH$)
$^1H$ ($CD_3OD$): 0.98 (d, 1.5 H, J = 5.3), 1.00 (d, 1.5 H, J = 6.4), 1.03 (d, 1.5 H, J = 7.1), 1.05 (d, 1.5 H, J = 6.4), 2.20-2.40 (m, 1 H), 2.91-3.35 (m, 6 H), 4.05-4.13 (m, 1 H), 4.58-5.10 (m, 5 H), 6.72-6.92 (m, 2 H), 7.14-7.50 (m, 5 H)
Anal. Calc'd. for $C_{25}H_{32}N_4O_5S_2$ . 0.55 $H_2O$. $C_2HF_3O_2$ : C, 49.39; H, 5.23; N, 8.53; S, 9.77; F, 8.68. Found: C, 49.64; H, 5.32; N, 8.60; S, 9.57; F, 8.25.

**Example 22**

**(R*)-N-[[2-[N-(L-Cysteinyl)glycyl]-1,2,3,4-tetrahydro-3-isoquinolinyl]carbonyl]-L-methionine.**

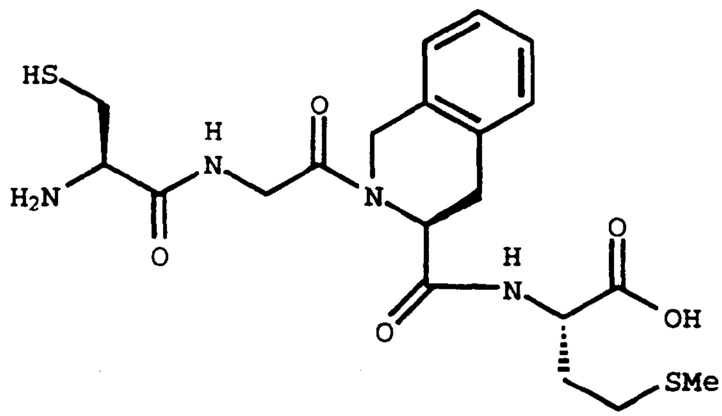

**A. (R*)-N-[(1,2,3,4-Tetrahydro-3-isoquinolinyl)carbonyl]-L-methionine, methyl ester.**

HCl/dioxane (anhydrous, 4M, 0.18 mL, 0.007 mmol) was added to a suspension of Compound 1A (1.5 g, 3.55 mmol), in $CH_3OH$/tetrahydrofuran (20/3 mL), at room temperature under argon. After 15 hours, the reaction mixture was concentrated *in vacuo.* The residue was triturated a few times with ether, and the solid was dried to afford Compound A (1.14 g, 100%).

**B. (R*)-N-[[2-[N-[(1,1-Dimethylethoxy)carbonyl]glycyl]-1,2,3,4-tetrahydro-3-isoquinolinyl]carbonyl]-L-methionine, methyl ester.**

Bis(2-oxo-3-oxazolidinyl)phosphinic chloride (1.08 g, 4.24 mmol) was added to a stirred solution of Compound A (1.14 g, 3.53 mmol), N-t-butyloxycarbonylglycine (0.68 g, 3.88 mmol) and N,N-diisopropylethylamine (2.02 mL, 1.50 g, 11.6 mmol) in $CH_2Cl_2$ (14 mL) at 0°C under argon. After stirring for 2.5 days, the reaction mixture was partitioned between ethyl acetate (35 mL) and 10% HCl (10 mL). The aqueous layer was extracted (2 x 30 mL) with ethyl acetate. The combined organic extracts were washed sequentially with 10% HCl (2 x 10 mL), water (2 x 20 mL), saturated $NaHCO_3$ (2 x 10 mL) and saturated NaCl (2 x 20 mL). The organic layer was dried over $MgSO_4$, filtered and concentrated to afford Compound B (1.7 g, 100%), which was used without further purification.
MS : $[M+H]^+$ $480^+$.
TLC: $R_f$ 0.75 (ethyl acetate), visualized by UV, Ceric ammonium molybdate.

**C. (R*)-N-[[2-[N-[N-[(1,1-Dimethylethoxy)carbonyl]-S-(triphenylmethyl)-L-cysteinyl]glycyl]-1,1,3,4-tetrahydro-3-isoquinolinyl]carbonyl]-L-methionine, methyl ester.**

Compound C (82%) was prepared from Compound B (1.64 g, 3.41 mmol) using the two step procedure described for the preparation of Compound B from Compound 1A, except that in the second step N-t-butyloxycarbonyl-S-trityl-L-cysteine (0.78 g, 1.67 mmol) was used instead of N-t-butyloxycarbonylglycine.
TLC: $R_f$ 0.69 (ethyl acetate), visualized by UV, Ceric ammonium molybdate.

**D. (R*)-N-[[2-[N-(L-Cysteinyl)glycyl]-1,2,3,4-tetrahydro-3-isoquinolinyl]carbonyl]-L-methionine.**

Compound D (39%) was prepared from Compound C (1.09 g, 1.58 mmol) using a procedure analogous to that described for the preparation of Compound 1H from Compound 1G, except that the title compound was purified by silica gel chromatography eluting with $CHCl_3$ and $CHCl_3/CH_3OH$ (9/1) and preparative HPLC.
m.p. 95-99°C.
TLC: $R_f$ 0.3 (9/1/.1,$CHCl_3/CH_3OH/CH_3COOH$),
Visualized by UV, Ceric ammonium molybdate.
MS: $[M+H]^+$ 469 $^+$.

**<u>Example 23</u>**

**<u>(R*)-$N^2$-[[2-[N-(L-Cysteinyl)-L-valyl]-1,2,3,4 -tetrahydro-3-isoquinolinyl]carbonyl-L-glutamine.</u>**

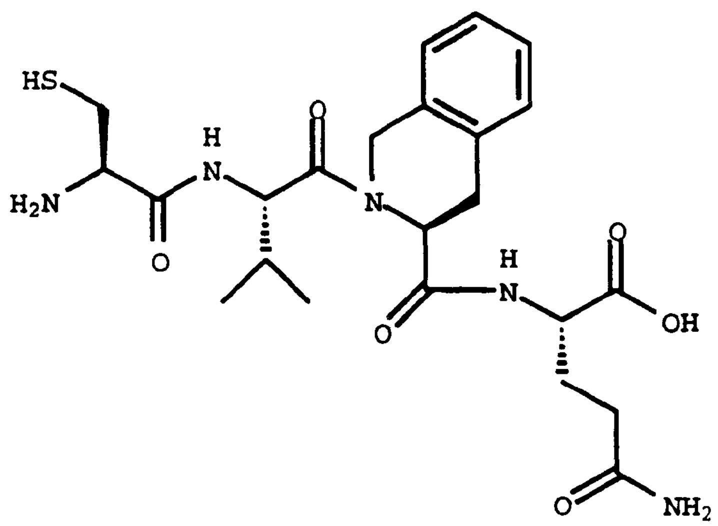

**A. (R*)-$N^2$-[[2-[N-[N-[(1,1-Dimethylethoxy)carbonyl]-S-(triphenylmethyl)-L-cysteinyl]glycyl]-1,2,3,4-tetrahydro-3-isoquinolinyl]carbonyl]-L-glutamine, 1,1-dimethylethyl ester.**

To a cooled solution (4°C) of Compound 18D (722 mg, 0.5 mmol) were added L-glutamine t-butylester (120 mg, 0.5 mmol), 4-methylmorpholine (137 mL, 1.25 mmol) in dimethylformamide (2.5 mL) and benzotriazol-1-yloxy-tris(dimethylamino)phosphonium hexafluorophosphate (332 mg, 0.75 mmol). The mixture was stirred at 4°C for 18 hours, allowed to warm to room temperature and stirred for an additional 24 hours. The mixture was poured into hydrochloric acid (50 mL, 0.5 N) and extracted with ethyl acetate (3 x 100 mL). The organic extracts were combined, washed with sodium bicarbonate (50 mL, saturated aqueous solution), dried (MgSO4) and concentrated *in vacuo* to give Compound A (510 mg, 68% yield, 60% pure) which was used without further purification.

**B. (R*)-$N^2$-[[2-[N-(L-Cysteinyl)-L-valyl]-1,2,3,4-tetrahydro-3-isoquinolinyl]carbonyl]-L-glutamine.**

A solution of Compound A (470 mg, 0.52 mmol), triethylsilane (832 mL, 5.2 mmol), trifluoroacetic acid (5 mL) and dichloromethane (5 mL) was stirred for two hours, concentrated *in vacuo* to an amber oil and purified by preparative HPLC (YMC S-15 ODS 50 x 500 mm column eluted at 35 mL/min with a 10-90% aqueous methanol gradient containing 0.1% trifluoroacetic acid over 1 hour, UV detection at 215 nm). Appropriate fractions were combined (as determined by analytical HPLC), concentrated *in vacuo*, and lyophilized from water to provide the title compound as a white solid, (80 mg, 0.11 mmol, 22 %).
mp 145-150 °C
$[\alpha]_D^{22}$ = - 27.5° (c 0.10, $CH_3OH$).

**Example 24**

**(R*)-N-[[2-[N-(L-Cysteinyl)-L-valyl]-1,2,3,4-tetrahydro-3-isoquinolinyl]carbonyl]-L-histidine.**

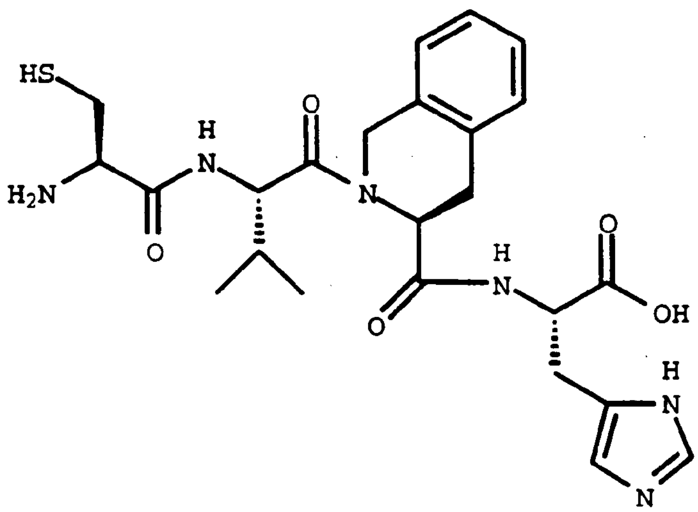

The title compound (0.196 g, 30%) was prepared from Compound 18D (0.60 g) and L-histidine methyl ester hydrochloride using the three step procedure described for the preparation of Compound 21C from Compound 18D and Compound 21A, with the exception that in the first step the 1N HCl quench was eliminated.
mp: 130-137°C
TLC: $R_f$ = 0.16 (6/3/1 n-propanol/ammonium hydroxide/ water, visualization by ceric ammonium sulfate/ ammonium molybdate/ sulfuric acid/ water).
MS: $(M+H)^+$ 516.
$^1H$ ($CD_3OD$): 1.03 (d, 3 H, J = 6.5), 1.08 (d, 3 H, J = 6.5), 2.20-2.28 (m, 1 H), 2.93 (d, 2 H, J = 5.3), 3.00-3.42 (m, 4 H), 4.15 (t, 1 H, J = 5.9), 4.59-5.13 (m, 5 H), 7.17-7.40 (m, 6 H).

**Example 25**

**(R*)-N-[[2-[N-(L-Cysteinyl)-L-valyl]-1,2,3,4-tetrahydro-3-isoquinolinyl]carbonyl]-3-(phenylmethyl)-L-alanine**

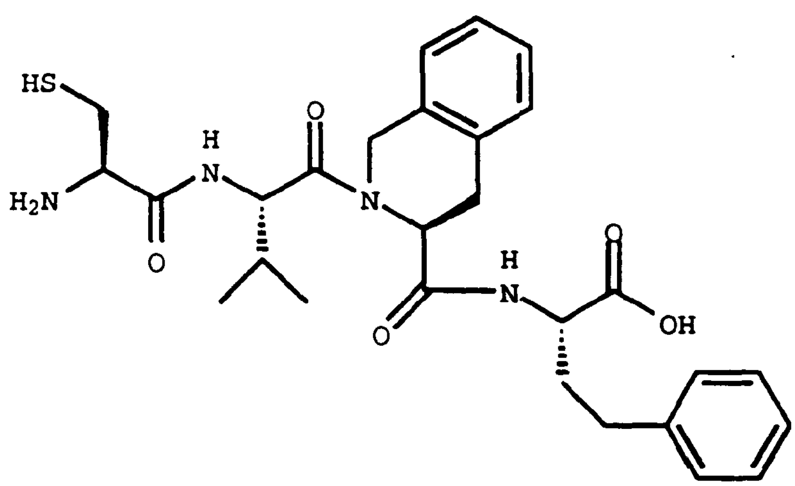

**A. 3-(Phenylmethyl)-L-alanine, methyl ester.**

To a solution of L-homophenylalanine (0.99 g, 5.53 mmol) in absolute methanol (50 mL) under argon was added p-TsOH·$H_2O$ (2.09 g, 11.0 mmol) and the mixture was heated under reflux for 6 hours. The reaction mixture was cooled and concentrated *in vacuo*. The residue was triturated with ether to afford Compound A (2.0 g, 90%).

**B. (R*)-N-[[2-[N-[N-[(1,1-Dimethylethoxy)carbonyl]-S-triphemylmethyl)-L-cysteinyl)-L-valyl]-1,2,3,4-tetrahydro-3-isoquinolinyl]carbonyl]-3-(phenylmethyl)-L-alanine, methyl ester.**

To a cold (0°C) solution of Compound 18D (360 mg, 0.5 mmol) in dichloromethane (5 mL) containing dimethylformamide (0.5 mL) was added Compound A (292 mg, 0.8 mmol), bis(2-oxo-3-oxazolidinyl)phosphinic chloride (128 mg, 0.5 mmol) and N-methylmorpholine (0.22 mL, 2.0 mmol). After 2 hours the reaction mixture was allowed to warm to 5°C and stirred overnight (16 hours). The mixture was diluted with chloroform (10 mL) and washed successively with 10 mL each of 1N HCl, saturated $NaHCO_3$ and brine. The organic layer was dried (MgSO4), filtered and concentrated *in vacuo*. Purification by flash silica gel column eluting with 30% ethyl acetate in hexanes afforded Compound B (330 mg, 73%).
TLC: $R_f$ 0.46 (40% ethyl acetate in hexanes, visualized by ceric ammonium molybdate)
MS: (FAB) $(M+H)^+ = 897^+$.

**C. (R*)-N-[[2-[N-(L-Cysteinyl)-L-valyl]-1,2,3,4-tetrahydro-3-isoquinolinyl]carbonyl]-3-(phenylmethyl)-L-alanine.**

The title compound (60% yield) was prepared from Compound B (195 mg) using the two step procedure described for the preparation of Compound 18G from Compound 18E.
mp: 120-123°C
MS: (FAB) $(M+H)^+ = 541$.
$[a]_D^{22} = -34.7$ ° (c = 0.426, methanol)
Elemental Analysis (%) for $C_{28}H_{36}N_4O_5S$ . 0.8 H2O . 1.13 trifluoroacetic acid:
Calc'd: C, 53.14; H, 5.71; N, 8.19; F, 9.42;
Found: C, 53.14; H, 5.68; N, 8.19; F, 9.44.

**Example 26**

**(R*)-N-[[2-[N-(L-Seryl)-L-valyl]-1,2,3,4-tetrahydro-3-isoquinolinyl]carbonyl]-L-methionine.**

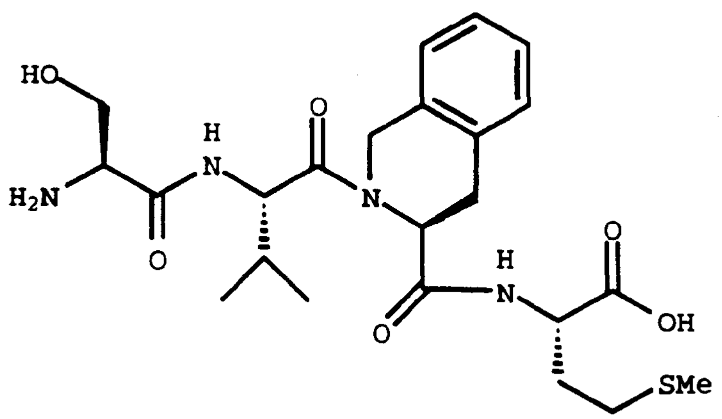

The title Compound (6 mg; white, fluffy powder) was prepared using the solid phase method described in Example 15, with the following modifications:

N-t-Butyloxycarbonyl-L-Met-PAM resin (0.59 g, 0.25 mmol) was obtaine commercially. The reactive side chain of serine was protected as a benzyl ether.
MS: $(M+H)^+$ $495^+$·
$^1$H NMR ($CD_3OD$): δ (ppm) 7.23 (m), 5.03 (m), 4.95 (m), 4.71 (m), 4.46 (m), 4.22 (m), 4.06-3.69 (m), 3.44 (m), 3.38 (m), 3.16 (m), 2.39 (m), 2.24 (m), 2.08 (m), 2.04 (s), 1.94 (m), 1.29 (m), 1.09 (J= 6.4 Hz), 1.00 (m). (Rotamers observed.)

## Example 27

**(R*,R*)-2-[N-(3-Mercapto-1-oxopropyl)-L-valyl]-1,2,3,4-tetrahydro-N-[3-(methylthio)-1-(1H-tetrazol-5-yl)propyl]-3-isoquinolinecarboxamide.**

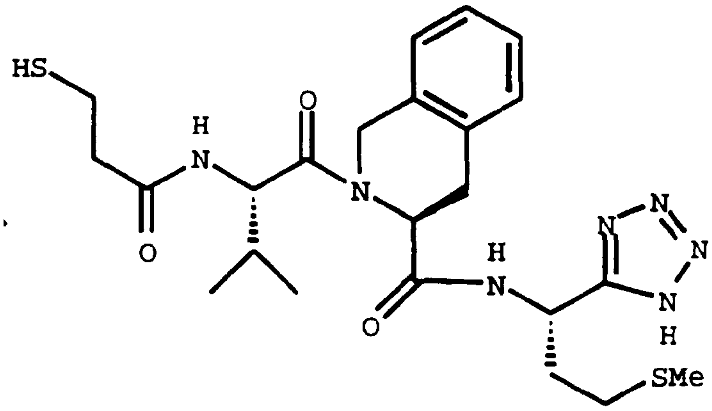

**A. N-(2-Cyanoethyl)-$N^2$-[(1,1-dimethylethoxy)carbonyl]-L-methionine.**

1-Ethyl-3-(3-dimethylaminopropyl)carbodiimide hydrochloride (16.24 g, 85 mmol), 3-aminopropionitrile (10.9 g, 85 mmol, fumarate salt), N,N-diisopropylethylamine (17.9 mL, 100 mmol) were added to a solution of N-t-butyloxycarbonyl-L-methionine (19.9 g, 80 mmol) and N-hydroxybenzotriazole (13.0 g, 85 mmol) in tetrahydrofuran (150 mL) at room temperature under argon. The reaction mixture became very thick and precipitate formed. More tetrahydrofuran (80 mL) and dimethylformamide (50 mL) were added. After 7 hours, additional amine salt (2 g) and N,N-diisopropylethylamine (3 mL) were added, and the mixture was stirred for another 18 hours. The reaction mixture was partitioned between saturated ammonium chloride (100 mL) and ethyl acetate (110 mL). The organic layer was washed sequentially with 1N HCl (100 mL) and $NaHCO_3$ (100 mL). The organic layer was dried ($MgSO_4$), filtered and concentrated. The oil obtained was purified by silica gel column chromatography eluting with a step gradient of 50% ethyl acetate in hexanes to afford Compound A (11.2 g, 47%).
MS (CI), $(M+H)^+$ = 320.
TLC : $R_f$ 0.11 (50%, ethyl acetate in hexanes, visualized by U.V. and ceric ammonium molybdate).

**B. (R)-[1-[1-(2-Cyanoethyl)-1H-tetrazol-5-yl]-3-(methylthio)propyl]carbamic acid, 1,1-dimethylethyl ester.**

To a solution of Compound A (10 g, 33.2 mmol) in tetrahydrofuran (300 mL) at room temperature under argon was added sequentially triphenylphosphine (18 g, 69.8 mmol), diethylazodicarboxylate (10.99 mL, 69.8 mmol) and azidotrimethylsilane (9.26 mL, 69.8 mmol). After 72 hours, the volatiles were removed on rotary evaporator behind a safety shield. The residue was treated with ammonium ceric nitrate (3 g in 50 mL). A precipitate formed along with evolution of gas. Water (50 mL) was added and the aqueous layer was extracted with dichloromethane (100 mL). The organic layer was dried ($MgSO_4$), filtered and concentrated *in vacuo*. Purification of the residue by flash silica gel column (50x300 mm), eluting with 40% ethyl acetate in hexanes, afforded crystalline (long needles) Compound B (7.2 g, 66%).
MS (CI): $(M+H)^+$ 327.
TLC : $R_f$ 0.3 (50%, ethyl acetate in hexanes, visualized by U.V. and ceric ammonium molybdate).

**C. (R*)-2-[N-[(1,1-Dimethylethoxy)carbonyl]-L-valyl]-1,2,3,4-tetrahydro-3-isoquinolinecarboxylic acid.**

To a solution of Compound 18A (3.1 g, 6.66 mmol) in 50 mL of methanol was added 150 mg $Pd(OH)_2$ (20% on carbon). The mixture was stirred under $H_2$ atmosphere (balloon) for 1.5 hours. The mixture was filtered through celite, and the filtrate was concentrated *in vacuo* to afford Compound C (2.3 g, 92%) as a white solid.
mp 89°C
MS: $(M+H)^+$ 377.

**D. (R*,R*)-2-[N-[(1,1-Dimethylethoxy)carbonyl]-L-valyl]-1,2,3,4-tetrahydro-N-[3-(methylthio)-1-[1-(2-cyanoethyl)-1H-tetrazol-5-yl]propyl]-3-isoquinolinecarboxamide.**

Anhydrous HCl (4M in dioxane, 1 mL, 4 mmol) and triethylsilane (0.3 mL) were added to solid Compound B (64 mg, 0.2 mmol) at room temperature under argon. After stirring overnight (16 hours) a white precipitate formed. Supernatent dioxane was removed by pipette, and the solid was triturated with ether and dried *in vacuo* to afford a solid (120 mg). (MS: $(M+H)^+$ $227^+$)

This material (120 mg) was dissolved in dichloromethane (1.5 mL) at room temperature under argon and was treated sequentially with Compound C (90 mg, 0.25 mmol), N-methylmorpholine (0.08 mL, 0.75 mmol) and benzotriazol-1-yloxy-tris(dimethylamino)phosphonium hexafluorophosphate (105 mg, 0.25 mmol). After stirring overnight (16 hours), the mixture was diluted with ethyl acetate (5 mL) and washed with 1N HCl, saturated $NaHCO_3$ and brine (5 mL each). The organic layer was dried ($MgSO_4$), filtered and concentrated to afford an oil. Purification by flash silica gel column chromatography eluting with 40% ethyl acetate in hexanes afforded Compound D (101 mg, 85%).

MS: (CI) $(M+H)^+ = 585^+$.

TLC : $R_f$ 0.67 (80%, ethyl acetate in hexanes, visualized by U.V. and ceric ammonium molybdate).

**E. (R*,R*)-2-[N-[3-[(Triphenylmethyl)thio]-1-oxopropyl]-L-valyl]-1,2,3,4-tetrahydro-N-[3-(methylthio)-1-[1-(2-cyanoethyl)-1H-tetrazol-5-yl]propyl]-3-isoquinolinecarboxamide.**

Compound E was prepared from Compound D using the two step procedure described above for the preparation of Compound D from Compound B, with the exception that in the second step S-trityl-3-mercaptopropionic acid was used in place of Compound C.

TLC $R_f$ 0.48 (80% ethyl acetate in hexanes visualized by ceric ammonium molybdate).

MS (FAB) $(M+H)^+ = 815$.

**F. (R*,R*)-2-[N-[3-[(Triphenylmethyl)thio]-1-oxopropyl]-L-valyl]-1,2,3,4-tetrahydro-N-[3-(methylthio)-1-(1H-tetrazol-5-yl)propyl]-3-isoquinolinecarboxamide.**

To a solution of Compound E (180 mg, 0.22 mmol) in tetrahydrofuran (1.5 mL) at room temperature under argon was added a solution of LiOH (0.44 mL, 0.44 mmol). Methanol (0.4 mL) was added to make the reaction mixture homogeneous and argon was bubbled through the solution for 2 minutes. After 1 hour, the mixture was diluted with 1N HCl (5 mL) and extracted with ethyl acetate (2 x 10 mL). The organic extracts were combined, dried ($MgSO_4$), and concentrated *in vacuo* to afford Compound F (160 mg, 95%), as a foamy solid.

TLC: $R_f$ 0.05 (70% ethyl acetate in hexanes, visualized by ceric ammonium molybdate).

MS: (FAB) $(M+H)^+ = 762$.

**G. (R*,R*)-2-[N-(3-Mercapto-1-oxopropyl)-L-valyl]-1,2,3,4-tetrahydro-N-[3-(methylthio)-1-(1H-tetrazol-5-yl)propyl]-3-isoquinolinecarboxamide.**

(All solvents used in the preparation of Compound G from Compound F, except trifluoroacetic acid, were deoxygenated by bubbling argon prior to use).

To a solution of Compound F (150 mg, 0.2 mmol) in dichloromethane (2 mL) at room temperature under argon was added trifluoroacetic acid (0.1 mL). After 5 min, water (5 mL) followed by saturated $NaHCO_3$ was added dropwise (until pH = 8) and then reacidified with 1N HCl (1 mL). The resulting mixture was extracted with chloroform (2 x 10 mL). The organic extracts were combined, dried ($MgSO_4$), filtered and concentrated. The residue was purified by preparative HPLC (YMC, S10, C18, 30 x 500 mm column) eluting with 50-90% aqueous methanol. Appropriate fractions were collected. Methanol was removed on a rotary evaporator and the aqueous solution was lyophilized to afford the title compound.

mp 102°C

TLC: $R_f$ 0.49 ($CHCl_3$: methanol: acetic acid, 9:1:0.1, visualized by ceric ammonium molybdate)

MS (FAB) $(M+H)^+ = 520$.

Elemental Analysis (%) $C_{23}H_{33}N_7O_3S_2$ . 0.6 $H_2O$

Calc'd: C, 52.07; H, 6.50; N, 18.48;

Found: C, 52.07; H, 6.38; N, 18.24.

**Example 28**

**(R*,S*)-N-[[2-[N-(2-Amino-3-mercaptopropyl)-N-methyl-L-valyl]-1,2,3,4-tetrahydro-3-isoquinolinyl]carbonyl]-L-methionine.**

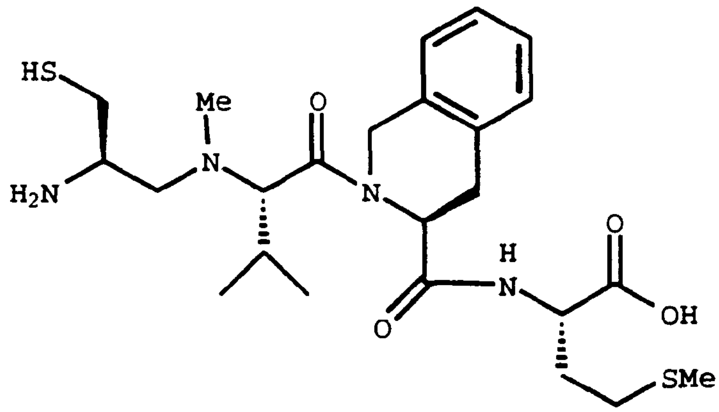

**A. (R*)-N-[[2-[N-[(1,1-Dimethylethoxy)carbonyl]-N-methyl-L-valyl]-1,2,3,4-tetrahydro-3-isoquinolinyl]carbonyl]-L-methionine, methyl ester**

Compound A (clear oil, 3.0 g, 99%) was prepared from Compound 1A (2.0 g, 4.73 mmol) using a two step procedure analogous to that described for the preparation of Compound 8A, except that in the second step N-t-butyloxycarbonyl-N-methyl-L-valine (1.80 g, 7.78 mmol) was used instead of N-Fmoc-N-methyl-L-valine.
MS: $(M+H)^+$ 536.

**B. (R*,S*)-N-[[2-[N-[2-[[(1,1-Dimethylethoxy)carbonyl]amino]-3-[(triphenylmethyl)thio]propyl]-N-methyl-L-valyl]-1,2,3,4-tetrahydro-3-isoquinolinyl]carbonyl]-L-methionine, methyl ester.**

Compound B (clear oil, 200 mg, 5%) was prepared from Compound A (2.5 g, 4.73 mmol) using a two step procedure analogous to that described for the preparation of compound 7B, with the following modifications: In the second step, Compound 1F (2.2 g, 4.92 mmol) was used instead of Compound 6B, and only one equivalent of sodium cyanoborohydride (298 mg, 4.73 mmol) was used.
MS: $(M+H)^+$ 867.

**C. (R*,S*)-N-[[2-[N-(2-Amino-3-mercaptopropyl)-N-methyl-L-valyl]-1,2,3,4-tetrahydro-3-isoquinolinyl]carbonyl]-L-methionine.**

Compound C (26 mg, 22%) was prepared from Compound B (200 mg, 0.231 mmol) using the procedure described for the preparation of Compound 1H from Compound 1G.
mp 59-60°C
$[a]_D^{25}$ = -12.0 ° (c=0.1, $CH_3OH$)
MS: $(M+H)^+$ $511^+$.
$^1$H-NMR ($CD_3OD$, 270 MHz) δ (ppm) 7.18 (4H, m), 4.43 (1H, m), 4.28 (1H, m), 3.55 (1H, m), 3.15 (4H, br m), 2.69 (2H, m), 2.48 (2H, m), 2.40 (1H, m), 2.27 (3H, m), 2.20 (2H, m), 1.95 (3H, d), 1.85 (3H, m), 0.91 (6H, m).

**Example 29**

**(R*,R*,S*)-N-[[2-[2-[(2-Amino-3-mercaptopropyl)amino]-3-methylbutyl]-1,2,3,4-tetrahydro-3-isoquinolinyl]carbonyl]-L-methionine.**

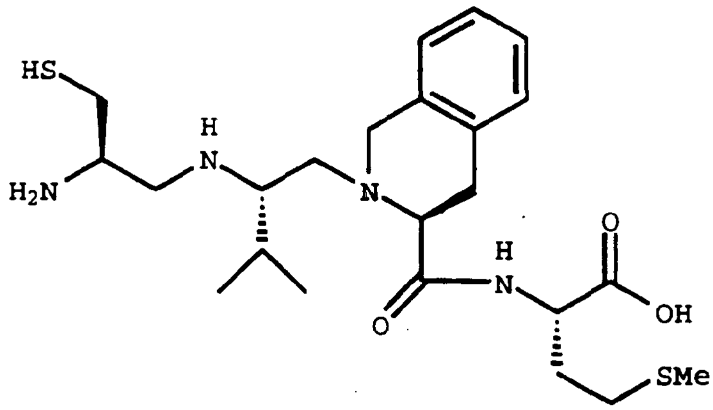

**A. (R*,R*,S*)-N-[[2-[2-[[2-[[(1,1-Dimethylethoxy)carbonyl]amino]-3-[(triphenylmethyl)thio]propyl]amino]-3-methylbutyl]-1,2,3,4-tetrahydro-3-isoquinolinyl]carbonyl]-L-methionine, methyl ester.**

Compound A (1.2 g, 32%) was prepared from Compound 5C (2.30 g, 4.54 mmol) using a two step procedure analogous to that described for the preparation of Compound 7B, with the following modifications: In the second step, Compound 1F (2.2 g, 4.92 mmol) was used instead of Compound 6B, and only one equivalent of sodium cyanoborohydride (285 mg, 4.54 mmol) was used.
Ms: $(M+H)^+$ 839.

**B. (R*,R*,S*)-N-[[2-[2-[(2-Amino-3-mercaptopropyl)amino]-3-methylbutyl]-1,2,3,4-tetrahydro-3-isoquinolinyl]carbonyl]-L-methionine.**

Compound B (50% yield) was prepared from Compound A (1.2 g, 1.43 mmol) using the two step procedure described for the preparation of Compound 1H from Compound 1G.
mp 72-73 °C
$[a]_D^{25}$ = -30.0 ° (c=0.18, $CH_3OH$)
MS: $(M+H)^+$ 483.
$^1$H-NMR ($CD_3OD$, 270 MHz) δ (ppm) 7.28 (4H, m), 4.68 (1H, m), 4.19 (1H, m), 3.66 (1H, m), 3.42 (2H, m), 3.24 (2H, m), 3.03 (2H, m), 2.94 (2H, d, J=4.28 Hz)), 2.60 (2H, m), 2.20 (2H, m), 2.10 (3H, s), 2.07 (3H, m), 1.01 (3H,d, J=7.03Hz), 0.93 (3H, d, J=7.04Hz).

**Example 30**

**(R*)-N-[[1-[N-(L-Cysteinyl)-L-valyl]-2,3-dihydro-1H-indol-2-yl]carbonyl]-L-methionine.**

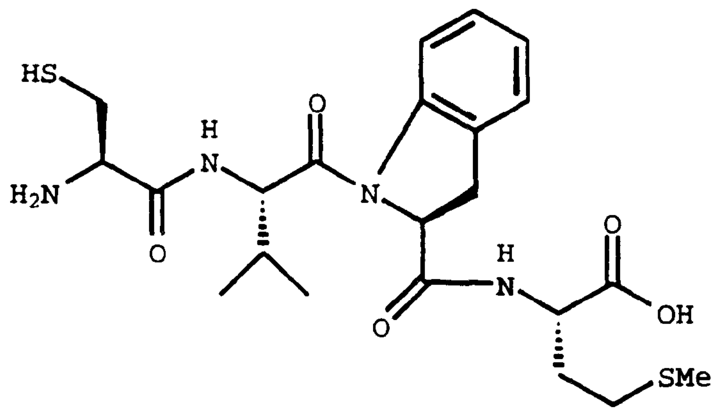

**A. (R*)-1-[N-[(1,1-Dimethylethoxy)carbonyl]-L-valyl]-2,3-dihydro-1H-indole-2-carboxylic acid, ethyl ester.**

Bis(2-oxo-3-oxazolidinyl)phosphinic chloride (2.54 g, 10 mmol), (S)-2,3-dihydro-1H-indole-2-carboxylic acid ethyl ester (1.15 g, 5 mmol) [see Stanton, *et. al.*, J. Med. Chem., 26, 1267, (1983)], and N-methylmorpholine (2.2 mL, 20.0 mmol) were added to a solution of N-t-butyloxycarbonyl-L-valine (2.17 g, 10 mmol) in dichloromethane (30 mL) containing dimethylformamide (1 mL) at 0°C under argon. The reaction was allowed to warm to 5°C over 2 hours, and stirred overnight (20 hours). The reaction mixture was washed with 1N HCl (30 mL) and 10% LiCl (30 mL), dried ($MgSO_4$), filtered and concentrated. The oil obtained was purified by silica gel column chromatography eluting with 10% acetone in hexanes, dissolved in ether (30 mL), and washed with 1N HCl (3 x 20 mL). The organic layer was concentrated *in vacuo* to afford Compound A (880 mg, 47%) as an oil.
MS (CI), $(M+H)^+ = 391^+$.
TLC : $R_f$ 0.6 (20%, ethyl acetate in $CHCl_3$, visualized by U.V. and ceric ammonium molybdate).

**B. (R*)-2-[N-[(1,1-Dimethylethoxy)carbonyl]-L-valyl]-2,3-dihydro-1H-indol-2-ylcarboxylic acid.**

Compound B (650 mg, 83%) was prepared from Compound A (840 mg, 2.15 mmol) using the procedure described for the preparation of Compound 18D from Compound 18C.
MS: (CI) $(M+H)^+ = 363^{+\cdot}$

**C. (R*)-N-[[1-[N-[(1,1-Dimethylethoxy)carbonyl]-L-valyl]-2,3-dihydro-1H-indol-2-yl]carbonyl]-L-methionine,1,1-dimethylethyl ester.**

Compound C (340 mg, 74%) was prepared from Compound B (300 mg, 0.83 mmol) using a procedure analogous to that described for the prepartion of Compound 23A with the following modifications: L-methionine t-butyl ester hydrochloride (242 mg, 1.0 mmol) was used instead of L-glutamine t-butyl ester hydrochloride, and the product was purified by silica gel column chromatography eluting with 30% ethyl acetate in hexanes.
m.p. 79-81°C
MS (CI) $(M+H)^+ = 550^+$.
TLC $R_f$ 0.2 (30% ethyl acetate in hexanes, visualized by UV, ceric ammonium molybdate).

**D. (R*)-N-[[1-(L-Valyl)-2,3-dihydro-1H-indol-2-yl]carbonyl]-L-methionine,1,1-dimethylethyl ester.**

To a solution of Compound C (130 mg, 0.24 mmol) in formic acid (98%, 0.5 mL) at room temperature under argon was added triethylsilane (0.08 mL) and the progress of the reaction mixture was monitored by TLC analysis. After 9 hours, saturated $NaHCO_3$ (5 mL) was added to the mixture and extracted with $CHCl_3$ (2 x 5 mL). The organic extracts were combined, dried ($MgSO_4$), filtered and concentrated to afford Compound D (100 mg, 92%).

MS (CI) $(M+H)^+ = 450^+$

**E. (R*)-N-[[1-[N-[N-[(1,1-Dimethylethoxy)carbonyl]-S-(triphenylmethyl)-L-cysteinyl]-L-valyl]-2,3-dihydro-1H-indol-2-yl]carbonyl]-L-methionine, 1,1-dimethylethyl ester.**

To a solution of Compound D (100 mg, 0.22 mmol) in dichloromethane (1 mL) at 0°C under argon were added sequentially N-t-butyloxycarbonyl-S-trityl-L-cysteine (122 mg, 0.26 mmol), N-methylmorpholine (0.033 mL, 0.3 mmol) and benzotriazol-1-yloxy-tris(dimethylamino)phosphonium hexafluorophosphate (115 mg, 0.26 mmol). The reaction mixture was allowed to warm to room temperature over 2 hours, and then stirred overnight (16 hours). The reaction mixture was diluted with chloroform (5 mL) and washed sequentially with 1N HCl, saturated $NaHCO_3$ and brine (5 mL each). Purification by flash silica gel column chromatography eluting with 30% ethyl acetate in hexanes afforded Compound E (160 mg, 81%).
TLC $R_f$ 0.11 (70% ethyl acetate in hexanes, visualized by UV, ceric ammonium molybdate).
MS: (FAB) (M+H)+= 895+.

**F. (R*)-N-[[1-(N-(L-Cysteinyl)-L-valyl]-2,3-dihydro-1H-indol-2-yl]carbonyl]-L-methionine.**

To solution of Compound E (140 mg, 0.16 mmol) in dichloromethane (1.5 mL) at room temperature under argon were added triethylsilane (0.2 mL, 1.25 mmol) and trifluoroacetic acid (0.5 mL). After 2 hours, the volatiles were removed by rotary evaporation and the residue was purified by preparative HPLC (C-18, S10, 30x500 mm column) eluting with linear gradient of 10-90% aqueous methanol containing 0.1% trifluoroacetic acid. Appropriate fractions were collected, methanol was removed on a rotary evaporator, and the aqueous solution was lyophilized to afford the title Compound (56 mg, 70%).
m.p. 72-74°C
MS: $(M+H)^+ = 497^+$.
$[a]_D$ = -49.8° (c= 0.49, $CH_3OH$)
HPLC: (C-18, S3, 6x150 mm column), 1.5 mL/min, 220 nm: 10-90% aqueous methanol containing 0.2% $H_3PO_4$; RT = 18.06 min.
Elemental Analysis (%) $C_{23}H_{33}N_7O_3S_2 \cdot 0.6H_2O$:
Calc'd: C, 52.07; H, 6.50; N, 18.48;
Found: C, 52.07; H, 6.38; N, 18.24.

**Example 31**

**(S*, R*)-N-[[2-[N-(2-Amino-3-mercaptopropyl)-L-valyl]-1,2,3,4-tetrahydro-3-isoquinolinyl]carbonyl]-L-methionine, methyl ester, trifluoroacetate (1:2) salt**

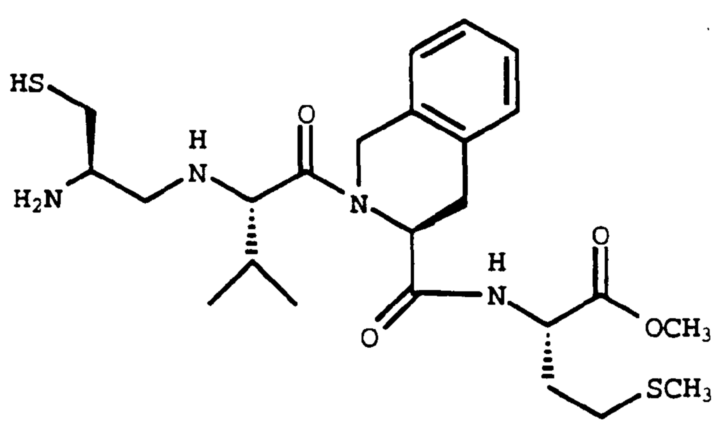

**A. (S*, R*)-N-[[2-(N-(2-Amino-3-mercaptopropyl)-L-valyl]-1,2,3,4-tetrahydro-3-isoquinolinyl]-carbonyl]-L-methionine, methyl ester, trifluoroacetate (1:2) salt**

To a solution of 180 mg (0.211 mmol) of Compound 1G in 3 mL of dichloromethane was added 0.20 mL (1.27 mmol) of triethylsilane. The resulting solution was cooled to 0°C and purged with argon. To this was added 1 mL of trifluoroacetic acid. The mixture was warmed to room temperature and stirred for 2 hours. The solvent

was removed in vacuo and the residue was triturated with hexane. The residue was purified by HPLC (YMC S-10 ODS 30 x 500 mm, 220 nm, 28 mL/minute, 40-90% aqueous methanol with 0.1% trifluoroacetic acid). The appropriate fractions were concentrated *in vacuo,* and lyophilized to afford the title compound (70 mg) as a white lyophilizate.

mp: 56-68°C

MS: $(M+H)^+$ 511

**Example 32**

**[3S-[2[R*(S*)],3R*(R*)]]-2-[N-(2-Amino-3-mercaptopropyl)-L-valyl]-1,2,3,4-tetrahydro-N-(tetrahydro-2-oxo-3-furanyl)-3-isoquinolinecarboxamide, trifluoroacetate (1:2) salt**

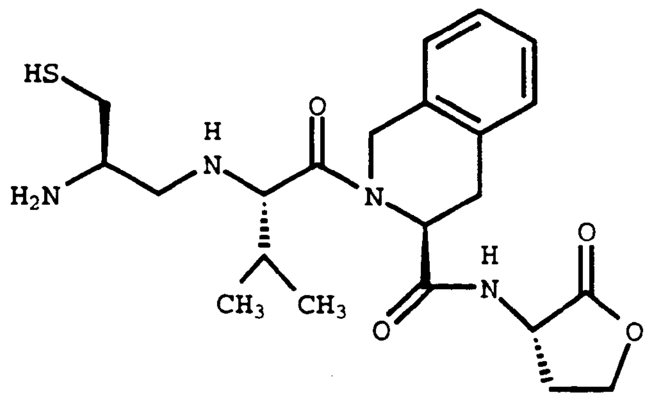

**A. (S*)-N-[2-[[(1,1-Dimethylethoxy)carbonyl]amino]-3-[(triphenylmethyl)thio]propyl]-L-valine, methyl ester**

Acetic acid (2.6 mL, 44 mmol) was added to a solution of Compound 1F (20 g, 44.7 mmol) and L-valine methylester hydrochloride (9.0 g, 53.7 mmol) in methanol (50 mL). The mixture was stirred at room temperature for 30 minutes. Sodium cyanoborohydride (2.8 g, 44.7 mmol) in tetrahydrofuran (50 mL) was added dropwise and the mixture was stirred at room temperature for 2 hours. The reaction was quenched with sodium bicarbonate (3.75 g, 44.7 mmol) in water (20 mL) and concentrated under vacuum. The residue was dissolved in 10% sodium bicarbonate (10 mL) and extracted with dichloromethane (3 x 100 mL). The combined organic extracts were dried (magnesium sulfate), filtered and concentrated under vacuum. The residue was purified by flash chromatography (eluting with 9:1 hexane/acetone) to afford Compound A (17.7 g, 70%).

TLC: $R_f$ : 0.25 (8/1 hexane/acetone, visualization by UV).

MS: $(M+H)^+$563

IR: ($CH_2Cl_2$ film), 1491, 1715 $cm^{-1}$

**B. (S*)-N-[2-[[(1,1-Dimethylethoxy)carbonyl]amino]-3-[(triphenylmethyl)thio]propyl]-N-[(phenylmethoxy)carbonyl]-L-valine, methyl ester**

Benzylchloroformate (about 90%, 3.2 mL, 20.3 mmol) was added to a solution of Compound A (3.8 g, 6.76 mmol) and diisopropylethylamine (3.8 mL, 21.6 mmol) in dichloromethane (20 mL) at 0°C. The mixture was warmed to room temperature and stirred for 16 hours. The reaction was diluted with dichloromethane (100 mL), and washed sequentially with 1N hydrochloric acid (2 x 100 mL) and 10% sodium bicarbonate (2 x 100 mL). The organic layer was dried (magnesium sulfate), filtered and concentrated under vacuum. The residue was purified by flash chromatography (eluting with 8/1 hexane/acetone) to afford Compound B (4.3 g, 92%), as a white solid.

mp: 48-52°C

TLC: $R_f$ : 0.35 (4/1 hexane/acetone, visualization by UV)

MS: $(M-H)^-$ 695

C. (S*)-N-[2-[[(1,1-Dimethylethoxy)carbonyl]amino]-3-[(triphenylmethyl)thio]propyl]-N-[(phenylmethoxy)carbonyl]-L-valine

Lithium hydroxide (2N, 1.5 mL, 3.0 mmol) was added dropwise to a solution of Compound B (0.696 g, 1.0 mmol) in tetrahydrofuran (5 mL) and methanol (3.5 mL). The homogeneous reaction was warmed to room temperature and stirred for 16 hours. The mixture was concentrated under vacuum, diluted with 1N hydrochloric acid (30 mL) and extracted with dichloromethane (3 x 50 mL). The combined organic extracts were dried (magnesium sulfate), filtered and concentrated under vacuum to afford Compound C (0.68 g, 100 %).
mp: 66-72°C
TLC: $R_f$ : = 0.61 (9/1/0.05 chloroform/methanol/acetic acid, visualization by UV)
MS: $(M-H)^-$ 681

D. [3S-[2[R*(S*)],3R*]]-2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinecarboxylic acid,methyl ester

Diisopropylethylamine (19.7 mL, 113 mmol) was added to a solution of Compound C (24.15 g, 35.4 mmol), (S)-1,2,3,4-Tetrahydroisoquinoline-3-carboxylic acid, methyl ester, hydrochloride (9.66 g, 42.5 mmol), and benzotriazol-1-yloxytris-(dimethylamino)phosphonium hexafluorophosphate hydrochloride (10.8 g, 42.5 mmol) in dichloromethane (200 mL). The mixture was stirred at 0°C for 16 hours. The reaction was quenched with 1N hydrochloric acid (300 mL) and extracted with dichloromethane (3 x 100 mL). The combined organic extracts were washed with 10% sodium bicarbonate (100 mL), dried, filtered and concentrated under vacuum. The residue was purified by flash chromatography (eluting with 4/1 hexane/acetone) to afford Compound D (21.8 g, 72%), as a white solid.
mp: 66-70°C
TLC: $R_f$ : 0.28 (4/1 hexane/acetone, visualization by UV)
MS: $(M+H)^+$ 856

E. [3S-[2[R*(S*)],3R*]]-2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinecarboxylic acid

Compound E was prepared using Compound D (12.5 g, 14.6 mmol) as in the method described in Example 32 C. Compound E (12.3 g, 100%) was prepared as a white solid.
mp: 88-94°C
TLC: $R_f$ : 0.52 (9/1/0.05
chloroform/methanol/acetic acid, visualization by UV)
MS: $(M-H)^-$ 840

F. [3S-[2[R*(S*)],3R*(R*)]]-2-[N-[2-[[(1,1-Dimethylethoxy)-carbonyl]amino]-3-[(triphenylmethyl)thio]propyl]-N-[(phenylmethoxy)carbonyl]-L-valyl]-1,2,3,4-tetrahydro-N-(tetrahydro-2-oxo-3-furanyl)-3-isoquinolinecarboxamide

Diisopropylethylamine (0.651 mL, 3.74 mmol) was added to a solution of Compound E (1.5 g, 1.78 mmol), L-homoserine lactone hydrochloride (0.245 g, 1.78 mmol) and benzotriazol-1-yloxytris-(dimethylamino)phosphonium hexafluorophosphate (0.789 g, 1.78 mmol) in acetonitrile (3 mL) and dimethylformamide (1 mL). The mixture was then stirred at room temperature for 16 hours. The reaction was quenched with 1N hydrochloric acid (50 mL), and extracted with ethyl acetate (3 x 75 mL). The combined organic extracts were washed with 10% lithium chloride (2 x 50 mL), dried, filtered and concentrated under vacuum. The residue was purified by flash chromatography (eluting with 2/1 hexane/acetone) to afford Compound F (1.45 g, 88%) as a gum.
TLC: $R_f$ : 0.61 (1/1 hexane/acetone, visualization by UV)
MS: $(M-H)^-$ 923

G. [3S-[2[R*(S*)], 3R*(R*)]]-2-[N-(2-Amino-3-mercaptopropyl)-L-valyl]-1,2,3,4-tetrahydro-N-(tetrahydro-2-oxo-3-furanyl)-3-isoquinolinecarboxamide, trifluoroacetate (1:2) salt

Trifluoroacetic acid (4.3 mL, 55.8 mmol) was added to a solution of Compound F (0.8 g, 0.866 mmol) in dichloromethane (9 mL) and triethylsilane (1.4 mL, 8.66 mmol). The resulting solution was stirred at room temperature for 1 hour. The reaction was concentrated under vacuum, and the residue was triturated with hexane

(3 x 10 mL). The hexane layer was discarded. In a separate flask, trifluoroacetic acid (17 mL, 220 mmol) was treated with thioanisole (1 mL, 8.5 mmol) and 1,2-ethanedithiol (0.726 mL, 8.66 mmol) at 0°C. This solution was added to the triturated residue, followed by the dropwise addition of bromotrimethylsilane (1.1 mL, 4.3 mmol). The solution was stirred at 0°C for 1 hour. The reaction was treated with diethylether (40 mL) and the resulting precipitate was centrifuged. The supernatant was removed and discarded, and the solid was washed and centrifuged with diethylether (3 x 40 mL), ethyl acetate (2 x 40 mL) and diethylether (1 x 40 mL). The solid was purified by preparative HPLC (YMC S-10 ODS 30 x 500 mm, 220 nm, 35 mL/minute, 10%-90% aqueous methanol with 0.1% trifluoroacetic acid gradient). The appropriate fractions were concentrated under vacuum at room temperature, and the residue was lyophilized to afford the title compound (0.13 g, 26%).
mp: 95-107°C
TLC: $R_f$ = 0.68 (6/3/1 n-propanol/ammonium hydroxide/ water, visualization by ceric ammonium sulfate/ ammonium molybdate/ sulfuric acid/ water)
MS: $(M+H)^+$ 449 free base
IR: (KBr), 1678, 1773, 2974 $cm^{-1}$

| Elemental analysis for $C_{26}H_{34}N_4F_6S_1O_6 \cdot 2.56\ H_2O$ | | | | |
|---|---|---|---|---|
| | C | H | N | S |
| Calculated: | 45.21 | 5.71 | 8.11 | 4.64 |
| Found: | 45.26 | 5.25 | 8.06 | 4.83 |

$^1H$ ($CD_3OD$): 1.06-1.13 (m, 6 H), 2.11-2.46 (m, 5 H), 2.85-2.89 (m, 3 H), 3.12-3.20 (m, 2 H), 3.50-3.80 (m 1 H), 4.25-4.91 (m, 6 H), 7.28 (s, 4 H)
$[\alpha]_D$ = - 6.7° , c = 0.76 (methanol)

**Example 33**

**(S*,R*)-N²-[[2-[N-(2-Amino-3-mercaptopropyl)-L-valyl]-1,2,3,4-tetrahydro-3-isoquinolinyl]carbonyl]-N-hydroxy-L-methioninamide, trifluoroacetate (1:2) salt**

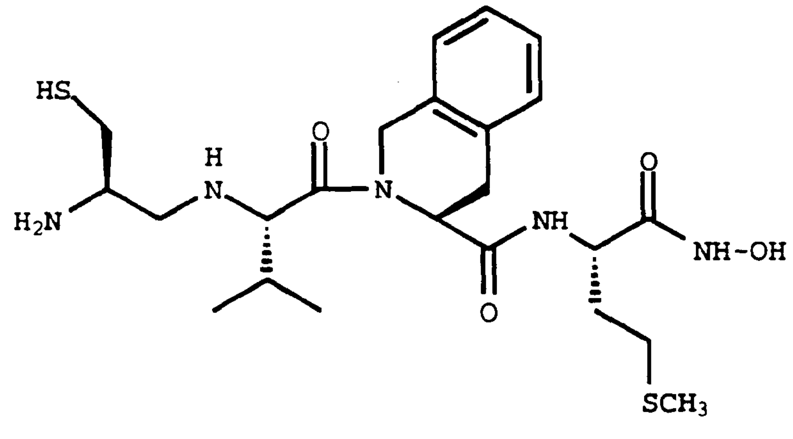

**A. (S*,R*)-N-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-L-methionine, methyl ester**

Compound A (707 mg, 60% yield) was prepared using Compound 32E (1.00 g, 1.19 mmol) and methionine methyl ester, hydrochloride (237 mg, 1.19 mmol) as in the method described in Example 32F. mp: 77-79°C
TLC: $R_f$ = 0.72 (1:1 hexane/ethyl acetate, visualized by phosphomolybdic acid)
MS: $(M+H)^+$ 987

**B. (S*,R*)-$N^2$-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-N-(tetrahydropyran-2-yloxy)-L-methioninamide**

To a solution of Compound A (600 mg, 0.608 mmol) in tetrahydrofuran (3 mL) at 0° was added 1 N lithium hydroxide (0.912 mL, 0.912 mmol). After stirring for 2 hours, 1 N hydrochloric acid (0.912 mL, 0.912 mmol) was added and the reaction was concentrated to 1 mL. The mixture was diluted with dichloromethane (100 mL) and 1 N hydrochloric acid (10 mL). The layers were separated and the aqueous layer was extracted with dichloromethane (1 x 100 mL) and ethyl acetate (100 mL), dried, filtered and concentrated to give crude acid (500 mg, 85% yield). HPLC showed the mixture contained a 7:3 ratio of the desired acid and the corresponding methionine sulfoxide. This was used without further purification.
MS: $(M+Li)^+$ 979 and $(M-H)^-$ 971 $(M+Li)^+$ 997 and $(M-H)^-$ 987

To a solution of the above material (500 mg, 0.514 mmol) in dimethylformamide (3 mL) at 0° under nitrogen was added Compound 20A (60 mg, 0.514 mmol), diisopropylethylamine (89 mL, 0.514 mol) and benzotriazol-1-yloxytris-(dimethylamino)phosphonium hexafluorophosphate (227 mg, 0.514 mmol). After stirring for 20 hours at 0°, diisopropylethylamine (30 µL, 0.171 mmol), Compound 20A (20 mg, 0.171 mmol) and benzotriazol-1-yloxytris-(dimethylamino)phosphonium hexafluorophosphate (45 mg, 0.102 mmol) were added to the reaction. After warming to room temperature and stirring for 4 hours, the mixture was diluted with ethyl acetate (100 mL), washed sequentially with saturated sodium bicarbonate (50 mL), 1% potassium bisulfate (50 mL) and 10% lithium chloride (3 x 50 mL), dried, filtered and concentrated *in vacuo*. The residue was purified on silica gel (20 mL, eluting with hexane/ethyl acetate 4:1 to 0:1) to give Compound B (300 mg, 54% yield).
mp: 150-151°C
TLC: $R_f$ = 0.37 (2:1 hexane/acetone, visualized by phosphomolybdic acid)
MS: $(M+H)^+$ 1072

**C. (S*,R*)-$N^2$-[[2-[N-(2-Amino-3-mercaptopropyl)-L-valyl]-1,2,3,4-tetrahydro-3-isoquinolinyl]carbonyl]-N-hydroxy-L-methioninamide, trifluoroacetate (1:2) salt**

Compound C (57 mg, 33%) was prepared from Compound B (250 mg, 0.233 mmol) using the method described in Example 32G.
mp: 94-97°C
TLC: $R_f$ = 0.14 (chloroform:methanol:acetic acid 9:1:0.05, visualized by ceric ammonium sulfate)
MS: $(M+)^+$ 512
IR: (KBr) 1676, 1206 $cm^{-1}$
$[\alpha]_D$ = -31.5° [c = 0.260, methanol]
$^1H$ NMR 400 MHz ($CDCl_3$): 0.92 - 1.23 (m, 6H), 1.72 -2.50 (m, 10H), 2.73 - 3.21 (m, 4H), 3.55 (m, 1H), 4.22 - 4.72 (m, 5H), 7.26 (m, 4H)
$^{13}C$ 100 MHz ($CDCl_3$): 15.3, 17.9, 18.1, 19.6, 19.9, 25.6, 25.7, 30.6, 30.9, 31.1, 32.2, 32.4, 32.6, 33.3, 46.0, 47.6, 48.4, 48.6, 48.8, 49.0, 49.2, 49.4, 49.7, 51.5, 51.7, 53.2, 53.5, 57.3, 57.7, 59.1, 65.9, 66.0, 126.9, 127.7, 128.3, 128.6, 128.8, 129.0, 129.3, 129.7, 130.9, 133.6, 134.0, 135.3, 135.4, 170.4, 170.8, 172.4, 173.3, 173.5, 173.8

| Elemental Analysis for: $C_{23}H_{37}N_5O_4S_2 \cdot 1.71\ H_2O \cdot 2.0\ CF_3CO_2H$ | | | | | |
|---|---|---|---|---|---|
| | C | H | N | S | F |
| Calculated: | 42.09 | 5.55 | 9.09 | 8.32 | 14.79 |
| Found: | 42.19 | 5.20 | 8.73 | 8.35 | 15.24 |

## Example 34

**[R-(R*,S*)]-$N^2$-[[2-[N-(2-Amino-3-mercaptopropyl)-L-valyl]-1,2,3,4-tetrahydro-3-isoquinolinyl]carbonyl]-L-glutamine, trifluoroacetate (1:2) salt**

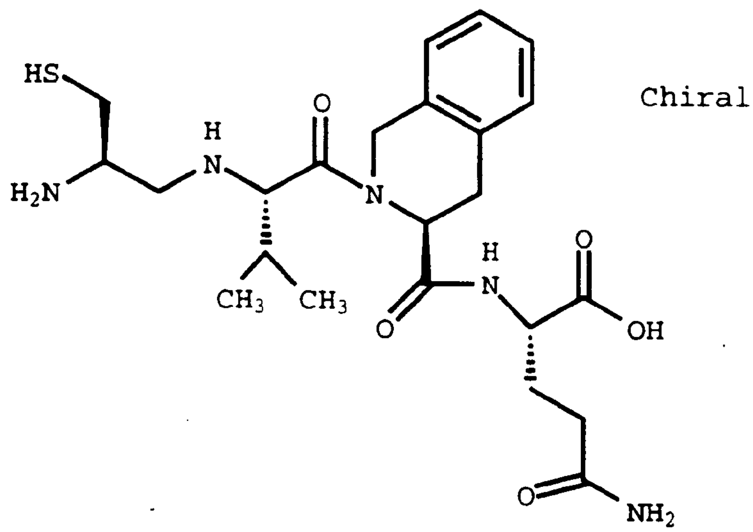

**A. (S*, R*)-$N^2$-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl)-carbonyl]-L-glutamine, 1,1-dimethylethyl ester**

Compound A (1.0 g, 90%) was prepared as a white solid using Compound 32E (1.0 g, 1.19 mmol) and L-glutamine t-butyl ester HCl (0.284 g, 1.19 mmol) in the method described in Example 32F.
mp: 80-86°C
TLC: $R_f$ : 0.82 (9/1 chloroform/methanol, visualization by UV)
MS: $(M+H)^+$ 1026

**B. [R-(R*,S*)]-$N^2$-[[2-[N-(2-Amino-3-mercaptopropyl)-L-valyl]-1,2,3,4-tetrahydro-3-isoquinolinyl]carbonyl]-L-glutamine, trifluoroacetate (1:2) salt**

The title compound (0.425 g, 56%) was prepared as a white lyophilate using Compound A (1.0 g, 0.976 mmol) in the method described in Example 32G.
mp: 65-75°C
TLC: $R_f$ : = 0.69 (6/3/1 n-propanol/ammonium hydroxide/water, visualization by ceric ammonium sulfate)
MS: $(M+H)^+$ 494, free amine
IR: (KBr), 1202, 1670, 2567 $cm^{-1}$
$^1H$ ($CD_3OD$): 1.07, 1.08, 1.09 (d, 6 H, J = 7.6), 1.93 (br m, 1 H), 2.12-2.31 (m, 6 H), 2.78-3.49 (m, 5H), 4.23, 4.25, 4.38, (br m, 2H), 4.64-4.91 (m, 3 H), 7.22-7.33 (m, 4 H)
$[\alpha]_D$ = - 21.4° ( c = 0.69, methanol)

**Example 35**

**[R-(R*,S*)]-2-[N-(2-Amino-3-mercaptopropyl)-L-valyl]-1,2,3,4-tetrahydro-N-(tetrahydro-2-oxo-3-thienyl)-3-isoquinolinecarboxamide, trifluoroacetate (2,5) salt**

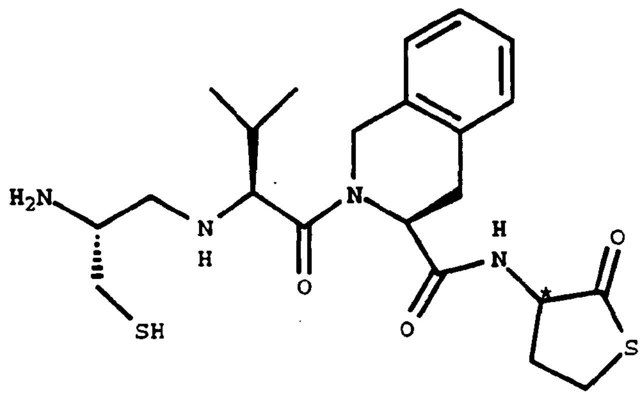

Chiral * ISOMER "A"
• ISOMER "B"

**A. [3S-[2(S*),3R*]]-2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-N-(tetrahydro-2-oxo-3-thienyl)-3-isoquinolinecarboxamide**

Compound A (0.62 g, 63%) was prepared using D,L-Homocysteine thiolactone hydrochloride salt (0.16 g, 1.04 mmol) and Compound 32E (0.88 g, 1.04 mmol) in the method described in Example 32F.

TLC: $R_f$ 0.37 (1/1 Hexane/ethyl acetate), visualized by UV, PMA

MS : $[M-H]^-$ $939^-$

**B. [R-(R*,S*)]-2-[N-(2-Amino-3-mercaptopropyl)-L-valyl)-1,2,3,4-tetrahydro-N-(tetrahydro-2-oxo-3-thienyl)-3-isoquinolinecarboxamide, trifluoroacetate (2,5) salt**

The title compound (0.056 g, 57%) was prepared as a mixture of isomers using Compound A (0.40 g, 0.42 mmol) in the method described in Example 32G.

(Isomer "A")

| Elemental analysis for $C_{22}H_{32}N_4O_3S_2$ 1.71 water ·2.50 trifluoroacetic acid | | | |
|---|---|---|---|
| | C | H | N |
| Calculated: | 41.55 | 4.90 | 7.18 |
| Found: | 41.55 | 4.60 | 7.18 |

TLC: $R_f$ 0.5

(9/1/.1,trichloromethane/methanol/acetic acid), Visualized by UV, PMA

HPLC $R_t$ 13.12 minutes (96%), 10-90% aqueous methanol with 0.2 % phosphoric acid buffer, 30 minutes gradient, 1.5 mL/minute, 220 nm, $C_{18}$ (6.0 x 150 mm) column

IR: (KBr) 2928, 1684, 1643, 1206, 1140. $cm^{-1}$

MS: $[M+H]^+$ $465^+$

(Isomer "B")

| Elemental analysis for $C_{22}H_{32}N_4O_3S_2$ 1.40 $H_2O$. 2.20 trifluoroacetic acid | | | |
|---|---|---|---|
| | C | H | N |
| Calculated: | 42.81 | 5.03 | 7.56 |
| Found: | 42.64 | 4.81 | 7.85 |

TLC:$R_f$ 0.4 (6/3/1 n-propanol/ammonium hydroxide/water, visualized by UV, PMA) HPLC $R_t$ 14.0 minutes (99%), 10-90% aqueous methanol with 0.2% phosphoric acid buffer, 30 minutes gradient, 1.5 mL/minute, $C_{18}$ (6.0 x 150 mm) column.
IR (KBr) 2971, 1682, 1645, 1206, 1136, 723 $cm^{-1}$.
MS: $[M+H]^+$ $465^+$

**Example 36**

**(S*,R*)-N²-[[2-[N-(2-Amino-3-mercaptopropyl)-L-valyl]-1,2,3,4-tetrahydro-3-isoquinolinyl]carbonyl]-L-asparagine, trifluoroacetate (1:2) salt**

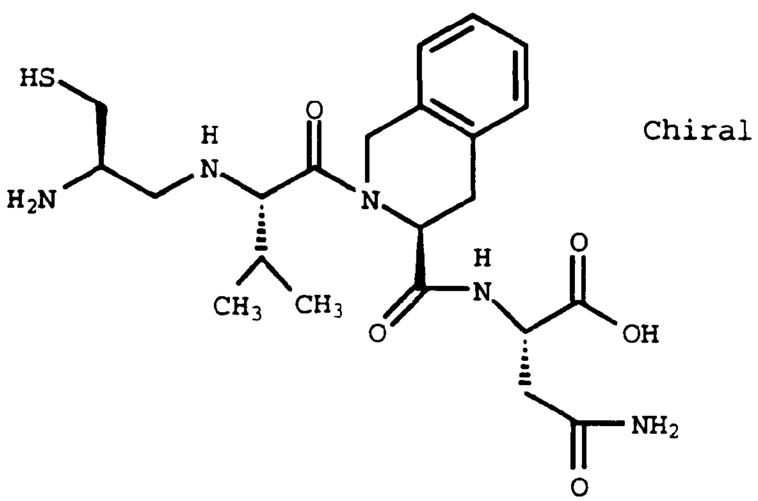

**A. (S*,R*)-N²-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-L-asparagine, 1,1-dimethylethyl ester**

Compound A (1.4 g, 93%) was prepared as a white solid using Compound 32E (1.25 g, 1.5 mmol) and L-asparagine t-butyl ester (0.282 g, 1.5 mmol) in the method described in Example 32F.
mp: 88-96°C
TLC: $R_f$ : = 0.34 (2/1 hexane/acetone, visualization by UV)
Ms: $(M+H)^+$ 1012

**B. (S*,R*)-N²-[[2-[N-(2-Amino-3-mercaptopropyl)-L-valyl]-1,2,3,4-tetrahydro-isoquinolinyl]carbonyl]-L-asparagine, trifluoroacetate (1:2) salt**

The title compound (0.14 g, 18%) was prepared as a white lyophilate using Compound A (1.14 g, 1.13 mmol) in the method described in Example 32G.
mp: 95-110°C
TLC: $R_f$ : = 0.50 (6/3/1 n-propanol/ammonium hydroxide/water, visualization by ceric ammonium sulfate)
MS: $(M+H)^+$ 480, free amine
IR: (KBr), 1204, 1674, 3424 $cm^{-1}$

$^1H$ ($CD_3OD$): 1.02, 1.04, 1.15 (d, 6 H, J = 6.84,7.27), 1.98, 2.20 (br m, 1 H), 2.51-2.89 (m, 5 H), 3.08-3.51 (m, 5H), 4.65-4.89 (m, 4 H), 7.22-7.24 (m, 4 H)
$[\alpha]_D$ = - 16.1° ( c = 0.54, methanol)

**Example 37**

**(S* ,R*)-N-[[2-[(2-Amino-3-mercaptopropyl)-L-valyl]-1,2,3, 4-tetrahydro-3-isoquinolinyl]carbonyl]-L-norleucine, trifluoroacetate (1:2) salt**

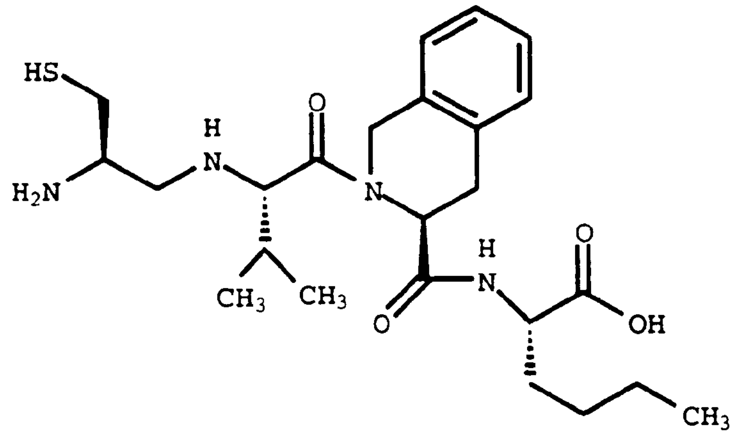

**A. (S*,R*)-N-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-L-norleucine, methyl ester**

Compound A (1.265 g, 79%) was prepared as a white solid using Compound 32E (1.39 g, 1.65 mmol), norleucine methyl ester hydrochloride (0.3 g, 1.65 mmol) and benzotriazol-1-yloxytris-(dimethylamino)phosphonium hexafluorophosphate (0.73 g, 1.65 mmol) in acetonitrile (9 mL) and dimethylformamide (3 mL) in the method described in Example 32F.
mp: 58-68°C
TLC: $R_f$ : 0.37 (4/1 hexane/acetone, visualization by UV)
MS: $(M+H)^+$ 969

**B. (S*,R*)-N-[[2-[N-[2-[[(1,1-Dimethylethoxy)carbonyl]amino]-3- (triphenylmethyl)thio]propyl]-N-(phenylmethoxy)carbonyl]-L-valyl]-1,2,3,4-tetrahydro-3-isoquinolinyl]carbonyl]-L-norleucine**

Compound B (1.1 g, 98%) was prepared as a hygroscopic solid using Compound A (1.14 g, 1.18 mmol) in the method described in Example 32C. mp: 86-94°C
TLC: $R_f$ = 0.53 (9/1/0.05 chloroform/methanol/acetic acid, visualization by ceric ammonium sulfate/ammonium molybdate/sulfuric acid/water)
MS: $(M+H)^+$ 955

**C. (S*,R*)-N-[[2-[(2-Amino-3-mercaptopropyl)-L-valyl]-1,2,3,4-tetrahydro-3-isoquinolinyl]-carbonyl]-L-norleucine, trifluoroacetate (1:2) salt**

The title compound (0.248 g, 33%) was prepared as a white lyophilate using Compound B (1.0 g, 1.05 mmol) in the method described in Example 32G.
mp: 80-90°C
TLC: $R_f$ : = 0.69 (6/3/1 n-propanol/ammonium hydroxide/water, visualization by ceric ammonium sulfate)
MS: $(M+H)^+$ 479, free amine
IR: (KBr), 1674, 3435 $cm^{-1}$

Elemental Analysis for

$C_{28}H_{40}N_4F_6S$ . 1.29 $H_2O$

| | C | H | N | S | F |
|---|---|---|---|---|---|
| Calculated: | 46.07 | 5.88 | 7.68 | 4.39 | 15.62 |
| Found: | 46.39 | 5.82 | 7.36 | 4.45 | 15.53 |

$^1H$ ($CD_3OD$): 0.83-1.31 (m, 13 H), 1.49-1.82 (m, 4 H), 2.10, 2.21 (br m, 1 H), 2.75-3.77 (m, 5 H), 4.06-4.34 (m, 2 H), 4.51-4.89 (m, 3 H), 7.22-7.29 (m 4 H).
$[\alpha]_D$ = - 27.7° ( c = 0.43, methanol).

**Example 38**

**(S*,R*)-N-[(2-[N-(2-Amino-3-mercaptopropyl)-L-valyl]-1,2,3,4-tetrahydro-3-isoquinolinyl]carbonyl]-γ-(aminosulphonyl)-L-α-aminobutyric acid, trifluoroacetate (2:5) salt**

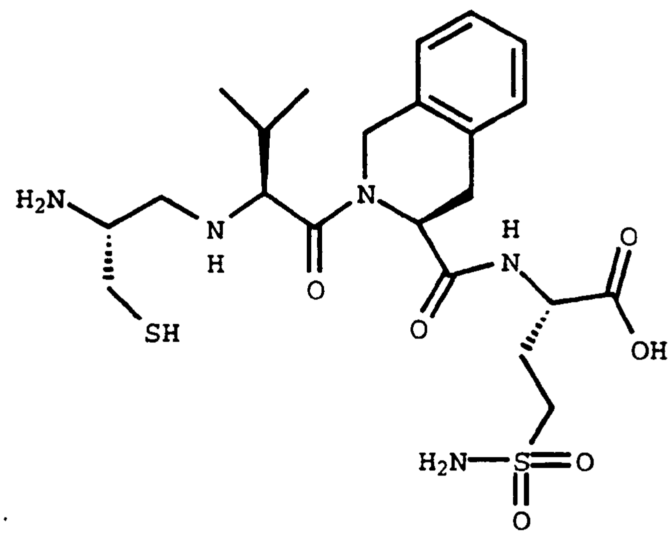

**A. (S)-4-(Aminosulfonyl)-2-aminobutyric acid, methyl ester, hydrochloride**

Hydrochloric acid gas is bubbled in a solution of (S)-4-(Aminosulfonyl)-2-amino-butyric acid (0.50 g, 2.7 mmol) in methanol (100 mL) at room temperature. The reaction mixture was stirred for 13 hours, and concentrated. The residue was triturated with ether to afford Compound A as a solid (0.55 g, 100 %).

**B. (S*,R*)-γ-(Aminosulfonyl)-N-[[[2-[N-[2-[[(1,1-dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl] -L-α-aminobutyric acid, methyl ester**

Compound B (0.428 g, 68%) was prepared using Compound A (0.123 g, 0.61 mmol) and Compound 32E (0.51 g, 0.61 mmol) in the method described in Example 32F.

| Elemental analysis calculated for $C_{55}H_{65}N_5O_{10}S_2$ 2.73 $H_2O$ | | | |
|---|---|---|---|
| | C | H | N |
| Calculated: | 61.77 | 6.64 | 6.55 |
| Found: | 61.72 | 6.11 | 6.52 |

**C. (S*,R*)-γ-(Aminosulfonyl)-N-[[2-[N-[2-[[(1,1-dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-L-α-aminobutyric acid**

Sodium hydroxide (1N, 0.47 mL, 0.018 g, 0.47 mmol) was added to a solution of Compound B (0.40 g, 0.39 mmol) in methanol (2 mL). After stirring for 13 hours at room temperature, the reaction was quenched with 10 % hydrochloric acid (10 mL) and ethyl acetate (20 mL). The layers were separated and the aqueous layer was extracted with ethyl acetate (2 x 20 mL). The combined organic layers were washed with 10% hydrochloric acid (1 x 10 mL) and brine (1 x 20 mL). The organic layer was dried over magnesium sulfate, filtered and concentrated to afford the acid. The product was purified by prep HPLC (YMC S-10, ODS, 30 x 500 mm, C18 column) eluting with 10-90% aqueous methanol containing 0.1% trifluoroacetic acid, gradient over 30 minutes. The fractions with product were concentrated, and lyophilized to afford the acid Compound C (0.065 g).

**D. (S*,R*)-N-[(2-[N-(2-Amino-3-mercaptopropyl)-L-valyl]-1,2,3,4-tetrahydro-3-isoquinolinyl]carbonyl]-γ-(aminosulphonyl)-L-α-amino-butyric acid, trifluoroacetate (2:5) salt**

The title compound (0.0329 g, 63%) was prepared using Compound C (0.100 g, 0.095 mmol) in the method described in Example 32G.

| Elemental analysis for $C_{22}H_{35}N_5O_6S_2$ 0.75 $H_2O$. 2.5 TFA | | | |
|---|---|---|---|
| | C | H | N |
| Calculated: | 39.16 | 4.75 | 8.46 |
| Found: | 39.15 | 5.00 | 8.36 |

$R_f$ = 0.6 (6/3/1 n-propanol/ammonium hydroxide/water), Visualized by UV, PMA HPLC $R_t$ 11.05 minutes (97%), 10-90% aqueous methanol with 0.2 % phosphoric acid buffer, 30 minutes gradient, 1.5 mL/minutes, 220 nm, $C_{18}$ (6.0 x 150 mm ) column
IR: (KBr) 3439, 1678, 1653, 1437, 1206, 1142, 723 $cm^{-1}$
MS: $[M+H]^+ 530^+$
$[\alpha]_D$ = -16.3° (c = 0.19, methanol)

**Example 39**

**(S*,R*)-N-[[2[N-(2-Amino-3-mercaptopropyl)-L-valyl]-1,2,3,4-tetrahydro-3-isoquinolinyl]carbonyl]-L-glutamic acid, trifluoroacetate salt**

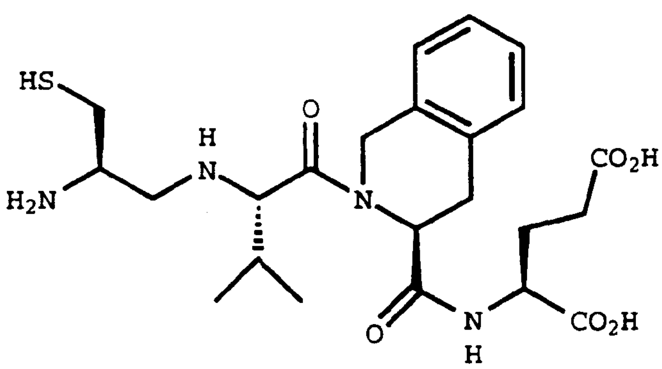

**A. L-glutamic acid, 1,1-dimethylethyl ester**

Aqueous sodium bicarbonate (10%, 150 mL) was added to a slurry of N-[(Phenylmethoxy)carbonyl]-L-glutamic acid, 1,1-dimethylethyl ester, dicyclohexylamine salt (2.3 g, 4.43 mmol) in ether (50 mL). The aqueous

layer was acidified with 3N hydrochloric acid (pH = 2) and extracted with ethyl acetate (3 x 50 mL). The combined organic extracts from the acid wash were concentrated under vacuum. The residue was dissolved in methanol (10 mL), treated with 10% palladium hydroxide/C (150 mg) and stirred under a hydrogen atmosphere at room temperature for 16 hours. The reaction was filtered and the filtrate concentrated under vacuum to afford Compound A (0.700 g, 78%) which was used without further purification.
MS: $(M+H)^+$ 204
$^1H(CD_3OD)$: 1.52 (s, 9 H), 2.00-2.43 (m, 2 H), 2.41 (t, 2 H, J = 6.41), 3.87-3.90 (m, 1 H)

**B. (S*,R*)-N-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-L-glutamic acid, 1,1-dimethylethyl ester**

1-(3-Dimethylaminopropyl)-3-ethylcarbodiimide hydrochloride (0.228 g, 1.19 mmol) was added to a solution of Compound 32E (1.0 g, 1.19 mmol) and N-hydroxysuccinimide (0.137 g, 1.19 mmol) in dimethylformamide (10 mL) and was stirred for 1 hour at 0°C. Sodium bicarbonate (0.084 g, 1.0 mmol) was added to Compound A (0.201 g, 1.0 mmol) in water (1 mL) and the solution was added to the activated ester. The mixture was then stirred at room temperature for 16 hours. The reaction was quenched with 1N hydrochloric acid (50 mL), and extracted with ethyl acetate (3 x 50 mL). The combined organic extracts were washed with 10% lithium chloride (3 x 100 mL), dried (magnesium sulfate), filtered and concentrated under vacuum. The residue was purified by flash chromatography (eluting with 1/1 hexane/acetone) to afford Compound B (0.55 g, 54%) as a white solid.
mp: 78-88°C
TLC: $R_f$ : 0.68 (1/1 hexane/acetone, visualization by UV)
MS: $(M+H)^+$ 1027

**C. (S*,R*)-N-[[2[N-(2-Amino-3-mercaptopropyl)-L-valyl]-1,2,3,4-tetrahydro-3-isoquinolinyl]-carbonyl]-L-glutamic acid, trifluoroacetate salt**

The title compound (0.123 g, 35%) was prepared using Compound B (0.5 g, 0.487 mmol) in the method described in Example 32G.
mp: 115-125°C
TLC: $R_f$ : = 0.40 (6/3/1 n-propanol/ammonium hydroxide/water, visualization by ceric ammonium sulfate)
MS: $(M+H)^+$ 495, free amine
IR: (KBr), 1431, 1672, 2976, 3428 $cm^{-1}$

| Elemental Analysis for $C_{23}H_{34}N_4O_6S$ . 1.4 $H_2O$ . 1.8 TFA | | | | | |
|---|---|---|---|---|---|
| | C | H | N | S | E |
| Calculated: | 44.06 | 5.37 | 7.73 | 4.42 | 14.15 |
| Found: | 44.13 | 5.21 | 7.60 | 4.45 | 14.02 |

$^1H$ $(CD_3OD)$: 1.026-1.15 (m, 6 H), 1.89-2.37 (m, 6 H), 2.71-3.31 (m, 6 H), 3.60, 4.05, 4.27, 4.61 (br m, 2 H), 4.67-4.91 (m, 3 H), 7.22-7.27 (m, 4 H)
$[\alpha]_D$ = - 41.8° ( c = 0.33, methanol)

**Example 40**

**(S*,R*)-N2-[[2-[N-(2-Amino-3-mercaptopropyl)-L-valyl]-1,2,3,4-tetrahydro-3-isoquinolinyl]carbonyl]-N5-hydroxy-L-glutamine, trifluoroacetate (1:2) salt**

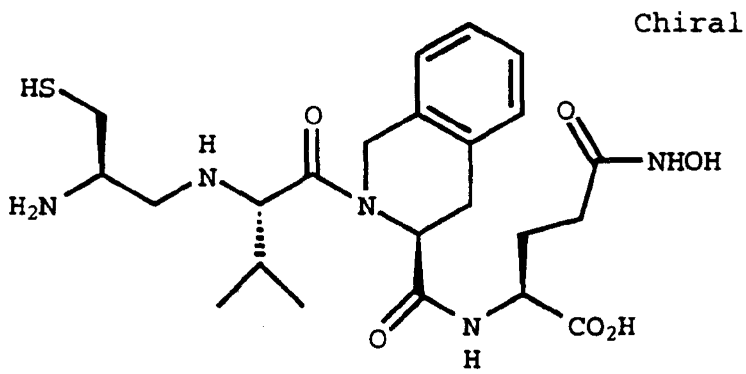

**A. (S*,R*)-N2-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-N5-(tetrahydropyran-2-yloxy)-L-glutamine,1,1-dimethylethyl ester**

Compound A (0.50 g, 91%) was prepared as a white solid using Compound 39B (0.5 g, 0.487 mmol) and Compound 20A (0.057 g, 0.487 mmol) in the method described in Example 32F.
mp: 70-80°C
TLC: $R_f$ : 0.75 (1/1 hexane/acetone, visualization by UV)
MS: $(M+H)^+$ 1126.5

**B. (S*,R*)-N2-[[2-[N-(2-Amino-3-mercaptopropyl)-L-valyl]-1,2,3,4-tetrahydro-3-isoquinolinyl]-carbonyl]-N5-hydroxy-L-glutamine, trifluoroacetate (1:2) salt**

The title compound (0.050 g, 16%) was prepared as a white lyophilate using Compound A (0.471 g, 0.419 mmol) in the method described in Example 32G.
mp: decomposition above 95°C
TLC: $R_f$ : = 0.52 (6/3/1 n-propanol/ammonium hydroxide/water, visualization by ceric ammonium sulfate)
MS: $(M+H)^+$ 510, free amine
IR: (KBr), 1204, 1676, 2976, 3430 $cm^{-1}$

| Elemental Analysis for $C_{23}H_{35}N_5O_6S$ . 1.0 $H_2O$ . 2.15 TFA | | | | | |
|---|---|---|---|---|---|
| | C | H | N | S | E |
| Calculated: | 42.43 | 5.11 | 9.06 | 4.15 | 15.86 |
| Found: | 42.46 | 4.96 | 8.99 | 4.41 | 15.85 |

$^1H$ ($CD_3OD$): 1.07-1.25 (m, 6 H), 1.97-2.30 (m, 7 H), 2.75-3.40 (m, 5 H), 3.80, 3.95, 4.40 (br m, 2 H), 4.60-5.00 (m, 3 H), 7.22-7.35 (m, 4 H).
$[\alpha]_D$ = - 23.2° ( c = 0.22, methanol).

**Example 41**

**[R-(R*,S*)]-N-[[2-[N-(2-Amino-3-mercaptopropyl)-L-valyl]-1,2,3,4-tetrahydro-3-isoquinolinyl]carbonyl]-L-methioninamide, trifluoroacetate (1:2) salt**

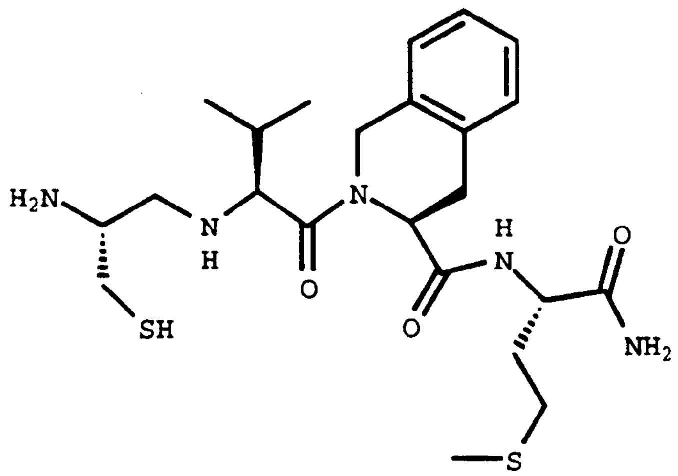

**A. (S*,R*)-$N^2$-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-L-methioninamide**

Compound A (633 mg, 65%) was prepared using Compound 32E (841 mg, 1.00 mmol) and L-methioninamide hydrochloride (185 mg, 1.00 mmol) in the method described in Example 32F.

**B. [R-(R*,S*))-N-[[2-[N-(2-Amino-3-mercaptopropyl)-L-valyl]-1,2,3,4-tetrahydro-3-isoquinolinyl]carbonyl]-L-methioninamide, trifluoroacetate (1:2) salt**

The title compound (0.13 g, 45%) was prepared using Compound A (0.579 g, 0.596 mmol) in the method described in Example 32G.

| Elemental analysis for $C_{23}H_{37}N_5O_3S_2$ 1.30 $H_2O$. 2.0 TFA | | | |
|---|---|---|---|
| | C | H | N |
| Calculated: | 43.39 | 5.61 | 9.37 |
| Found: | 43.39 | 5.64 | 9.44 |

TLC: $R_f$ 0.5 (6:3:1, n-propanol/ammonium hydroxide/water), Visualized by UV, PMA HPLC $R_t$ 14.1 minutes (96%), 10-90% aqueous methanol with 0.2% phosphoric acid buffer, 30 minutes gradient, 1.5 mL/minutes, 220 nm, $C_{18}$ (6.0 x 150 mm) column.
IR: (KBr) 3435, 2972, 1678, 1435, 1206, 1140, 841, 723 $cm^{-1}$
MS: $[M+H]^+$ $496^+$
$[\alpha]_D$ = -23.6° (c = 0.44, methanol)

**Example 42**

**N-[[(S)-2-[N-[(R)-2-Amino-3-mercaptopropyl)-L-valyl]-1,2,3,4-tetrahydro-3-isoquinolinyl]carbonyl]-2-methyl-DL-methionine, trifluoroacetate (1:2) salt**

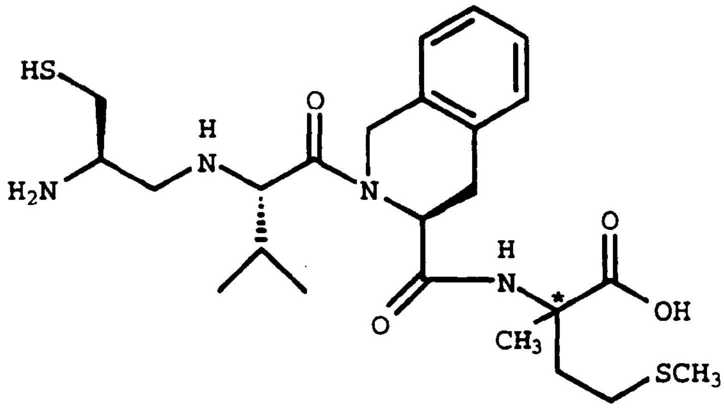

**A. 2-Methyl-DL-methionine, methyl ester**

A solution of D,L-α-methylmethionine (0.500 g, 3.06 mmol) in 20 mL of methanol was saturated with hydrochloric acid (g), stirred 48 hours at room temperature, and then heated at reflux for 12 hours. The mixture was concentrated in vacuo. The residue was disolved in 50 mL of dichloromethane and washed with 10% sodium bicarbonate (50 mL). The organic layer was dried (sodium sulfate), filtered and concentrated under vacuum to afford 320 mg (59%) of Compound A.

**B. N-[[(S)-2-[N-[(R)-2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-DL-methionine, methyl ester**

Compound B (0.766 g, 77%) was prepared as a white solid using Compound 32E (0.841 g, 1.00 mmol) and Compound A (0.195 g, 1.10 mmol) in the method described in Example 32F.

**C. N-[[(S)-2-[N-[(R)-2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-DL-methionine**

Compound C (0.716 g, 98%) was prepared using Compound B (0.74 g, 0.740 mmol) in the method described in Example 32C.

**D. N-[[(S)-2-[N-[(R)-2-Amino-3-mercaptopropyl]-L-valyl]-1,2,3,4-tetrahydro-3-isoquinolinyl]carbonyl]-2-methyl-DL-methionine, trifluoroacetate (1:2) salt**

The title compound (0.095 g, 29%) was prepared as a white lyophilate using Compound C (0.400 g, 0.406 mmol) in the method described in Example 32G.
mp: 95-110°C
MS: $(M+H)^+$ 511

## Example 43

**(S*, R*)-N-[[2-[N-(2-Amino-3-mercaptopropyl)-L-alanyl]-1,2,3,4-tetrahydro-3-isoquinolinyl]carbonyl]-L-methionine**

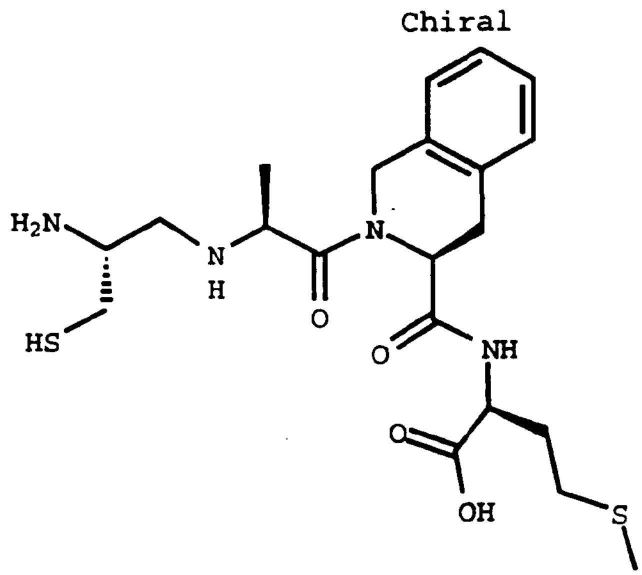

**A. (S*,R*)-N-[[2-[N-[2-[[(1,1-Dimethylethoxy)carbonyl]amino]-3-[(triphenylmethyl)thio]propyl]-L-alanyl]-1,2,3,4-tetrahydro-3-isoquinolinyl]carbonyl]-L-methionine, methyl ester**

Compound A was prepared as a clear oil (700 mg, 28%) using Compound 7A (1.50 g, 3.04 mmol) and Compound 1F (2.10 g, 4.74 mmol) in the two step procedure described in Example 7B.

**B. (S*,R*)-N-[[2-[N-(2-Amino-3-mercaptopropyl)-L-alanyl]-1,2,3,4-tetrahydro-3-isoquinolinyl]carbonyl]-L-methionine**

The title compound (96 mg, 15%) was prepared using Compound A (700 mg, 0.85 mmol) in the two step procedure described in Example 1H.
m.p. 89-90 °C, Purity > 98%, $[\alpha]_D^{25}$ = - 32.1° (c=1.0, methanol), MS $(M+H)^+$ $469^+$, Analysis: calculated for 1.90 trifluoroacetic acid · 3.17 water: C, 40.12; H, 5.46; N, 7.55; F, 14.59; Found: C, 40.12; H, 5.05; N, 7.31; F,14.24
$^1$H-NMR ($CD_3OD$, 270 MHz) d (ppm) 7.16 (4H, m), 4.71 (2H, m), 4.57 (2H, m), 4.43 (1H, m), 4.32 (1H, m), 3.98 (1H, m), 3.56 (1H, m), 3.33-3.02 (2H, m), 2.84 (2H, m), 2.37 (1H, m), 1.98 (2H, m), 1.89 (1H, m), 2.10-1.76 (3H, m), 1.57 (2H, m), 1.43 (1H, m)

**Example 44**

**(S*,R*)-N-[[2-[N-(2-Amino-3-mercaptopropyl)-L-leucyl]-1,2,3,4-tetrahydro-3-isoquinolinyl]carbonyl]-L-methionine, trifluoroaceate (1:2) salt**

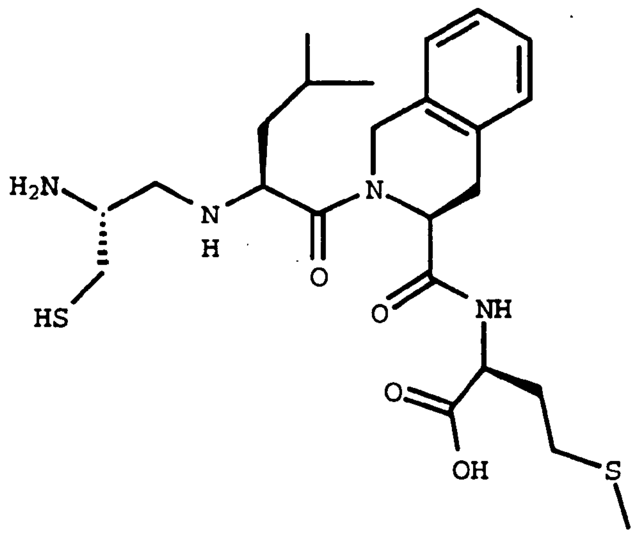

**A. (R*)-N-[[2-[N-[(1,1-Dimethylethoxy)carbonyl]-L-leucyl]-1,2,3,4-tetrahydro-3-isoquinolinyl]carbonyl]-L-methionine, methyl ester**

N-t-Butyloxycarbonyl-L-leucine (1.0 g, 4.01 mmol) and Compound 1B (hydrochloride, 1.43 g, 4.01 mmol) were dissolved in dichloromethane (10 mL). Bromo-tris-pyrrolidino-phosphonium hexafluorophosphate (2.10 g, 4.41 mmol) and 4-dimethylaminopyridine (100 mg, 0.815 mmol) were added. N,N-Diisopropylethylamine (1.49 g, 2.0 mL, 11.5 mmol) was added dropwise. The reaction mixture was stirred for 2 hours at room temperature, concentrated and chromatographed (silica gel, 5.0 X 15 cm, 40% ethyl acetate, 60% hexane). Fractions containing the desired compound were collected and concentrated to yield Compound A as a clear oil (2.0 g, 93 %).
$(M+H)^+$ $536^+$

**B. (S*,R*)-N-[[2-[N-[2-[[(1,1-Dimethylethoxy)carbonyl]amino]-3-[(triphenylmethyl)thio]propyl]-L-leucyl]-1,2,3,4-tetrahydro-3-isoquinolinyl]carbonyl]-L-methionine, methyl ester**

Compound B (600 mg, 25%) was prepared as a clear oil using Compound A (1.5 g, 2.81 mmol) and Compound 1F (1.43 g, 2.81 mmol) in the two step procedure described in Example 7B.
$(M+H)^+$ $867^+$

**C. (S*,R*)-N-[[2-[N-(2-Amino-3-mercaptopropyl)-L-leucyl]-1,2,3,4-tetrahydro-3-isoquinolinyl]carbonyl]-L-methionine, trifluoroaceate (1:2) salt**

The title compound (200 mg, 38%) was prepared using Compound B (600 mg, 0.693 mmol) in the two step procedure described in Example 1H.
M.P. 85-86°C, Purity > 98.4 %,
$[\alpha]_D^{25}$ = -35.3° (c=0.15, methanol),
MS $(M+H)^+$ $511^+$, Analysis: calculated for 1.95 trifluoroacetic acid · 0.95 water:
C, 44.67; H, 5.62; N, 7.47
Found: C, 44.36; H, 5.75; N, 8.09
$^1$H-NMR ($CD_3OD$, 400 MHz) d (ppm) 7.26 (4H, m), 4.68 (2H, m), 4.51 (1H, m), 4.40 (1H, m), 4.24 (1H, m), 3.62 (1H, m), 3.34 (2H, m), 3.10 (2H, m), 2.82 (2H, m), 2.46 (2H, m), 2.06 (3H, m), 1.90 (2H, m), 1.72 (1H, m), 1.62 (1H, m), 1.46 (1H, m), 1.03 (6H, m)

**Example 45**

**[R*[R*(S*)]]-N-[[2-[2-[(2-Amino-3-mercaptopropyl)amino]-1-oxobutyl]-1,2,3,4-tetrahydro-3-isoquinolinyl]carbonyl]-L-methionine, trifluoroacetate (1:1) salt**

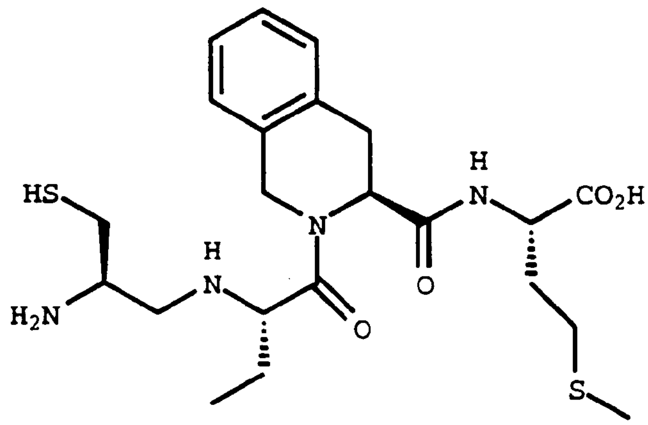

contains 1.5 H2O

**A. (S)-2-[[(1,1-Dimethylethoxy)carbonyl]amino]butyric acid**

A mixture of (S)-(+)-2-aminobutyric acid (5.0 g, 48.5 mmol), sodium hydroxide pellets (2.0 g, 50 mmol), water (5 mL), t-butanol (20 mL), and di-t-butyldicarbonate (11.0 g, 50 mmol) was stirred at 25°C for 18 hours. The mixture was then poured into water and washed with ether. The aqueous layer was acidified by addition of dilute hydrochloric acid and was extracted with ethyl acetate (2x). The extract was dried and concentrated to give Compound A as a colorless oil (10.3 g, >100%), which was used without further purification.

**B. (R*,R*)-N-[[2-[2-[[(1,1-Dimethylethoxy)carbonyl]amino]-1-oxobutyl]-1,2,3,4-tetrahydro-3-isoquinolinyl]carbonyl]-L-methionine, methyl ester**

A mixture of Compound A (550 mg, 2.7 mmol), Compound 1B hydrochloride (895 mg, 2.5 mmol), diisopropylethyl amine (0.70 mL, 4 mmol), 4-dimethylaminopyridine (61 mg, 0.5 mmol) and bromotris-pyrrolidino-phosphonium hexafluorophosphate (1.86 g, 4 mmol) in methylene chloride (10 mL) was stirred at 25°C for 20 hours. The mixture was then poured into 1N hydrochloric acid solution and extracted with ethyl acetate. The extract was washed with saturated aqueous sodium bicarbonate solution and brine, dried, and concentrated *in vacuo.* The residue was purified by flash chromatography on silica gel (300 g, EM), eluting with hexane:ethyl acetate (1:1) to give Compound B as a clear oil (600 mg, 47%).

**C. [R*[R*(S*)]]-N-[[2-[2-[[2-[[(1,1-Dimethylethoxy)carbonyl]amino]-3-[(triphenylmethyl)thio]propyl]amino]-1-oxobutyl]-1,2,3,4-tetrahydro-3-isoquinolinyl]carbonyl]-L-methionine, methyl ester**

A solution of Compound B (600 mg, 1.18 mmol) in acetic acid saturated with hydrogen chloride (10 mL) was stirred at 25°C for one hour, after which it was concentrated *in vacuo* to give Compound B (450 mg, 94%). To a solution of this material (443 mg, 1.0 mmol) in methanol (2 mL) was added Compound 1F (445 mg, 1.0 mmol). After 30 minutes, acetic acid (0.2 mL) and sodium cyanoborohydride (63 mg, 1.0 mmol) were added, and the resulting mixture was stirred at 25°C for 48 hours. The mixture was then poured into excess 1N hydrochloric acid and extracted with ethyl acetate. The aqueous layer was made basic by addition of sodium bicarbonate and was extracted again with ethyl acetate. The extracts were combined, dried and concentrated *in vacuo.* The residue was purified by flash chromatography on silica gel (60 g, EM), eluting with hexane:ethyl acetate (1:1), to give a high $R_f$ product (BOC-trityl-cysteinol), followed by Compound C (100 mg, 12%).

**D. [R*[R*(S*)]]-N-[[2-[2-[(2-Amino-3-mercaptopropyl)amino]-1-oxobutyl]-1,2,3,4-tetrahydro-3-isoquinolinyl]carbonyl]-L-methionine, trifluoroacetate (1:1) salt**

A mixture of Compound C (100 mg), sodium hydroxide (1 mL of 1N aqueous solution), tetrahydrofuran (2

mL) and methanol (2 mL) was stirred at 25°C for 2 hours, after which it was poured into excess 1N hydrochloric acid and extracted with ethyl acetate. The extract was dried and concentrated to a glass (90 mg). The glass was dissolved in trifluoroacetic acid (2 mL), and triethylsilane (0.2 mL) was added. The mixture was stirred for one hour at 25°C, after which it was concentrated *in vacuo.* The residue was purified by preparative HPLC (YMC S-10 ODS column, 30 x 500 mm, eluting with 50 mL/minute of a linear gradient from 10% to 90% aqueous methanol containing 0.1% trifluoroacetic acid over 30 minutes). Fractions were monitored by UV absorbance at 220 nm. Those containing the first major product to elute were combined and concentrated. The residue was lyophilized to give the title compound as a hygroscopic white solid (30 mg, 52%).

mp: 85-95°C

$[\alpha]_D$: -31° (c = 0.1, ethanol)

IR (KBr): 3435, 2924, 1674, 1431 $cm^{-1}$

MS: $483^+$ $(M+H)^+$

$^1H$ NMR: ($CD_3OD$) 1.1 (6H, m), 1.97 & 2.07 (3H, s (rotamers)), 1.8-4.9 (20H. m), 7.25 (4H, m).

TLC: $R_f$ 0.15, EM silica gel, 2:1 ethyl acetate : [pyridine 20:acetic acid 6:water 11], UV + PMA stain

HPLC: > 90% at 14.9 minutes (YMC S3 ODS column, 6.0 x 150 mm, eluted with a 30 minutes linear gradient from 10% to 90% aqueous methanol containing 0.2% phosphoric acid, monitored at 220 nm)

Analysis: Calculated for $C_{22}H_{34}N_4O_4S_2$ . 1.90 trifluoroacetic acid . 1.5 water:

Calculated: C = 42.66 H = 5.40, N = 7.71, S = 8.83, F = 14.91

Found: C = 42.81, H = 5.32, N = 7.77, S = 8.99, F = 14.81

**Example 46**

**(S*,R*)-N-[[2-[2-[(2-Amino-3-mercaptopropyl)amino]-4-methylpentyl]-1,2,3,4-tetrahydro-3-isoquinolinyl]carbonyl]-L-methionine, trifluoroacetate (2:3) salt**

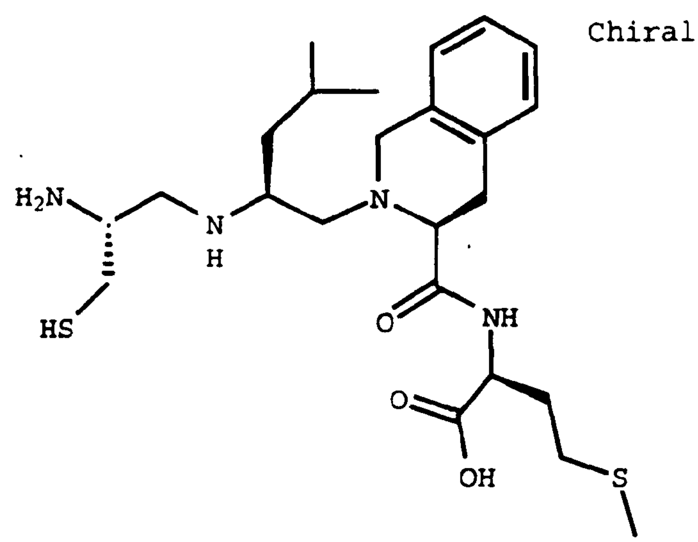

**A. $N^2$-[(1,1-Dimethylethoxy)carbonyl]-N-methoxy-N-methyl-L-leucinamide**

N-t-Butyloxycarbonyl-L-leucine (3.0 g, 12.05 mmol) and N,O-Dimethylhydroxylamine hydrochloride (1.3 g, 13.25 mmol) were dissolved in dichloromethane (100 mL). Bromo-tris-pyrrolidinophosphonium hexafluorophosphate (6.18 g, 13.25 mmol) and 4-dimethylaminopyridine (300 mg, 2.45 mmol) were added. N,N-Diisopropylethylamine (3.71 g, 5.0 mL, 28.65 mmol) was added dropwise. The reaction mixture was stirred for 4 hours at room temperature, concentrated and chromatographed (silica gel, 5.0 X 15 cm, 40% ethyl acetate, 60% hexane). Fractions containing the desired compound were collected and concentrated to yield Compound A as a slightly yellow oil (2.8 g, 80 %).

$(M + H)^+ 275^+$

**B. (1-Formyl-3-methylbutyl)carbamic acid, 1,1-dimethylethyl ester**

Compound A (2.8 g, 9.61 mmol) was used in the method described in Example 1F to prepare Compound

B as a clear oil (2.1 g, 99%) which was used immediately in the next step without further purification.

**C. (R*,R*)-N-[[2-[2-[[(1,1-Dimethylethoxy)carbonyl]amino]-4-methylpentyl]-1,2,3,4-tetrahydro-3-isoquinolinyl]carbonyl]-L-methionine, methyl ester**

Compound 1A (3.0 g, 7.11 mmol) was stirred in dimethyl sulfide (1.0 mL) and 4N hydrochloric acid in dioxane (20 mL) for 30 minutes. The mixture was concentrated, dissolved in methylene chloride (30 mL) and concentrated. This procedure was repeated five times to yield the amine as a clear glass. This amine and Compound B (2.1 g, 9.60 mmol) were dissolved in methanol (20 mL) and glacial acetic acid (0.4 mL). Sodium cyanoborohydride (447 mg, 7.11 mmol) was added portionwise over 30 minutes. The reaction mixture was stirred for 16 hours. The mixture was cooled to 0°C and saturated sodium bicarbonate (100 mL) was slowly added. The product was then extracted into ethyl acetate (100 mL). The ethyl acetate layer was washed with water (200 mL) and brine (100 mL), dried (magnesium sulfate), concentrated and chromatographed (silica gel, 4.1 X 15 cm, 25% ethyl acetate, 75% hexane). Fractions containing the desired compound were collected and concentrated to yield Compound C as a clear oil (2.3 g, 62%).
$(M+H)^+$ $522^+$

**D. [R*[R*(S*)]]-N-[[2-[2-[[2-[[(1,1-Dimethylethoxy)carbonyl]amino]-3-[(triphenylmethyl)thio]propyl]amino]-4-methylpentyl]-1,2,3,4-tetrahydro-3-isoquinolinyl]carbonyl]-L-methionine, methyl ester**

Compound D (500 mg, 24%) was prepared as a clear glass using Compound C (1.3 g, 2.49 mmol) and Compound 1F (5.1 g, 11.4 mmol) in the two step method described in Example 46C.
$(M+H)^+$ $853^+$

**E. (S*,R*)-N-[[2-[2-[(2-Amino-3-mercaptopropyl)amino]-4-methylpentyl]-1,2,3,4-tetrahydro-3-isoquinolinyl]carbonyl]-L-methionine, trifluoroacetate (2:3) salt**

The title compound (35 mg, 24%) was prepared using Compound D (500 mg, 0.587 mmol) in the two step method described in Example 1H.
M.P. 118-119°C, Purity > 98%
$[\alpha]_D^{25}$ - 29.2 ° (c=0.1,2 methanol), MS $(M+H)^+$ $497^+$
Analysis: Calculated for 2.80 trifluoroacetic acid · 3.50 water: C, 40.44; H, 5.71; N, 6.37;
Found: C, 40.47; H, 5.35; N, 6.52
H-NMR ($CD_3OD$, 400 MHz) d (ppm) 7.14 (4H, m), 4.53 (1H, m), 4.10 (2H, m), 3,87 (1H, m), 3.60 (1H, m), 3.09 (4H, m), 2.85 (4H, m), 2.42 (1H, m), 2.31 (1H, m), 2.08 (1H, m), 1.98 (3H, s), 1.92 (1H, m), 1.53 (2H, m), 1.30 (1H, m), 0.84 (6H, m)

**Example 47**

**(R)-N-[[2-[1-[(2-amino-3-mercaptopropyl)-L-prolyl]-1,2,3,4-tetrahydro-3-isoquinolinyl]carbonyl]-L-methionine, trifluoroacetate (1:2) salt**

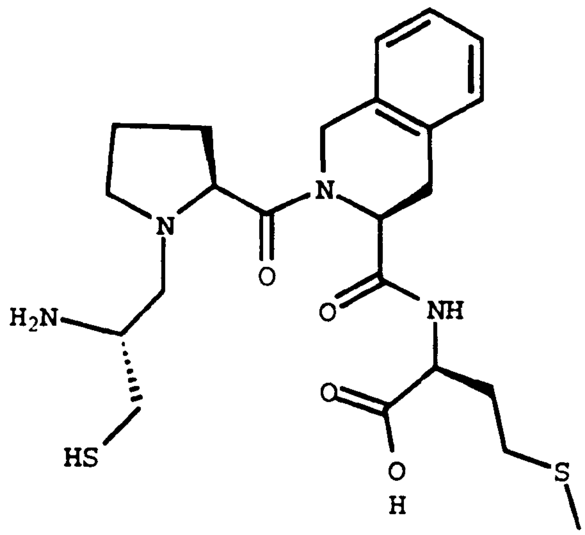

A. **(R*)-N-[[2-[1-[(1,1-Dimethylethoxy)carbonyl]-L-prolyl]-1,2,3,4-tetrahydro-3-isoquinolinyl]carbonyl]-L-methionine, methyl ester**

Compound 1B (hydrochloride, 1.88g, 4.1 mmol) was combined with N-t-butyloxycarbonyl-L-proline (0.881 g, 4.1 mmol), bromo-tris-pyrrolidinophosphonium hexafluorophosphate (1.91 g, 4.1 mmol) and diisopropylethylamine(2.1 mL, 12.3 mmol) in methylene chloride (40 mL). This was allowed to stir for 2 hours at room temperature followed by concentration under vacuum. The residue was chromatographed (silica gel, 7.1 X 20 cm, 50% ethyl acetate, 50% hexane) and fractions containing the desired compound were collected and concentrated to yield Compound A as a glass (yield; 1.88g, 72%).
MS $(M+H)^+$ $520^+$

B. **(S*,R*)-N-[[2-[1-[2-[[(1,1-Dimethylethoxy)carbonyl]amino]-3-[(triphenylmethyl)thio]propyl]-L-prolyl]-1,2,3,4-tetrahydro-3-isoquinolinyl]carbonyl]-L-methionine, methyl ester**

Compound B (5.21 mg, 25%) was prepared using Compound A (1.38g, 2.66 mmol), Compound 1F (1.28 g, 2.88 mmol) and sodium cyanoborohydride (167 mg, 2.6 mmol) in the two step procedure described in Example 7B.
$(M+H)^+$ $851^+$
rf 0.75 (chloroform, methanol acetic acid, 85:10:5)

C. **(R)-N-[[2-[1-[(2-amino-3-mercaptopropyl)-L-prolyl]-1,2,3,4-tetrahydro-3-isoquinolinyl]carbonyl]-L-methionine, trifluoroacetate (1:2) salt**

The title compound (70 mg, 15%) was prepared as a white powder using Compound B (520 mg, 0.61 mmol) in the two step procedure described in Example 1H.
$(M+H)^+$ $495^+$.
mp 69-71°C, $^1$H-NMR($CD_3OD$, 400 MHz) d (ppm) 7.15 (4H, m), 4.91 (1H, t), 4.63 (4H, m), 4.40 (1H, m), 4.20 (1H,dd), 3.85(1H, m), 3.65(4H,m), 3.42(1H,dd), 3.27(1H,m), 3.08(1H,dd), 2.80 (2H,m), 2.40(1H,m), 2.18(1H,m), 2.00(1.5H,s), 1.99(3H, m), 1.89(1.5H,s).
Elemental Analysis for $C_{23}H_{34}N_4O_4S_2{\cdot}2.35C_2HF_3O_2$, 0.65 water;
Calculated: C 42.97, H 4.90, N 7.24, S 8.28, F 17.30
Found:C 42.67, H 4.78, N 7.05, S 8.63, F 17.13
$[\alpha]_D^{25}$; -60.4° (c = 0.5, methanol).
>98% at 13.1 minutes (YMC S3-ODS column 6.0 x 150 mm 30 minutes linear gradient from 10 to 90% aqueous

methanol containing 0.2% phorphoric acid 1.5 mL/minute monitoring at 220 nm)

**Example 48**

**[R*[S*(S*)]]-N-[[2-[[1-(2-Amino-3-mercaptopropyl)-2-methyl-2-pyrrolidinyl]carbonyl]-1,2,3,4-tetrahydro-3-isoquinolinyl]carbonyl]-L-methionine, trifluoroacetate (1:2) salt**

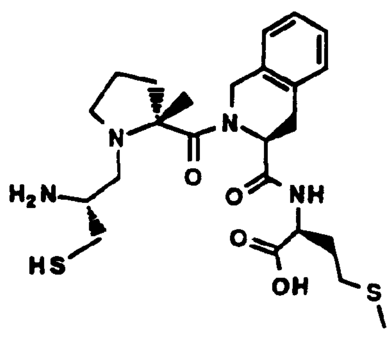

**A. (S*,R*)-N-[[2-[[1-[(1,1-Dimethylethoxy)carbonyl]-2-methyl-2-pyrrolidinyl]carbonyl]-1,2,3,4-tetrahydro-3-isoquinolinyl]carbonyl]-L-methionine, methyl ester**

(R)-2-Methyl-1,2-pyrrolidinedicarboxylic acid, 1-(1,1-dimethylethyl) ester [See D. Seeback et al., J. Am. Chem. Soc., 1983, 105, 5390] (0.27 g, 1.18 mmol) was combined with Compound 1D (1 g, 2.7 mmol), bromo-tris-pyrrolidino-phosphonium hexafluorophosphate (1.2 g, 2.6 mmol), dimethylaminopyridine (0.2 g) and diisopropylethylamine (0.8 mL, 5.2 mmol) in methylene chloride (40 mL). This was allowed to stir for 16 hours followed by concentration under vacuum. The residue was chromotagraphed (silica gel, 4.5 x 21 cm, 50% ethyl acetate, 50% hexane) and fractions containing the desired compound were collected and concentrated to yield Compound A as a glass (508 mg, 81%).
MS $(M+H)^+534^+$

**B. [R*[S*(S*)]]-N-[[2-[[1-[2-[[(1,1-Dimethylethoxy)carbonyl]amino]-3-[(triphenylmethyl)thio]propyl]-2-methyl-2-pyrrolidinyl]carbonyl]-1,2,3,4-tetrahydro-3-isoquinolinyl]carbonyl]-L-methionine**

Compound B (270 mg, 34%) was prepared using Compound A (0.5 g, 0.93 mmol) and Compound 1F (1.31 g, 2.88 mmol) in the two step procedure described in Example 7B.
MS $(M+H)^+$ $865^+$

**C. [R*[S*(S*)]]-N-[[2-[[1-(2-Amino-3-mercaptopropyl)-2-methyl-2-pyrrolidinyl]carbonyl]-1,2,3,4-tetrahydro-3-isoquinolinyl]-carbonyl]-L-methionine, trifluoroacetate (1:2) salt**

To 10 mL of methanol was added Compound B (180 mg, 0.21 mmol) along with 2N sodium hydroxide adjusted to pH 12. This was allowed to stir until there was quantitative disappearance of starting material (4 hours). The pH was adjusted to 3-4 using 1N potassium bisulfate, and ethyl acetate (30 mL) was added. The layers were separated and the aqueous layer was washed with ethyl acetate (3 x 30 mL). The organic extracts were pooled, dried (magnesium sulfate) and concentrated.

The residue (179 mg, 0.21 mmol) was combined with trifluoroacetic acid (5 mL), methylene chloride (10 mL) and triethylsilane (0.25 mL, 1.56 mmol). The solution was stirred for 1 hour, followed by concentration under vacuum. Methylene chloride was added and the solution was reconcentrated and thoroughly dried under vacuum. The residue was applied to a YMC C18 column (S-10, ODS 30 x 500 mm) with monitoring at 220 nm. HPLC purification was performed under the following conditions; Solvent A; 0.1 % trifluoroacetic acid in water, Solvent B; 0.1% trifluoroacetic acid in methanol; 20-50% B in A over 40 minutes. Fractions containing the major peak were pooled and lyophilized to yield 25 mg of the title compound as a white powder.
MS $(M+H)^+509^+$
mp 107-108°C
$^1$H-NMR($CD_3OD$, 400 MHz) δ (ppm) 7.29 (4H, m), 4.60 (4H, m), 3.40 (1H, m), 3.22 (1H, m), 3.14 (1H, m), 2.97 (1H, m), 2.97 (1H, m), 2.78 (2H, m), 2.59 (1H, m), 2.33 (2H, m), 2.21 (1H, m), 2.11 (1H, m), 2.05 (3H, s), 1.92 (2H, m), 1.80 (1H, m), 1.53 (3H, s).

| Elemental Analysis for $C_{24}H_{36}N_4O_4S_2.2.3C_2HF_3O_2$, 3 water: | | | |
|---|---|---|---|
| | C | H | N |
| Calculated: | 41.63 | 5.41 | 6.79 |
| Found: | 41.31 | 4.98 | 6.64 |

$[\alpha]_D^{25}$; + 26.3° (c = 0.38 methanol)
>99% at 13.9 minutes (YMC S3-ODS column 6.0 x 150 mm 30 minutes linear gradient from 10 to 90% aqueous methanol containing 0.2% phorphoric acid 1.5 mL/minute monitoring at 220 nm)

**Example 49**

**(S*,R*)-N-[[2-[N-(2-Amino-3-mercaptopropyl)-3-methyl-L-valyl]-1,2,3,4-tetrahydro-3-isoquinolinyl]carbonyl]-L-methionine, trifluoroacetate (1:2) salt**

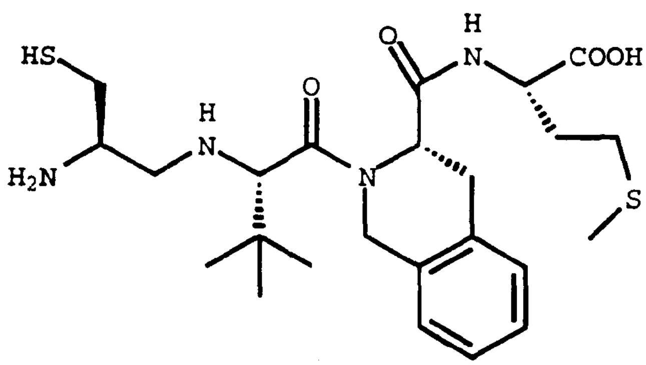

**A. N-[(1,1-Dimethylethoxy)carbonyl]-3-methyl-L-valine**

To a suspension of L-tert-leucine (1.0 gm, 7.6 mmol) in dioxane (10 mL) at 0°C was added 1N sodium hydroxide (7.6 mL, 7.6 mmol) and water (7 mL). To the resulting clear solution was added t-butoxycarbonyl-anhydride (1.86 gm, 8.5 mmol) and the cold bath was removed. After 1 hour, the reaction mixture was concentrated to half and ethyl acetate (15 mL) and 1N hydrochloric acid (10 mL) were added with vigorous stirring. The aqueous layer was separated and extracted with ethyl acetate (10 mL). The organic layers were combined, dried (magnesium sulfate), filtered and concentrated *in vacuo* to afford Compound A (2.0 g).
MS (FAB), $(M+H)^+ = 232^+$

**B. (R*)-N-[[2-[N-[(1,1-Dimethylethoxy)-carbonyl]-3-methyl-L-valyl]-1,2,3,4-tetrahydro-3-isoquinolinyl]carbonyl]-L-methionine, methyl ester**

To a solution of Compound A (0.4 g, 1.7 mmol) in dichloromethane (5 mL) at 0°C under argon was added sequentially bromo-tris-pyrrolidinophosphnium hexafluorophosphate (0.79 g, 1.7 mmol), diisopropylethylamine (0.72 mL, 5.1 mmol) and dimethylaminopropylamine (22 mg, 0.2 mmol). After 5 minutes, Compound 1B, hydrochloride (0.6 gm, 1.68 mmol) was added. The reaction was allowed to warm up to room temperature. After 3 hours, the reaction mixture was washed successively with 1N hydrochloric acid, saturated sodium bicarbonate and brine (5 mL each). Each aqueous layer was extracted with chloroform (5 mL). The organic layers were combined, dried (magnesium sulfate), filtered and concentrated. The residue was purified by flash silica gel column chromatography eluting with 30% ethyl acetate in hexanes to afford Compound B (430 mg, 48%).
MS (CI), $(M+H)^+ = 536^+$

**C. (S*,R*)-N-[[2-[N-[2-[[(1,1-Dimethylethoxy)carbonyl]amino]-3-[(triphenylmethyl)thio]propyl]-3-methyl-L-valyl]-1,2,3,4-tetrahydro-3-isoquinolinyl]carbonyl]-L-methionine, methyl ester**

Anhydrous hydrochloric acid (4M in dioxane, 1 mL, 4 mmol) and triethylsilane (0.24 mL, 1.5 mmol) were

added to solid Compound B (400 mg, 0.75 mmol) at room temperature under argon. After stirring for 2 hours, the reaction mixture was concentrated and the residue was triturated with ether and dried *in vacuo* to afford 325 mg of material (MS: $(M-H)^- = 434^-$). To a solution of this tripeptide (310 mg, 0.66 mmol) in methanol (2 mL) was added a solution of Compound 1F (0.3 g, 0.6 mmol) in methanol (1 mL) and acetic acid (0.1 mL). After 5 minutes, solid sodium cyanoborohydride (33 mg, 0.6 mmol) was added. After 1 hour, additional aldehyde (0.3 g, 0.6 mmol) in methanol (1 mL) and sodium cyanoborohydride (67 mg, 1.2 mmol) were added. After 30 minutes, aldehyde (0.3 g, 0.6 mmol) in methanol (1 mL) was added. After another 1 hour, the reaction mixture was diluted with ethyl acetate (5 mL) and ether (5 mL) and washed with saturated sodium bicarbonate (10 mL). The organic layer was washed with brine (10 mL), dried (magnesium sulfate), filtered and concentrated *in* vacuo. The residue was purified by flash silica gel column chromatography eluting with step gradient of 30%, 50% ethyl acetate in hexanes to afford a white foam Compound C (540 mg, 95%).
MS: (FAB) $(M+H)^+ = 867^+$

**D. (S*,R*)-N-[[2-[N-(2-Amino-3-mercaptopropyl)-3-methyl-L-valyl]-1,2,3,4-tetrahydro-3-isoquinolinyl]carbonyl]-L-methionine, trifluoroacetate (1:2) salt**

To a solution of Compound C (0.3 g, 0.34 mmol) in methanol (1.5 mL) at 0°C was added aqueous 1N sodium hydroxide (0.4 mL, 0.4 mmol). A precipitate formed which was dissolved by the addition of tetrahydrofuran (0.8 mL). The reaction mixture was allowed to warm to room temperature. After 1 hour, 1N hydrochloric acid (1 mL) was added and the mixture was extracted with ethyl acetate (5 mL). The organic layer was dried ($MgSO_4$), filtered and concentrated in *vacuo* to afford a white solid (0.28 g, 98%).
MS $(M-H)^- = 851^-$

To a solution of this solid (270 mg, 0.31 mmol) in dichloromethane (1.5 mL) at room temperature under argon was added triethylsilane (0.5 mL) and trifluoroacetic acid (1.5 mL). After 1.5 hours, the volatiles were removed on a rotovap and the residue was dissolved in deoxygenated 50% aqueous methanol. The precipitate formed was filtered through a nylon 0.45 μ filter and the filtrate was purified by RPHPLC (YMC, S10, C18, 30 x 500 mm column) eluting with linear gradient of 26%-66% aqueous methanol containing 0.1% trifluoroacetic acid over 1 hour. Appropriate fractions were collected, concentrated on a rotovap to remove methanol and then lyophilized to obtain the title compound (153 mg, 63%) as a white lyophilate.
m.p. 87-90°C
MS: (FAB) ; $(M+H)^+ = 511^+$
IR (KBr) : 1676, 1204 $cm^{-1}$
$[\alpha]_D$ = -27° (c= 0.46, methanol)
HPLC: YMC, S3, C18 (6 x 150 mm) column, 220 nm, 1.5 mL/minute: 0-90% aqueous methanol with 0.2% phosphoric acid, gradient over 30 minutes :
Retention time : 17.5 minutes

| Elemental analysis for $C_{24}H_{38}N_4O_4S_2$. 1.5 $H_2O$ . 1.85 trifluoroacetic acid | | | | | |
|---|---|---|---|---|---|
| | C | H | N | S | F |
| Calculated: | 44.44 | 5.77 | 7.48 | 8.57 | 14.08 |
| Found: | 44.38 | 5.47 | 7.39 | 8.50 | 14.13 |

$^1H$ NMR ($CD_3OD$, 400 MHz): d 7.35-7.20 (4H, m), 5.0-4.55 (5H, m), 3.45-3.1 (2H, m), 2.90-2.45 (5H, m), 2.07,2.03 (3H, 2 s, rotomeric), 2.2-1.95 (2H, m), 1.12, 1.06 (9H, 2 s, rotomeric)

**Example 50**

**[R*[R*(S*)]]-N-[(2-[[(2-Amino-3-mercaptopropyl)amino)phenylacetyl]-1,2,3,4-tetrahydro-3-isoquinolinyl]carbonyl]-L-methionine, trifluoroacetate (1:2) salt**

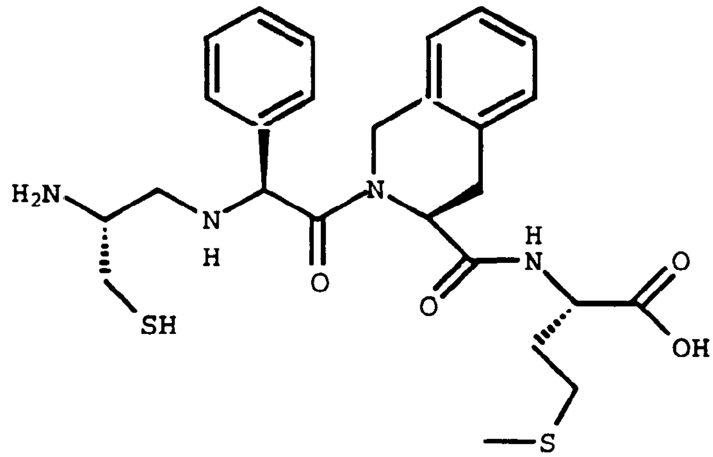

**A. α-Aminobenzeneacetic acid, methyl ester, hydrochloride**

Hydrochloric acid gas was bubbled into a solution of α-Aminobenzeneacetic acid (5.00 g, 33.0 mmol) in methanol (100 mL). After stirring for 6 days, the reaction mixture was concentrated, the residue was triturated with ether, and the solid was filtered and air dried to afford Compound A (5.8 g, 88%).

**B. [R-(R*,S*)]-α-[[2-[[(1,1-Dimethylethoxy)carbonyl]amino]-3-[(triphenylmethyl)thio]propyl]amino]benzeneacetic acid, methyl ester**

Acetic acid (0.5 mL) was added to a solution of Compound 1F (0.30 g, 0.67 mmol) and Compound A (0.144 g, 0.87 mmol) under argon at room temperature. After stirring for 30 minutes, sodium cyanoborohydride (0.050 g, 0.87 mmol) was added and the reaction mixture was stirred for 13 hours at room temperature. The reaction mixture was diluted with sodium bicarbonate (10 mL) and ethyl acetate (20 mL), and the layers were separated. The aqueous layer was extracted with ethyl acetate (2 x 20 mL). The combined organic layer was washed with brine (1 x 20 mL), dried over magnesium sulfate, filtered and concentrated. The residue was purified on a flash silica column eluting with 9/1, 8/2 hexane/ ethyl acetate to afford Compound B (0.22 g, 56%).

**C. [R-(R*,S*)]-α-[[2-[[(1,1-Dimethylethoxy)carbonyl]amino]-3-[(triphenylmethyl)thio]propyl]amino]benzeneacetic acid**

Sodium hydroxide (1N, 0.47 mL, 0.019 g, 0.47 mmol) was added to a solution of Compound B (0.22 g, 0.37 mmol) in methanol (2 mL). After stirring for 13 hours at room temperature, the reaction mixture was quenched with 10% hydrochloric acid (10 mL) and ethyl acetate (20 mL). The layers were separated and the aqueous layer was extracted with ethyl acetate (2 x 20 mL). The combined organic layers were washed with 10% hydrochloric acid (1 x 10 mL) and brine (1 x 20 mL). The organic layer was dried over magnesium sulfate, filtered and concentrated to afford the acid Compound C (0.21 g, 94%).

**D. [R*[R*(S*)]]-N-[[2-[N-[[2-[[(1,1-Dimethylethoxy)carbonyl]amino]-3-[(triphenylmethyl)thio]propyl]amino]phenylacetyl]-1,2,3,4-tetrahydro-3-isoquinolinyl]carbonyl]-L-methionine, methyl ester**

Benzotriazol-1-yloxytris-(dimethylamino)-phosphonium hexafluorophosphate hydrochloride (0.093 g, 0.36 mmol) was added to a solution of Compound C (0.21 g, 0.36 mmol), Compound 1B, hydrochloride (0.14 g, 0.40 mmol) and diisopropylethylamine (0.13 mL, 0.10 g, 0.80 mmol) in dichloromethane (2 mL) under argon. This reaction mixture was stirred for 13 hours at room temperature. The reaction mixture was diluted with 10% hydrochloric acid (10 mL) and ethyl acetate (20 mL). The aqueous layer was extracted with ethyl acetate (2 x 20 mL). The combined organic layers were washed with 10% hydrochloric acid (2 x 10 mL), water (1 x 10 mL), sodium bicarbonate (2 x 10 mL) and brine (2 x 20 mL), dried over magnesium sulfate, filted and concentrated.

The product was purified on a flash silica column eluting with 7/3, 1/1 hexane/ethyl acetate to afford Compound D (0.087 g, 28%).

E. **[R*[R*(S*)]]-N-[(2-[[(2-Amino-3-mercaptopropyl)amino)phenylacetyl]-1,2,3,4-tetrahydro-3-isoquinolinyl]carbonyl]-L-methionine, trifluoroacetate (1:2) salt**

The title compound (0.03 g) was prepared using Compound D (0.09 g, 0.10 mmol) in the two step procedure described in Example 49D.

| Elemental analysis for $C_{26}H_{34}N_4O_4S_2$ 2.5 water 2.1 trifluoroacetic acid: | | | |
|---|---|---|---|
| | C | H | N |
| Calculated: | 44.50 | 5.08 | 6.87 |
| Found: | 44.30 | 4.75 | 6.90 |

TLC: $R_f$ 0.6 (6/3/1, n-propanol/ammonium hydroxide/water), visualized by UV, PMA HPLC $R_t$ 18.6 minutes (92%), 10-90% aqueous methanol with 0.2% phosphoric acid buffer, 30 minute gradient, 1.5 mL/minute, 220 nm, C18 (6.0 x 150 mm) colum
IR: (KBr) 3435, 2924, 1680, 1645, 1206, 1134 $cm^{-1}$
MS: $[M+H]^+531^+$

**Example 51**

**[R*[R*(S*)]]-N-[[2-[2-[(2-Amino-3-mercaptopropyl)amino]-2-cyclohexylacetyl]-1,2,3,4-tetrahydro-3-isoquinolinyl]carbonyl]-L-methionine, trifluoroacetate (1:2) salt**

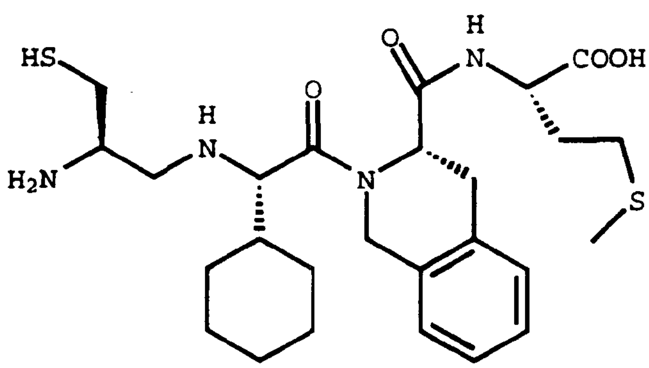

A. **(S)-α-[[(1,1-Dimethylethoxy)carbonyl]amino]cyclohexaneacetic acid**

Compound A (3.5 g) was prepared using L-cyclohexylalanine (1.6 g, 10.1 mmol) [P. J. Corringer, et al., J. Med. Chem., 1993, 36, 166] in the method described in Example 49A.
MS (FAB), $(M+H)^+ = 258.^+$

B. **(R*,R*)-N-[[2-[Cyclohexyl[[(1,1-dimethylethoxy)carbonyl]amino]acetyl]-1,2,3,4-tetrahydro-3-isoquinolinyl]carbonyl]-L-methionine, methyl ester**

Compound B (430 mg, 48%) was prepared using Compound A (0.77 g, 3.01 mmol) and Compound 1B, hydrochloride in the method described in Example 49B.
MS (CI), $(M+H)^+ = 562^+$.

**C. [R*[R*(S*)]]-N-[[2-[Cyclohexyl[[2-[[(1,1-dimethylethoxy)-carbonyl]amino]-3-[(triphenylmethyl) thio]propyl]amino]acetyl]-1,2,3,4-tetrahydro-3-isoquinolinyl]carbonyl]-L-methionine, methyl ester**

Compound C (230 mg, 59%) was prepared as a white foam using Compound B (250 mg, 0.44 mmol) and Compound 1F (0.2 g, 0.4 mmol) in the two step procedure described in Example 49C.
MS: (FAB) $(M+H)^+ = 893^{+\cdot}$

**D. [R*[R*(S*)]]-N-[[2-[2-[(2-Amino-3-mercaptopropyl)amino]-2-cyclohexylacetyl]-1,2,3,4-tetrahydro-3-isoquinolinyl]carbonyl]-L-methionine, trifluoroacetate (1:2) salt**

The title compound (55 mg, 50%) was prepared as a white lyophilate using Compound C (0.22 g, 0.25 mmol) in the two step procedure described in Example 49D.
m.p. 100-105°C
MS: (FAB) ; $(M+H)^+ = 537^+$
IR (KBr): 2934,1676, 1434, 1204 $cm^{-1}$
HPLC: YMC, S3, C18 (6 x 150 mm) column, 220 nm, 1.5 mL/minute: 0-90% aqueous methanol with 0.2% phosphoric acid, gradient over 30 minutes Retention time:20.3 minutes
$[\alpha]_D$ = -31° (c= 0.3, methanol)

| Elemental analysis for $C_{26}H_{40}N_4O_4S_2$. 1.8 water . 2.0 trifluoroacetic acid. | | | | |
|---|---|---|---|---|
| | C | H | N | S |
| Calculated: | 45.20 | 5.77 | 7.03 | 8.04 |
| Found | 45.29 | 5.49 | 6.98 | 7.76 |

$^1H$ NMR ($CD_3OD$, 400 MHz): d 7.30-7.15 (4H, m), 4.90-4.50 (5H, m), 3.40 (1H, m), 3.15 (1H, m), 3.00-2.70 (3H, m), 2.60-2.40 (2H, m), 2.20-1.50 (16H, m), 2.06, 2.00 (3H, 2s, rotomeric), 1.40-1.10 (6H, m)

**Example 52**

**(S*,R*)-N-[[2-[N-(2-Amino-3-mercaptopropyl)-3-methyl-L-valyl]-1,2,3,4-tetrahydro-3-isoquinolinyl]carbonyl]-L-methionine, methyl ester, trifluoroacetate (1:2) salt**

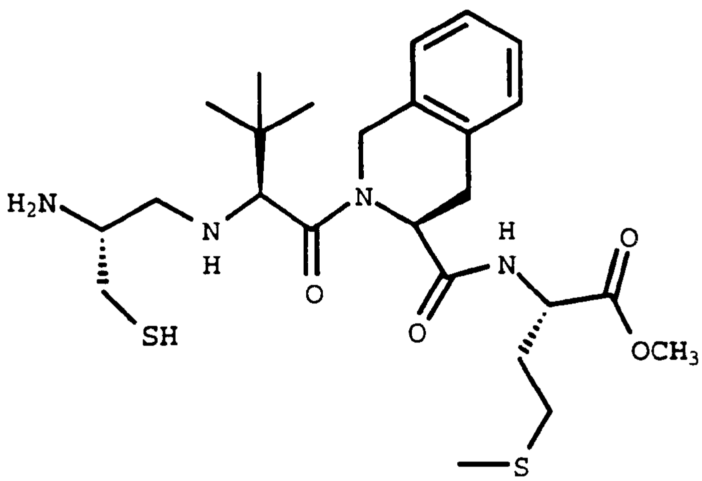

**A. (S*,R*)-N-[[2-[N-(2-Amino-3-mercaptopropyl)-3-methyl-L-valyl]-1,2,3,4-tetrahydro-3-isoquinolinyl]carbonyl]-L-methionine, methyl ester, trifluoroacetate (1:2) salt**

Triethylsilane (0.066 mL, 0.048 g, 0.415 mmol) was added to a solution of Compound 49C (0.180 g, 0.207 mmol), and trifluoroacetic acid (0.4 mL) in dichloromethane (0.8 mL). After stirring for 3 hours, the reaction was

concentrated to afford a solid which was purified by prep HPLC (YMC, S-10, ODS, 30 x 500 mm, column) eluting with 30-90% aqueous methanol containing 0.1% trifluoracetic acid, gradient over 30 minutes. The fractions with product were concentrated and lyophilized to afford the title compound (0.036 g, 23%).

| Elemental analysis for $C_{25}H_{40}N_4O_4S_2$ 0.73 $H_2O$. 2.0 trifluoracetic acid | | | |
|---|---|---|---|
| | C | H | N |
| Calculated: | 45.48 | 5.72 | 7.31 |
| Found: | 45.48 | 5.71 | 7.37 |

HPLC: $R_t$ 19.85 minutes (99%), 10-90% aqueous methanol with 0.2% phosphoric acid buffer, 30 minute gradient, 1.5 mL/minute, 220 nm, $C_{18}$ (6.0 x 150 mm ) column
IR: (KBr) 3435, 2924, 1437, 1682, 1636, 1208, 1134 $cm^{-1}$
MS: $[M+H]^+$ $525^+$
$[\alpha]_D$ = -32° (c = 0.25, methanol)

## Example 53

**(S*,R*)-$N^2$-[[2-[N-(2-Amino-3-mercaptopropyl)-3-methyl-L-valyl]-1,2,3,4-tetrahydro-3-isoquinolinyl]carbonyl]-$N^5$-methyl-L-glutamine, trifluoroacetate (1:2) salt**

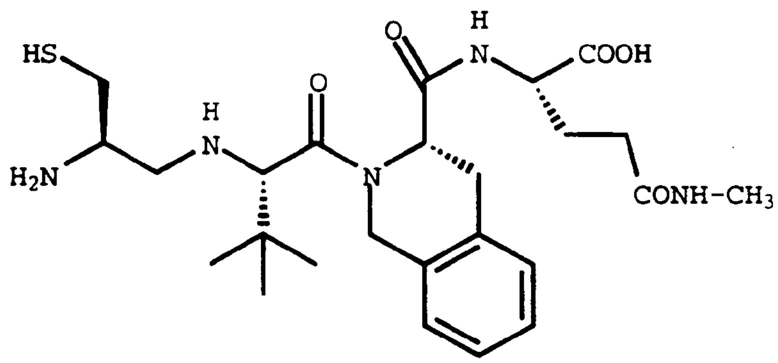

**A. $N^2$-[(1,1-Dimethylethoxy)carbonyl]-$N^5$-methyl-L-glutamine, 1,1-dimethylethyl ester**

To a solution of N-[(1,1-Dimethylethoxy)carbonyl]-L-glutamic acid, 1-(1,1-dimethylethyl) ester (3.03 g, 10 mmol) in dichloromethane (40 mL) at room temperature under argon were added benzotriazol-1-yloxytris-(dimethylamino)phosphonium hexafluorophosphate (4.42 g, 10 mmol) and diisopropylethylamine (7.1 mL, 40 mmol). After 15 minutes, methylamine hydrochloride (2.01 g, 30 mmol) was added. After stirring overnight (18 hours), the reaction mixture was washed sequentially with 1N hydrochloric acid (30 mL) and sodium bicarbonate (30 mL). The aqueous washings were extracted separately with chloroform (30 mL), dried (magnesium sulfate), filtered and concentrated. The oil obtained was purified by silica gel column chromatography eluting with 30% acetone in hexanes to afford Compound A (2.8 g, 88%).
MS (CI), $(M+H)^+$ = 317

**B. $N^5$-Methyl-L-glutamine,1,1-dimethylethyl ester**

To a solution of Compound A (0.6 g, 1.9 mmol) in dichloromethane (2 mL) at room temperature under argon were added 2,6-lutidine (0.49 mL, 4.2 mmol) followed by t-butyldimethylsilyltriflate (0.52 mL, 2.28 mmol). After 30 minutes, the reaction mixture was washed with saturated ammonium chloride (2 x 5 mL) and extracted with chloroform (5 mL). The organic layer was concentrated *in vacuo* (product partially decomposes on silica gel TLC plate). The oil obtained was dissolved in tetrahydrofuran (3 mL) and 1N hydrochloric acid (2 mL). The mixture was stirred vigorously for 20 minutes, and washed with ether (5 mL). The aqueous layer was basified to pH 8.5 by the addition of saturated sodium bicarbonate, washed with ether (2 x 5 mL) and concentrated *in*

*vacuo.* Acetonitrile was added to the residue and concentrated (2 x 5 mL). Chloroform and methanol (1:1) was added to the solid obtained and the insoluble inorganics were filtered off. The filtrate was concentrated *in vacuo* and the solid was suspended in acetonitrile (5 mL) and concentrated *in vacuo* to afford Compound B (0.37 g) which was used without further purification.

**C. 3-Methyl-N-[(phenylmethoxy)carbonyl]-L-valine**

To a solution of L-tert-leucine (10.2 g, 77.6 mmol) in aqueous sodium hydroxide (2N, 40 mL) at 0°C was added benzyl chloroformate (12.1 mL, 85.4 mmol) and sodium hydroxide (2N, 40 mL) alternatively in 5 portions over 30 minutes. The cold bath was removed and after 1 hour the pH of the mixture was adjusted to 10. The mixture was extracted with ether (2 x 50 mL). The aqueous layer was cooled to 0°C and acidified (pH 2) with 5 N hydrochloric acid and extracted with ether (3 x 70 mL). The organic layer was dried (magnesium sulfate) and concentrated *in vacuo* to afford a thick oil Compound C (18.2 g, 88%).
MS (CI) : $(M+H)^+ = 266^+$

**D. (S)-1,2,3,4-Tetrahydro-2-[3-methyl-N-[(phenylmethoxy)carbonyl]-L-valyl]-3-isoquinolinecarboxylicacid, methyl ester**

To a solution of Compound C (5.31 g, 20 mmol) in dichloromethane (80 mL) at 0°C under argon were sequentially added diisopropylethylamine (10.6 mL, 60 mmol), benzotriazol-1-yloxytris- (dimethylamino)phosphonium hexafluorophosphate (5.08 g, 20 mmol) and (S)-1,2,3,4-Tetrahydroisoquinoline-3-carboxylic acid, methyl ester, hydrochloride (5.68 g, 25 mmol). The reaction mixture was allowed to warm to 5°C over 2 hours, and then stirred overnight (16 hours). The mixture was washed with 1N hydrochloric acid, saturated sodium bicarbonate and brine (50 mL each). The organic layer was dried (magnesium sulfate), filtered and concentrated to afford an oil. Purification by flash silica gel column chromatography eluting with 20% ethyl acetate in hexanes afforded Compound D (4.0 g, 45%).
MS: (CI) $(M+H)^+ = 439^+$

**E. (S)-1,2,3,4-Tetrahydro-2-[3-methyl-N-[(phenylmethoxy)carbonyl]-L-valyl]-3-isoquinolinecarboxylic acid**

To a solution of Compound D (830 mg, 1.9 mmol) in tetrahydrofuran/methanol (4 mL/5 mL) at room temperature was added lithium hydroxide (1N, 1.9 mL ). After 1 hour, lithium hydroxide (1N, 1.5 mL) was added. After 2 hours, sodium hydroxide (1N, 1.9 mL) was added. After another hour, solvent was removed and the residue was partitioned between 1N hydrochloric acid and ethyl acetate (10 mL each). The organic layer was dried (magnesium sulfate) and concentrated *in vacuo* to afford Compound E (820 mg).

**F. (R*)-$N^5$-Methyl-$N^2$-[[1,2,3,4-tetrahydro-2-[3-methyl-N-[(phenylmethoxy)carbonyl]-L-valyl]-3-isoquinolinyl]carbonyl]-L-glutamine, 1,1-dimethylethyl ester**

To a solution of Compound B (370 mg, 1.71 mmol) and Compound E (800 mg, 1.88 mmol) in a mixture of dimethylformamide:tetrahydrofuran (2 mL:1 mL) at 0°C under argon was added 1-hydroxybenzotriazole (290 mg, 1.9 mmol) followed by 1-(3-dimethylaminopropyl)-3-ethylcarbodiimide hydrochloride (363 mg, 1.9 mmol). The reaction mixture was allowed to warm to room temperature over 1 hour and then stirred overnight (18 hours). The mixture was diluted with ethyl acetate (10 mL) and washed with 1N hydrochloric acid, saturated sodium bicarbonate and brine (10 mL each). Purification by silica gel column chromatography eluting with 30% acetone in hexanes afforded white foamy solid Compound F (585 mg, 55%).
MS (CI) $(M+H)^+ = 623$

**G. (S*,R* )-$N^2$-[[2-[N-[2-[[(1,1-Dimethylethoxy)carbonyl]amino]-3-[(triphenylmethyl)thio]propyl]-3-methyl-L-valyl]-1,2,3,4-tetrahydro-3-isoquinolinyl]carbonyl]-$N^5$-methyl-L-glutamine,1,1-dimethylethyl ester**

To solution of Compound F (300 mg, 0.48 mmol) in dichloromethane (2 mL) at room temperature under argon was added triethylsilane (115 μL, 0.72 mmol), diisopropylethylamine (14 μL, 0.1 mmol) and palladium acetate (11 mg, 0.05 mmol). A black precipitate formed. After 1 hour, silica gel TLC analysis indicated absence of Compound F and presence of a less polar product which partially decomposed on TLC plate. The mixture was filtered through Celite® and the filtrate was concentrated *in vacuo.* The product used without further purification.

To a solution of the residue (0.48 mmol assumed) in methanol (1.5 mL) at room temperature was added acetic acid (0.5 mL). A solution of aldehyde Compound 1F (447 mg, 1.0 mmol) in methanol (1.5 mL) was prepared. After 20 minutes, the above solution of aldehyde (0.8 mL) was added. After 5 minutes, sodium cyanoborohydride (20 mg, 0.33 mmol) was added. The remaining solution of aldehyde and sodium cyanoborohydride (20 mg each) were added in two portions with 20 minutes interval. After an additional 20 minutes, the volatiles were removed *in vacuo* and the residue was partitioned between ethyl acetate and saturated sodium bicarbonate (20 mL each). The aqueous layer was separated and extracted with ethyl acetate (15 mL), and the organic layers were combined, dried (magnesium sulfate) and concentrated *in vacuo*. The residue was purified by silica gel column chromatography eluting with 80% ethyl acetate in hexanes on a small column to afford Compound G (310 mg).
MS (FAB) ; $(M+H)^+ = 920$

**H. (S*,R*)-$N^2$-[[2-[N-(2-Amino-3-mercaptopropyl)-3-methyl-L-valyl]-1,2,3,4-tetrahydro-3-isoquinolinyl]carbonyl]-$N^5$-methyl-L-glutamine, trifluoroacetate (1:2) salt**

To a solution of Compound G (270 mg, 0.29 mmol) in dry dichloromethane (2 mL) under argon was added trifluoroacetic acid (2 mL) and triethylsilane (0.5 mL). After 2 hours, the volatiles were removed *in vacuo.* Aqueous methanol (50%, 1.5 mL, deoxygenated) was added to the residue. The white precipitate was filtered off through nylon filter and the filtrate was purified by preparative RP HPLC (EM Science E8, 50 x 250 mm, S12 lichrosphere) eluting with 30 to 74% aqueous methanol containg 0.1% trifluoroacetic acid. Appropriate fractions were collected and lyophilized to afford the title compound (177 mg, 75%).
MS (FAB): $(M+H)^+= 522$
IR (KBr) 3432, 1678, 1647, 120, 1138 $cm^{-1}$
$[\alpha]_D = -15°$ (c = 0.92, methanol)
HPLC: HPLC: (YMC S3 ODS 6 x 150 mm, C18 column at 60°C); 70-90% aqueous methanol containing 0.2% phosphoric acid, retention time 13.9 minutes.

| Elemental analysis for $C_{25}H_{39}N_5O_5S \cdot 2.31$ trifluoroacetic acid · 1.39 water | | | | | |
|---|---|---|---|---|---|
| | C | H | N | S | F |
| Calculated: | 43.95 | 5.48 | 8.65 | 3.96 | 16.26 |
| Found: | 43.95 | 5.29 | 8.68 | 3.98 | 16.26 |

**Example 54**

**(S*,R*)-$N^2$-[[2-[N-(2-Amino-3-mercaptopropyl)-3-methyl-L-valyl]-1,2,3,4-tetrahydro-3-isoquinolinyl]carbonyl]-L-glutamine, trifluoroacetate (1:2) salt**

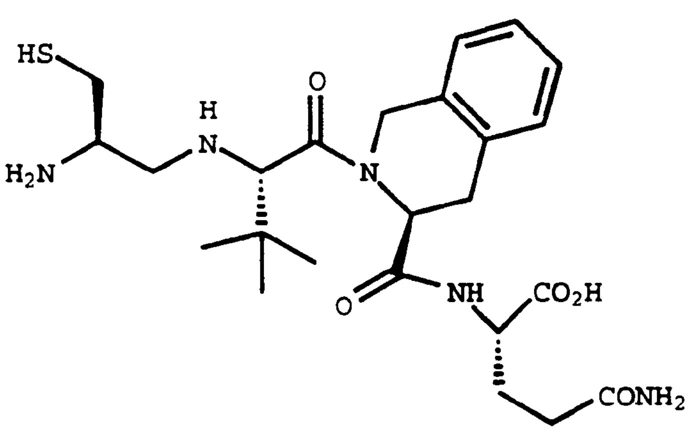

**A. (R*)-$N^2$-[[1,2,3,4-Tetrahydro-2-[3-methyl-N-[(phenylmethoxy)carbonyl]-L-valyl]-3-isoquinolinyl]carbonyl]-L-glutamine,1,1-dimethylethyl ester**

Compound A (1.1 g, 87%) was prepared as a white solid using Compound 53E (0.9 g, 2.1 mmol) and L-glutamine, t-butylester, hydrochloride (0.501 g, 2.1 mmol) in the method described in Example 32F.
mp: 60-68°C

**B. (R*)-$N^2$-[[1,2,3,4-Tetrahydro-2-(3-methyl-L-valyl)-3-isoquinolinyl]carbonyl]-L-glutamine, 1,1-dimethylethyl ester, hydrochloride**

Palladium hydroxide on carbon (10%, 0.091 g) was added to a solution of Compound A (0.914 g, 1.5 mmol) in tetrahydrofuran (9.1 mL) with 1N hydrochloric acid (1.5 mL). A hydrogen balloon was attached to the reaction and the mixture was stirred at room temperature for 3 hours. The reaction was filtered through Celite® and concentrated under vacuum to afford Compound B (0.767 g, 100%) which was used without further purification.
mp: foams and liberates gas above 128°C

**C. (S*,R*)-$N^2$-[[2-[N-[2-[[(1,1-Dimethylethoxy)carbonyl]amino]-3-[(triphenylmethyl)thio]propyl]-3-methyl-L-valyl]-1,2,3,4-tetrahydro-3-isoquinolinyl]carbonyl]-L-glutamine, 1,1-dimethylethyl ester**

Sodium cyanoborohydride (0.077 g, 1.22 mmol) was added portionwise to a solution of Compound B (0.464 g, 0.91 mmol), Compound 1F (0.609 g, 1.4 mmol) and acetic acid (0.135 mL) in methanol (4.5 mL), and stirred at room temperature for 2.25 hours. The reaction was quenched with water (100 mL) and extracted with dichloromethane (3 x 100 mL). The combined organic extracts were dried (magnesium sulfate), filtered and concentrated under vacuum. The residue was purified by flash chromatogrphy (eluting with ethyl acetate) to afford Compound C (0.55 g, 67%) as a gum.
MS: $(M+H)^+$ 906

**D. (S*,R*)-$N^2$-[[2-[N-(2-Amino-3-mercaptopropyl)-3-methyl-L-valyl]-1,2,3,4-tetrahydro-3-isoquinolinyl]carbonyl]-L-glutamine, trifluoroacetate (1:2) salt**

Trifluoroacetic acid (5 mL, 65 mmol) was added to a solution of Compound C (0.487 g, 0.538 mmol) and triethylsilane (0.843 mL, 5.38 mmol) in dichloromethane (5 mL) at 0°C, warmed to room temperature and stirred for 3 hours. Chloroform (10 mL) was added and the reaction was concentrated under vacuum. The residue was triturated with hexane (discarding the hexane layer) and concentrated under vacuum. The residue was purified by preparative HPLC (EM S-12 Lichrosphere Select B, 50 x 250 mm, 220 nm, 40 mL/minute, gradient 10-90% aqueous methanol with 0.1% tetrahydrofuran). Following concentration of appropriate fractions the residue was dissolved in water, millipore filtered and lyophilized to afford the title compound (0.28 g, 71%).
mp: decomposition above 97°C
TLC: $R_f$ = 0.55 (6/3/1 n-propanol/ammonium hydroxide/water, visualization by ceric ammonium sulfate)
MS: $(M+H)^+$ 508
IR: (KBr), 1431, 1674, 2972, 3437 $cm^{-1}$

| Elemental analysis for $C_{28}H_{39}N_5O_9SF_6$ . 1.07 water | | | | | |
|---|---|---|---|---|---|
| | C | H | N | S | F |
| Calculated: | 44.54 | 5.49 | 9.28 | 4.25 | 15.10 |
| Found: | 44.56 | 5.17 | 9.26 | 4.23 | 15.48 |

$[\alpha]_D$ = - 17.6° (c = 0.33, methanol)
$^1H$(DMSO): 1.00 (s, 9H), 1.81-1.96 (m, 2H), 2.08-2.13 (m, 2H), 2.35-2.47 (m, 2H), 2.72 (br m, 1H), 2.96 (br m, 2H), 3.34 (br m, 2H), 4.18-4.20 (m, 1H), 4.52-4.59 (m, 1H), 4.85-5.02 (m, 3H), 7.17 (s, 4H)

**Example 55**

**(S*,R*)-N²-[[2-[N-(2-Amino-3-mercaptpropyl)-3-methyl-L-valyl]-1,2,3,4-tetrahydro-3-isoquinolinyl]carbonyl]-L-glutamine, methyl ester, trifluoroacetate (1:2) salt**

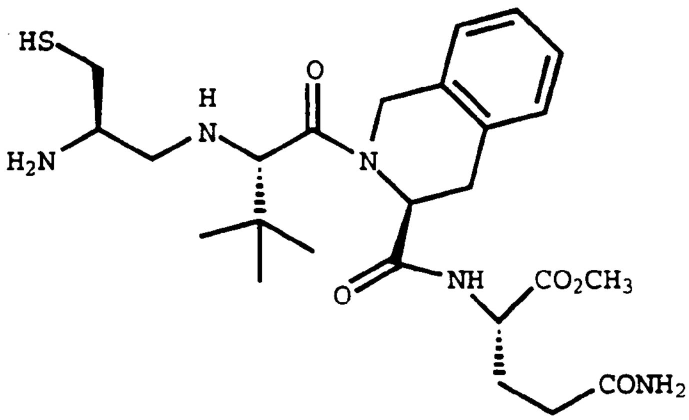

**A. $N^2$-[(1,1-Dimethylethoxy)carbonyl]-L-glutamine, methyl ester**

1-Methyl-3-nitro-1-nitrosoguanidine (9 g, 61.0 mmol) was added portionwise to a mixture of (1:1) diethyl ether/50% aqueous potassium hydroxide (200 mL) at -10°C. The organic layer was decanted and dried over solid potassium hydroxide. In a separate flask, $N^2$-[(1,1-Dimethylethoxy)carbonyl]-L-glutamine (5.0 g, 20.3 mmol) was dissolved in tetrahydrofuran (15 mL) at -10°C. The diazomethane solution was added portionwise to the acid solution until a yellow color persisted. The reaction was quenched with acetic acid (3 mL), diluted with diethyl ether (100 mL) and washed with 10% sodium bicaronate (100 mL). The aqueous layer was extracted with ethyl acetate (3 x 150 mL) and the combined organic extracts were dried (magnesium sulfate), filtered and concentrated under vacuum to afford Compound A (2.0 g, 38%) which was used without further purification.
MS: $(M+H)^+261$

**B. L-glutamine, methyl ester, hydrochloride**

Hydrogen chloride in dioxane (4M, 20 mL) was added to Compound A (2.0 g, 7.7 mmol) and stirred at room temperature for 0.5 hours. The reaction was concentrated under vacuum to afford Compound B (1.4 g, 93%).
MS: $(M+H)^+161$

**C. (R*)-$N^2$-[[1,2,3,4-Tetrahydro-2-[3-methyl-N-[(phenylmethoxy)-carbonyl]-L-valyl]-3-isoquinolinyl]carbonyl]-L-glutamine, methyl ester**

Compound C (1.05 g, 88%) was prepared as a white solid using Compound B (0.26 g, 3.2 mmol) and Compound 52E (0.90 g, 2.1 mmol) in the method described in Example 32F.
mp: 55-60°C

**D. (R*)-$N^2$-[[1,2,3,4-Tetrahydro-2-(3-methy-L-valyl)-3-isoquinolinyl]carbonyl]-L-glutamine, methyl ester, hydrochloride**

Compound D (0.715 g, 91%) was prepared using Compound C (0.95 g, 1.68 mmol) in the method described in Example 54B.
mp: foams and liberates gas above 100°C

**E. (S*,R*)-$N^2$-[[2-[N-[2-[[(1,1-Dimethylethoxy)carbonyl]amino]-3-[(triphenylmethyl)thio]propyl]-3-methyl-L-valyl]-1,2,3,4-tetrahydro-3-isoquinolinyl]carbonyl]-L-glutamine, methyl ester**

Compound E (1.25 g, 70%) was prepared as a gum using Compound D (0.645 g, 1.38 mmol) and Compound 1F (0.925 g, 2.07 mmol) in the method described in Example 54C.

F. (S*,R*)-$N^2$-[[2-[N-(2-Amino-3-mercaptpropyl)-3-methyl-L-valyl]-1,2,3,4-tetrahydro-3-isoquinolinyl]carbonyl]-L-glutamine, methyl ester, trifluoroacetate (1:2) salt

The title compound (0.291 g, 60%) was prepared using Compound E (0.55 g, 0.649 mmol) in the method described in Example 54D.
mp: 67-77°C
TLC: $R_f$ = 0.73 (6/3/1 n-propanol/ammonium hydroxide/water, visualization by ceric ammonium sulfate)
MS: $(M+H)^+$ 522
IR: (KBr), 1431, 1674, 2972, 3437 $cm^{-1}$

| Elemental analysis for $C_{25}H_{39}N_5O_5S$ . 2.45 trifluoroacetic acid . 0.59 water | | | | | |
|---|---|---|---|---|---|
| | C | H | N | S | E |
| Calculated: | 44.25 | 5.29 | 8.63 | 3.95 | 17.20 |
| Found: | 44.25 | 5.20 | 8.51 | 3.80 | 17.21 |

$[\alpha]_D$ = - 22.7° (c = 0.30, methanol)
$^1$H(DMSO): 1.00 (s, 9H), 1.78-1.99 (m, 2H), 2.01-2.13 (m, 2H), 2.44 ( br m, 2H), 2.72 (br m, 3H), 2.97 (br m, 1H), 3.33 (br m, 1H), 3.51, 3.55 (s, 3H), 4.20-4.28 (m, 1H), 4.51-4.60 (m, 1H), 4.85-4.96 (m, 2H), 5.02-5.04 (m, 1H), 7.15-7.18 (s, 4H)

## Example 56

[R*[R*(S*)]]-N-[[2-[2-[(2-Amino-3-mercaptopropyl)amino]-3-methylbutyl]-1,2,3,4-tetrahydro-3-isoquinolinyl]carbonyl]-N-methyl-L-methionine

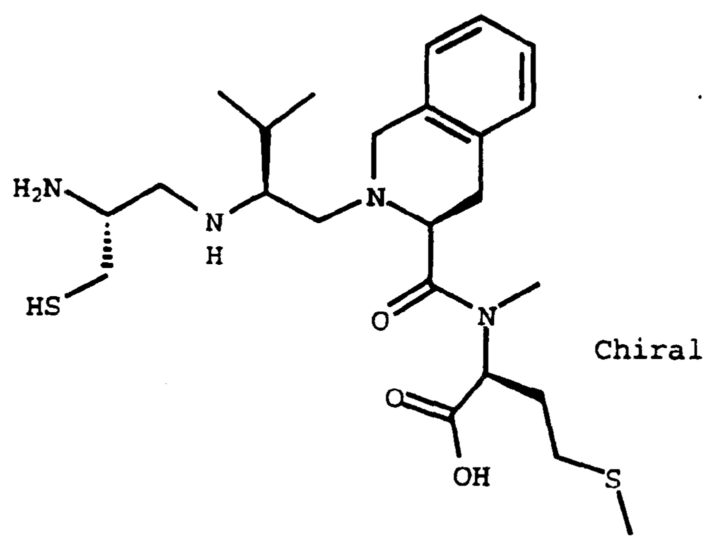

A. N-[(1,1-Dimethylethoxy)carbonyl]-N-methyl-L-methionine, phenylmethyl ester

Sodium hydride (3.37 g of 60% suspension in mineral oil, 84.2 mmol) was slurried in hexane (3 X 50 mL) and allowed to settle. The hexane was decanted off and tetrahydrofuran (100 mL) was added. A solution of N-t-butyloxycarbonyl-L-methionine (7.00 g, 28.1 mmol) in tetrahydrofuran (120 mL) was added to the sodium hydride solution at 0°C and was treated with methyl iodide (12.0 g, 5.24 mL, 84.2 mmol) dropwise. The mixture was stirred for 20 hours at room temperature, diluted with ethyl acetate (100 mL) and quenched with water (50 mL). The organic layer was separated and extracted with saturated sodium bicarbonate (3 X 100 mL) and the combined sodium bicarbonate layer was acidified to pH 2 using potassium bisulfate. Saturated sodium chloride was added to the acidic solution and the product was extracted with ethyl acetate (3 X 100 mL). The

combined ethyl acetate washes were dried (magnesium sulfate) and concentrated to yield the desired product as a slightly yellow oil (5.43 g, 73%), MS $(M+H)^+$ $264^+$.

Benzylbromide (1.43 g, 1.00 mL, 8.36 mmol) was added to the oil obtained above (2.00 g, 7.60 mmol) and potassium carbonate (1.16 g, 8.36 mmol) in dimethylformamide (12 mL). The mixture was stirred for 48 hours and partitioned between water (100 mL) and ethyl acetate (100 mL). The ethyl acetate layer was washed with saturated sodium bicarbonate (3 X 50 mL), water (3 X 50 mL) and brine (2 X 50 mL), dried (magnesium sulfate), and concentrated to yield Compound A as a clear oil (2.32 g, 86%), MS $(M+H)^+$ $354^+$.

**B. (R*)-N-[[2-[(1,1-Dimethylethoxy)carbonyl]-1,2,3,4-tetrahydro-3-isoquinolinyl]carbonyl]-N-methyl-L-methionine, phenylmethyl ester**

Compound A (1.5 g, 4.234 mmol) was stirred in dimethyl sulfide (0.6 mL) and 4N hydrochloric acid in dioxane (10 mL) for 30 minutes. The mixture was concentrated, dissolved in methylene chloride (30 mL) and concentrated. This procedure was repeated five times to yield the amine hydrochloride as a clear glass. This amine was dissolved in methylene chloride (20 mL) and cooled to 0°C. t-Butyloxycarbonyl-L-1,2,3,4-tetrahydroisoquinolin-3-carboxylic acid (1.76 g, 6.35 mmol), Bis-(2-oxo-3-oxazolidinyl)-phosphinic chloride (3.23 g, 12.7 mmol) and N,N-diisopropylethylamine (1.64 g, 2.22 mL, 12.7 mmol) were added. The reaction mixture was stirred for 24 hours at 0°C. Additional Bis-(2-oxo-3-oxazolidinyl)-phosphinic chloride (1.61 g, 6.35 mmol) and N,N-diisopropylethylamine (820 mg, 1.1 mL, 6.35 mmol) were added and the reaction mixture was stirred for an additional 24 hours at 0°C. The reaction mixture was concentrated and chromatographed (silica gel, 4.1 X 20 cm, 40% ethyl acetate, 60% hexane). Fractions containing the desired compound were collected and concentrated to yield Compound B as a clear oil (2.4 g, 99%). $(M+H)^+$ $513^+$

**C. R*,R*)-N-[[2-[2-[[(1,1-Dimethylethoxy)carbonyl]amino]-3-methylbutyl]-1,2,3,4-tetrahydro-3-isoquinolinyl]carbonyl]-N-methyl-L-methionine, phenylmethyl ester**

Compound C (800 mg, 50%) was prepared as a clear oil using Compound B (1.4 g, 2.64 mmol) and Compound 5B (770 mg, 3.84 mmol) in the two step procedure described in Example 7B.
$(M+H)^+598^+$

**D . [R*[R*(S*)]]-N-[[2-[2-[2-[[(1,1-Dimethylethoxy)carbonyl]amino]-3-[(triphenylmethyl)thio]propyl]-3-methylbutyl]-1,2,3,4-tetrahydro-3-isoquinolinyl]carbonyl]-N-methyl-L-methionine, phenylmethyl ester**

Compound D (150 mg, 16%) was prepared as a clear oil using Compound C (600 mg, 1.00 mmol) and Compound 1F (890 mg, 2.00 mmol) in the two step procedure described in Example 7B.

**E. [R*[R*(S*)]]-N-[[2-[2-[(2-Amino-3-mercaptoprpyl)amino]-3-methylbutyl]-1,2,3,4-tetrahydro-3-isoquinolinyl]carbonyl]-N-methyl-L-methionine**

The title compound (28 mg, 19%) was prepared using Compound D (150 mg, 0.161 mmol) in the two step procedure described in Example 1H.
$[\alpha]_D^{25}$ = - 20.0° (c=0.1, methanol)
MS $(M+H)^+$ $497^+$
Analysis: calculated for 2.70 trifluoroacetic acid · 5.50 water: C, 39.08; H, 5.99; N, 6.20; F, 17.03;
Found: C, 39.12; H, 5.38; N, 6.22; F, 17.03.
$^1$H-NMR ($CD_3OD$, 270 MHz) d (ppm) 7.19 (4H, m), 4.28 (4H, m), 3.46 (1H, m), 3.32 (2H, m), 2.82 (2H, m), 2.61 (2H, m), 2.49 (2H, m), 2.25 (1H, m), 2.12 (1H, m), 2.11 (3H, s), 2.01 (3H, s), 1.97 (1H, m), 0.88 (6H, m)

## Example 57

**(S*,R*)-N-[[2-[2-[N-(2-Amino-3-mercaptopropyl)methyl-amino]-3-methylbutyl]-1,2,3,4-tetrahydro-3-isoquinolinyl]-carbonyl]-L-methionine, trifluoroacetate (2:3) salt**

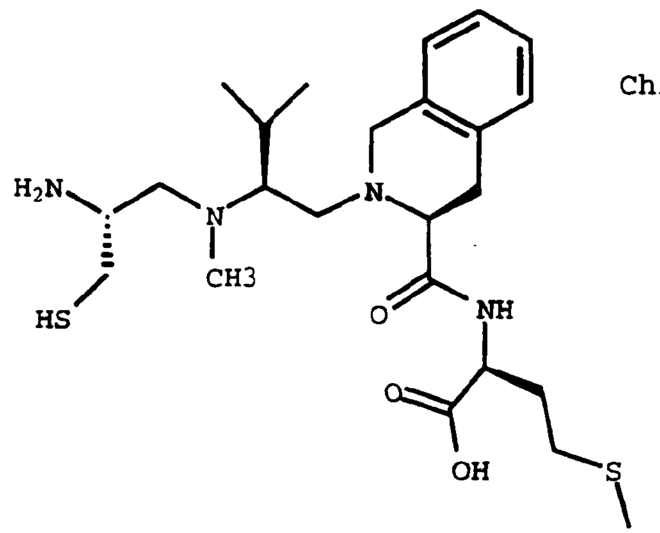

Chiral

**A. (S)-(1-Formyl-2-methylpropyl)methylcarbamic acid, 1,1-dimethylethyl ester**

Compound A (1.8 g, 58%) was prepared as a clear oil using $N^2$-[(1,1-Dimethylethoxy)carbonyl]-N-methoxy-N,$N^2$-dimethyl-L-valinamide (4.0 g, 14.62 mmol) in the method described in Example 1F.
$(M+H)^+$ $522^+$

**B. (R*)-N-[[2-[N-[(1,1-Dimethylethoxy)carbonyl]methylamino]-3-methylbutyl]-1,2,3,4-tetrahydro-3-isoquinolinyl]carbonyl]-L-methionine, methyl ester**

Compound B (1.9 g, 60%) was prepared as a clear oil using Compound 1A (6.0 g, 14.22 mmol) and Compound A (1.8 g, 8.43 mmol) in the two step procedure described in Example 7B.
$(M+H)^+$ $522^+$

**C. [R*[R*(S*)]]-N-[[2-[2-[N-[2-[[(1,1-Dimethylethoxy)carbonyl]amino]-3-[(triphenylmethyl)thio]propyl]methylamino]-3-methylbutyl]-1,2,3,4-tetrahydro-3-isoquinolinyl]carbonyl]-L-methionine, methyl ester**

Compound C (1.1 g, 35%) was prepared as a clear oil using Compound B (1.9 g, 3.64 mmol) and Compound 1F (8.4 g, 18.8 mmol) in the two step method described in Example 7B.
$(M+H)^+$ $853^+$

**D. (S*,R*)-N-[[2-[2-[N-(2-Amino-3-mercaptopropyl)methylamino]-3-methylbutyl]-1,2,3,4-tetrahydro-3-isoquinolinyl]carbonyl]-L-methionine, trifluoroacetate (2:3) salt**

The title compound (380 mg) was prepared using Compound C (500 mg, 0.587 mmol) in the method described in Example 1H.
M.P. 113-114 °C, Purity > 98%, $[\alpha]_D^{25}$ = - 38.0 ° (c=0.1, methanol), MS $(M+H)^+$ $497^+$, Analysis: calculated for 1.90 trifluoroacetic acid · 1.00 water: C, 45.72; H, 5.92; N, 7.67;
Found: C, 45.75; H, 5.94; N, 7.99.
$^1$H-NMR ($CD_3OD$, 400 MHz) d (ppm) 7.27 (4H, m), 4.64 (1H, m), 4.39 (1H, m), 4.29 (2H, m), 3.56 (1H, m), 3.39 (3H, m), 3.01 (2H, m), 2.85 (4H, m), 2.60 (2H, m), 2.56 (3H, s), 2.18 (1H, m), 2.09 (3H, s), 1.94 (2H, m), 0.99 (3H, d, J=6.8 Hz), 0.82 (3H, d, J=6.4 Hz)

**Example 58**

**(S*,R*)-N-[[2-[N-Acetyl-N-(2-amino-3-mercaptopropyl)-L-valyl]-1,2,3,4-tetrahydro-3-isoquinolinyl]carbonyl]-L-methionine, trifluoroacetate (1:1) salt**

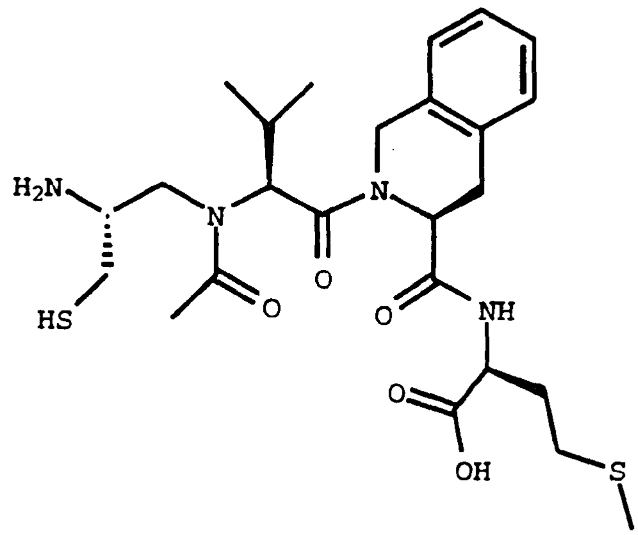

**A. (S*,R*)-N-[[2-[N-Acetyl-N-[2-[[(1,1-dimethylethoxy)carbonyl]-amino]-3-[(triphenylmethyl)thio]propyl]-L-valyl]-1,2,3,4-tetrahydro-3-isoquinolinyl]carbonyl]-L-methionine, methyl ester**

Compound 1G (700 mg, 0.821 mmol) was stirred in acetic anhydride (5.41 g, 53.0 mmol) and N,N-diisopropylethylamine (3.70 g, 5.0 mL, 28.6 mmol) for 24 hours at room temperature. The mixture was concentrated, dissolved in methylene chloride (30 mL) and concentrated. This latter procedure was repeated five times to yield the crude product as a brown oil (700 mg, mixture).
MS $(M+H)^+$ $895^+$

**B. (S*,R*)-N-[[2-[N-Acetyl-N-(2-amino-3-mercaptopropyl)-L-valyl]-1,2,3,4-tetrahydro-3-isoquinolinyllcarbonyl]-L-methionine, trifluoroacetate (1:1) salt**

The title compound (41 mg) was prepared using Compound A (700 mg, 0.82 mmol assumed) in the two step procedure described in Example 1H.
Purity > 98%, $[\alpha]_D 25°$ = - 38.0° (c=0.1, methanol),
MS $(M+H)^+$ $539^+$
Analysis: Calculated for 1.10 trifluoroacetic acid · 1.30 water: C, 47.52; H, 6.11; N, 8.15.
Found: C, 47.52; H, 6.07; N, 8.09.
$^1$H-NMR ($CD_3OD$, 270 MHz) δ (ppm) 7.19 (4H, m), 5.47-5.13 (2H, m), 4.58 (2H, m), 4.45 (2H, m), 4.20 (1H, m), 3.92-3.58 (2H, m), 3.42 (2H, m), 3.18 (1H, m), 2.84-2.55 (2H, m), 2.43 (1H, m), 2.31 (1H, m), 2.11 (3H, s), 1.98 (3H, m), 1.85 (1H, m), 0.75-1.13 (6H, m)

**Example 59**

**(S*,R*)-N-[[2-[N-[3-Mercapto-2-(methylamino)propyl]-L-valyl]-1,2,3,4-tetrahydro-3-isoquinolinyl]carbonyl]-L-methionine, trifluoroacetate (2:3) salt**

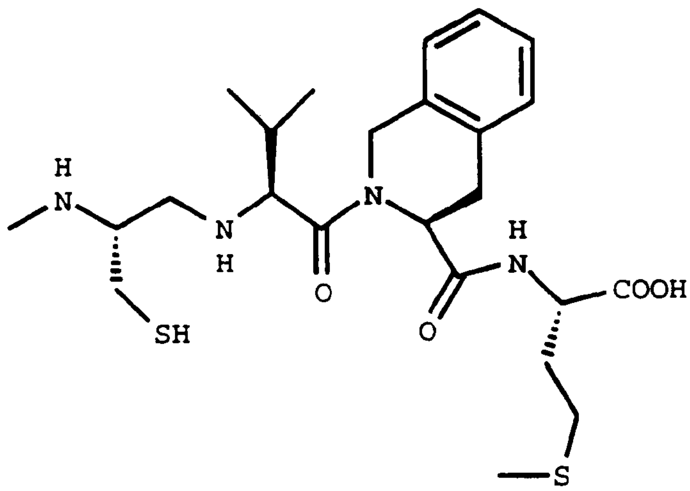

**A. N-Methyl-L-cysteine**

As reported in Blondeau et al., Can. J. Chem., *45*, 49, 1967, sodium metal was added to a solution of (R)-4-Thiazolidinecarboxylic acid (22.0 g, 165.2 mmol) in liquid ammonia and cooled in an acetone/dry ice bath at -30°C. A blue colored solution was obtained, and persisted for 20 minutes. The reaction was quenched with ammonium chloride until the reaction was clear. Ammonia was evaporated with a stream of nitrogen to obtain a purple residue which was acidified with concentrated hydrochloric acid to pH = 0. The reaction mixture was concentrated and extracted with hot ethanol (2 x 50 mL) and the extracts were concentrated to afford Compound A (19.8 g, 89 %).
MS: $[M+H]^+$ $136^+$

**B. N-Methyl-S-(triphenylmethyl)-L-cysteine**

Trityl alcohol (1.93 g, 7.39 mmol) was added to a solution of Compound A (1.00g, 7.39 mmol) in trifluoroacetic acid (10 mL) and stirred under argon. The reaction was concentrated, the residue was triturated with hexane and extracted with trichloromethane (2 x 10 mL). The organic layer was concentrated to afford Compound B (1.6 g, 57%).
TLC: $R_f$ 0.16 (ethyl acetate) visualized by UV, ceric ammonium molybdate

**C. N-[(1,1-Dimethylethoxy)carbonyl]-N-methyl-S-(triphenylmethyl)-L-cysteine**

Di-t-butyldicarbonate (1.50 g, 6.88 mmol) in tetrahydrofuran (5 mL) was added dropwise to a solution of Compound B (2.0 g, 5.29 mmol) in tetrahydrofuran and aqueous sodium bicarbonate (10 mL, saturated) at 0°C. The reaction was warmed to room temperature, and stirred for 15 hours. The reaction mixture was poured into a mixture of brine (25 mL) and methylene chloride (50 mL). The layers were separated and the aqueous layer was extracted with dichloromethane (2 x 50 mL). The combined organic layers were washed with 10% potassium bisulfate (2 x 20 mL) and brine (25 mL) and dried over magnesium sulfate, filtered and concentrated to afford an oil which was purified on a silica gel flash column eluting with trichloromethane/methanol/acetic acid (9:1:0.1) to afford Compound C (1.82 g, 72 %).
MS: $[M-H]^-$ $476^-$

**D. (R)-N-[1-Formyl-2-[(triphenylmethyl)thio]ethyl]methylcarbamic acid, 1,1-dimethylethyl ester**

Compound D (0.55 g, 57%) was prepared using Compound C (1.0 g, 2.09 mmol) in two steps as described in Example 1E and F.
MS : $[M+H]^+$ $521^+$

**E. (S*,R*)-N-[[2-[N-[2-[N-[(1,1-Dimethylethoxy)carbonyl]methylamino]-3-[(triphenylmethyl)thio]propyl]-L-valyl]-1,2,3,4-tetrahydro-3-isoquinolinyl]carbonyl]-L-methionine, methyl ester**

Sodium cyanoborohydride (0.023 g, 0.37 mmol) was added dropwise as a solution in methanol (0.5 mL) to a stirred solution of Compound D of Example 1 (0.17 g, 0.41 mmol) and Compound D (0.14 g, 0.31 mmol) in acetic acid (1 mL) and methanol (1 mL) at 0°C. The mixture was slowly warmed to room temperature, and stirred for 15 hours. The reaction mixture was diluted with saturated sodium bicarbonate at 0°C and extracted with ethyl acetate (3 x 20 mL). The combined extracts were washed with brine and dried over magnesium sulfate, filtered and concentrated. The product was purified on a flash silica gel column eluting with 7/3, 1/1 hexane/ethyl acetate to afford Compound E (0.141 g, 63 %).
MS: $[M+H]^+$ $867^+$
IR: (KBr) 2926, 1636, 1165, 702 $cm^{-1}$

**F. (S*,R*)-N-[[2-[N-[3-Mercapto-2-(methylamino)-propyl]-L-valyl]-1,2,3,4-tetrahydro-3-isoquinolinyl]carbonyl]-L-methionine, trifluoro acetate (2:3) salt**

Sodium hydroxide (1N, 0.32 mL, 0.012 g, 0.31 mmol) was added to a solution of Compound E (0.23 g, 0.26 mmol) in methanol (2 mL). After stirring for 3 hours at room temperature, the reaction was quenched with 10% hydrochloric acid (15 mL) and ethyl acetate (20 mL). The layers were separated and the aqueous layer was extracted with ethyl acetate (2 x 20 mL). The combined organic layers were washed with 10% hydrochloric acid (1 x 10 mL) and brine (1 x 20 mL). The organic layer was dried over magnesium sulfate, filtered and concentrated to afford the acid. The product was purified by prep HPLC (YMC S-10, ODS, 30 x 500 mm, C18 column) eluting with 10-90% aqueous methanol containing 0.1% trifluoroacetic acid, gradient over 30 minutes. The fractions with product were concentrated and lyophilized (0.13 g, 59 %).

This solid was converted to Compound F (0.072 g, 39%) using the method described in Example 34D.

| Elemental analysis for $C_{24}H_{38}N_4O_4S_2$ . 0.35 $H_2O$. 1.6 trifluoroacetic acid | | | |
|---|---|---|---|
| | C | H | N |
| Calculated: | 46.71 | 5.81 | 8.01 |
| Found: | 46.71 | 5.88 | 7.84 |

TLC: $R_f$ 0.32 (9/1/0.1,trichloromethane/ methanol/acetic acid), Visualized by UV, PMA HPLC $R_t$ 17.15 minutes (99%), 10-90% aqueous methanol with 0.2% phosphoric acid buffer, 30 minutes gradient, 1.5 mL/minute, 220 nm, $C_{18}$ (6.0 x 150 mm) column
IR: (KBr) 2421, 1670, 1435, 1202, 1136, 721 $cm^{-1}$
MS: $[M+H]^+$ $511^+$
$[\alpha]_D$ = -25.5° (c = 0.2, methanol)

**Example 60**

**(S*,R*)-N-[[1,2,3,4-Tetrahydro-2-[N-[3-mercapto-2-[(phenylmethyl)amino]propyl]-L-valyl]-3-isoquinolinyl]carbonyl]-L-methionine, trifluoroacetate (2:3) salt**

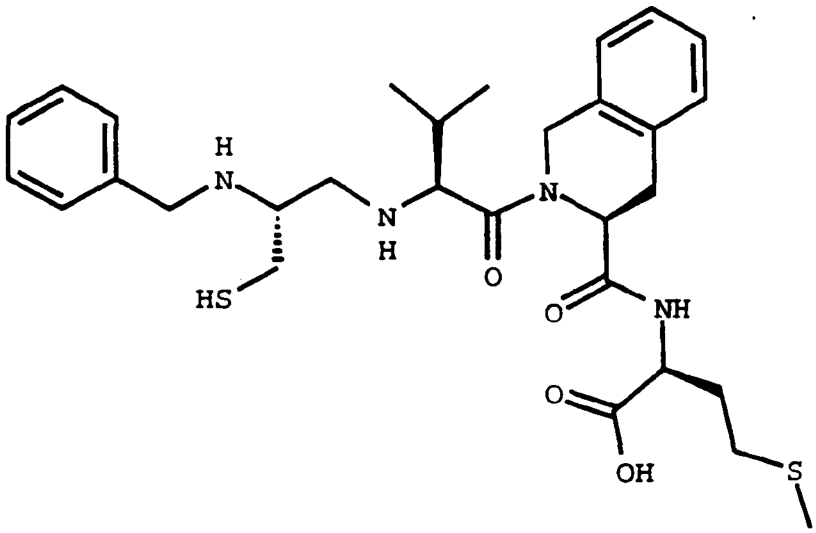

**A. (S*,R*)-N-[[1,2,3,4-Tetrahydro-2-[N-[2-[(phenylmethyl)amino]-3-[(triphenylmethyl)thio]propyl]-L-valyl]-3-isoquinolinyl]carbonyl]-L-methionine, methyl ester**

Compound 1G (400 mg, 0.469 mmol) was stirred in trifluoroacetic acid (7.5 g, 5.0 mL, 65.8 mmol) and dichloromethane for 30 minutes. The mixture was concentrated, dissolved in methylene chloride (50 mL) and concentrated. This latter procedure was repeated five times to yield a clear glass. This, and benzaldehyde (50 mg, 0.05 mL, 0.469 mmol), were dissolved in methanol (10 mL) and glacial acetic acid (0.2 mL). Sodium cyanoborohydride (30 mg, 0.469 mmol) was added portionwise over 15 minutes. The reaction mixture was stirred for 16 hours. The mixture was cooled to 0°C and saturated sodium bicarbonate (10 mL) was slowly added. The product was then extracted into ethyl acetate (60 mL). The ethyl acetate layer was washed with water (100 mL) and brine (100 mL), and dried (magnesium sulfate), concentrated and chromatographed (silica gel, 4.1 X 15 cm, 95% dichloromethane, 5% methanol). Fractions containing the desired compound were collected and concentrated to yield Compound A as a clear oil (270 mg, 68%).
$(M+H)^+$ $843^+$

**B. (S*,R*)-N-[[1,2,3,4-Tetrahydro-2-[N-[3-mercapto-2-[(phenylmethyl)amino]propyl]-L-valyl]-3-isoquinolinyl]carbonyl]-L-methionine, trifluoroacetate (2:3) salt**

The title compound (40 mg) was prepared using Compound A (270 mg, 0.32 mmol) in the two step procedure described in Example 1H.
Purity > 94% , $[\alpha]_D 25°$ = - 18.0° (c=0.1, methanol)
MS $(M+H)^+$ $587^+$
Analysis: Calculated for 1.69 trifluoroacetic acid · $2.18H_2O$: C, 48.97; H, 5.91; N, 6.84
Found: C, 48.96; H, 5.57; N, 6.71
$^1$H-NMR ($CD_3OD$, 270 MHz) δ (ppm) 7.42 (5H, m), 7.21 (4H, m), 4.93 (1H, m), 4.73 (1H, m), 4.51 (2H, m), 4.28 (1H, m), 3.52 (1H, m), 3.43 (1H, m), 3.18 (4H, m), 2.87 (3H, m), 1.98 (2H, m), 1.94 (3H, s), 1.87 (2H, m), 1.67 (1H, m), 1.06 (3H, d), 0.97 (3H, d)

## Example 61

**(S*,R*)-N-[[2-[N-[2-(Acetylamino)-3-mercaptopropyl]-L-valyl]-1,2,3,4-tetrahydro-3-isoquinolinyl]carbonyl]-L-methionine, trifluoroacetate (4:5) salt**

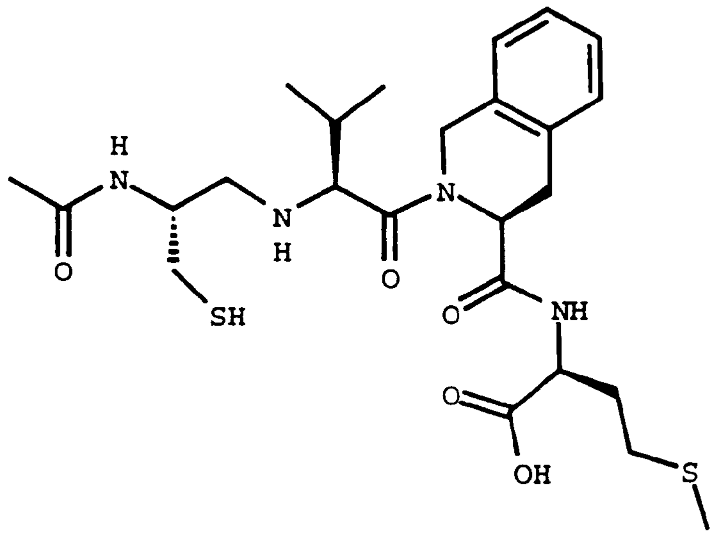

**A. (S*,R*)-N-[[2-[N-[2-Amino-3-[(triphenylmethyl)thio]propyl]-L-valyl]-1,2,3,4-tetrahydro-3-isoquinolinyl]carbonyl]-L-methionine, methyl ester**

Compound 1G (230 mg, 0.469 mmol) was stirred in trifluoroacetic acid (7.5 g, 5.0 mL, 65.8 mmol) and methylene chloride (5.0 mL) for 30 minutes. The mixture was concentrated, dissolved in methylene chloride (50 mL) and concentrated. This latter procedure was repeated five times to yield Compound A as a clear glass (200 mg, 99%), $(M+H)^+$ $753^+$.

**B. (S*,R*)-N-[[2-[N-[2-(Acetylamino)-3-[(triphenylmethyl)thio]propyl]-L-valyl]-1,2,3,4-tetrahydro-3-isoquinolinyl]carbonyl]-L-methionine, methyl ester**

Compound A (200 mg, 0.266 mmol) was dissolved in dichloromethane (10 mL). N,N-Diisopropylethyl amine (74 mg, 0.1 mL, 0.574 mmol) and acetic anhydride (55 mg, 0.1 mL, 0.541 mmol) were added. The mixture was stirred for 2 hours, concentrated and chromatographed (silica gel, 4.1 X 15 cm, 50% ethyl acetate, 50% hexane). Fractions containing the desired compound were collected and concentrated to yield Compound B as a clear oil (100 mg, 47%).
$(M+H)^+$ $795^+$

**C. (S*,R*)-N-[[2-[N-[2-(Acetylamino)-3-mercaptopropyl]-L-valyl]-1,2,3,4-tetrahydro-3-isoquinolinyl]carbonyl]-L-methionine, trifluoroacetate (4:5) salt**

The title compound (38 mg) was prepared using Compound B (100 mg, 0.126 mmol) in the method described in Example 1H.
M.P. 98-99°C, Purity > 98.9 %
$[\alpha]_D^{25}$ = - 41.0° (c=0.1, methanol)
MS $(M+H)^+$ $539^+$
Analysis: Calculated for 1.25 trifluoroacetic acid · 0.96 water: C, 47.28; H, 5.94; N, 8.02;
Found: C, 47.28; H, 5.60; N, 7.81
$^1$H-NMR ($CD_3OD$, 400 MHz) δ (ppm) 7.28 (4H, m), 4.84 (2H, m), 4.65 (1H, m), 4.52 (2H, m), 4.31 (1H, m), 4.09 (1H, d, J=6.4 Hz), 3.63 (1H, m), 3.48 (2H, m). 3.14 (1H, m), 2.68 (2H, m), 2.38 (2H, m), 1.99 (3H, m), 1.92 (1H, m), 1.89 (3H, s), 1.77 (1H, m), 1.66 (1H, m), 1.14 (6H, m)

## Example 62

**[R*[S*(S*)]]-N-[[2-[N-[2-amino-3-[(2-amino-2-carboxyethyl)dithio]propyl]-L-valyl]-1,2,3,4-tetrahydro-3-isoquinolinyl]carbonyl]-L-methionine**

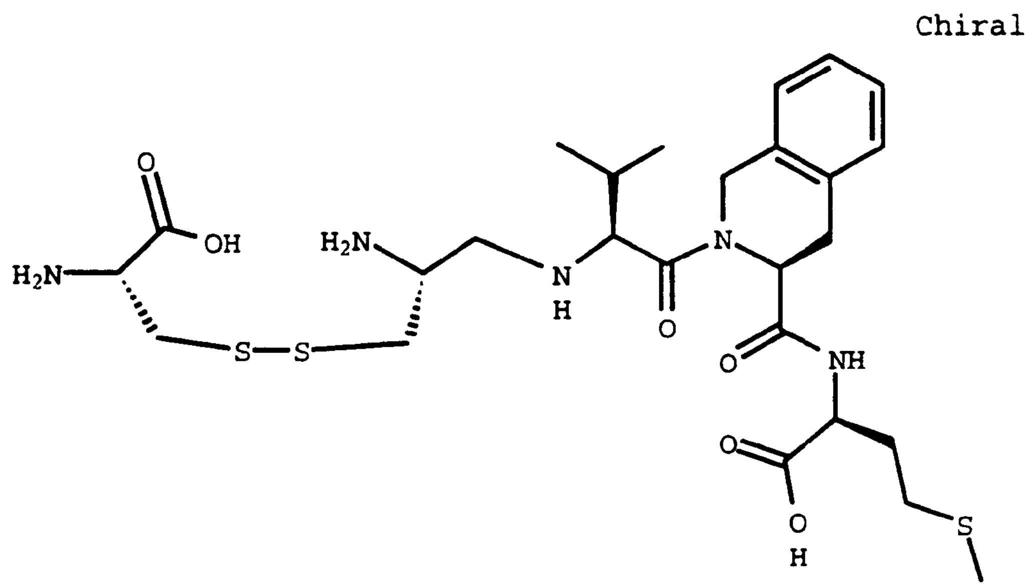

**A. [R*[S*(S*)]]-N-[[2-[N-[2- amino-3-[(2-amino-2-carboxyethyl)dithio]propyl]-L-valyl]-1,2,3,4-tetrahydro-3-isoquinolinyl]carbonyl]-L-methionine**

Cystine (59 mg, 0.24 mmol) was combined with Compound 1H (30 mg, 0.06 mmol) in 2 mL of 0.2 M Hepes buffer adjusted to pH 7.4. The solution was allowed to stand for 5 hours followed by HPLC purification (YMC S-10, ODS 30 x 500 mm, monitoring at 220 nm, flow rate, 20 mL/minute, 30-50% B over 45 minutes). The appropriate fractions were collected and lyophilized to give 14 mg (38%) of the title compound.

mp 130-132°C

MS: $(M+H)^+$ $616^+$

$^1$H-NMR($CD_3OD$, 270 MHz) δ (ppm) 7.14 (4H, m), 4.71 (1H, m), 4.60 (1H, m), 4.45 (1H, d), 4.30 (1H, d), 4.07 (2H, m) 3.70 (1H, m), 3.55(1H,m), 3.47(1H, m), 3.07(2H, dd). 2.87(1H,m), 2.75(1H,m), 2.6(1H, m), 2.40(1H,m), 2.1-1.75(2H,m), 1.95 (3H, 2s), 0.99 (6H, m)

Elemental Analysis for $C_{26}H_{41}N_5O_6S_3 \cdot 4C_2HF_3O_2$, 1.3 water:

Calculated: C 37.28, H 4.38, N 6.39,

Found:C 37.6, H 4.81, N 6.70

$[\alpha]_D^{25}$; -84° (c = 0.05, methanol)

C-18 (YMC ODS, 5 micron, 120 A , 6 x 150 mm) , 220 nm, isocratic conditions at 10-90% aqueous methanol over 30 minutes containing 0.2% phosphoric acid, 1.5 mL/minute monitoring at 220 nm

RT 15.05 minutes

HI = >98%

**Example 63**

**(S*,R*)-N,N'-Dithiobis(2-amino-3,1-propanediyl)bis[N-[[2-(L-valyl)-1,2,3,4-tetrahydro-3-isoquinolinyl]carbonyl]-L-methionine]**

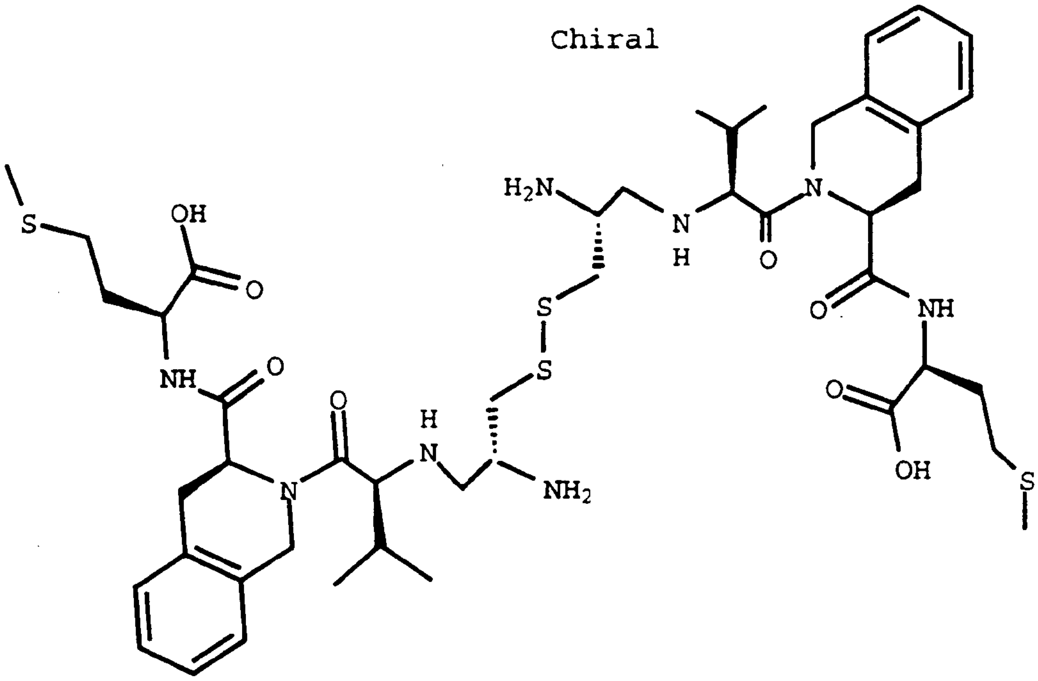

**A. (S*,R*)-N,N'-Dithiobis(2-amino-3,1-propanediyl)bis[N-[[2-(L-valyl)-1,2,3,4-tetrahydro-3-isoquinolinyl]carbonyl]-L-methionine]**

Compound 1H (70 mg, 0.141 mmol) was dissolved in 0.1% trifluoroacetic acid in water (10 mL). The solution was allowed to stand at room temperature for 72 hours and lyophilized. This crude solid was purified by preparative HPLC (YMC-C18 column, 2.2 X 25 cm, 5 micron, 120 angstrom; solvent A, 0.1% trifluoroacetic acid in water; solvent B, 0.1% trifluoroacetic acid in acetonitrile: 30-40% B in 40 minutes, flow rate 9 mL/minute; UV monitored at 220 nm). Fractions containing the desired product were combined and lyophilized to provide the title compound (30 mg, 43%).

M.P. 115-116°C

Purity > 96%

$[\alpha]_D^{25}$ = °-42.2 (c=0.09, methanol)

MS $(M+H)^+$ $991^+$

Analysis: Calculated for 4.0 trifluoroacetic acid · 3.71 water: C, 42.83; H, 5.42; N, 7.40;

Found: C, 42.83; H, 5.28; N, 7.47

$^1$H-NMR ($CD_3OD$, 400 MHz) δ (ppm) 7.26 (8H, m), 4.84 (8H, m), 4.55 (2H, m), 4.38 (2H, m), 3.80 (2H, m), 3.57 (2H, m), 2.95 (4H, m), 2.72 (2H, m), 2.48 (2H, m), 2.31 (2H, m), 2.13 (4H, m), 2.05 (6H, m), 1.96 (4H, m), 1.07 (12H, m)

## Example 64

**(S*,R*)-N-[[2-[N-[2-Amino-3-(methyldithio)propyl]-L-valyl]-1,2,3,4-tetrahydro-3-isoquinolinyl]carbonyl]-L-methionine, trifluoroacetate (1:2) salt**

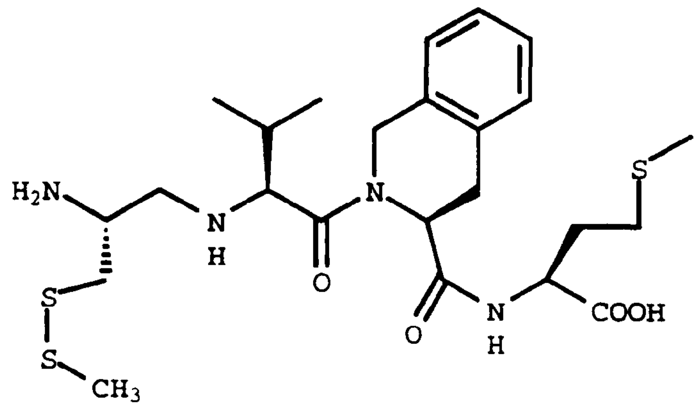

**A. (S*,R*)-N-[[2-[N-[2-Amino-3-(methyl dithio)propyl]-L-valyl]-1,2,3,4-tetrahydro-3-isoquinolinyl]carbonyl]-L-methionine, trifluoroacetate (1:2) salt**

To a solution of Compound 1H (100 mg, 0.20 mmol) in a mixture of methanol/water (0.5/0.3 mL, deoxygenated) at 0°C under argon was added methyl methanethiosulfonate (36 μL, 0.35 mmol). The reaction mixture was warmed to and stirred at 5°C overnight (16 hours). The volatiles were removed *in vacuo.* The residue was purified by preparative RP HPLC (YMC, S10, 30 x 500 mm, C18 column) eluting with 36% to 70% aqueous methanol over 1 hour. Appropriate fractions were collected and lyophilized to afford white lyophilate of the title compound (65 mg).

m.p. 77-80°C

MS (FAB): $(M+H)^+= 543$

IR (KBr) 2974, 1676, 1431, 1204, 1140 $cm^{-1}$

$[\alpha]_D = -45.9°$ (c = 0.18, methanol)

HPLC: YMC, S3, C18 (6 x 150 mm) column, 220 nm, 1.5 mL/minute: 10-90% aqueous methanol with 0.2% phosphoric acid, linear gradient over 30 minutes Retention time: 18.8 minutes.

| Elemental analysis for $C_{24}H_{38}N_4O_4S_3$. 1.6 $H_2O$ . 2.1 trifluoroacetic acid: | | | | | |
|---|---|---|---|---|---|
| | C | H | N | S | F |
| Calculated: | 41.76 | 5.38 | 6.91 | 11.86 | 14.76 |
| Found: | 41.75 | 5.23 | 6.77 | 11.88 | 14.70 |

$^1H$ NMR ($CDCl_3$, with 5% $CD_3OD$, 400 MHz): δ 7.29-7.20 (4H, m), 4.92 (1H, d, J=16.7 Hz), 4.77 (1H, m), 4.56 (1H, d, J =16.7 Hz), 4.48 (1H, m), 3.50-2.90 (6H, m), 2.44 and 2.34 (3H, two s), 2.2-2.1 (2H, m), 2.04 andl.91 (3H, two s), 1.80 -1.60 (3H, m), 1.11 (3H, d, J = 6.8 Hz) 1.09 (6H, d, J = 6.4 Hz)

**Example 65**

**N-[[(S)-1,2,3,4-Tetrahydro-2-[N-[[(4R)-2-methyl-4-thiazolidinyl]methyl]-L-valyl]-3-isoquinolinyl]carbonyl]-L-methionine, trifluoroacetate (1:2) salt**

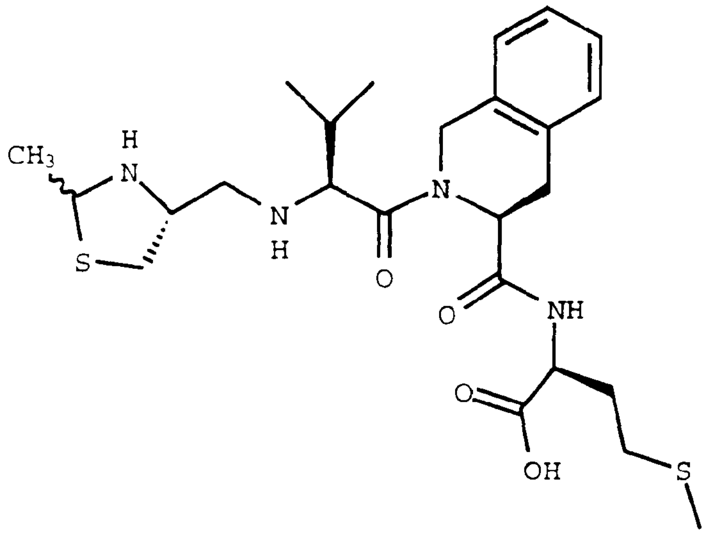

**A. N-[[(S)-1,2,3,4-Tetrahydro-2-[N-[[(4R)-2-methyl-4-thiazolidinyl]methyl]-L-valyl]-3-isoquinolinyl]carbonyl]-L-methionine, trifluoro acetate (1:2) salt**

Compound 1H (60 mg, 0.121 mmol) was dissolved in dichloromethane (10 mL). The solution was cooled to 0°C and acetaldehyde (0.39 g, 0.50 mL, 8.94 mmol) was added. The mixture was stirred for 1 hour at 0°C. The solvent was evaporated to half the volume under a stream of nitrogen and the solid was filtered and dried under a vacuum for 5 hours to provide the title compound (35mg, 55%).

M.P. 156-157°C

Purity > 93%

$[\alpha]_D^{25}$ = - 8.0 (c=0.1, methanol)

MS $(M+H)^+$ $523^+$

Analysis: Calculated for 1.90 trifluoroacetic acid · 1.25 water: C, 45.40; H, 5.61; N, 7.35;

Found: C, 45.39; H, 5.47; N, 7.00

$^1$H-NNR ($CD_3OD$, 400 MHz) d (ppm) 7.27 (4H, m), 4.75 (2H, m), 4.65 (1H, m), 4.56 (2H, m), 4.11 (1H, m), 4.03 (1H, m), 3.21 (1H, m), 3.16 (3H, m), 3.00 (1H, m), 2.75 (1H, m), 2.55 (2H, m), 2.42 (1H, m), 2.14 (1H, m), 2.07 (3H, m), 1.93 (1H, m), 1.43 (3H, m), 1.19 (3H, d, J=6.8 Hz), 1.14 (3H, d, J=7.3 Hz)

**Example 66**

**(S*,R*)-N-[[1,2,3,4-Tetrahydro-2-[N-(4-thiazolidinylmethyl)-L-valyl]-3-isoquinolinyl]carbonyl]-L-methionine, trifluoroacetate (1:5) salt**

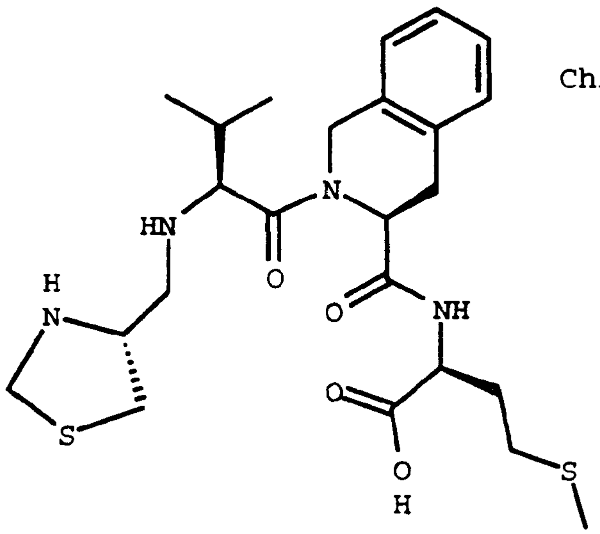

Chiral

**A. (R)-4-[(N-Methoxy-N-methylamino)carbonyl]-3-thiazolidinecarboxylic acid, 1,1-dimethylethyl ester**

(R)-3,4-Thiazolidinedicarboxylic acid, 3-(1,1-dimethylethyl) ester (1g, 4.97 mmol), bromotris-pyrrolidinophosphonium hexafluorophosphate (5.1g, 10.93 mmol), and dimethylaminopyridine (200 mg, 1.6 mmol) were dissolved in methylene chloride (20 mL). N,O-Dimethylhydroxylamine hydrochloride (1.07 g, 10.93 mmol) and N,N-diisopropylethylamine (4.5 mL, 25.7 mmol) were added. The reaction mixture stirred for 1 hour at room temperature and was concentrated and chromatographed (silica gel, 7.1 X 20 cm, 40% ethyl acetate, 60% hexane). Fractions containing the desired compound were collected and concentrated to yield Compound A as a white solid (700 mg, 51%).
$(M+H)^+$ $277^+$

**B. (R)-4-Formyl-3-thiazolidinecarboxylic acid, 1,1-dimethylethyl ester**

Compound B (500 mg, 92%) was prepared as a white glass using Compound A (0.7 g, 2.5 mmol) in the method described in Example 1F.

**C. (S*,R*)]-N-[[2-[N-[[3-[(1,1-Dimethylethoxy)carbonyl]-4-thiazolidinyl]methyl]-L-valyl]-1,2,3,4-tetrahydro-3-isoquinolinyl]carbonyl]-L-methionine, methyl ester**

Compound C (400 mg, 25%) was prepared using Compound B (0.5g, 2.3 mmol) and Compound 1D (1.6 g, 2.5 mmol) in the method described in Example 1G.
MS: $(M+H)^+$ $622^+$.

**D. (S*, R*)]-N-[[2-[N-[[3-[(1,1-Dimethylethoxy)carbonyl]-4-thiazolidinyl]methyl]-L-valyl]-1,2,3,4-tetrahydro-3-isoquinolinyl]carbonyl]-L-methionine**

To 25 mL of methanol was added Compound C (300 mg, 0.48 mmol) along with 2N sodium hydroxide adjusted to pH 12. This was allowed to stir until there was quantitative formation of the desired compound as detected by tlc (chloroform, methanol, acetic acid/85:10:5) (3 hours). The pH was adjusted to 3-4 using 1N potassium bisulfate and ethyl acetate (30 mL) was added. The layers were separated and the aqueous layer was washed with ethyl acetate (3 x 30 mL). The organic extracts were pooled, dried (magnesium sulfate) and concentrated to give 312 mg of Compound D (98%).
MS $(M+H)^+$ $609^+$.

**E. (S*,R*)-N-[[1,2,3,4-Tetrahydro-2-[N-(4-thiazolidinylmethyl)-L-valyl]-3-isoquinolinyl]carbonyl]-L-methionine, trifluoroacetate (1:5) salt**

Compound D 250 mg (0.41 mmol) was combined with trifluoroacetic acid (10 mL), methylene chloride (5 mL) and dimethylsulfide (0.5 mL). The solution was stirred for 1 hour, followed by concentration under vacuum. Methylene chloride was added and the solution was reconcentrated and thoroughly dried under vacuum. Yield of residue, 240 mg (quantitative yield). HPLC purification was performed under the following conditions; Solvent A; 0.1% trifluoroacetic acid in water, Solvent B; 0.1% trifluoroacetic acid in methanol (YMC S-10, ODS 30 x 500 mm, monitoring at 220 nm, flow rate, 20 mL/minute, 30-50% B over 45 minutes). Fractions containing the major peak (220 nm) were pooled and lyophilized to yield 194 mg (67%), of the title compound as a white powder.

MS $(M+H)^+$ $509^+$

$^1$H-NMR($CD_3OD$, 270 MHz) d (ppm) 7.17 (4H, m), 4.67 (4H, m), 4.20 (2H, m), 3.28 (2H, m), 3.06(2H, m), 2.89(1H. q) 2.40 (1H, m), 2.08 (1H, m), 1.95(2H, s), 1.89 (1H, s) 1.09 (6H, m)

Elemental Analysis for $C_{24}H_{36}N_4O_4S_2 \cdot 1.4C_2HF_3O_2$, 1.53 water:

Calculated: C 46.26, H 5.86, N 8.05, S 9.21 F 11.47

Found: C 46.26, H 5.70, N 7.82, S 9.03, F 11.46

$[\alpha]_D^{25}$; -30° (c = 0.94, methanol)

C-18 (YMC ODS, 5 micron, 120 A, 6 x 150 mm) , 220 nm, isocratic conditions at 10-90% aqueous methanol over 30 minutes containing 0.2% phosphoric acid, 1.5 mL/minute monitoring at 220 nm

RT 17.34 minutes

HI = >97%

**Example 67**

**(S*,R*)-N-[[2-[N-[2-(Acetylamino)-3-(acetylthio)propyl]-L-valyl]-1,2,3,4-tetrahydro-3-isoquinolinyl]carbonyl]-L-methionine, trifluoroacetate (2:3) salt**

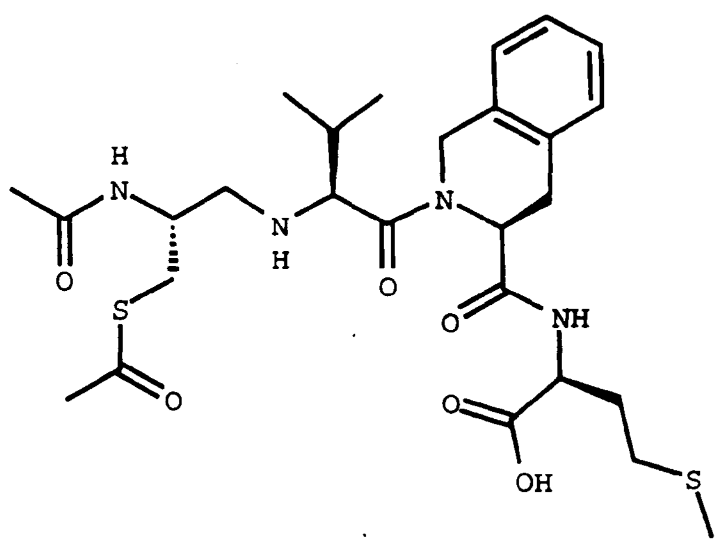

**A. (S*,R*)-N-[[2-[N-[2-(Acetylamino)-3-(acetylthio)propyl]-L-valyl]-1,2,3,4-tetrahydro-3-isoquinolinyl]carbonyl]-L-methionine, trifluoroacetate (2:3) salt**

Compound 1H (70 mg, 0.141 mmol) was dissolved in dichloromethane (10 mL). N,N-Diisopropylethyl amine (74 mg, 0.1 mL, 0.574 mmol) and acetic anhydride (55 mg, 0.1 mL, 0.541 mmol) were added. The mixture was stirred for 2 hours, concentrated and purified by preparative HPLC (YMC S-10 ODS column, 30 X 500 mm; solvent A, 0.1% trifluoroacetic acid in 90% water, 10% methanol; solvent B, 0.1% trifluoroacetic acid in 10% water, 90% methanol: 50-100% B in 70 minutes, flow rate 20 mL/minute; UV monitored at 220 nm). Fractions containing the desired product were combined and lyophilized to provide the title compound (30 mg).

M.P. 91-92°C

Purity > 97.9 %

$[\alpha]_D^{25}$ = -37° (c=0.1, methanol)
MS $(M+H)^+$ $581^+$
Analysis: Calculated for 1.50 trifluoroacetic acid · 0.13 $H_2O$: C, 47.78; H, 5.58; N, 7.43;
Found: C, 47.78; H, 5.78; N, 7.55
$^1$H-NMR ($CD_3OD$, 400 MHz) δ (ppm) 7.28 (4H, m), 4.89-4.68 (2H, m), 4.53 (2H, m), 4.32 (1H, m), 4.12 (1H, d, J=7.3 Hz), 3.66 (1H, m), 3.49 (1H, d, J=12.8 Hz). 3.34 (1H, d, J=10.7 Hz), 3.18-3.00 (3H, m), 2.40 (3H, s), 2.35 (1H, m), 2.01 (1H, m), 1.95 (3H, s), 1.89 (3H, s), 1.85 (1H, m), 1.71 (1H, m), 1.61 (1H, m), 1.13 (6H, m)

**Example 68**

**N-[[(S)-1,2,3,4-Tetrahydro-2-[N-(2-hydroxy-3-mercaptopropyl)-L-valyl]-3-isoquinolinyl]carbonyl]-L-methionine, trifluoroacetate (1:1) salt**

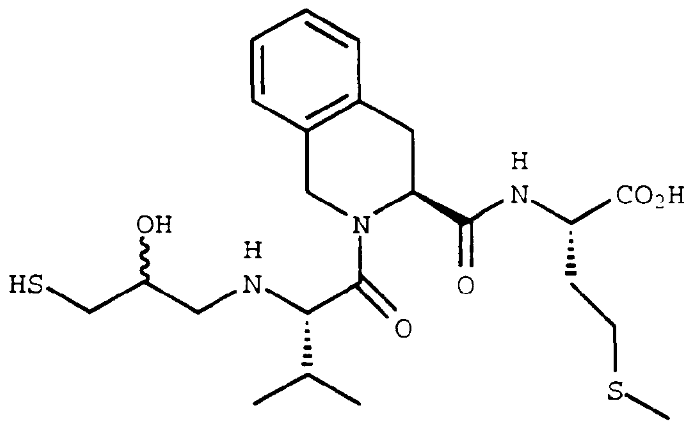

**A. [[(Triphenylmethyl]thio]methyl]oxirane**

A mixture of triphenylmethyl mercaptan (2.76 g, 10 mmol), (±)-epichlorohydrin (1 mL, 12 mmol), and cesium carbonate (4.87 g, 15 mmol) in dimethylformamide (25 mL) was stirred at 25°C for 4 hours, after which it was poured into water and extracted with ethyl acetate. The extract was dried and concentrated to an oil. The oil was purified by flash chromatography on silica gel (300 g), eluting with 4:1 hexane:ethyl acetate, to give an oil. Trituration with petroleum ether containing a few drops of diethyl ether gave Compound A as a white solid (2.0 g, 60%).

**B. N-[[(S)-1,2,3,4-Tetrahydro-2-[N-[2-hydroxy-3-[(triphenylmethyl)thio]propyl]-L-valyl]-3-isoquinolinyl]carbonyl]-L-methionine, methyl ester**

A mixture of Compound A (500 mg, 1.5 mmol), Compound 1D (640 mg, 1.4 mmol) and triethylamine (0.28 mL, 2.0 mmol) in methanol (7 mL) was heated at reflux for 15 hours, after which it was concentrated *in vacuo*. The residue was partitioned between ethyl acetate and sodium bicarbonate, and the aqueous layer was further extracted with ethyl acetate (3x). The combined extract was dried and concentrated. The residue was purified by flash chromatography on silica gel (300 g), eluting with a step gradient from 20:20:1 to 0:10:1 hexane:ethyl acetate: [pyridine 20:acetic acid 6:water 11], to give Compound B as a golden oil (540 mg, 51%).

**C. N-[[(S)-1,2,3,4-Tetrahydro-2-[N-[2-hydroxy-3-[(triphenylmethyl)thio]propyl]-L-valyl]-3-isoquinolinyl]carbonyl]-L-methionine**

A mixture of Compound B (400 mg, 0.53 mmol), tetrahydrofuran (10 mL), methanol (10 mL) and 1N sodium hydroxide solution (2 mL) was stirred at 25°C for 30 minutes, after which it was poured into excess 1N hydrochloric acid and extracted with ethyl acetate. The extract was dried and concentrated to give Compound C (350 mg, 89%).

**D. N-[[(S)-1,2,3,4-Tetrahydro-2-[N-(2-hydroxy-3-mercaptopropyl)-L-valyl]-3-isoquinolinyl]-carbonyl]-L-methionine, trifluoroacetate (1:1) salt**

A mixture of Compound C (330 mg, 0.45 mmol), trifluoroacetic acid (20 mL) and triethylsilane (1 mL) was stirred for one hour at 25°C, after which it was concentrated *in vacuo*. The residue was purified by preparative HPLC (YMC S-10 ODS column, 30 x 500 mm, eluting with 50 mL/minute of a linear gradient from 32% to 90% aqueous methanol containing 0.1% trifluoroacetic acid over 30 minutes). Fractions were monitored by UV absorbance at 220 nm. Those containing the first major product to elute were combined and concentrated. The residue was lyophilized to give the title compound as a hygroscopic white solid (100 mg, 42%).
mp: 91-105°C
$[\alpha]_D$: -33° (c = 0.6, ethanol)
IR (KBr): 3432, 2974, 2575, 1672, 1437 $cm^{-1}$
MS: $498^+$ $(M+H)^+$
$^1H$ NMR: ($CD_3OD$) 1.18 (6H, m), 2.07 (3H, s), 1.8-4.9 (17H, m)
$^{13}C$ NMR: ($CD_3OD$) 174.8, 173.3, 168.2, 168.2, 135.4, 135.3, 129.3, 129.2, 128.4, 128.2, 126.9, 126.8, 69.8, 68.7, 64.1, 63.3, 57.5, 51.7, 51.6, 32.5, 31.7, 31.0, 29.6, 29.3, 18.8, 18.5, 18.3, 18.2, 18.0, 15.2 ppm
TLC: $R_f$ 0.67, EM silica gel, 1:1 ethyl acetate: [pyridine 20:acetic acid 6:water 11], UV + PMA stain
HPLC: > 97% at 17.8 minutes (YMC S3 ODS column, 6.0 x 150 mm, eluted with a 30 minute linear gradient from 10% to 90% aqueous methanol containing 0.2% phosphoric acid, monitored at 220 nm)

| Analysis Calculated for $C_{23}H_{35}N_3O_5S_2$ . 1.00 trifluoroacetic acid . 0.5 water: | | | | | |
|---|---|---|---|---|---|
| | C | H | N | S | F |
| Calculated: | 43.38 | 6.01 | 6.77 | 10.33 | 9.18 |
| Found: | 43.38 | 6.18 | 6.67 | 10.48 | 9.29 |

**Example 69**

**(S*, R*) -N-[[1,2,3,4 -Tetrahydro-2-[N-[3-mercapto-2-[(3-phenylpropyl)amino]propyl]-L-valyl]-3-isoquinolinyl]carbonyl]-L-methionine, trifluoroacetate (1:2) salt**

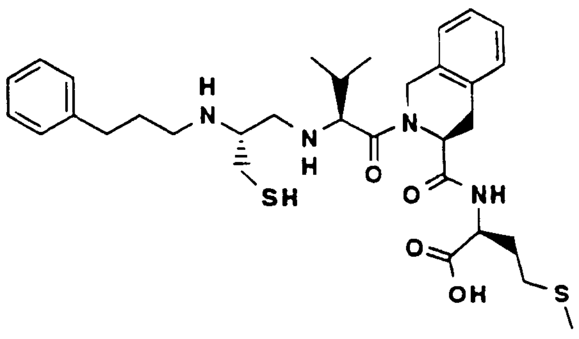

**A. (S*,R*)-N-[[1,2,3,4-Tetrahydro-2-[N-[2-[(3-phenylpropyl)amino]-3-[(triphenylmethyl)thio]propyl]-L-valyl]-3-isoquinolinyl]carbonyl]-L-methionine, methyl ester**

Compound 61A (450 mg, 0.598 mmol) and hydrocinnamaldehyde (80 mg, 0.080 mL, 0.598 mmol) were used in the method described in Example 5C to prepare Compound A as a clear oil (300 mg, 58%), $(M+H)^+$ $871^+$.

**B. (S*,R*)-N-[[1,2,3,4-Tetrahydro-2-[N-[3-mercapto-2-[(3-phenylpropyl)amino]propyl]L-valyl]-3-isoquinolinyl]carbonyl]-L-methionine, trifluoro acetate (1:2) salt**

Compound A (300 mg, 0.34 mmol) was used in the two step procedure described in Example 1H to prepare the title compound (57 mg, 20 %), Purity > 99 % (YMC S3 ODS column, 6 X 150 mm, 220 nm, 1.5 mL/min, 0-100% B in A over 30 minutes (A, 0.2% $H_3PO_4$ in 90% $H_2O$, 10% methanol; B, 0.2% $H_3PO_4$ in 90% methanol, 10% $H_2O$), RT 22.96 minute. HI>99%), $[\alpha]_D25°$ = - 41.0° (c=0.1, methanol), MS $(M+H)^+$ $615^+$, Analysis: Calculated for 1.80TFA· $0.55H_2O$: C, 51.52; H, 5.94; N, 6.75 Found: C, 51.54; H, 5.77; N, 6.65. $^1H$-NMR ($CD_3OD$, 270 MHz) δ (ppm) 7.33-7.16 (9H, m), 4.84 (2H, m), 4.72 (1H, m), 4.69-4.59 (2H, m), 4.55 (1H, m), 4.37 (1H, m), 3.36 (2H, m), 3.16-2.97 (3H, m), 2.85 (2H, m), 2.80-2.68 (2H, m), 2.48 (1H, m), 2.29 (1H, m), 2.21-2.08 (2H, m), 2.02 (3H, s), 1.99 (2H, m), 1.90 (1H, m), 1.15-0.92 (6H, m).

**Example 70**

**(S*,R*)-$N^2$-[[2-[N-(2-Amino-3-mercaptopropyl)-L-valyl]-1,2,3,4-tetrahydro-3-isoquinolinyl]-carbonyl]-N-hydroxy-N-methyl-L-methioninamide, trifluoroacetate (1:2) salt**

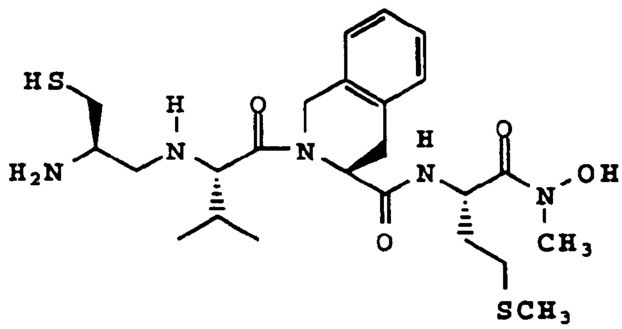

**A. (S*,R*)-$N^2$-[[2-[N-[2-[[(1,1-Dimethylethoxy)carbonyl]amino]-3-[(triphenylmethyl)thio]propyl]-L-valyl]-1,2,3,4-tetrahydro-3-isoquinolinyl]carbonyl]-N-methyl-N-hydroxy-L-methioninamide**

To a solution of Compound 1G (9.8 g, 0.176 mmol) in tetrahydrofuran:methanol (70 mL, 5:2) was added 2N lithium hydroxide (15 mL, 30 mmol). After stirring for 20 hours, more 2N lithium hydroxide (7.5 ml, 15 mmol) was added. After stirring for 20 more hours, 1N hydrochloric acid (40 mL, 40 mmol) was added, and the reaction was concentrated to 60 mL. The aqueous solution was extracted with chloroform (3 x 200 mL), dried over sodium sulfate, filtered and concentrated to give a white solid (9.30 g, 100%).

To a solution of this solid (400 mg, 0.477 mmol) in dimethylformamide (2 mL) at 0° under nitrogen was added N-methylhydroxylamine hydrochloride (40 mg, 0.477 mmol), diisopropylethylamine (166 μl, 0.984 mmol), and benzotriazol-1-yloxytris(dimethylamino)phosphonium hexafluorophosphate (211 mg, 0.477 mmol). After stirring the mixture at 0° for 20 hours, the reaction was diluted with ethyl acetate (100 mL) and washed sequentially with saturated sodium bicarbonate (40 mL), 10% citric acid (40 mL) and 10% lithium chloride (3 x 40 mL), dried over sodium sulfate, filtered and concentrated *in vacuo*. The residue was purified on silica gel (100 ml, eluting with hexane:acetone:methanol, 5:2:0 to 1:1:0.01) to give Compound A (288 mg, 70%, white solid).
mp: 83-86°
MS: $(M+H)^+$ $868^+$

**B. (S*,R*)-$N^2$-[[2-[N-(2-Amino-3-mercaptopropyl)-L-valyl]-1,2,3,4-tetrahydro-3-isoquinolinyl]carbonyl]-N-hydroxy-N-methyl-L-methioninamide, trifluoroacetate (1:2) salt**

To a solution of Compound A (288 mg, 0.332 mmol) in trifluoroacetic acid (3 mL) at 0° under nitrogen was added triethylsilane dropwise until the yellow color was gone. After stirring 30 minutes at 0°, the mixture was concentrated *in vacuo*. The residue was purified by prep HPLC (YMC ODS S-10, 30 x 500 mm, 22 mL/min,10-90% aqueous methanol, 0.1% trifluoroacetic acid, 60 min). The proper fractions were combined, concentrated to 1/4 volume and lyophilized to give the title compound (85 mg, 32%).
mp: 103-105°
MS: $(M+H)^+$ $526^+$

IR: (KBr) 1678, 1653, 1520, 1433, 1204, 1184 $cm^{-1}$
TLC: $R_f$ =0.18 (chloroform:methanol:acetic acid, 9:1:0.05, visualized by ceric ammonium sulfate) $(\alpha)_D$ = -30.2° [c = 0.24, methanol]
HPLC: 96.6% at 11.2 min, YMC S3 ODS, 6.0 x 150 mm, λ = 220nm, 27% aqueous methanol, 0.2% $H_3PO_4$, 1.5 ml/min
$^1H$ NMR 270MHz ($CD_3OD$): 1.15-1.38 (m, 6H), 1.87-2.62 (m, 8H), 2.92-3.86 (m, 11H plus $CD_3OD$), 4.42-5.23 (m, 4H plus water), 7.32-7.42 (m, 4H)
$^{13}C$ NMR 100MHz ($CD_3OD$) : 15.5, 15.6, 17.4, 17.6, 17.9, 18.2, 18.4, 19.8, 20.2, 25.9, 26.1, 31.3, 31.5, 32.3, 32.5, 32.6, 32.8, 33.0, 33.7, 36.9, 37.0, 46.3, 47.7, 50.6, 51.1, 53.2, 57.6, 57.7, 57.8, 66.3, 66.4, 127.2, 127.8, 128.2, 128.9, 129.0, 129.1, 129.5, 129.7, 133.9, 134.3, 134.6, 135.8, 171.7, 172.6, 173.4, 173.5, 173.8

| Elemental Analysis for : $C_{24}H_{39}N_5O_4S_2 \cdot 1.00\ H_2O \cdot 2.25\ CF_3CO_2H$ | | | | | |
|---|---|---|---|---|---|
| | C | H | N | S | F |
| Calculated: | 42.77 | 5.45 | 8.75 | 8.01 | 16.02 |
| Found: | 42.86 | 5.14 | 8.51 | 7.97 | 16.23 |

**Example 71**

**(S*,R*)-N-[[2-[N-(2-Amino-3-mercaptopropyl)-L-valyl]-1,2,3,4-tetrahydro-3-isoquinolinyl]carbonyl]-O-methyl-L-homoserine, trifluoroacetate (1:2) salt**

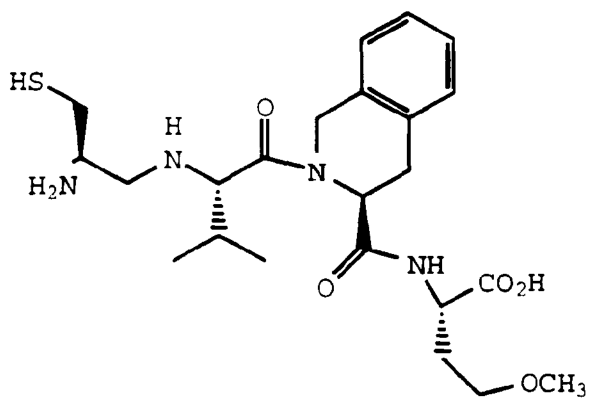

**A. O-Methyl-L-homoserine, methyl ester, hydrochloride salt**

Sodium hydride (60%, 0.365 g, 9.12 mmol) was added to a solution of N-t-butyloxycarbonyl-L-homoserine (1.0 g, 4.56 mmol) in tetrahydrofuran (20 mL) at 0°C. The reaction was stirred for 30 minutes at 0°C, iodomethane (1.14 mL, 18.2 mmol) was added and the reaction warmed to room temperature and stirred for 16 hours. The reaction was quenched with 1N hydrochloric acid (50 ml) and extracted with ethyl acetate (4 x 50 ml). The combined organic extracts were dried (magnesium sulfate), filtered and concentrated under vacuum to afford 1.0 g of a gum, which was used without further purification. MS: $(M+H)^+$ 234

Anhydrous hydrogen chloride was added to a solution of this gum (1.0 g, 4.29 mmol) in methanol (60 ml) at 0°C. The reaction was warmed to room temperature and stirred for 72 hours. The solution was concentrated under vacuum to afford Compound A (0.788 g, 100 %), a gum, which was used without further purification. MS: $(M+H)^+$ 148 free amine.

**B. (S*,R*)-N-[[2-[N-[2-[[(1,1-Dimethylethoxy)carbonyl]amino]-3-[(triphenylmethyl)thio]propyl]-N-[(phenylmethoxy)carbonyl]-L-valyl]-1,2,3,4-tetrahydro-3-isoquinolinyl]carbonyl]-O-methyl-L-homoserine, methyl ester**

Diisopropylethylamine (1.12 mL, 6.45 mmol) was added to a solution of Compound A (0.394 g, 2.15 mmol),

Compound 32E (1.81 g, 2.15 mmol) and benzotriazol-1-yloxytris-(dimethylamino)phosphonium hexafluorophosphate (0.951 g, 2.15 mmol) in acetonitrile (30 mL) and dimethylformamide (10 mL). The mixture was then stirred at room temperature for 16 hours. The reaction was quenched with 1N hydrochloric acid (50 mL), and extracted with ethyl acetate (4 x 50 mL). The combined organic extracts were washed with 10% lithium chloride (3 x 100 mL), dried, filtered and concentrated under vacuum.
The residue was purified by flash chromatography (eluting with 3/1 hexane/acetone) to afford Compound B (1.16 g, 56%), as a white solid.
mp: 57-65°C
TLC: $R_f$ : 0.85 (1/1 hexane/acetone, visualization by ceric stain).
MS: $(M+H)^+$ 971

**C. (S*, R*)-N-[[2-[N-(2-Amino-3-mercaptopropyl)-L-valyl]-1,2,3,4-tetrahydro-3-isoquinolinyl]carbonyl]-O-methyl-L-homoserine, trifluoro acetate (1:2) salt**

Sodium hydroxide (1N, 1.02 mL, 1.96 mmol) was added slowly to a solution of Compound B (0.50 g, 0.52 mmol) in methanol (15 mL) and stirred for 16 hours. The mixture was concentrated under vacuum, diluted with 1N HCl (100 mL) and extracted with dichloromethane (3 x 100 mL). The combined organic extracts were dried (magnesium sulfate), filtered, and concentrated under vacuum to afford 0.497 g of a white solid.
TLC: $R_f$ : = 0.37 (9/1 chloroform/methanol, visualization by UV).
MS: $(M+H)^+$ 979

Trifluoroacetic acid (2.6 mL, 34 mmol) was added to a solution of the solid from above (0.5 g, 0.523 mmol) in dichloromethane (5 mL), and triethylsilane (0.836 mL, 5.23 mmol) at 0°C. The resulting solution was warmed and stirred at room temperature for 1 hour. The reaction was concentrated under vacuum, and the residue was triturated with hexane (2 x 20 mL). The hexane layer was discarded. In a separate flask trifluoroacetic acid (10.3 mL, 133 mmol) was treated with thioanisole (0.614 mL, 5.23 mmol), 1,2 ethanedithiol (0.439 mL, 5.23 mmol), and p-thiocresol (0.649 g, 5.23 mmol) at 0°C. This solution was added to the triturated residue, followed by the dropwise addition of bromotrimethylsilane (0.552 mL, 4.18 mmol, 8). The solution was stirred at 0°C for 1.5 hours. The reaction was treated with diethylether (30 mL) and the resulting precipitate was centrifuged. The supernatant was removed and discarded and the solid was washed and centrifuged with diethylether (2 x 30 mL), ethyl acetate (1 x 30 mL) and diethylether (1 x 30 mL). The solid was purified by preparative HPLC (YMC S-15 ODS 50 x 500 mm, 220 nm, 50 mL/minute, 26%-74% aqueous methanol with 0.1% trifluoroacetic acid gradient). The appropriate fractions were concentrated under vacuum at room temperature, and the residue was lyophilized to afford the title compound (0.197 g, 53%).
mp: 78-88°C
TLC: $R_f$ = 0.65 (6/3/1 n-propanol/ammonium hydroxide/ water, visualization by ceric ammonium sulfate/ ammonium molybdate/ sulfuric acid/ water)
MS: $(M+H)^+$ 481 free base
IR: (KBr), 1204, 1431, 1676, 2974 $cm^{-1}$

| Elemental analysis for $C_{22}H_{36}N_4S_1O_5$ . 2.4 TFA . 0.3 $H_2O$ | | | | | |
|---|---|---|---|---|---|
| | C | H | N | S | F |
| Calculated: | 43.95 | 5.17 | 7.38 | 4.22 | 18.01 |
| Found: | 43.95 | 5.28 | 7.59 | 4.45 | 18.01 |

$^1H$ (DMSO-$d_6$, @125°C) : 0.93 (d, 3 H, J = 6.45), 1.00 (d, 3 H, J = 6.45), 1.75-1.80 (m, 2 H), 1.88-1.93 (m, 3 H), 2.72-3.00 (m, 5 H), 3.17 (s, 3 H), 3.23-3.29 (m, 2H), 3.50 (br m, 1 H), 4.23-4.25 (m, 1 H), 4.53-4.96 (m, 3 H), 7.16, 17.18 (s, 4 H).
$[\alpha]_D$ = - 19.1° , c = 0.22 (methanol)

## Claims

1. A compound of the formula

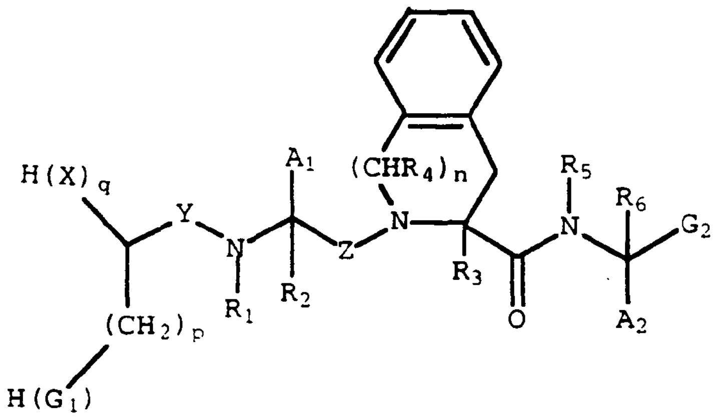

or an enantiomer, diastereomer, pharmaceutically acceptable salt, prodrug or solvate thereof, wherein:
$A_1$ and $A_2$ are each independently H, alkyl, substituted alkyl, cycloalkyl, substituted cycloalkyl, phenyl or substituted phenyl;
$G_1$ is S or O;
$G_2$ is H, -C(O)OH, -C(O)$NH_2$, 5-tetrazolyl, -C(O)N($R_7$)OH or -$CH_2$OH;
X is O or $R_8$N;
Y and Z are each independently -$CH_2$- or -C(O)-;
$R_1$, $R_2$, $R_3$, $R_4$, $R_5$, $R_6$ and $R_7$ are each independently H or alkyl;
$R_1$ may also be alkanoyl;
$R_1$ and $A_1$ taken together may be $-(CH_2)_m$;
$R_8$ is H, alkyl, phenyl, phenylalkyl, substituted phenyl, (substituted phenyl)alkyl or -C(O)$R_9$;
$R_9$ is H, alkyl, phenyl, phenylalkyl, substituted phenyl or (substituted phenyl)alkyl;
m is 3 or 4;
n is 0, 1 or 2;
p is 0, 1 or 2; and
q is 0 or 1, with the proviso that when p is 0, then q is also 0.

2. A compound of Claim 1, wherein $A_1$ and $A_2$ are each independently H or D-, L- or DL- $-CH_3$, $-CH(CH_3)_2$, $-CH_2CH(CH_3)_2$, $-CH(CH_3)CH_2CH_3$, $-C(CH_3)_3$, $-CH_2OH$, $-CH_2CH_2OH$, $-CH_2CH_2CH_2OH$, $-CH(OH)CH_3$,

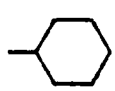 , 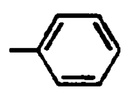 ,

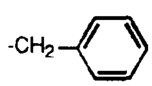 , 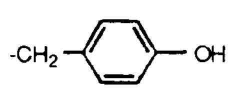 ,

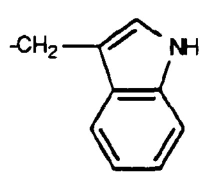 ,

$-CH_2\ CH_2\ CH_2\ CH_2\ NH_2$,

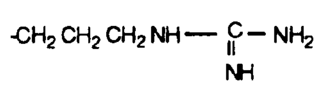 ,

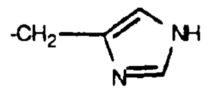 , 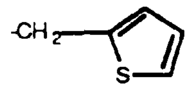 ,

$-CH_2C(O)OH$, $-CH_2CH_2C(O)OH$, $-CH_2C(O)NH_2$, $-CH_2CH_2C(O)NR_{11}R_{12}$ where $R_{11}$ and $R_{12}$ are each independently H, alkyl or phenyl, and $R_{11}$ and $R_{12}$ taken together may be $-(CH_2)_o-$ where o is 2 to 6, $-CH_2CH_2C(O)NHOH$, $-CH_2SH$, $-CH_2CH_2SH$, $-CH_2CH_2S(O)_2NH_2$, $-CH_2CH_2OCH_3$ or $-CH_2CH_2SCH_3$.

**3.** A compound of Claim 2, wherein $A_1$ and $A_2$ are each independently H or L- $-CH_3$, $-CH(CH_3)_2$, $-CH_2CH(CH_3)_2$, $-CH(CH_3)CH_2CH_3$, $-C(CH_3)_3$, $-CH_2OH$, $-CH_2CH_2OH$, $-CH_2CH_2CH_2OH$, $-CH(OH)CH_3$,

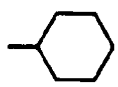 , 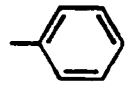 ,

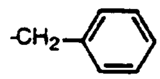 , 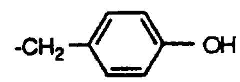 ,

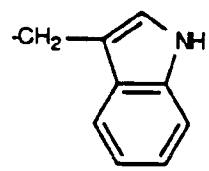 ,

$-CH_2\ CH_2\ CH_2\ CH_2\ NH_2$,

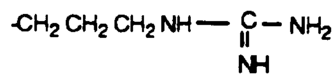 ,

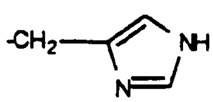 , 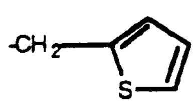 ,

$-CH_2C(O)OH$, $-CH_2CH_2C(O)OH$, $-CH_2C(O)NH_2$, $-CH_2CH_2C(O)NR_{11}R_{12}$ where $R_{11}$ and $R_{12}$ are each independently H, alkyl or phenyl, and $R_{11}$ and $R_{12}$ taken together may be $-(CH_2)_o-$ where o is 2 to 6, $-CH_2CH_2C(O)NHOH$, $-CH_2SH$, $-CH_2CH_2SH$, $-CH_2CH_2S(O)_2NH_2$, $-CH_2CH_2OCH_3$ or $-CH_2CH_2SCH_3$.

**4.** A compound of Claim 3, wherein

$A_1$ is L- $-CH_3$, $-CH(CH_3)_2$, $-CH_2CH(CH_3)_2$, $-C(CH_3)_3$, $-CH(CH_3)CH_2CH_3$, $-CH_2OH$ or $-CH(OH)CH_3$; and

$A_2$ is L- $-CH_2CH_2SCH_3$, $-CH_2CH_2OH$, $-CH_2CH_2CH_2OH$, $-CH_2CH_2C(O)NR_{11}R_{12}$ where $R_{11}$ and $R_{12}$ are each independently H, alkyl or phenyl, and $R_{11}$ and $R_{12}$ taken together may be $-(CH_2)_o-$ where o is 2 to 6, $-CH_2CH_2OCH_3$ or $-CH_2CH_2SH$.

**5.** A compound of Claim 1, wherein $A_1$ is L- $-CH_3$, $-CH(CH_3)_2$, $-CH_2CH(CH_3)_2$, $-C(CH_3)_3$, $-CH(CH_3)CH_2CH_3$, $-CH_2OH$ or $-CH(OH)CH_3$; $A_2$ is L- $-CH_2CH_2SCH_3$, $-CH_2CH_2OH$, $-CH_2CH_2CH_2OH$, $-CH_2CH_2C(O)NR_{11}R_{12}$ where $R_{11}$ and $R_{12}$ are each independently H, alkyl or phenyl, and $R_{11}$ and $R_{12}$ taken together may be $-(CH_2)_o-$ where o is 2 to 6, $-CH_2CH_2OCH_3$ or $-CH_2CH_2SH$; $G_1$ is S; $G_2$ is $-C(O)OH$; X is $R_8N$; Y is $-CH_2-$; $R_1$, $R_2$, $R_3$, $R_4$, $R_5$, $R_6$, $R_7$ and $R_8$ are each H; n is 1; and p is 1.

6. A compound of any preceding claim, wherein n is 1.

7. A compound of any preceding claim, wherein $R_8$ is H.

8. A compound of Claim 1 of the formula

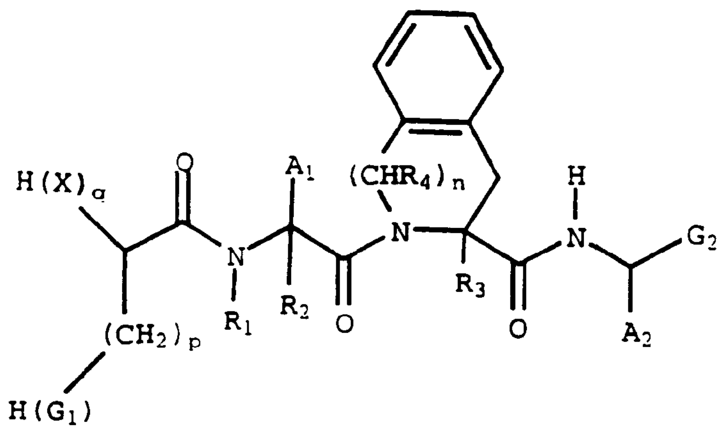

or an enantiomer, diastereomer, pharmaceutically acceptable salt, prodrug or solvate thereof, wherein:
$A_1$ and $A_2$ are each independently H, alkyl, substituted alkyl, cycloalkyl, substituted cycloalkyl, phenyl or substituted phenyl;
$G_1$ is S or O;
$G_2$ is H, -C(O)OH, -C(O)$NH_2$, -C(O)N($R_7$)OH or -$CH_2$OH;
X is O or $R_8$N;
$R_1$, $R_2$, $R_3$, $R_4$ and $R_7$ are each independently H or alkyl;
$R_1$ may also be alkanoyl;
$R_1$ and $A_1$ taken together may be $-(CH_2)_m$;
$R_8$ is H, alkyl, phenyl, phenylalkyl, substituted phenyl, (substituted phenyl)alkyl or -C(O)$R_9$;
$R_9$ is H, alkyl, phenyl, phenylalkyl, substituted phenyl or (substituted phenyl)alkyl;
m is 3 or 4;
n is 0, 1 or 2;
p is 0, 1 or 2; and
q is 0 or 1, with the proviso that when p is 0, q is also 0.

9. A compound of Claim 1 of the formula

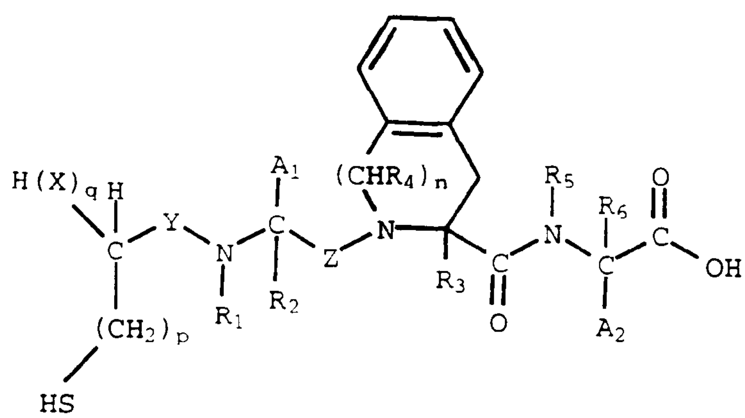

or an enantiomer, diastereomer, pharmaceutically acceptable salt, prodrug or solvate thereof, wherein:
$A_1$ and $A_2$ are each independently H, alkyl, substituted alkyl, cycloalkyl, substituted cycloalkyl, phenyl or substituted phenyl;
X is O or $R_8$N;

Y and Z are independently $-CH_2-$ or -C(O)-;
$R_1$, $R_2$, $R_3$, R4, $R_5$, $R_5$ and $R_7$ are each independently H or alkyl;
$R_1$ may also be alkanoyl;
$R_1$ and $A_1$ taken together may be $-(CH_2)_m$;
$R_8$ is H, alkyl, phenyl, phenylalkyl, substituted phenyl, (substituted phenyl)alkyl or $-C(O)R_9$;
$R_9$ is H, alkyl, phenyl, phenylalkyl, substituted phenyl or (substituted phenyl)alkyl;
m is 3 or 4;
n is 0, 1 or 2;
p is 0, 1 or 2; and
q is 0 or 1, with the proviso that when p is 0, q is also 0.

**10.** A compound of Claim 9, wherein $A_1$ is L- $-CH_3$, $-CH(CH_3)_2$, $-CH_2CH(CH_3)_2$, $-C(CH_3)_3$, $-CH(CH_3)CH_2CH_3$, $-CH_2OH$ or $-CH(OH)CH_3$; $A_2$ is L- $-(CH_2)_2SCH_3$, $-CH_2CH_2OH$, $-CH_2CH_2CH_2OH$, $-CH_2CH_2C(O)NR_{11}R_{12}$ where $R_{11}$ and $R_{12}$ are each independently H, alkyl or phenyl, and $R_{11}$ and $R_{12}$ taken together may be $-(CH_2)_o-$ where o is 2 to 6, $-CH_2CH_2OCH_3$ or $-CH_2CH_2SH$; Y is $-CH_2-$; Z is -C(O)-; X is $R_8N$; $R_1$, $R_2$, $R_3$, $R_4$, $R_5$, $R_6$ and $R_8$ are H; n is 1; and p is 1.

**11.** A compound of Claim 1, selected from:
(R*,S*)-N-[[2-[N-(2-Amino-3-mercaptopropyl)-L-valyl]-1,2,3,4-tetrahydro-3-isoquinolinyl]carbonyl]-L-methionine,
(R*)-N-[[2-[N-(L-Cysteinyl)-L-valyl]-1,2,3,4-tetrahydro-3-isoquinolinyl)carbonyl)-L-methionine,
(R*)-N-[[2-[N-(L-Cysteinyl)-L-valyl]-1,2,3,4-tetrahydro-3-isoquinolinyl)carbonyl]-L-methionine, methyl ester,
(R*)-N-[[2-[N-(3-Mercapto-1-oxopropyl)-L-valyl]-1,2,3,4-tetrahydro-3-isoquinolinyl]carbonyl]-1-methionine,
(R*,R*)-N-[[2-[2-(L-Cysteinylamino)-3-methylbutyl]-1,2,3,4-tetrahydro-3-isoquinolinyl]carbonyl]-L-methionine,
(R*)-N-[[2-[N-(3-Mercaptopropyl)-L-valyl]-1,2,3,4-tetrahydro-3-isoquinolinyl]carbonyl]-L-methionine,
(R*)-N-[[2-[N-(3-Mercaptopropyl)-L-alanyl]-1,2,3,4-tetrahydro-3-isoquinolinyl]carbonyl]-L-methionine,
(R*)-N-[[2-[N-(3-Mercaptopropyl)-N-methyl-L-valyl]-1,2,3,4-tetrahydro-3-isoquinolinyl]carbonyl]-L-methionine,
(R*,R*)-N-[[2-[2-[(3-Mercaptopropyl)amino]-3-methylbutyl]-1,2,3,4-tetrahydro-3-isoquinolinyl]carbonyl]-L-methionine,
(R*)-N-[[2-[N-(3-Mercaptopropyl)-L-propyl]-1,2,3,4-tetrahydro-3-isoquinolinyl]carbonyl]-L-methionine,
(R*)-N-[[2-[N-(2-Mercaptoethyl)-L-valyl]-1,2,3,4-tetrahydro-3-isoquinolinyl]carbonyl]-L-methionine,
(R*)-N-[[2-[N-(3-Mercapto-1-oxopropyl)-L-valyl]-1,2,3,4-tetrahydro-3-isoquinolinyl]carbonyl]-L-methionine, methyl ester,
(R*,S*)-2-[N-(2-Amino-3-mercaptopropyl)-L-valyl]-1,2,3,4-tetrahydro-N-[3-(methylthio)propyl]-3-isoquinolinecarboxamide,
(R*)-2-[N-(3-Mercaptopropyl)-L-valyl]-1,2,3,4-tetrahydro-N-[3-(methylthio)propyl]-3-isoquinolinecarboxamide,
(R*)-N-[[2-[N-(L-Cysteinyl)-L-valyl]-1,2,3,4-tetrahydro-3-isoquinolinyl]carbonyl]-L-alanine,
(R*,R*)-N-[[2-[N-(2-Amino-4-hydroxy-1-oxobutyl)-L-valyl]-1,2,3,4-tetrahydro-3-isoquinolinyl]carbonyl]-L-methionine,
(R*,R*)-2-[N-(L-Cysteinyl)-L-valyl]-1,2,3,4-tetrahydro-N-[1-(hydroxymethyl)-3-(methylthio)propyl]-3-isoquinolinecarboxamide,
(R*)-N-[[2-(L-Cysteinyl)-L-valyl]-1,2,3,4-tetrahydro-3-isoquinolinyl]carbonyl]-L-phenylalanine,
(R*)-N-[[2-[N-(L-Cysteinyl)-L-valyl]-1,2,3,4-tetrahydro-3-isoquinolinyl]carbonyl]-L-serine,
(R*)-$N^2$-[[2-[N-(L-Cysteinyl)-L-valyl]-1,2,3,4-tetrahydro-3-isoquinolinyl]carbonyl]-N-hydroxy-L-methioninamide,
(R*)-N-[[2-[N-(L-Cysteinyl)-L-valyl]-1,2,3,4-tetrahydro-3-isoquinolinyl]carbonyl]-3-(2-thienyl)-DL-alanine,
(R*)-N-[[2-[N-(L-Cysteinyl)glycyl]-1,2,3,4-tetrahydro-3-isoquinolinyl]carbonyl]-L-methionine,
(R*)-$N^2$-[[2-[N-(L-Cysteinyl)-L-valyl]-1,2,3,4-tetrahydro-3-isoquinolinyl]carbonyl]-L-glutamine,
(R*)-N-[[2-[N-(L-Cysteinyl)-L-valyl]-1,2,3,4-tetrahydro-3-isoquinolinyl]carbonyl]-L-histidine,
(R*)-N-[[2-[N-(L-Cysteinyl)-L-valyl]-1,2,3,4-tetrahydro-3-isoquinolinyl]carbonyl]-3-(phenylmethyl)-L-alanine,
(R*)-N-[[2-[N-(L-Seryl)-L-valyl]-1,2,3,4- tetrahydro-3-isoquinolinyl]carbonyl]-L-methionine,
(R*,R*)-2-[N-(3-Mercapto-1-oxopropyl)-L-valyl]-1,2,3,4-tetrahydro-N-[3-(methylthio)-1-(1H-tetrazol-5-yl)propyl]-3-isoquinolinecarboxamide,
(R*,S*)-N-[[2-[N-(2-Amino-3-mercaptopropyl)-N-methyl-L-valyl]-1,2,3,4-tetrahydro-3-isoquinolinyl]carbonyl]-L-methionine,
(R*,R*,S*)-N-[[2-[2-[(2-Amino-3-mercaptopropyl)amino]-3-methylbutyl]-1,2,3,4-tetrahydro-3-isoquinolinyl]carbonyl]-L-methionine,
(R*)-N-[[1-[N-(L-Cysteinyl)-L-valyl]-2,3-dihydro-1H-indol-2-yl]carbonyl]-L-methionine,

(S*,R*)-N-[[2-[N-(2-Amino-3-mercaptopropyl)-L-valyl]-1,2,3,4-tetrahydro-3-isoquinolinyl]carbonyl]-L-methionine, methyl ester,
[3S-[2[R*(S*)],3R*(R*)]]-2-[N-(2-Amino-3-mercaptopropyl)-L-valyl]-1,2,3,4-tetrahydro-N-(tetrahydro-2-oxo-3-furanyl)-3-isoquinolinecarboxamide,
(S*,R*)-$N^2$-[[2-[N-(2-Amino-3-mercaptopropyl)-L-valyl]-1,2,3,4-tetrahydro-3-isoquinolinyl]carbonyl]-N-hydroxy-L-methioninamide,
[R-(R*,S*)]-$N^2$-[[2-[N-(2-Amino-3-mercaptopropyl)-L-valyl]-1,2,3,4-tetrahydro-3-isoquinolinyl]carbonyl]-L-glutamine,
[R-(R*,S*)]-2-[N-(2-Amino-3-mercaptopropyl)-L-valyl]-1,2,3,4-tetrahydro-N-(tetrahydro-2-oxo-3-thienyl)-3-isoquinolinecarboxamide,
(S*,R*)-$N^2$-[[2-[N-(2-Amino-3-mercaptopropyl)-L-valyl]-1,2,3,4-tetrahydro-3-isoquinolinyl]carbonyl]-L-asparagine,
(S*,R*)-N-[[2-[(2-Amino-3-mercaptopropyl)-L-valyl]-1,2,3,4-tetrahydro-3-isoquinolinyl]carbonyl]-L-norleucine,
(S*,R*)-N-[(2-[N-(2-Amino-3-mercaptopropyl)-L-valyl]-1,2,3,4-tetrahydro-3-isoquinolinyl]carbonyl]-γ-(aminosulphonyl)-L-α-amino-butyric acid,
(S*,R*)-N-[[2[N-(2-Amino-3-mercaptopropyl)-L-valyl]-1,2,3,4-tetrahydro-3-isoquinolinyl]carbonyl]-L-glutamic acid,
(S*,R*)-$N^2$-[[2-[N-(2-Amino-3-mercaptopropyl)-L-valyl]-1,2,3,4-tetrahydro-3-isoquinolinyl]-carbonyl]-$N^5$-hydroxy-L-glutamine,
[R-(R*,S*)]-$N^2$-[[2-[N-(2-Amino-3-mercaptopropyl)-L-valyl]-1,2,3,4-tetrahydro-3-isoquinolinyl]carbonyl]-L-methioninamide,
N-[[(S)-2-[N-[(R)-2-Amino-3-mercaptopropyl]-L-valyl]-1,2,3,4-tetrahydro-3-isoquinolinyl]carbonyl]-2-methyl-DL-methionine,
(S*,R*)-N-[[2-[N-(2-Amino-3-mercaptopropyl)-L-alanyl]-1,2,3,4-tetrahydro-3-isoquinolinyl]carbonyl]-L-methionine,
(S*,R*)-N-[[2-[N-(2-Amino-3-mercaptopropyl)-L-leucyl]-1,2,3,4-tetrahydro-3-isoquinolinyl]carbonyl]-L-methionine,
[R*[R*(S*)]]-N-[[2-[2-[(2-Amino-3-mercaptopropyl)amino]-1-oxobutyl]-1,2,3,4-tetrahydro-3-isoquinolinyl] carbonyl]-L-methionine,
(S*,R*)-N-[[2-[2-[(2-Amino-3-mercapto-propyl)amino]-4-methylpentyl]-1,2,3,4-tetrahydro-3-isoquinolinyl] carbonyl]-L-methionine,
(R)-N-[[2-[1-[(2-amino-3-mercaptopropyl)-L-prolyl]-1,2,3,4-tetrahydro-3-isoquinolyl]carbonyl]-L-methionine,
[R*[S*(S*)]]-N-[[2-[[1-(2-Amino-3-mercaptopropyl)-2-methyl-2-pyrrolidinyl]carbonyl]-1,2,3,4-tetrahydro-3-isoquinolinyl]carbonyl]-L-methionine,
(S*,R*)-N-[[2-[N-(2-Amino-3-mercaptopropyl)-3-methyl-L-valyl]-1,2,3,4-tetrahydro-3-isoquinolinyl]carbonyl]-L-methionine,
[R*[R*(S*)]]-N-[(2-[[(2-Amino-3-mercaptopropyl)amino)phenylacetyl]-1,2,3,4-tetrahydro-3-isoquinolinyl] carbonyl]-L-methionine,
[R*[R*(S*)]]-N-[[2-[2-[(2-Amino-3-mercaptopropyl)amino]-2-cyclohexylacetyl]-1,2,3,4-tetrahydro-3-isoquinolinyl]carbonyl]-L-methionine,
(S*,R*)-N-[[2-[N-(2-Amino-3-mercaptopropyl)-3-methyl-L-valyl]-1,2,3,4-tetrahydro-3-isoquinolinyl]carbonyl]-L-methionine, methyl ester,
(S*,R*)-$N^2$-[[2-[N-(2-Amino-3-mercaptopropyl)-3-methyl-L-valyl]-1,2,3,4-tetrahydro-3-isoquinolinyl]carbonyl]-$N^5$-methyl-L-glutamine,
(S*,R*)-$N^2$-[[2-[N-(2-Amino-3-mercaptopropyl)-3-methyl-L-valyl]-1,2,3,4-tetrahydro-3-isoquinolinyl]carbonyl]-L-glutamine,
(S*,R*)-$N^2$-[[2-[N-(2-Amino-3-mercaptopropyl)-3-methyl-L-valyl]-1,2,3,4-tetrahydro-3-isoquinolinyl]carbonyl]-L-glutamine, methyl ester,
[R*[R*(S*)]]-N-[[2-[2-[(2-Amino-3-mercaptopropyl)amino]-3-methylbutyl]-1,2,3,4-tetrahydro-3-isoquinolinyl]carbonyl]-N-methyl-L-methionine,
(S*,R*)-N-[[2-[2-[N-(2-Amino-3-mercaptopropyl)methylamino]-3-methylbutyl]-1,2,3,4-tetrahydro-3-isoquinolinyl]carbonyl]-L-methionine,
(S*,R*)-N-[[2-[N-Acetyl-N-(2-amino-3-mercaptopropyl)-L-valyl]-1,2,3,4-tetrahydro-3-isoquinolinyl]carbonyl]-L-methionine,
(S*,R*)-N-[[2-[N-[3-Mercapto-2-(methylamino)propyl]-L-valyl]-1,2,3,4-tetrahydro-3-isoquinolinyl]carbonyl]-L-methionine,
(S*,R*)-N-[[1,2,3,4-Tetrahydro-2-[N-[3-mercapto-2-[(phenylmethyl)amino]propyl]-L-valyl]-3-isoquinolinyl]carbonyl]-L-methionine,
(S*,R*)-N-[[2-[N-[2-(Acetylamino)-3-mercaptopropyl]-L-valyl]-1,2,3,4-tetrahydro-3-isoquinolinyl]carbonyl]-L-methionine,
[R*[S*(S*)]]-N-[[2-[N-[2-amino-3-[(2-amino-2-carboxyethyl)dithio]propyl]-L-valyl]-1,2,3,4-tetrahydro-3-isoquinolinyl]carbonyl]-L-methionine,
(S*,R*)-N,N'-Dithiobis(2-amino-3,1-propanediyl)bis-[N-[[2-(L-valyl)-1,2,3,4-tetrahydro-3-isoquinolinyl]carbonyl]-L-methionine],
(S*,R*)-N-[[2-[N-[2-Amino-3-(methyldithio)propyl]-L-valyl]-1,2,3,4-tetrahydro-3-isoquinolinyl]carbonyl]-L-methionine,
N-[[(S)-1,2,3,4-Tetrahydro-2-[N-[[(4R)-2-methyl-4-thiazolidinyl]methyl]-L-valyl]-3-isoquinolinyl]carbonyl]-L-methionine,
(S*,R*)-N-[[1,2,3,4-Tetrahydro-2-[N-(4-thiazolidinylmethyl)-L-valyl]-3-isoquinolinyl]carbonyl]-L-methionine,
(S*,R*)-N-[[2-[N-[2-(Acetylamino)-3-(acetylthio)propyl]-L-valyl]-1,2,3,4-tetrahydro-3-isoquinolinyl]carbonyl]-L-methionine,
N-[[(S)-1,2,3,4-Tetrahydro-2-[N-(2-hydroxy-3-mercaptopropyl)-L-valyl]-3-isoquinolinyl]carbonyl]-L-methionine,
(S*,R*)-N-[[1,2,3,4-Tetrahydro-2-[N-[3-mercapto-2-[(3-phenylpropyl)amino]propyl] L-valyl]-3-isoquinolinyl]carbonyl]-L-methionine,
(S*,R*)-$N^2$-[[2-[N-(2-Amino-3-mercaptopropyl)-L-valyl]-1,2,3,4-tetrahydro-3-isoquinolinyl]carbonyl]-N-hydroxy-N-methyl-L-methioninamide, and
(S*,R*)-N-[[2-[N-(2-Amino-3-mercaptopropyl)-L-valyl]-1,2,3,4-tetrahydro-3-isoquinolinyl]carbonyl]-O-methyl-L-homoserine.

**12.** A compound of any preceding claim for use as an active pharmaceutical substance.

**13.** Use of a compound of any one of claims 1-11 for the manufacture of a medicament for treatment of conditions requiring inhibition of farnesyl protein transferase in a mammalian subject.

**14.** Use of a compound of any one of claims 1-11 for the manufacture of a medicament for treatment of conditions requiring inhibition of prenyl transferases in a mammalian subject.

**15.** Use of a compound of any one of claims 1-11 for the manufacture of a medicament for treatment of conditions requiring inhibition of tumors in a mammalian subject.

**16.** Use of a compound of any one of claims 1-11 for the manufacture of a medicament for treatment of diseases associated with signal transduction pathways operating through Ras in a mammalian subject.

**17.** Use of a compound of any one of claims 1-11 for the manufacture of a medicament for treatment of diseases associated with proteins that are post-translationally modified by the enzyme farnesyl protein transferase, in a mammalian subject.

**18.** A compound of the formula

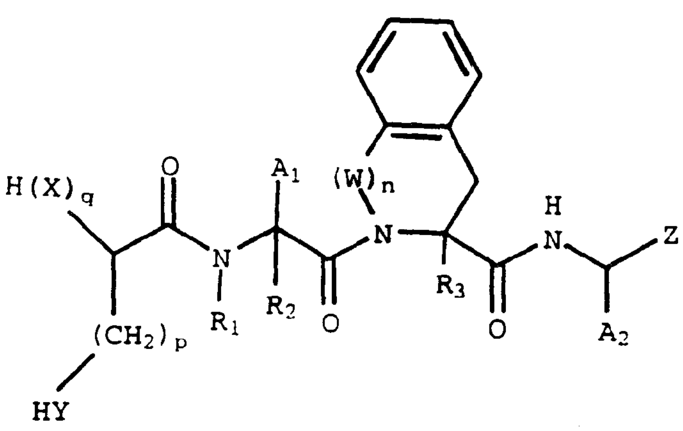

its enantiomers and diastereomers, and pharmaceutically acceptable salts, prodrugs and solvates there-

of, wherein:

$A_1$ and $A_2$ are each independently H or any of the natural D-, L- or DL-amino acid side chains;
W is $CH-R_4$;
X is O or $R_5N$;
Y is S or O;
Z is H, C(O)OH, tetrazolyl, $C(O)N(R_7)OH$ or $C(O)CH_2OH$;
$R_1$, $R_2$, $R_3$, $R_4$ and $R_7$ are each independently H or alkyl;
$R_1$ and $A_1$ taken together may be $(CH_2)_m$;
$R_5$ is H, alkyl, phenyl, phenylalkyl, or $C(O)R_6$, where the phenyl and phenylalkyl may be optionally substituted on the phenyl ring;
$R_6$ is H, alkyl, phenyl or phenylalkyl, where the phenyl and phenylalkyl may be optionally substituted on the phenyl ring;
m is 3 or 4;
n is 0, 1 or 2;
p is 1 or 2; and
q is 0 or 1.

**19.** A compound of the formula

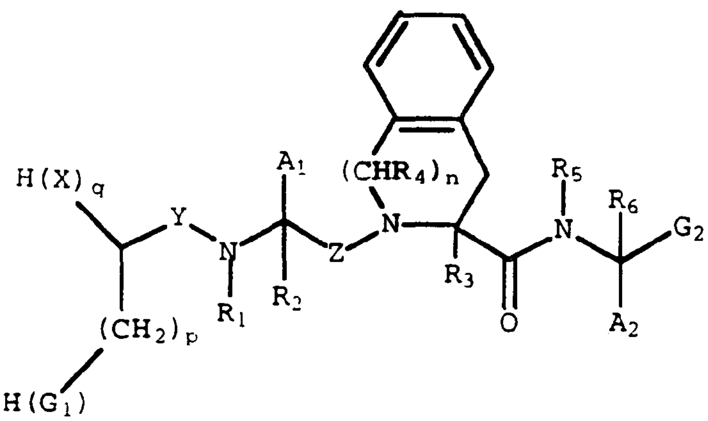

or its enantiomers, diastereomers, pharmaceutically acceptable salts, prodrugs or solvates thereof, wherein:

$A_1$ and $A_2$ are each independently H, alkyl, substituted alkyl, phenyl or substituted phenyl;
$G_1$ is S or O;
$G_2$ is H, -C(O)OH, $-C(O)NH_2$, 5-tetrazolyl, $-C(O)N(R_7)OH$ or $-CH_2OH$;
X is O or $R_8N$;
Y and Z are each independently $-CH_2-$ or -C(O)-;
$R_1$, $R_2$, $R_3$, $R_4$, $R_5$, $R_6$ and $R_7$ are each independently H or alkyl;
$R_1$ and $A_1$ taken together may be $-(CH_2)_m$;
$R_8$ is H, alkyl, phenyl, phenylalkyl, substituted phenyl, (substituted phenyl)alkyl or $-C(O)R_9$;
$R_9$ is H, alkyl, phenyl, phenylalkyl, substituted phenyl or (substituted phenyl)alkyl;
m is 3 or 4;
n is 0, 1 or 2;
p is 0, 1 or 2; and
q is 0 or 1, with the proviso that when p is 0, q is also 0.